# EUROPEAN PATENT SPECIFICATION

(11) **EP 4 323 371 B1**
(45) Date of publication and mention of the grant of the patent: **05.02.2025**
(21) Application number: 21820253.9
(22) Date of filing: 01.12.2021
(51) Int. Cl.: C07F 9/26, C07F 5/02, C07F 5/00, C09K 11/06, H10K 85/60, C07F 9/06

(54) **METAL-ORGANIC COORDINATION COMPOUND AND METHOD FOR PRODUCING THE SAME**
METALLORGANISCHE KOORDINATIONSVERBINDUNG UND VERFAHREN ZU DEREN HERSTELLUNG
COMPOSÉ DE COORDINATION ORGANOMÉTALLIQUE ET SON PROCÉDÉ DE PRODUCTION

(30) Priority: 16.04.2021 WO PCT/EP2021/059962
(43) Date of publication of application: 21.02.2024
(73) Proprietor: beeOLED GmbH, 01257 Dresden (DE)
(72) Inventor: ROTHE, Carsten, 01099 Dresden (DE); SENKOVSKYY, Volodymyr, 01187 Dresden (DE)
(74) Representative: Altmann, Andreas
(86) International application number: PCT/EP2021/083808
(87) International publication number: WO 2022/218562

(56) References cited:
- WO-A1-2012/157631
- WO-A1-2019/191328
- LENORA CHAMIKA U. ET AL: "Measurement of the Dissociation of Eu II -Containing Cryptates Using Murexide", INORGANIC CHEMISTRY, vol. 59, no. 1, 19 February 2019 (2019-02-19), Easton , US, pages 86 - 93, XP055824366, ISSN: 0020-1669, DOI: 10.1021/acs.inorgchem.8b03605
- HUH DANIEL N. ET AL: "Synthesis of uranium-in-cryptand complexes", CHEMICAL COMMUNICATIONS, vol. 54, no. 73, 18 August 2018 (2018-08-18), pages 10272 - 10275, XP055824374, ISSN: 1359-7345, DOI: 10.1039/C8CC05341C
- JENKS TYLER C. ET AL: "Photophysical characterization of a highly luminescent divalent-europium-containing azacryptate", vol. 54, no. 36, 17 April 2018 (2018-04-17), pages 4545 - 4548, XP055824377, ISSN: 1359-7345, Retrieved from the Internet <URL:http://pubs.rsc.org/en/content/articlepdf/2018/CC/C8CC01737A> DOI: 10.1039/C8CC01737A
- CORBIN BROOKE A ET AL: "Screening of ligands for redox-active europium using magnetic resonance imaging", BIOORGANIC & MEDICINAL CHEMISTRY, ELSEVIER, AMSTERDAM, NL, vol. 26, no. 19, 4 April 2018 (2018-04-04), pages 5274 - 5279, XP085529199, ISSN: 0968-0896, DOI: 10.1016/J.BMC.2018.04.001
- LENORA CHAMIKA U. ET AL: "Structural Features of Europium(II)-Containing Cryptates That Influence Relaxivity", vol. 23, no. 61, 14 July 2017 (2017-07-14), pages 15404 - 15414, XP055824404, ISSN: 0947-6539, Retrieved from the Internet <URL:https://api.wiley.com/onlinelibrary/tdm/v1/articles/10.1002%2Fchem.201702158> DOI: 10.1002/chem.201702158
- LEHNA JEAN-MARIE ET AL: "0. Synthesis and Properties of Acyclic and Cryptate Europium(II1) Complexes Incorporating the 3,3'-Biisoquinoline 2,Z'-Dioxide Unit", HELVETICA CHIMICA ACTA, vol. 73, 31 January 1990 (1990-01-31), pages 106 - 111, XP055894978

## Description

### Technical Field

Various aspects of this disclosure generally relate to an electroluminescent coordination compound, a mixture, a compound and an organic electronic device and a contrast enhancement medium for magnet resonance tomography having the same as well as methods for forming the same.

### Background

Electroluminescent devices that make use of organic light emitting diodes (OLEDs) are state-of-the-art for flat panel display applications used in everyday consumer electronics. For OLEDs usually special organic materials are employed for the purpose of converting electrical excitation into light emission. For most organic emitters, the excitation that is formed upon recombination of an electron and a hole on such an emitter molecule is called an "exciton". Depending on the spins of the recombining charges, there are two types of excitons formed in a statistical manner: 75% probability of triplet excitons with spin 1, and 25% probability singlet excitons with spin zero are generated. If the emitter molecule is heteroaryl-based without any significant content of heavy metals, then, because of spin-conservation, only the singlet excitons contribute to light emission, known as fluorescence (FL). Thus, fluorescent OLEDs are comparably inefficient as 75% of the invested electrical power is wasted.

In a related art, incorporation of quantum mechanical heavy metal effect into the emitter molecules, by introducing d-metal elements such as Iridium, Osmium, Gold or Platinum, has been used. The presence of heavy metal elements softens the selection rules for the excited states and allows triplet excitons to emit light too; known as phosphorescence (Ph).

In another related art, thermally activated delayed fluorescence (TADF) has been used wherein by thoughtful design of the organic emitter molecule, the energy difference between the non-emissive triplet and the emissive singlet exciton is engineered to be very small. This allows triplets to thermally convert into singlet excitons and thereby contribute to light emission.

However, there are no TADF or Ph blue emitters with sufficient chemical stability known which hinders their implementation into OLED applications. The underlying cause of low chemical stability is due to the formation of charge-separated states upon excitation. For Ph emitters, the exciton resides on two different parts of the emitter, namely the central heavy metal cation and the organic ligand. During excitation, especially with high energetic blue light, the chemical bond between metal center and organic ligand is weakened, giving rise to chemical decomposition. Similarly, for TADF emitters, a low energy difference between singlet and triplet exciton energy is needed, which is achieved by bridging the excitation in-between an electron accepting and an electron donating part. Again, upon excitation with energies corresponding to blue photons, the chemical organic bond between those two parts of the emitter molecules is substantially weakened, giving rise to bond cleavage and in consequence short operational lifetime in OLED device.

Therefore, TADF or Ph blue emitting materials cannot be used in display applications, as otherwise the blue spectral component would fade away after prolonged operation times which is known as burn-in.

For display applications, the emission spectra of the primary red, green, and blue colors should ideally be narrow in order to allow for the highest color purities. Otherwise color filters are used to sharpen the emission spectra, compromising efficiency. In this context, Ph and TADF emitters are not ideal, as they picture rather wide emission spectra. The observed broad emission spectra are an inherent consequence of the design principle based on charge-transfer states being localized between flexible organic bonds, which lead to a wide range of energetic states. Consequently, the implementation of TADF or Ph emitting organic molecules in OLED flat panel display applications, forces the use of color filters, inevitably leading to reduced efficiencies.

Thus, for emitter molecules suitable for OLED, it is desirable to avoid charge separated states, but to localize the excitation on one part of the molecule, preferably on a single atom. Generally, elements with suitable intra-atomic transitions are found within the f-Elements, i.e. the lanthanides. The preferred oxidation state of all lanthanides is 3+. Emitters based on such lanthanides have been extensively used in OLEDs. For example, OLEDs based on intra-atomic transitions in the blue, green, and red spectral region based on Thulium (Tm3+), Terbium (Ter3+) and Europium (Eu3+) respectively have been demonstrated. However, OLED emitters based on three-valent lanthanides have a serious flaw which renders them as being unsuitable for display applications. Here, the excited state relaxation time is around one millisecond, which is about three orders of magnitude too long for display applications. Such slow relaxation times are incompatible with the requirements of fast display content refresh rate and as well lead to a severe efficiency roll-off of the OLEDs at high brightness. Here, a long-excited state lifetime leads to a high density of excitations in the active OLED layer, which leads to bimolecular annihilation loss and low efficiency. Finally, the emission spectra of all three valent lanthanides are not narrow. Instead, a whole range of rather sharp individual narrow lines is distributed over a wide spectral region; which again is not suitable for achieving deep and pure colors.

The above limitations of three valent lanthanides in terms of excited state lifetime and color purity do not apply for specific divalent lanthanides, namely Europium (Eu2+) and Ytterbium (Yb2+), which both possess desired deep blue, narrow emission spectra with sufficiently short excited state lifetime.

Further, Lanthanides with oxidation state +2 are extremely attractive for a large range of applications, mainly due to their unique magnetic and optic properties. For example, Eu(II) is paramagnetic, which can be beneficial for medical diagnostics, memory devices or devices or materials based on magnetic behavior. Divalent Ytterbium and Europium feature attractive emission characteristics due to their Laporte allowed d-f intra-atomic transition. Applied in a stabilizing chemical environment those ions may emit pure and deep blue light which is applicable for a large range of opto-electronic devices, for example sensors, solar cells, electroluminescent devices, or color conversion materials.

The preferred oxidation state of all lanthanide metals is trivalent. Thus, a major obstacle for the application of divalent lanthanides is their chemical instability, and -in particular- their tendency to oxidize under normal ambient conditions. Therefore, the application of divalent lanthanide requires sufficient stabilization of the cation.

JP3651801 B2 discloses the stabilization of lanthanide (II) salts by embedding them into a matrix of inorganic salts. However, due to the high evaporation temperature of inorganic materials, this technique cannot be applied to fabricate state of the art multilayer organic electronic devices, such as OLEDs, due to the unavoidable degradation of underlying organic layer. Further, such inorganic salts are not suitable for injection into the blood system, as required for application as MRT contrast media.

US 6,492,526 discloses a metal organic complex comprising divalent Europium and charged pyrazolyl borate ligands. In this case, the desired stabilization of the divalent oxidation state is achieved by application of the strong electron-donating chelating ligand. Yet, such a strong chelating ligand leads to polarization in the excited state of the central cation. This undesired polarization changes the dominate intra-metallic optical transition of the isolated cation partially into a metal-to-ligand charge transfer state. Thus, excitation energy is transferred to the organic ligand. Consequently, the emission of the compound is substantially red shifted, compared to the desirable deep blue emission from the free Eu2+ cation.

Another related art to combine the desired properties of divalent lanthanides with the benefits of organic processing are metal organic coordination compounds, in which the reactive metal cation is stabilized by cyclic polyether or bicyclic macrocyclic ligands, such as cryptands. In related arts, porphyrins are known as red emitters, specific cryptands have been proposed as ligands for d-metal Ph emitters, crown ether or cryptands have been proposed to stabilize reactive metals for n-type doping, ultraviolet emitting OLED have been achieved by using a Ce(3+) crown ether coordination compound as active emitter. However, in those cases, the central metal ion has been mainly coordinated to electron rich hard atoms, such as oxygen and nitrogen. Hence, cryptands employing simply hard coordinating atoms such as nitrogen or oxygen cannot sufficiently prevent oxidation of the central reactive ion, such that processing in ambient conditions becomes possible. Thus, no coordination compounds are known that do keep the desired properties of the divalent lanthanide, but at the same time sufficiently prevent oxidation, such that device fabrication or general use in ambient condition becomes possible.

In another related art, a specific strategy to prevent oxidation is known by incorporating soft coordinating ligands, such as sulfur or aromatic aryl or heteroaryl rings into the coordination sphere of the central metal. Thereby oxidation stability of the central metal may be observed. However, in providing such a soft coordination, asymmetrical environment leads to polarization effects in the excited state of the central metal. In other words, the originally pure intra-metallic transition on the central metal ion becomes partly of metal-to-ligand charge transfer (MLCT) type character. Consequently, the originally deep blue and spectrally pure emission shifts substantially to the green and red spectral region and broadens, which renders the application of those metal coordination compounds in opto-electronic devices undesirable. Crown ethers and cryptands with Eu (II) in OLEDs are described in general terms in WO 2004/058912. Nitrogen-containing (macro)circular molecules with Eu(II) in OLEDs, similar to crown ethers, are described in WO 2011/090977. WO 2014/164712 describes nitrogen-containing (macro)circular molecules with Eu(II) for MRI applications. There is no mention of OLED applications. Chem. Commun., 2018, 54, pages 4545-4548, discloses nitrogen-containing (macro)circular molecules with Eu(II). In this respect, (marco)circular molecules are understood to accommodate the central atom, for example Eu(II) in the centre of the circular structure.

WO 2012/157631 relates to aliphatic amine-coordinated cerium complexes. The complexes (D8) and (D9) depicted on page 20 and prepared as disclosed in example 4 contain the counter ions not covalently linked with the cyclic organic ligand as it is evident from the formulae on page 20.

An object underlying the present invention is to provide an electroluminescent coordination compound which shows deep blue, saturated emission profile combined with high operational stability in an OLED device especially when applying an electrical field and which therefore has increased stability. In particular, neither the molecule itself nor parts of it shall drift within the OLED device upon application of electrical field which is known to seriously compromise the stability of such an OLED device and would just prevent application of such OLED devices in consumer electronics.

Furthermore, preferably, the solubility in non-polar, non-halogen solvents shall be improved, so that the electroluminescent coordination compounds can be solution-processed to form OLED structures.

According to a further object, preferably, the electroluminescent coordination compounds shall show an increased sublimation yield such that large area OLED displays can be process using low cost sublimation or evaporation technology.

### Summary

In various aspects an electroluminescent coordination compound, a mixture, a compound and an organic electronic device and a contrast enhancement medium for magnet resonance tomography having the same as well as methods for forming the same are provided.

The object is achieved according to the present invention by a metal-organic coordination compound, which is neutrally charged and wherein the coordination compound comprises at least one divalent lanthanide coordinated by a cyclic organic ligand, wherein the cyclic organic ligand contains two monoanionic groups covalently linked with the cyclic organic ligand and being separated from each other by a sequence of at least two intervening atoms.

The metal-organic coordination compound is neutrally charged since it contains two monoanionic groups, which compensate for the divalent lanthanide cation.

The monoanionic groups covalently linked with a cyclic organic ligand are separated from each other by a sequence of at least two intervening atoms so that for example a phosphate group or a carbonate group is excluded.

The monoanionic group can be part of the cyclic organic ligand, for example as a group B-. In this case, the two negatively charged boron atoms are separated from each other by a sequence of at least two intervening atoms. If the monoanionic group is a group O-, two groups O- are separated by at least two intervening atoms. The intervening atoms together with the monoanionic groups form a sequence of covalent linkages. In this definition it is assumed that the monoanionic charge is located on a single atom in most of the cases. However, it is also possible that the monoanionic charge is delocalized in a larger system of atoms. In this case these larger systems atoms are separated by at least two intervening atoms.

The monoanionic group can also be present in a heterocyclic group like a pyrazole group.

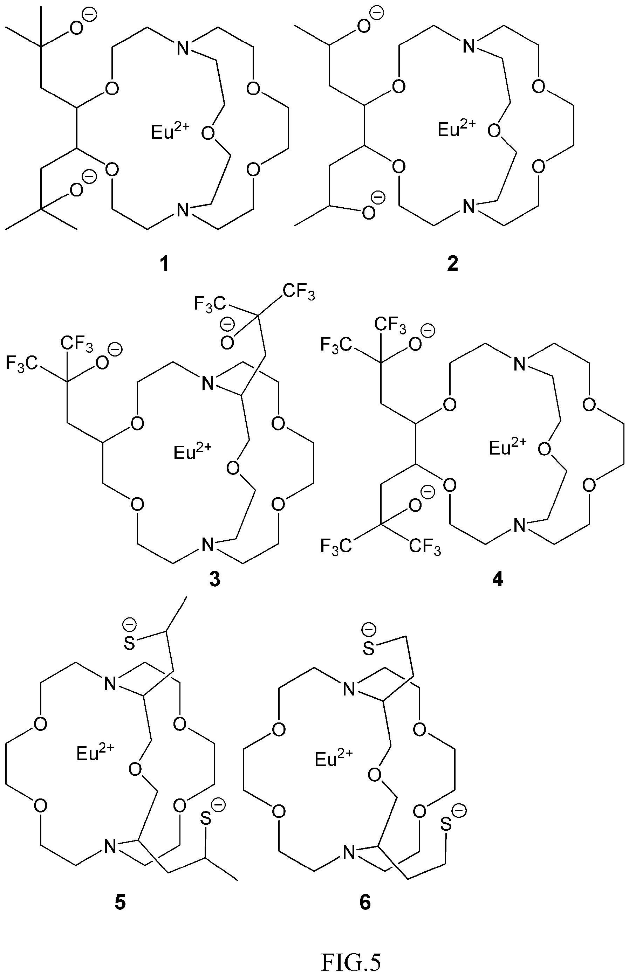

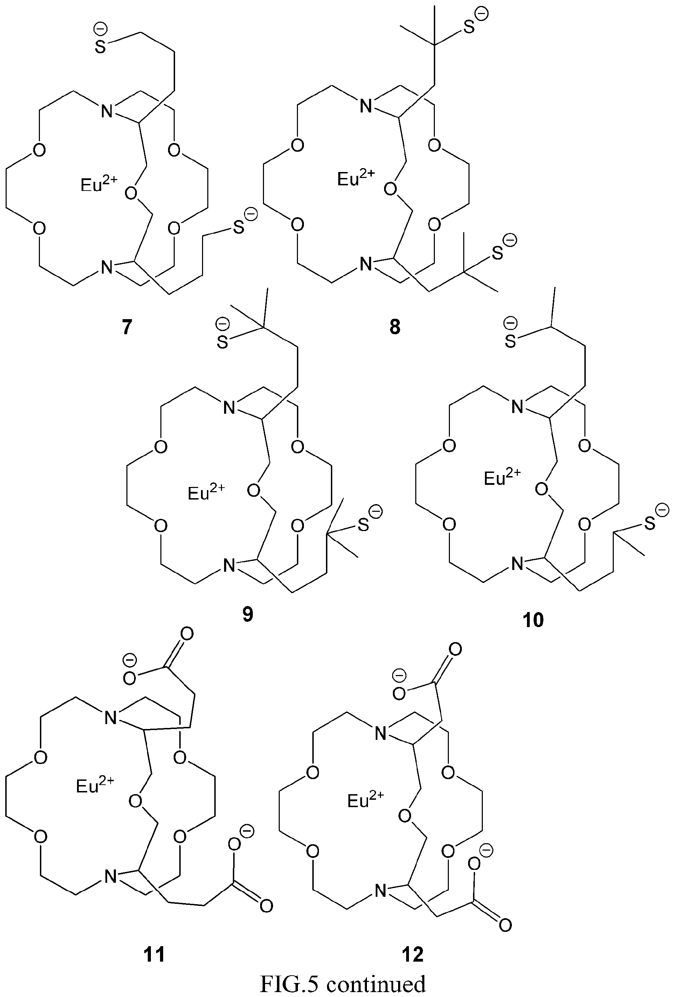

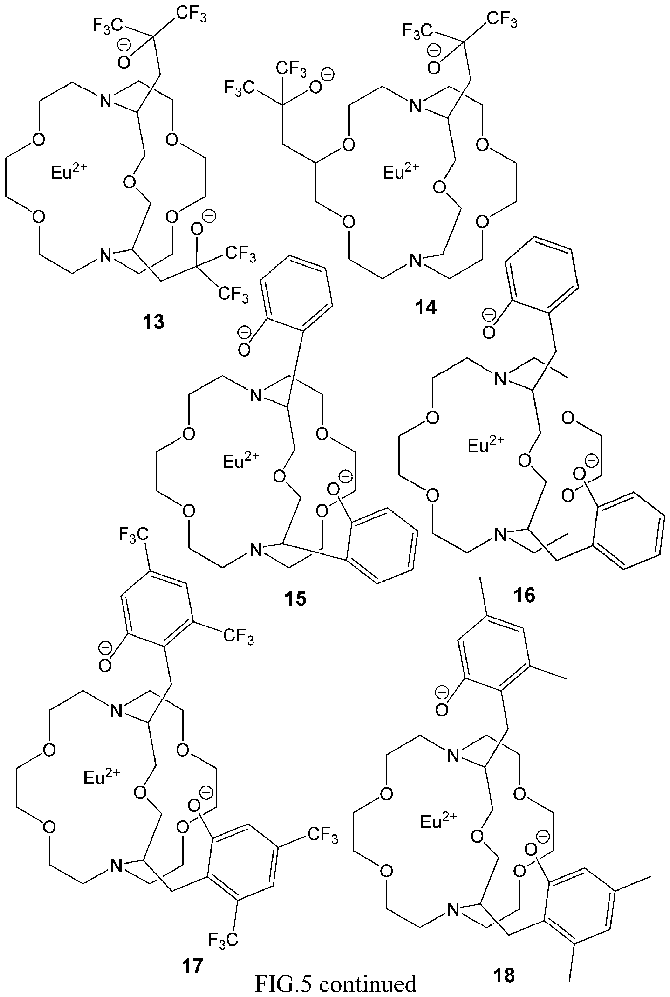

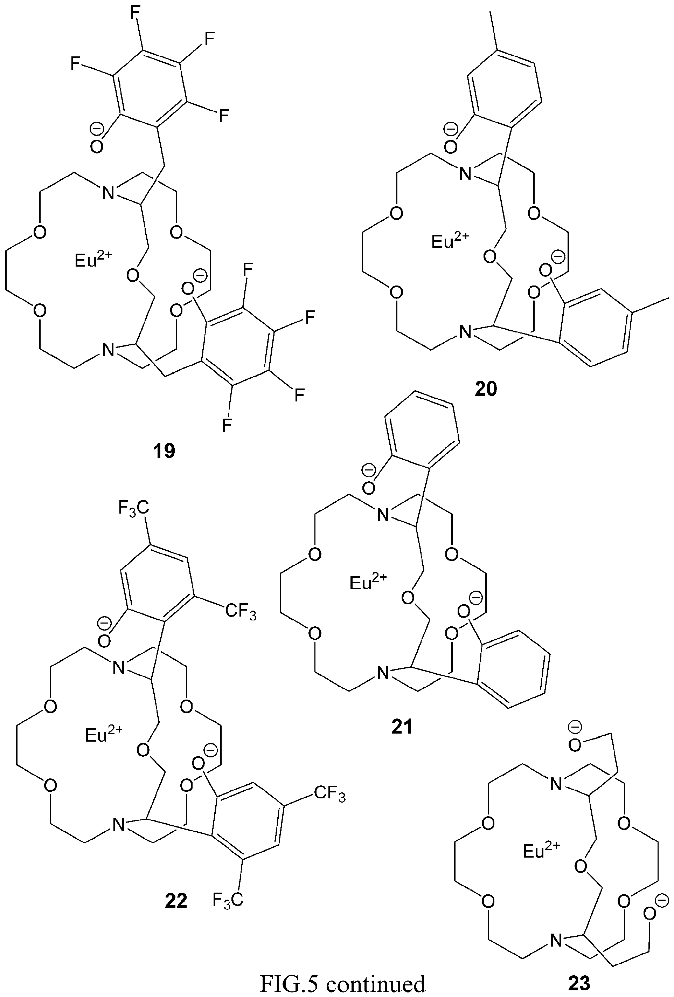

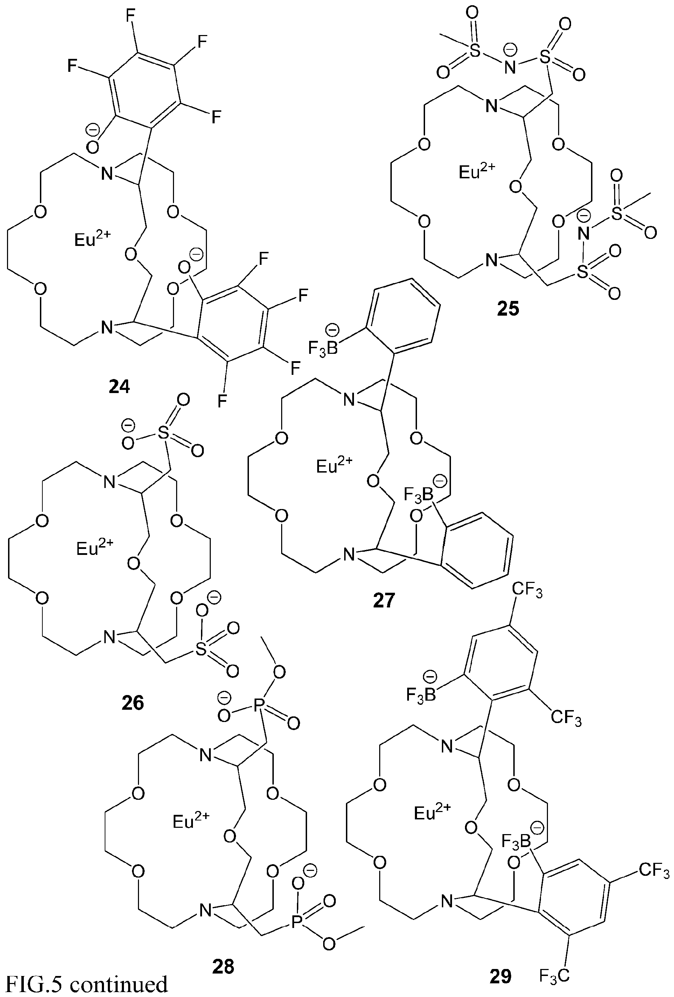

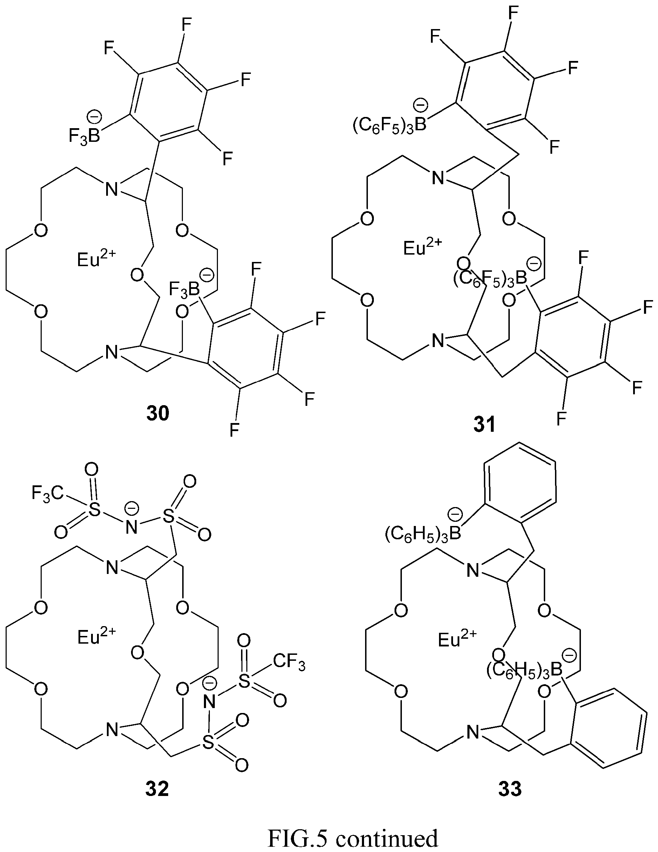

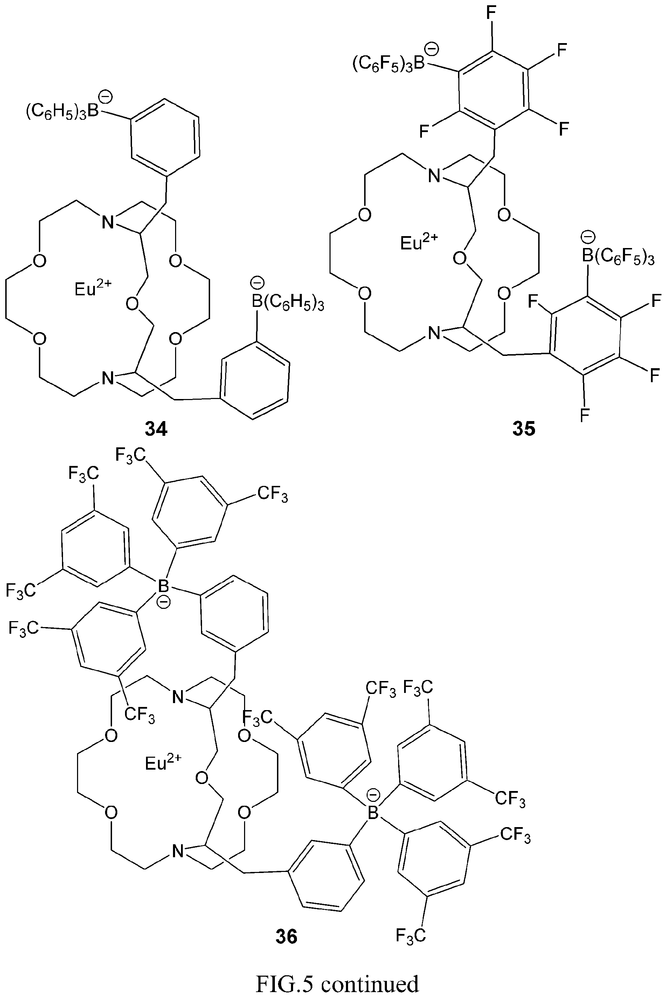

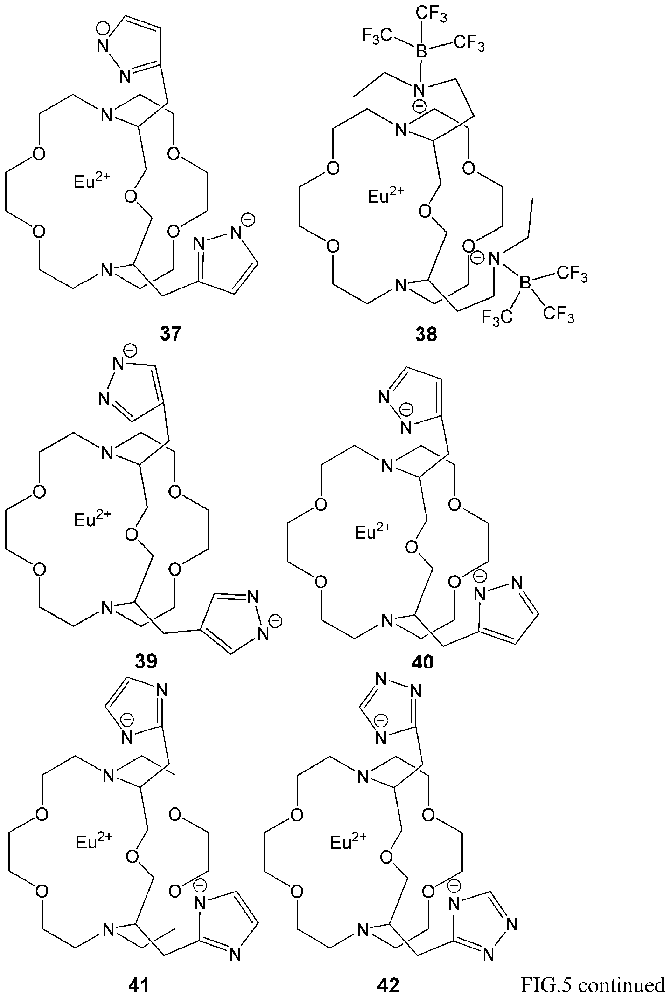

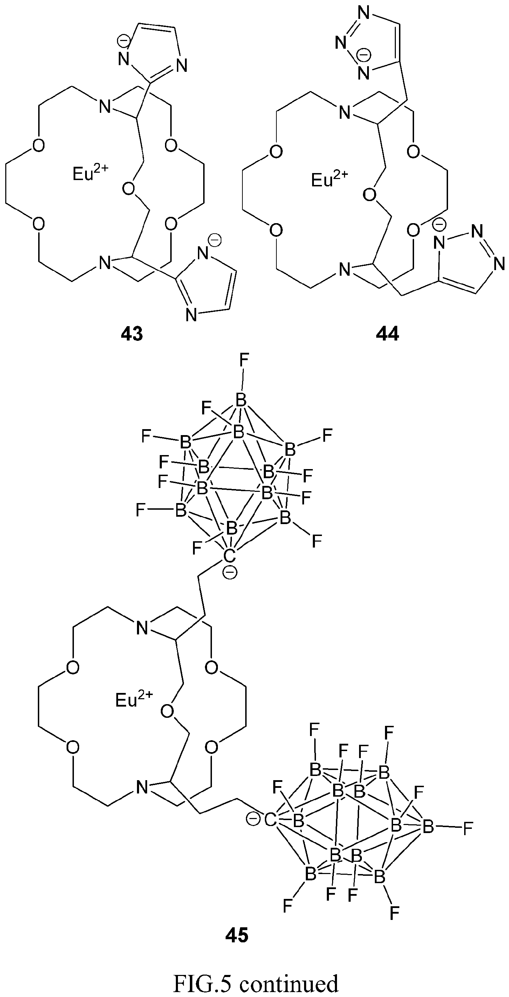

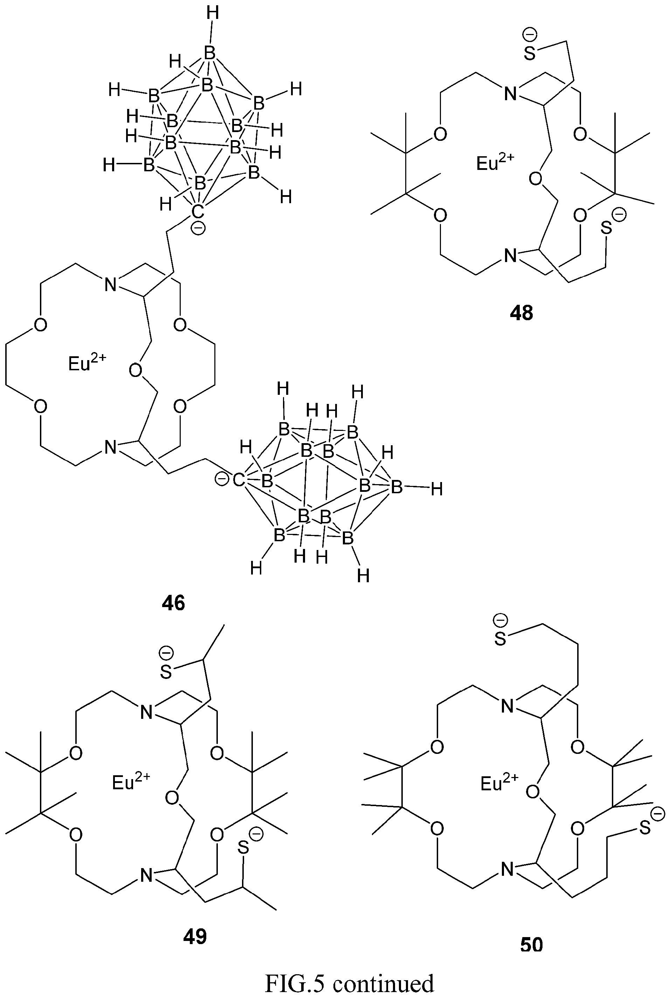

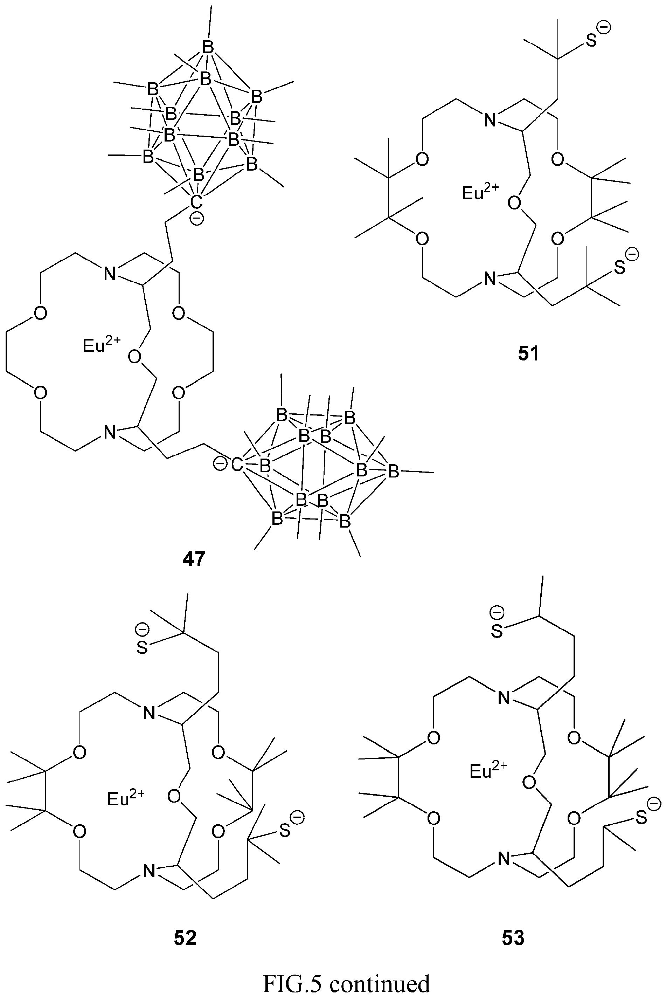

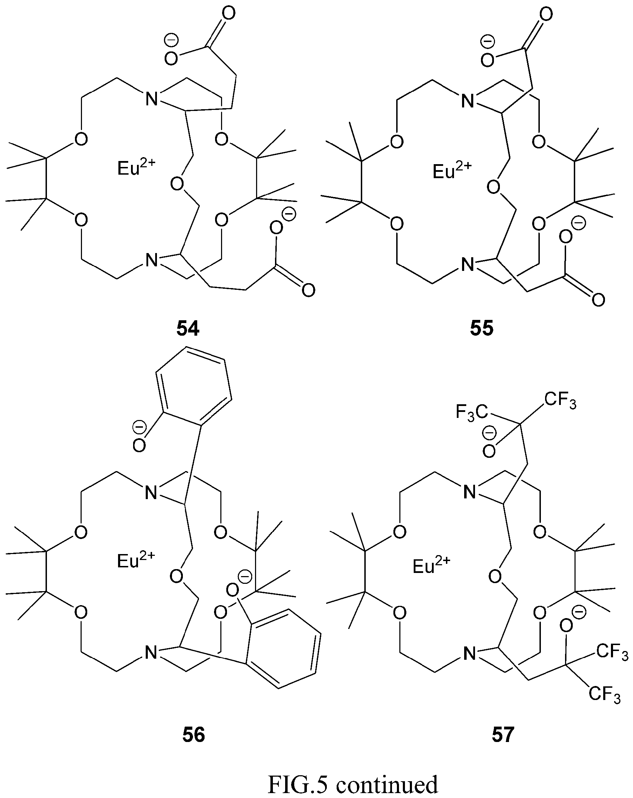

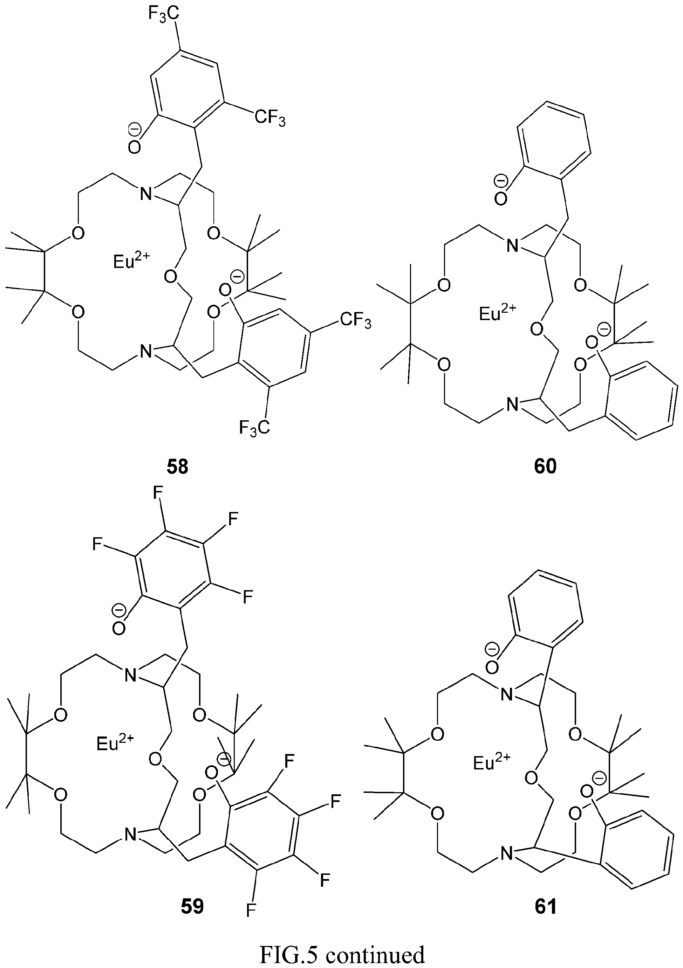

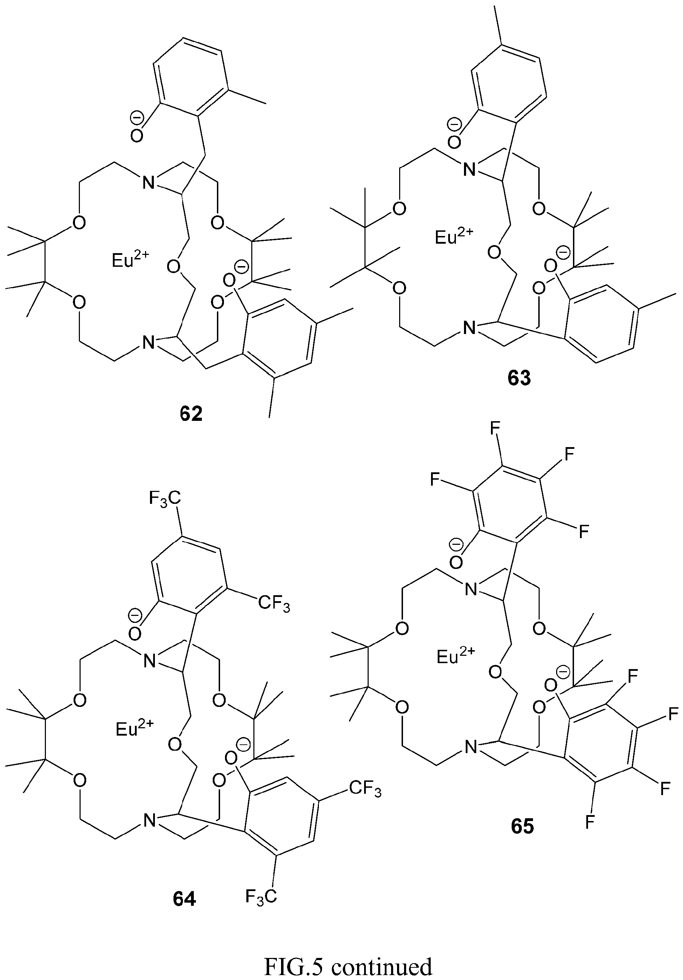

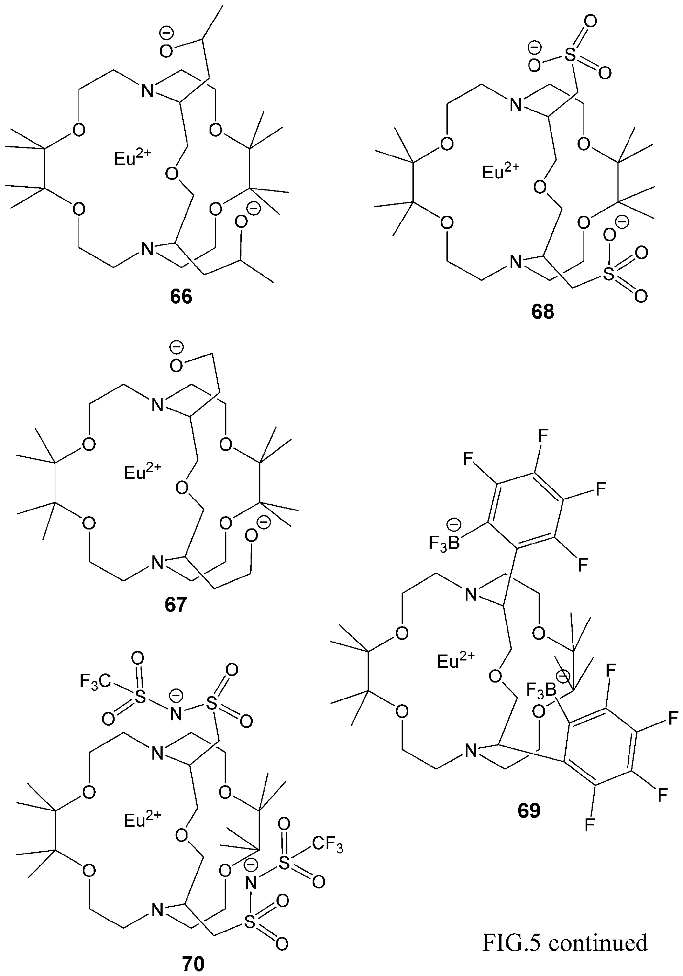

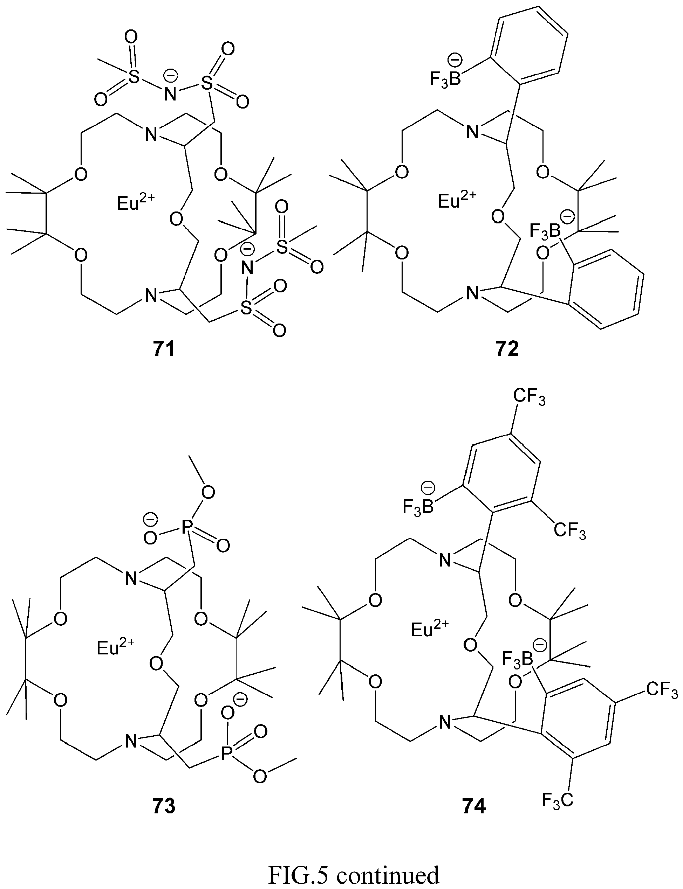

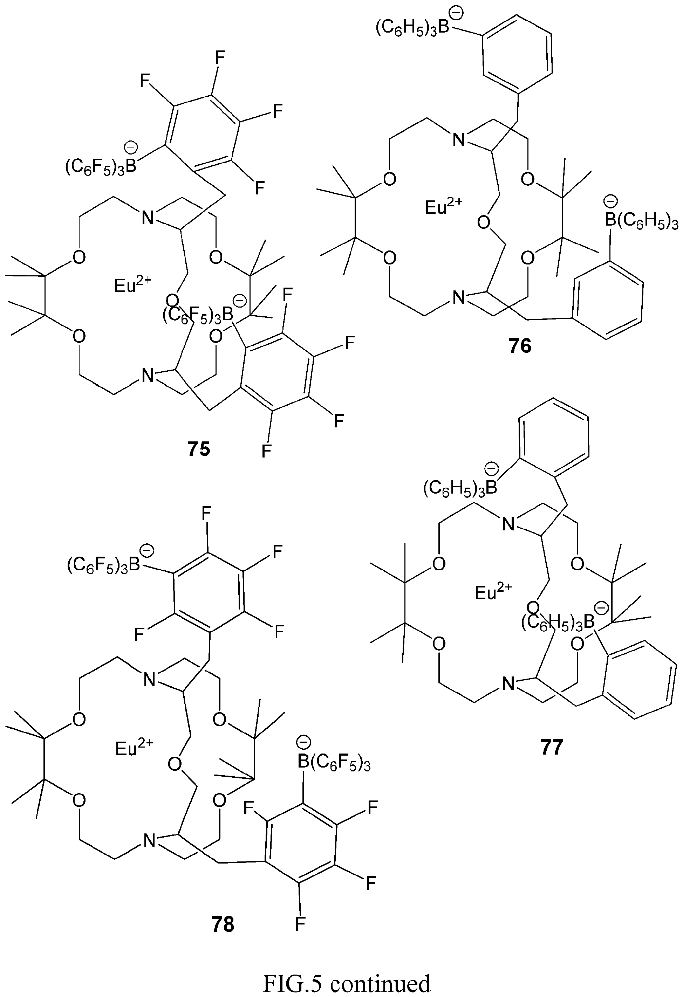

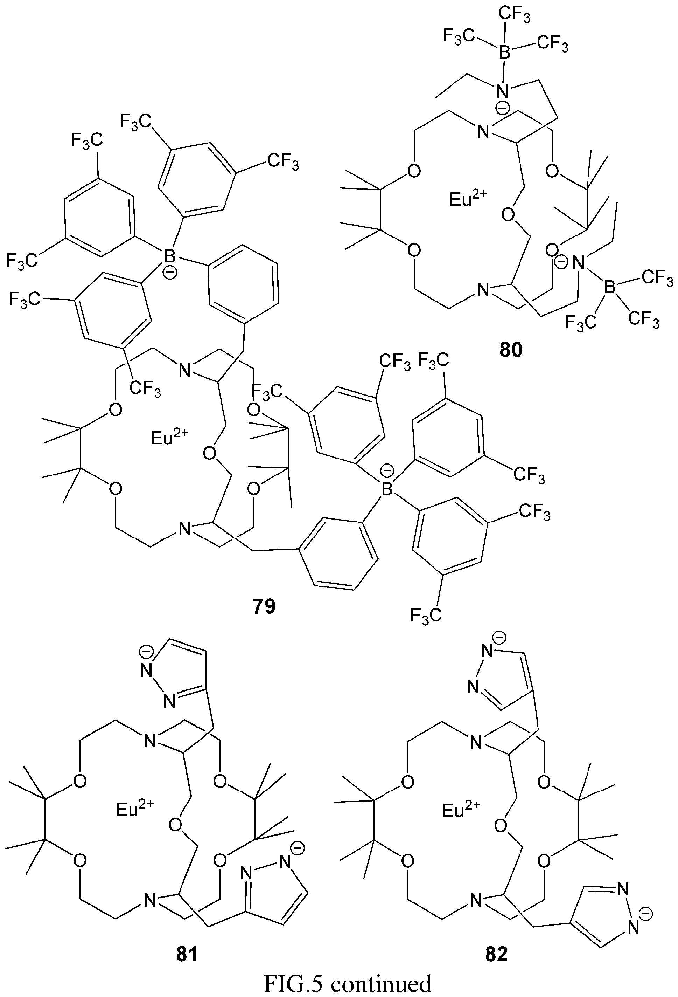

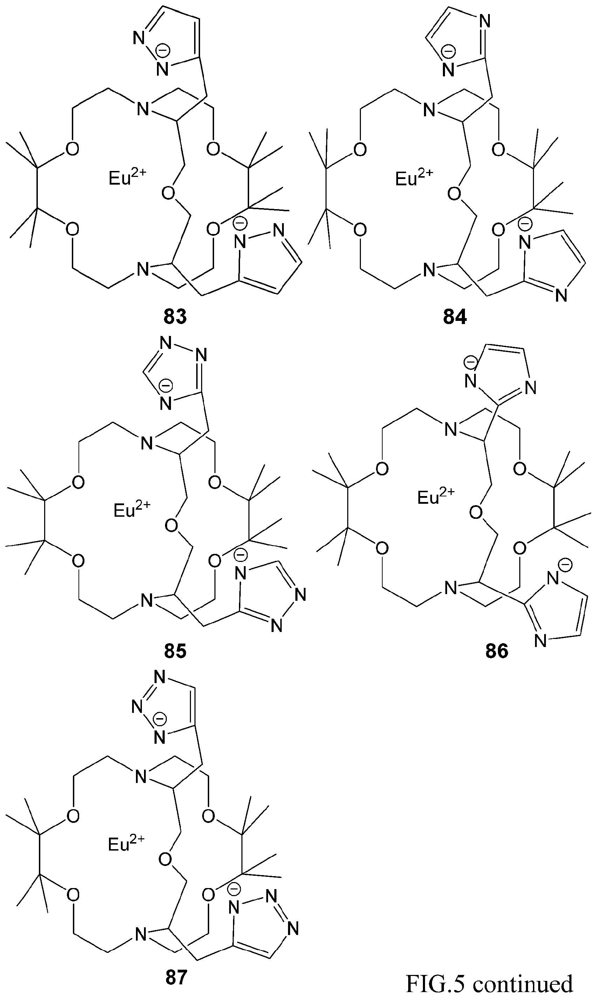

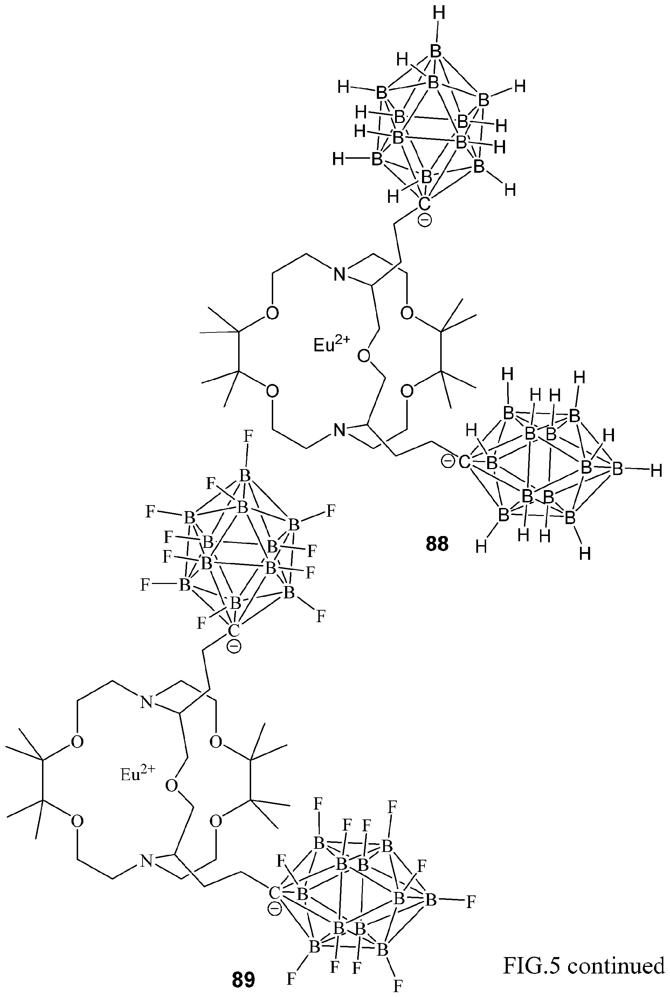

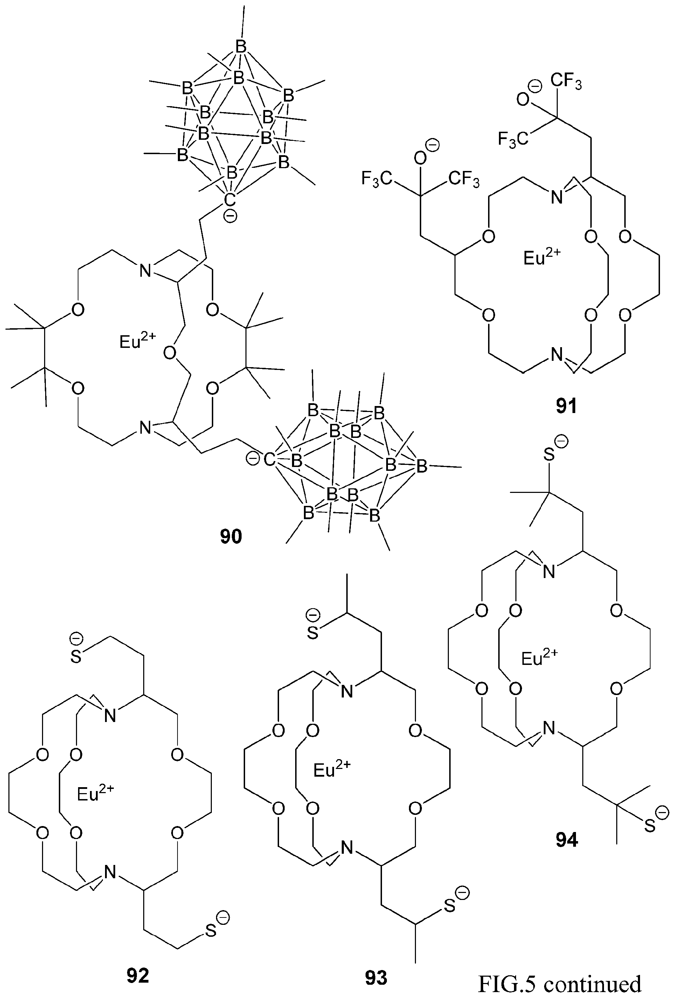

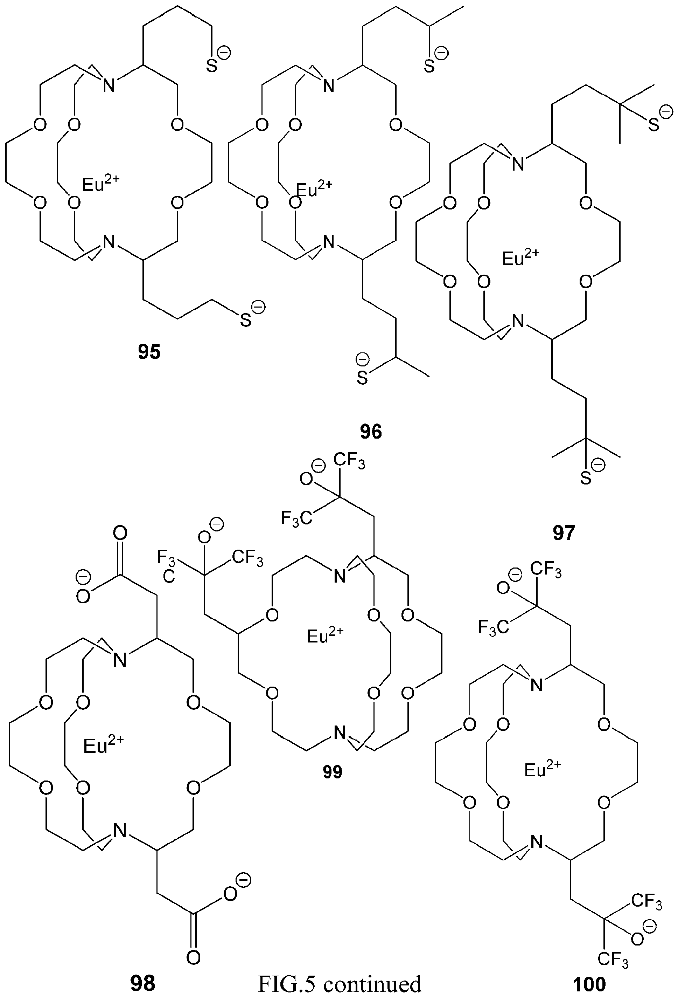

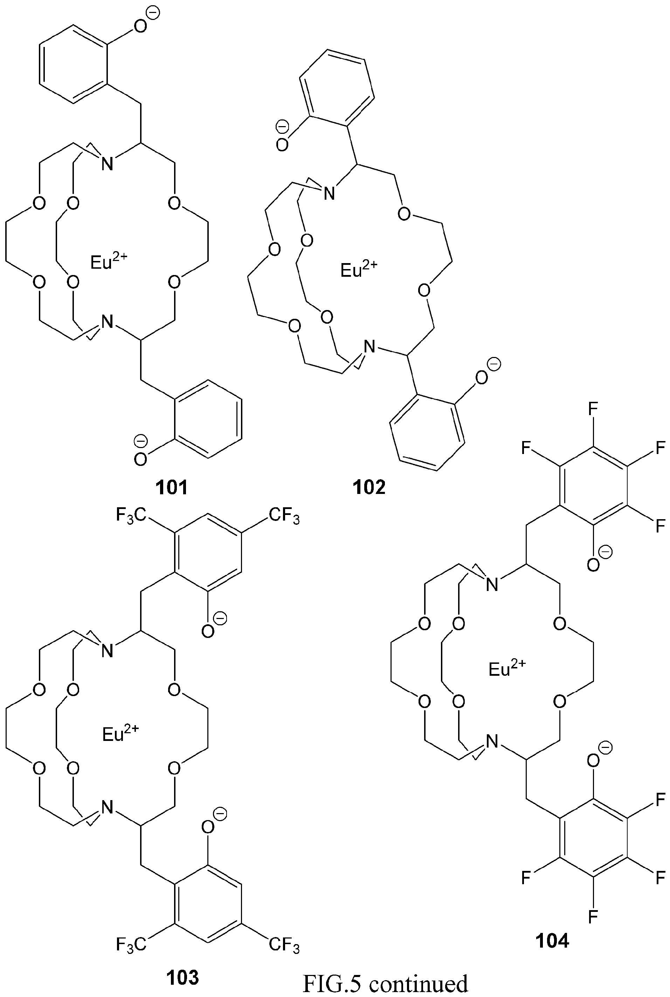

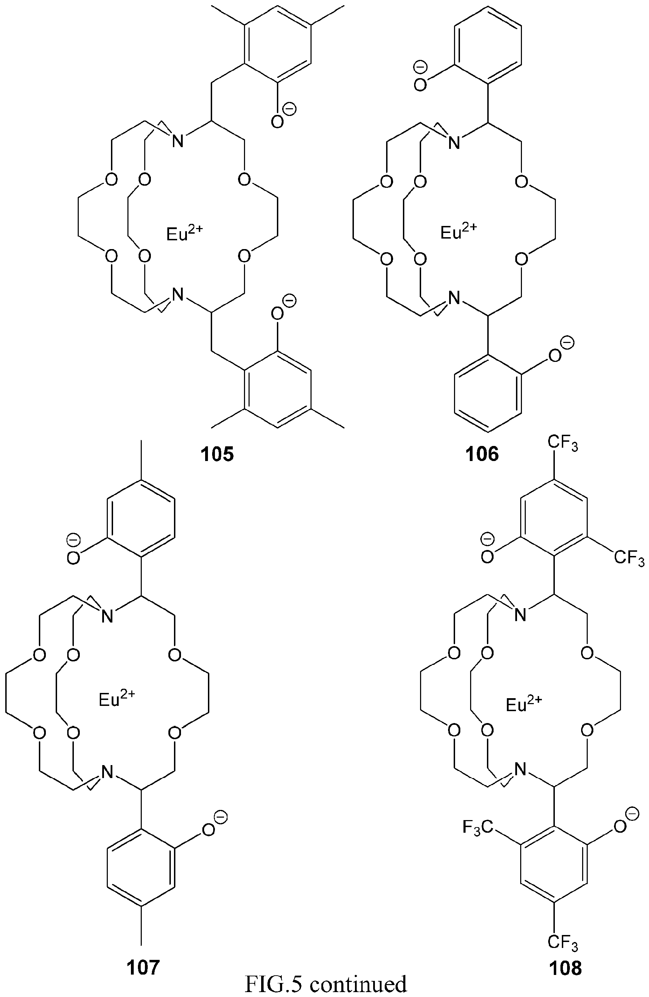

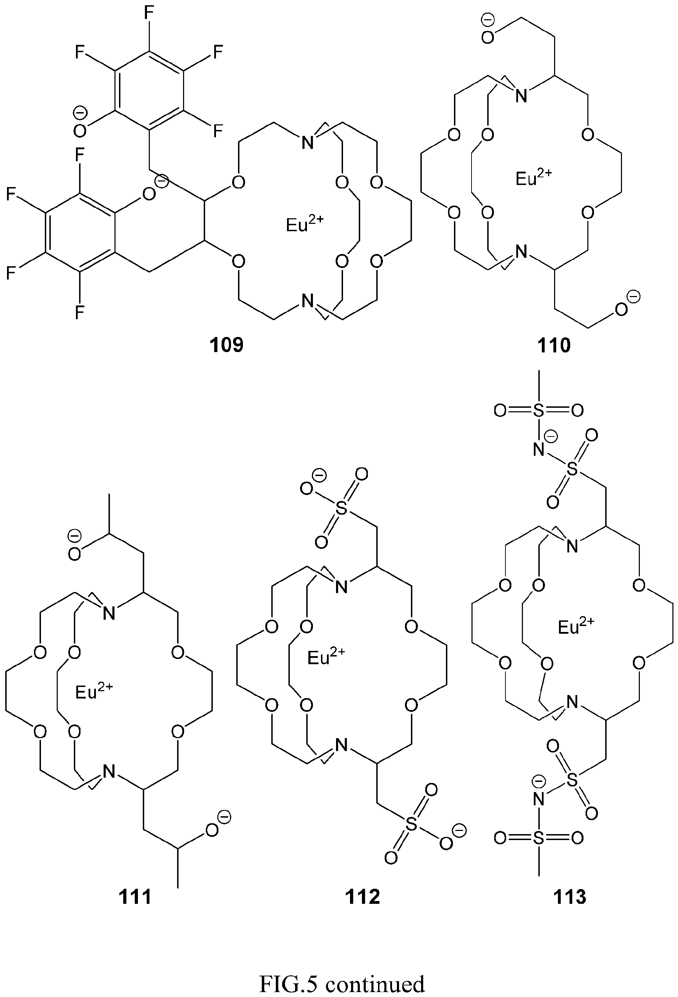

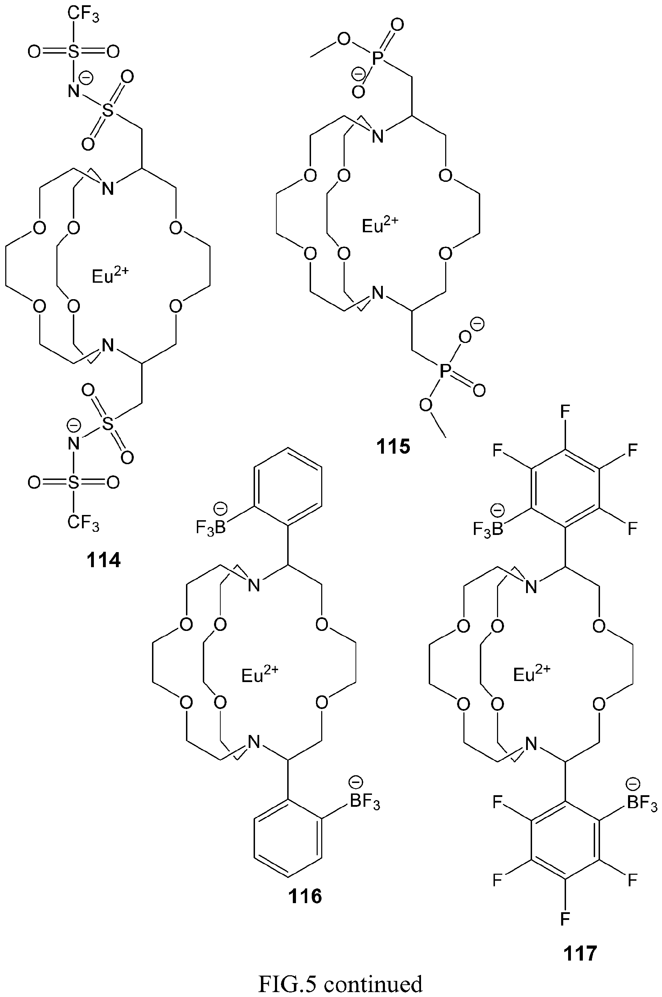

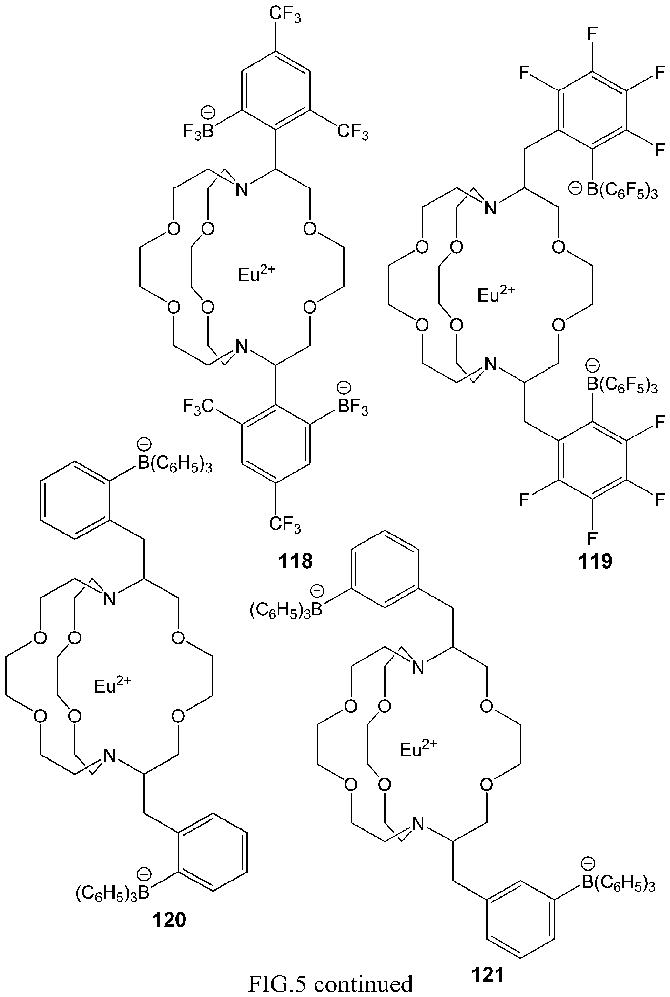

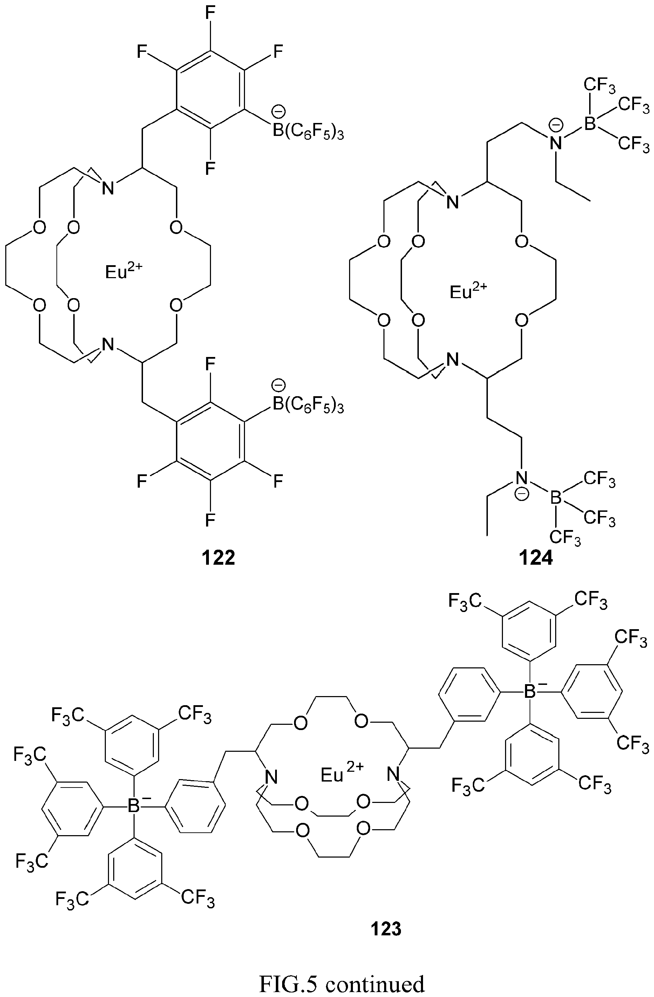

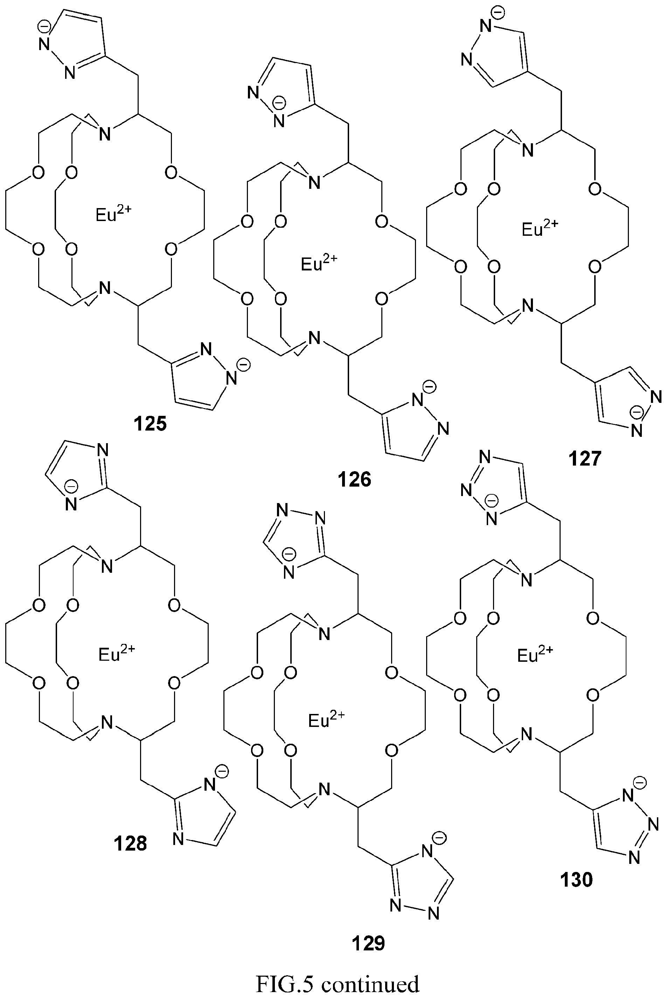

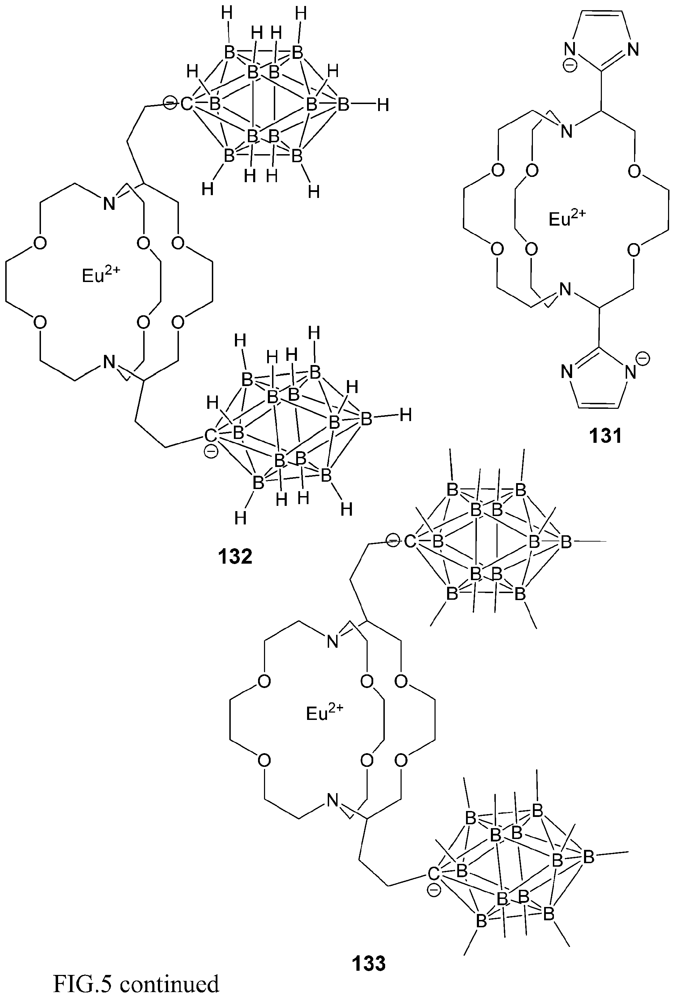

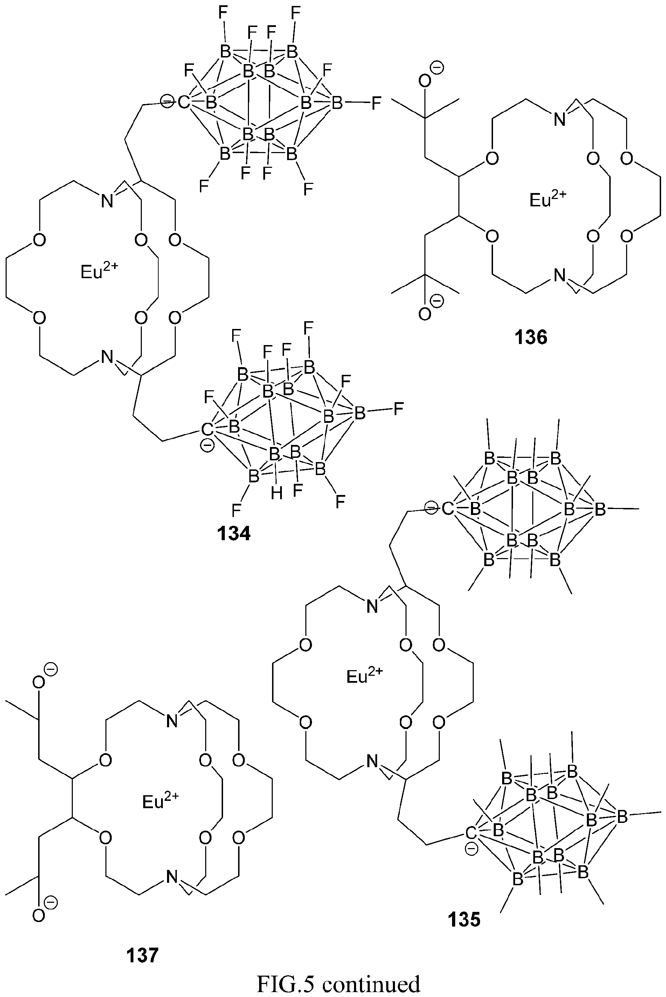

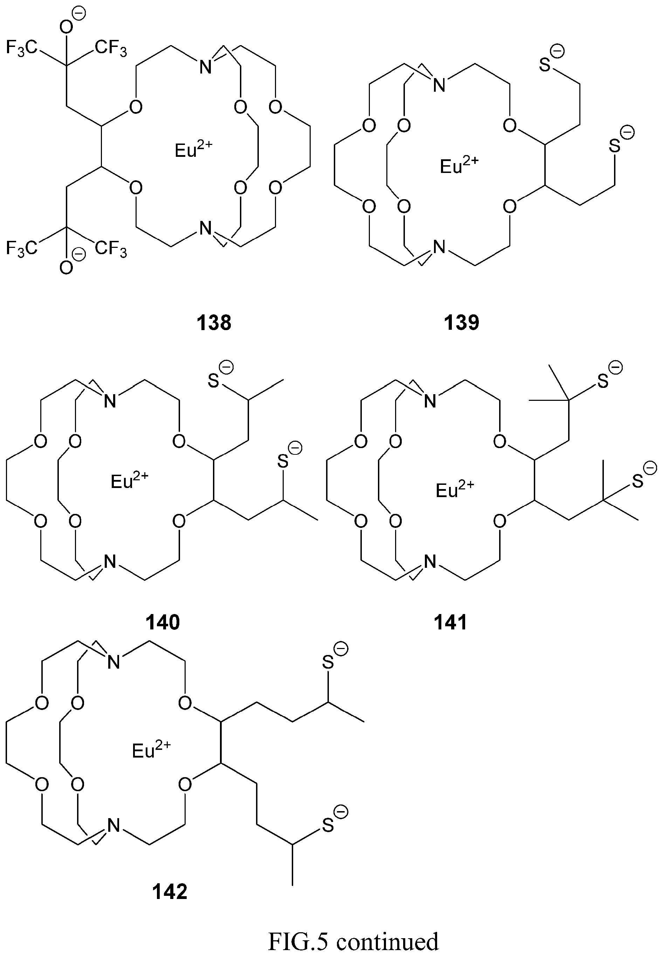

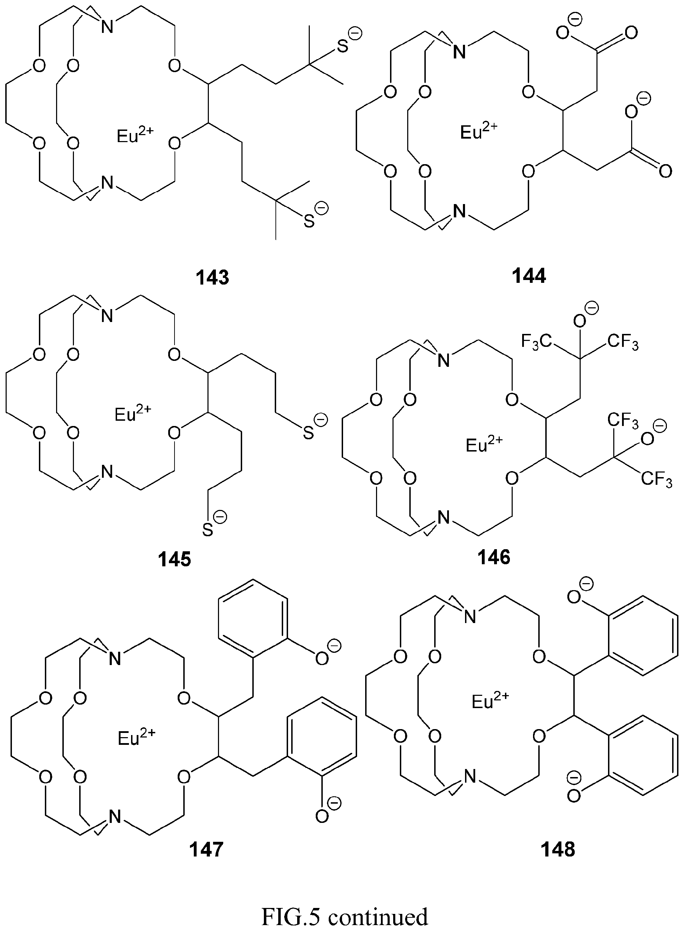

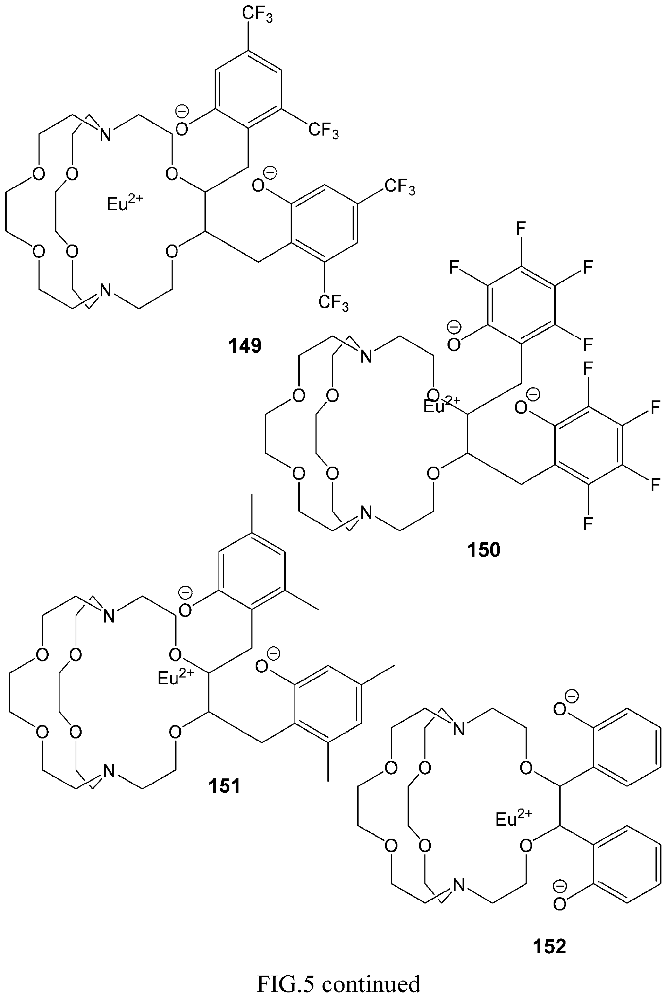

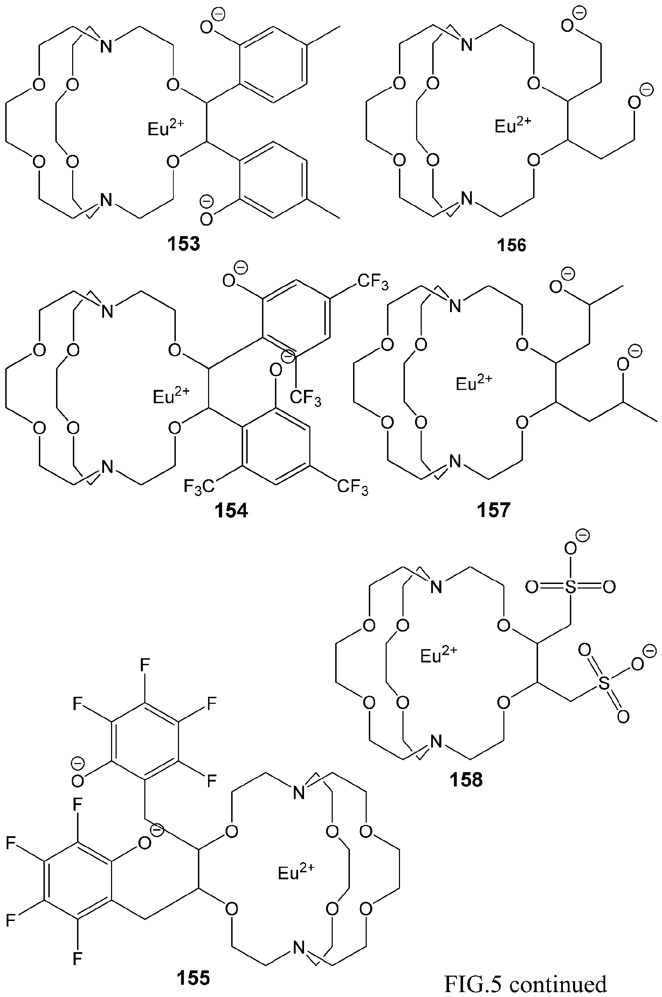

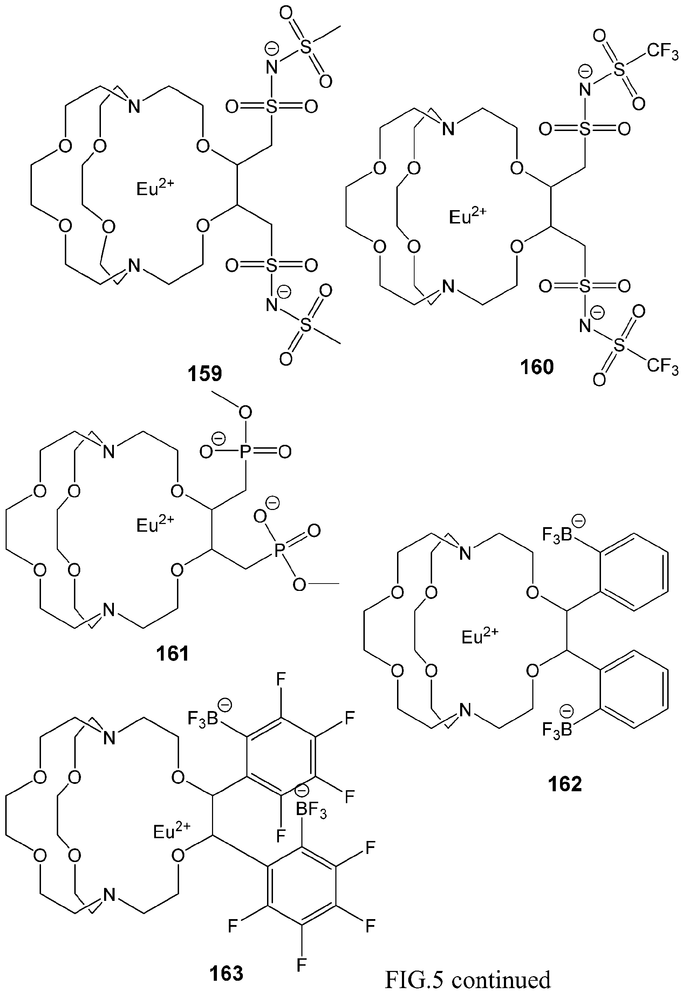

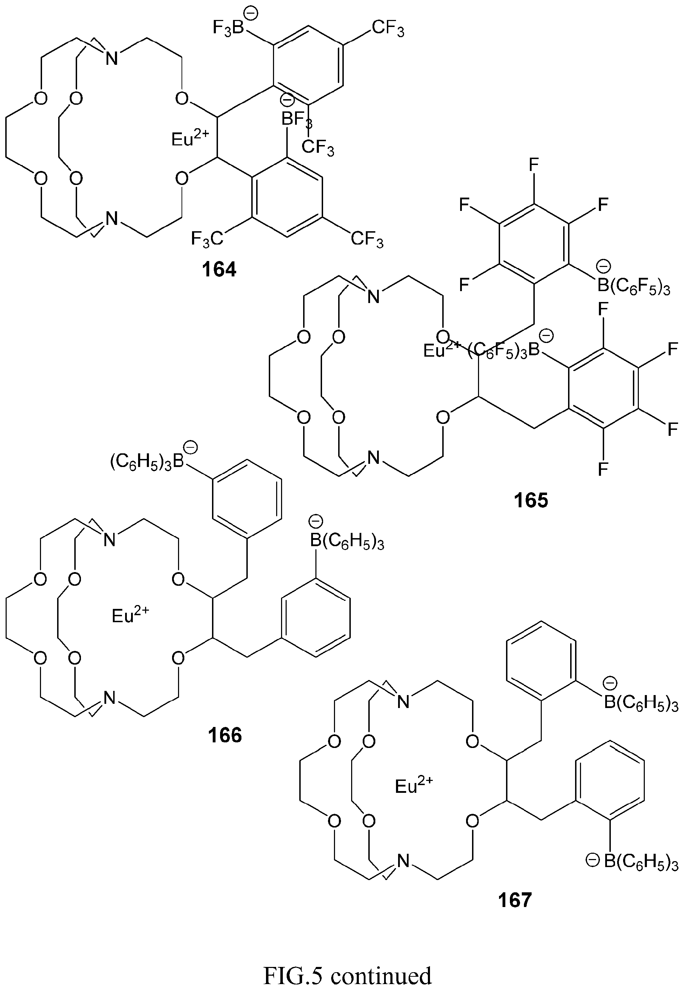

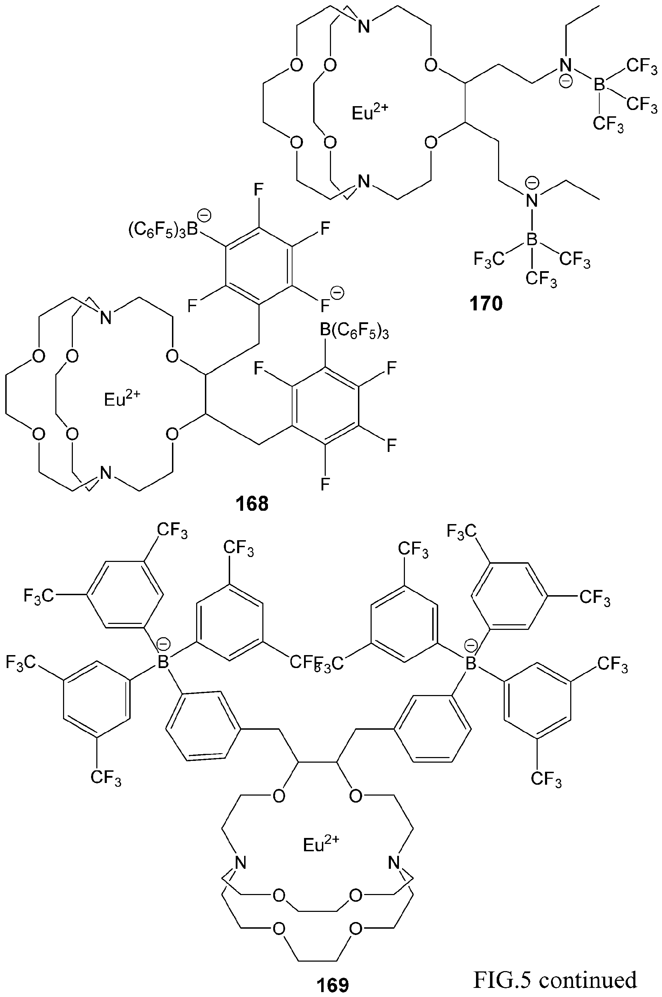

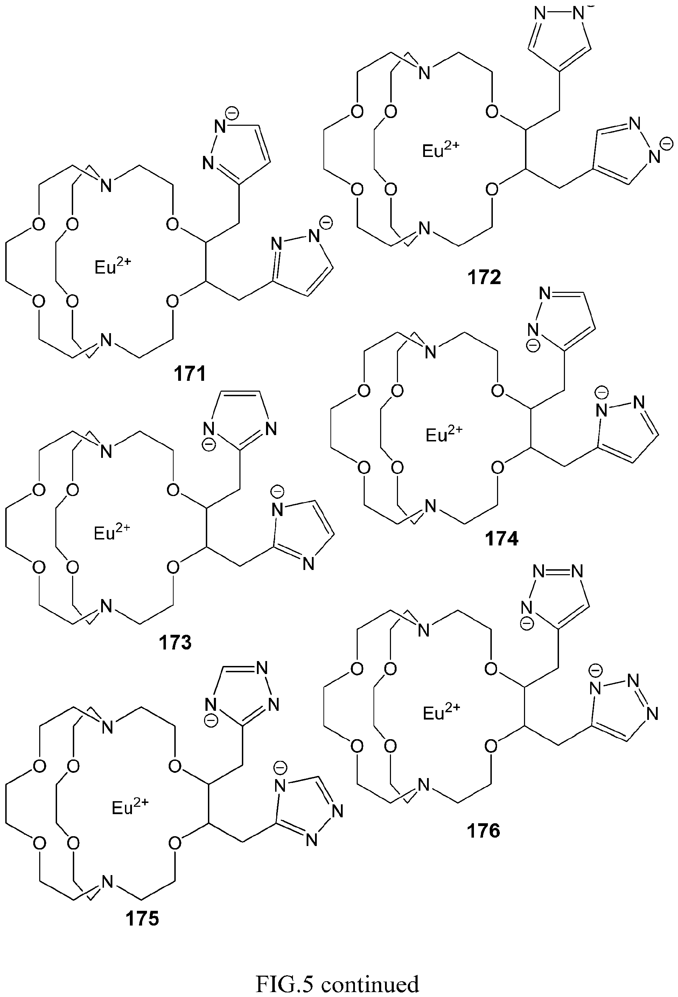

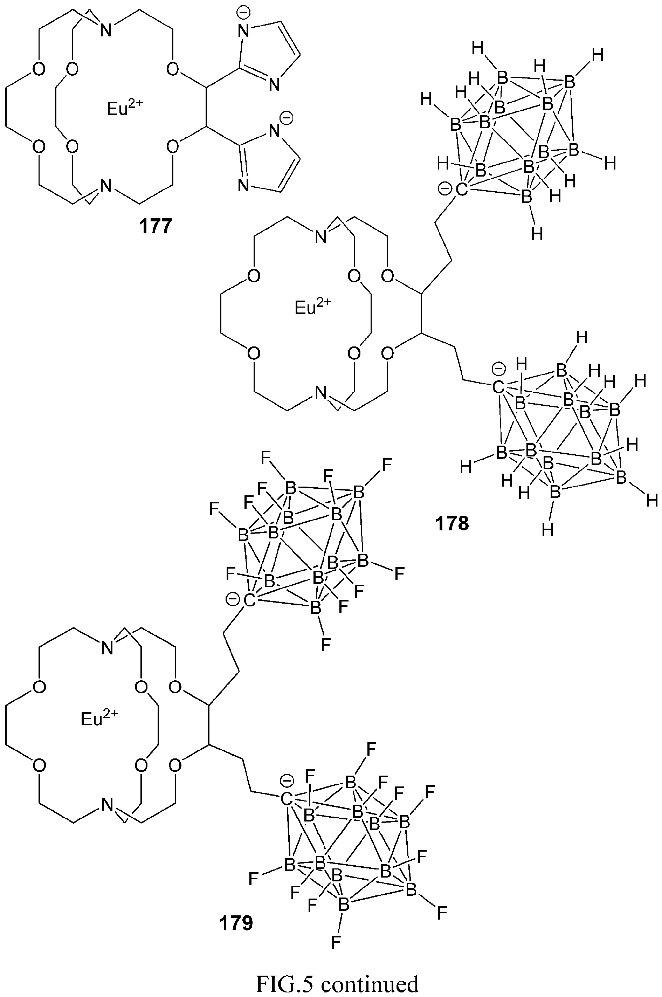

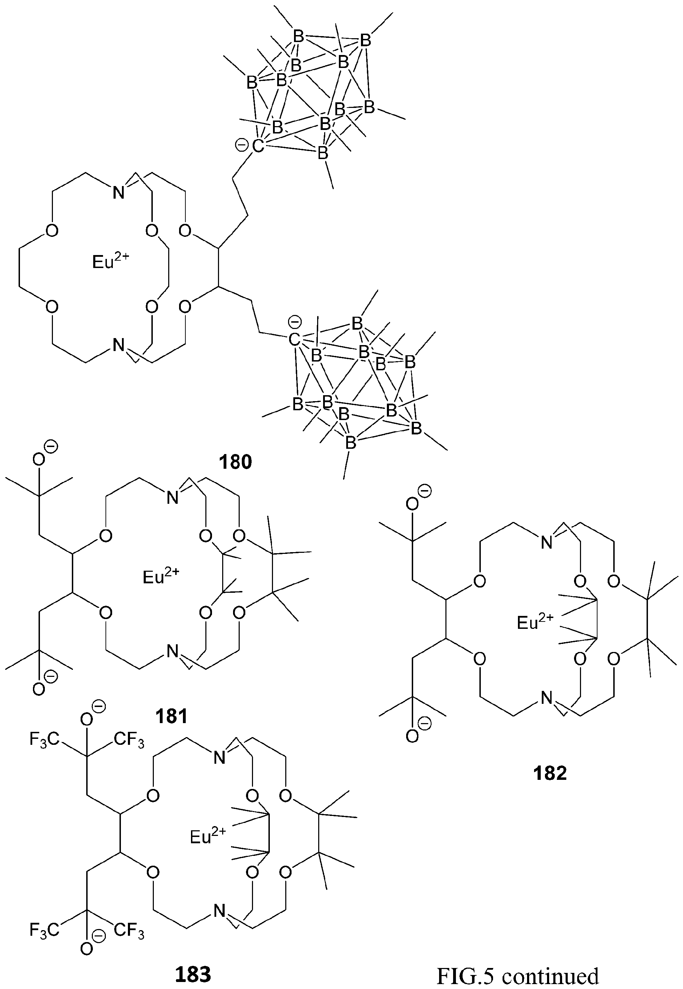

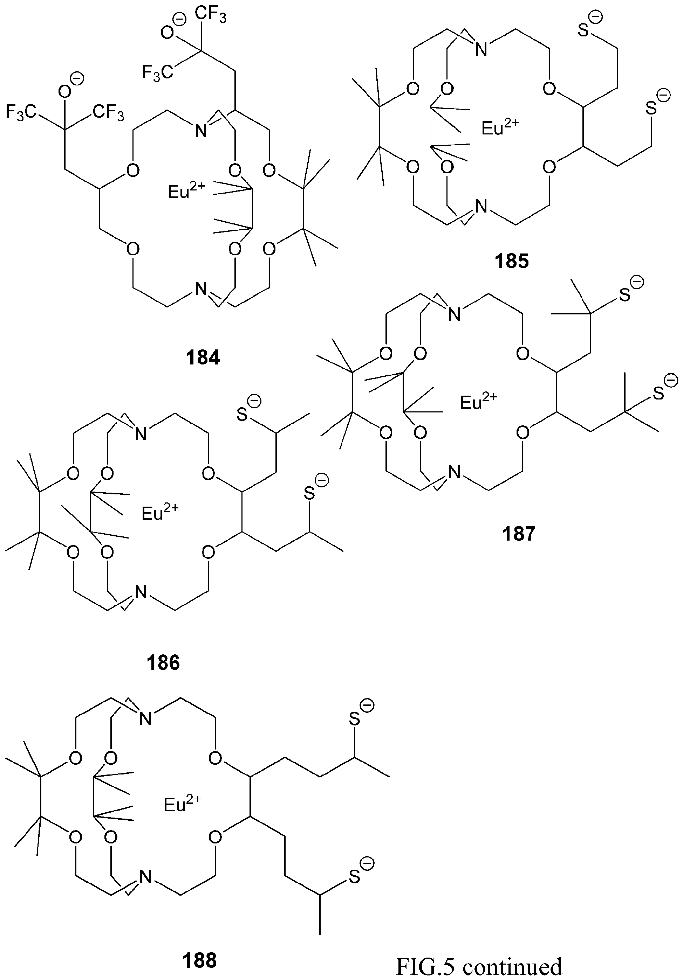

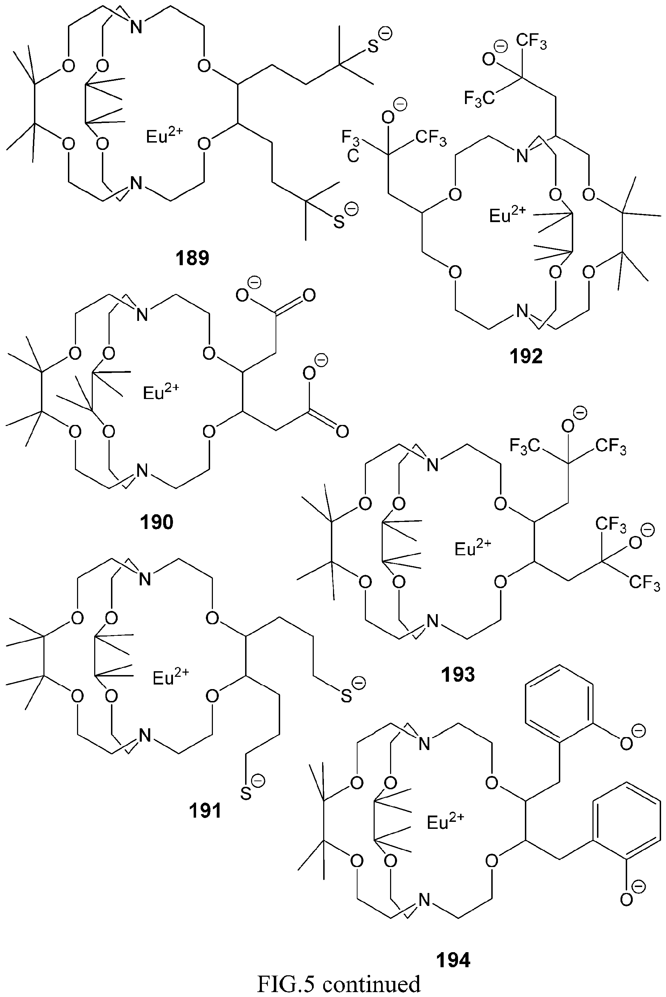

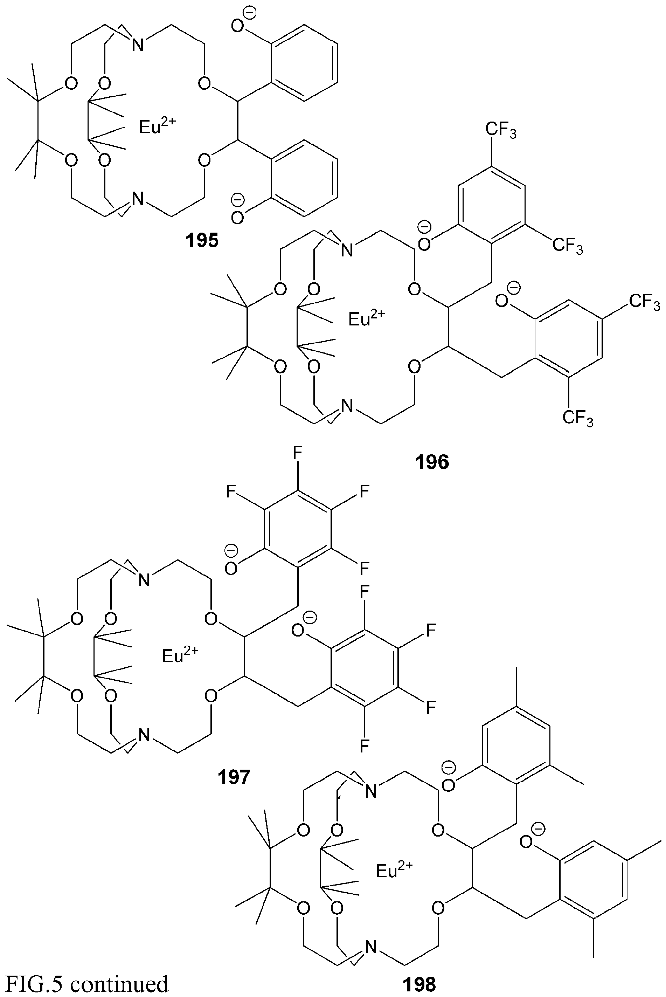

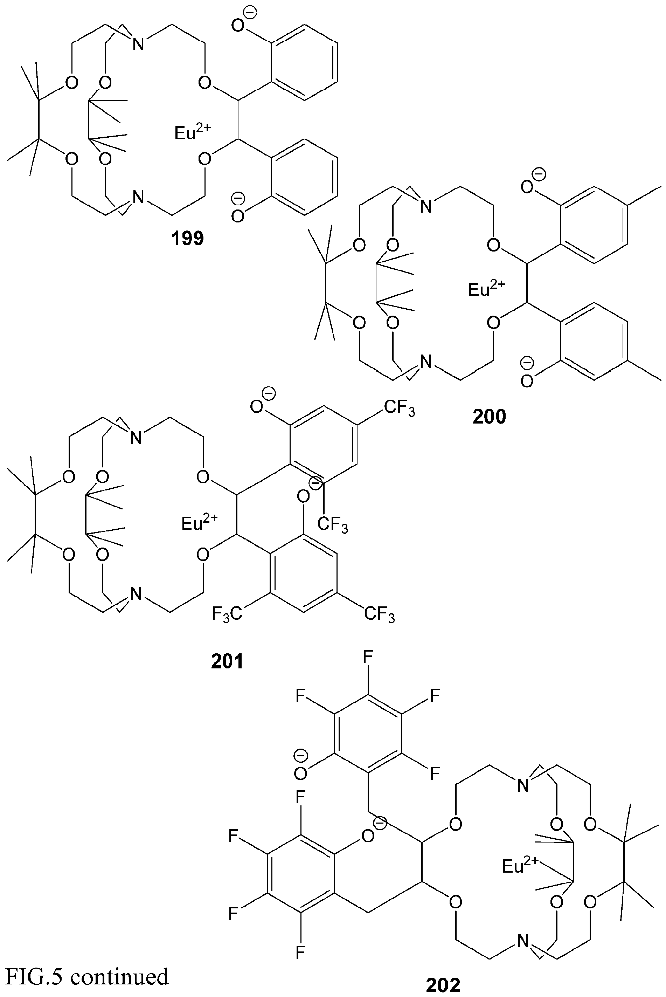

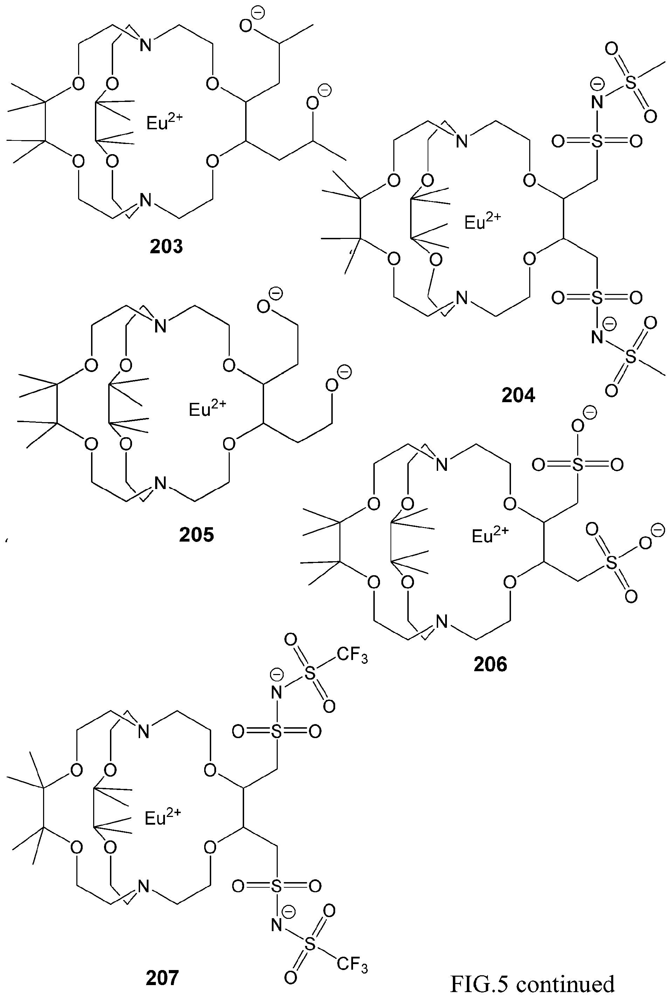

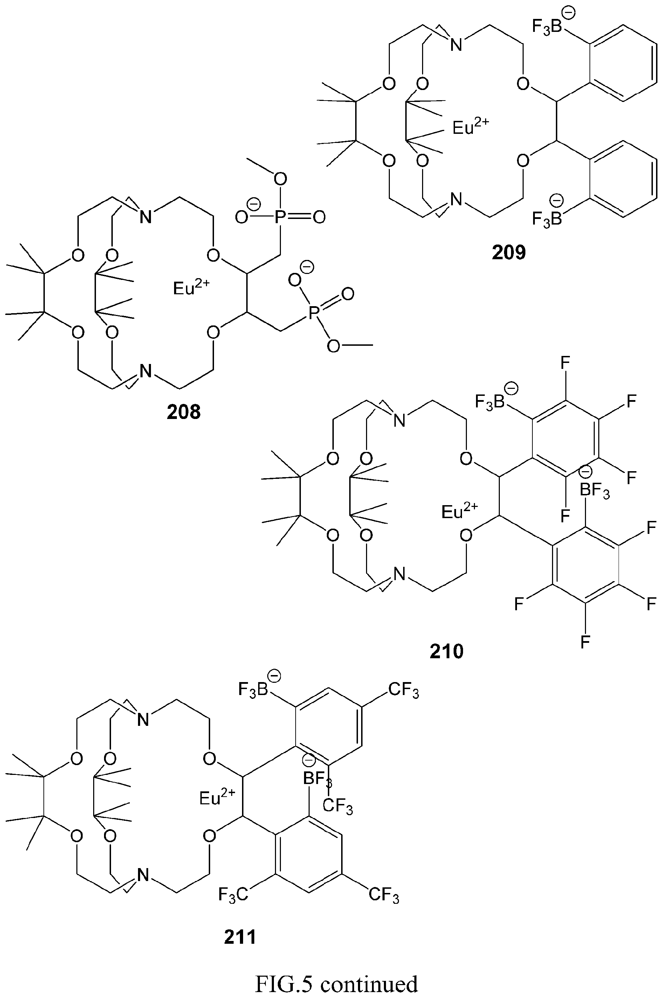

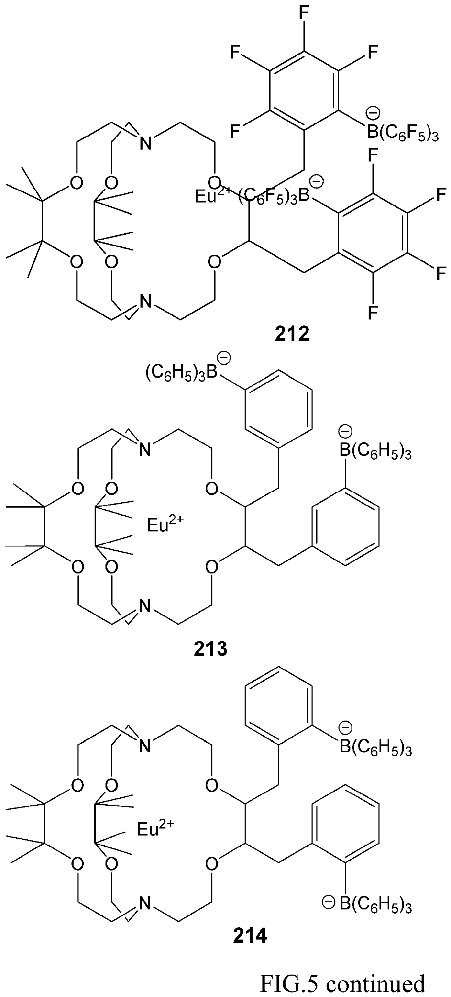

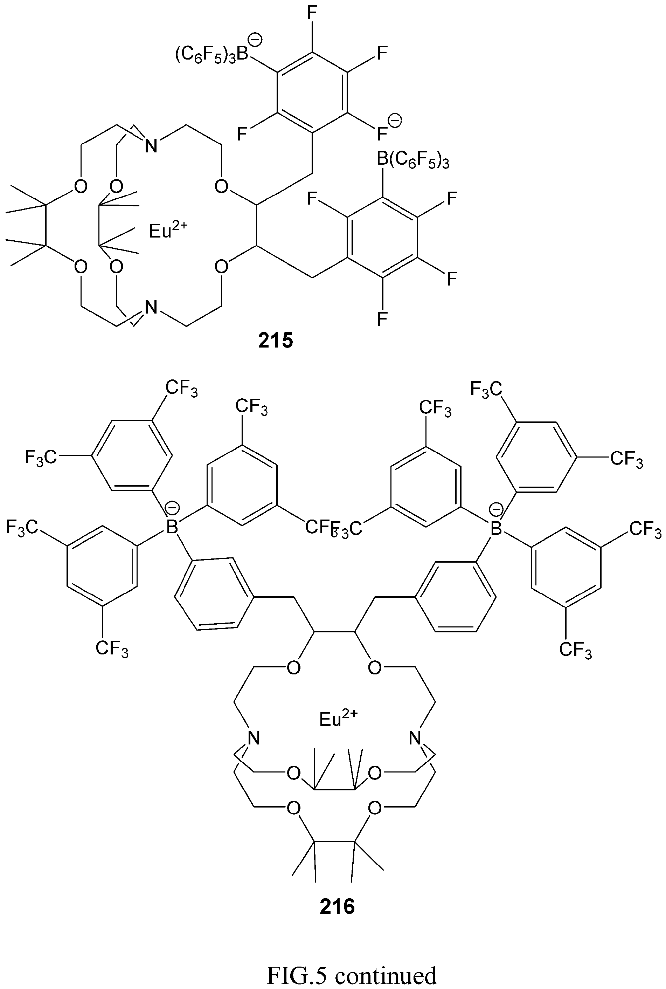

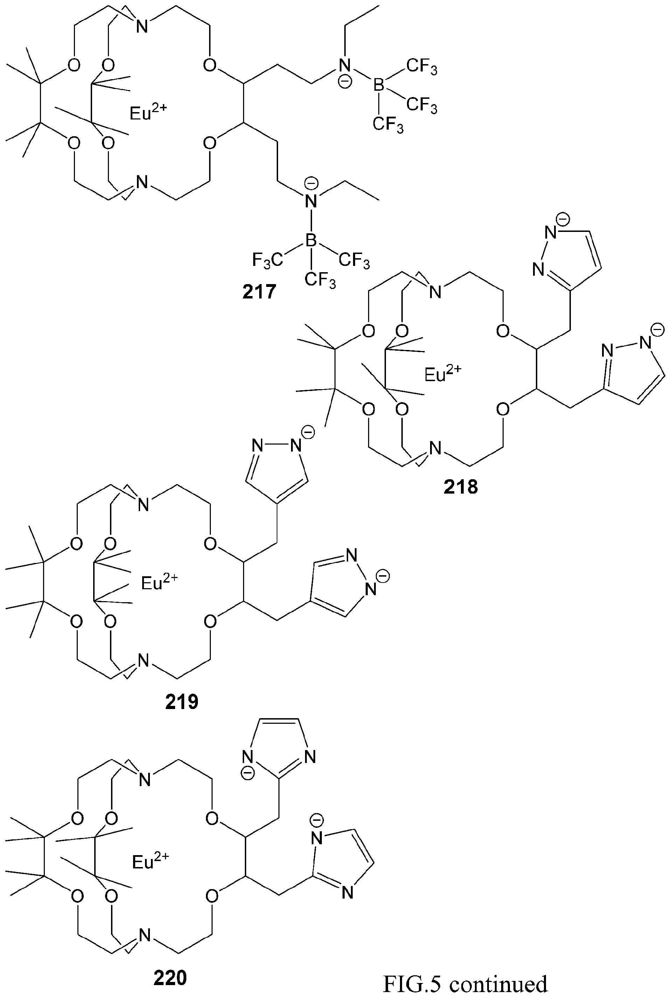

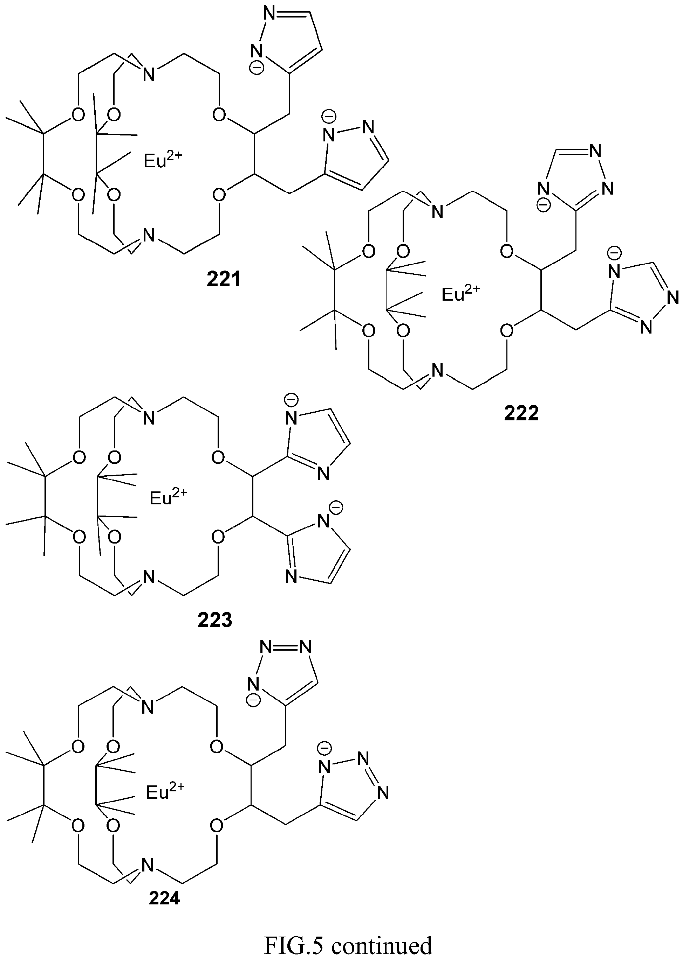

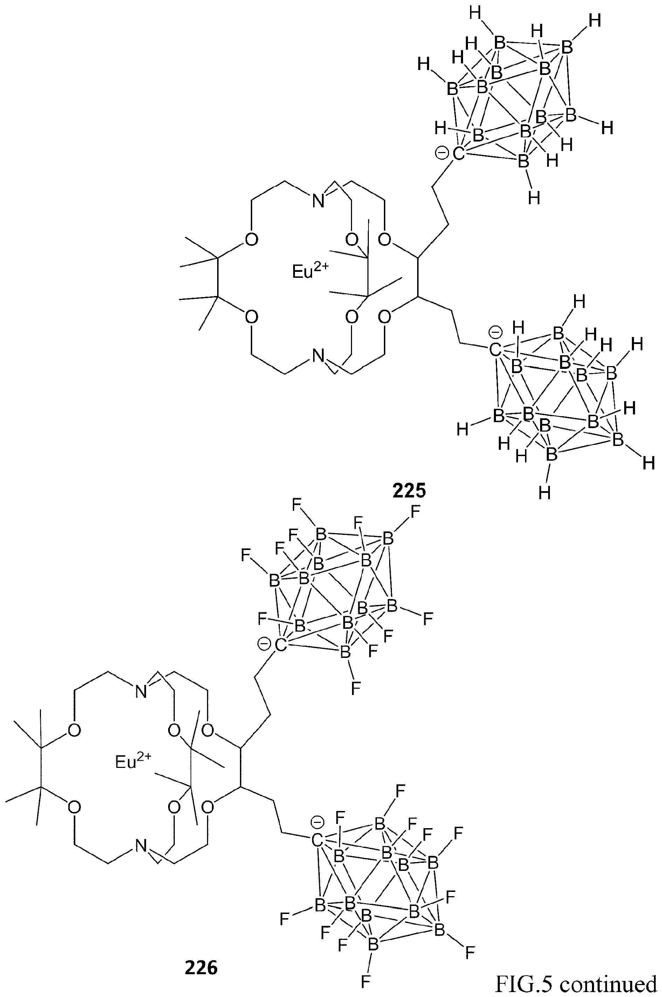

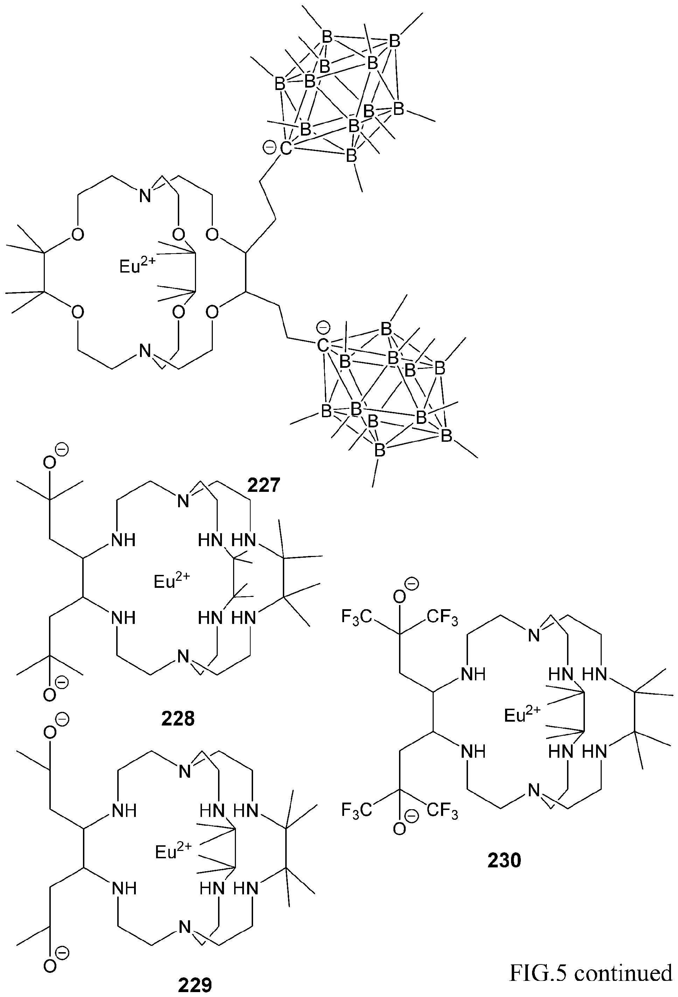

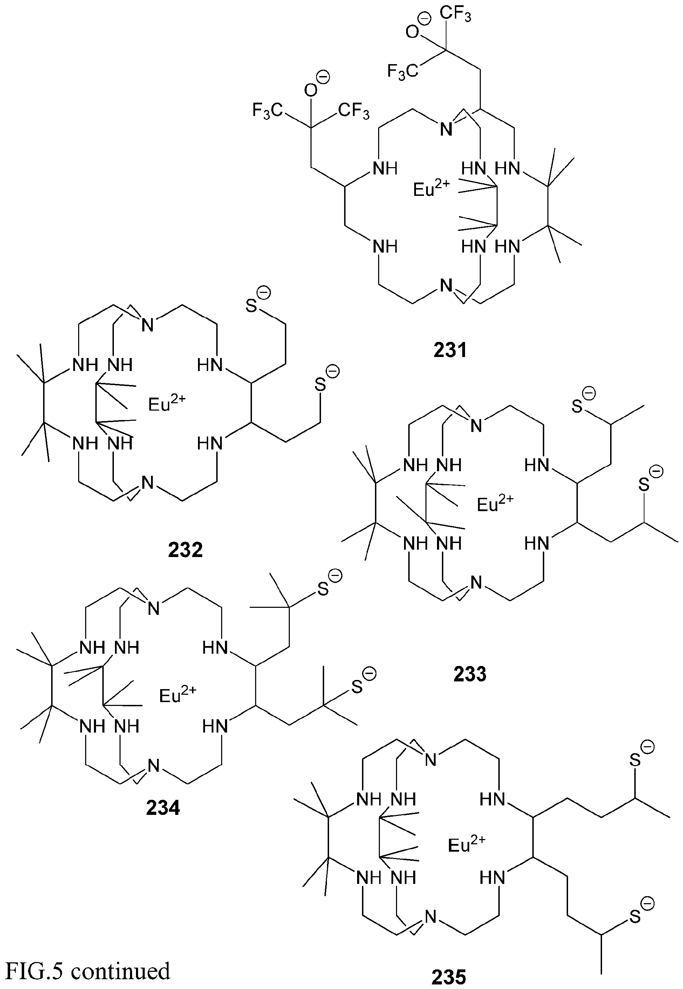

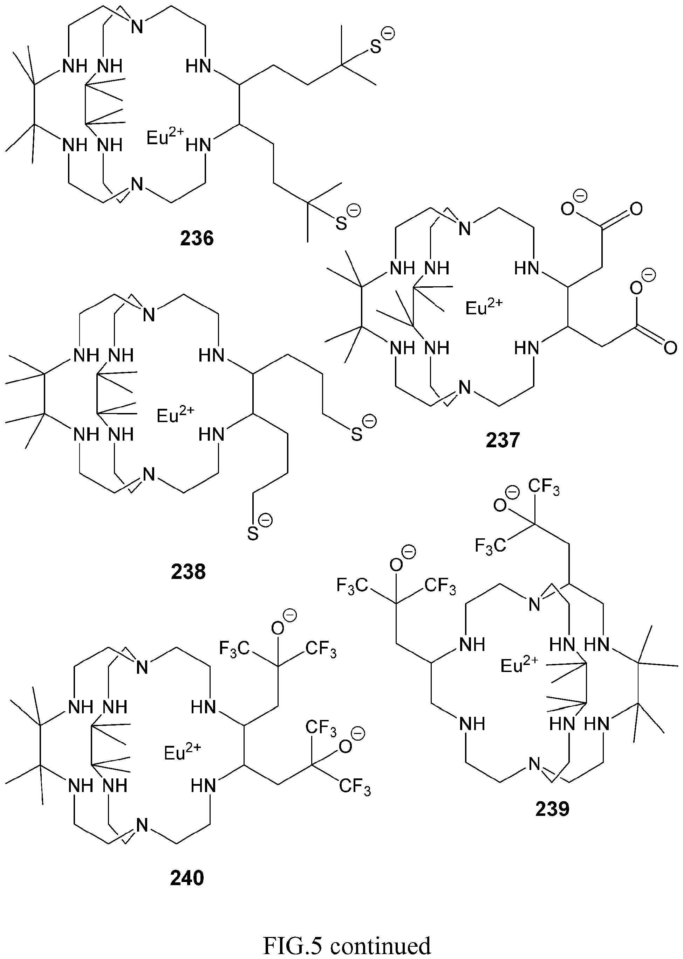

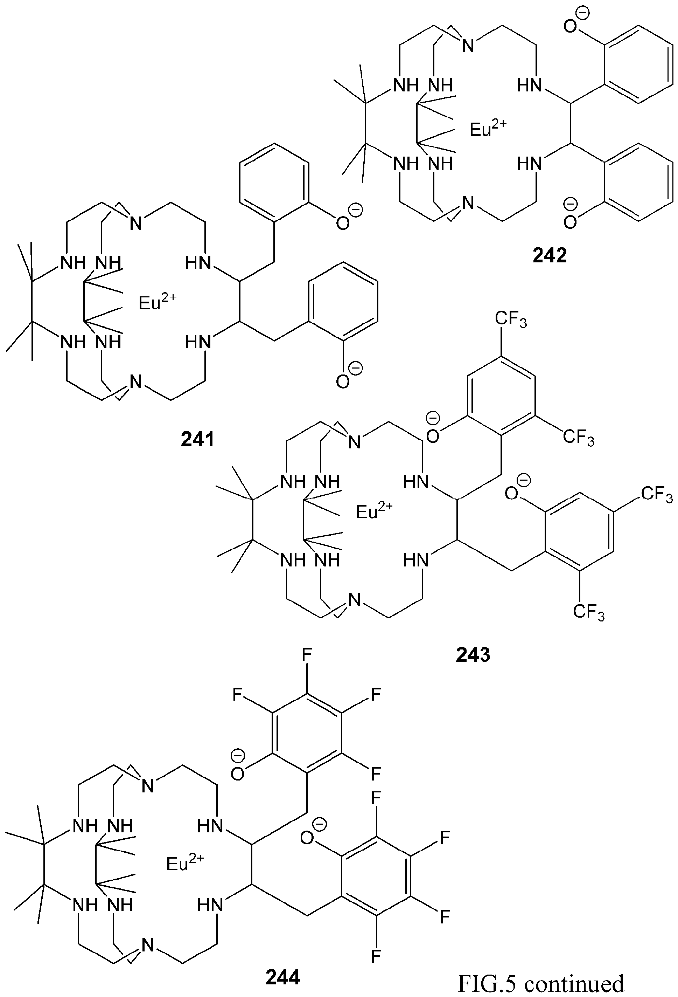

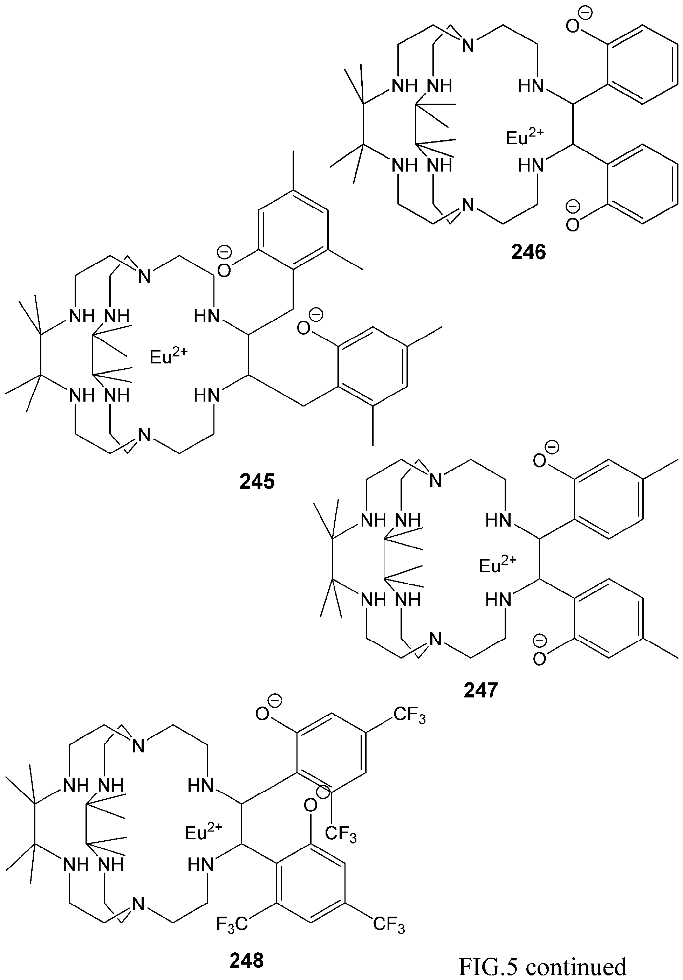

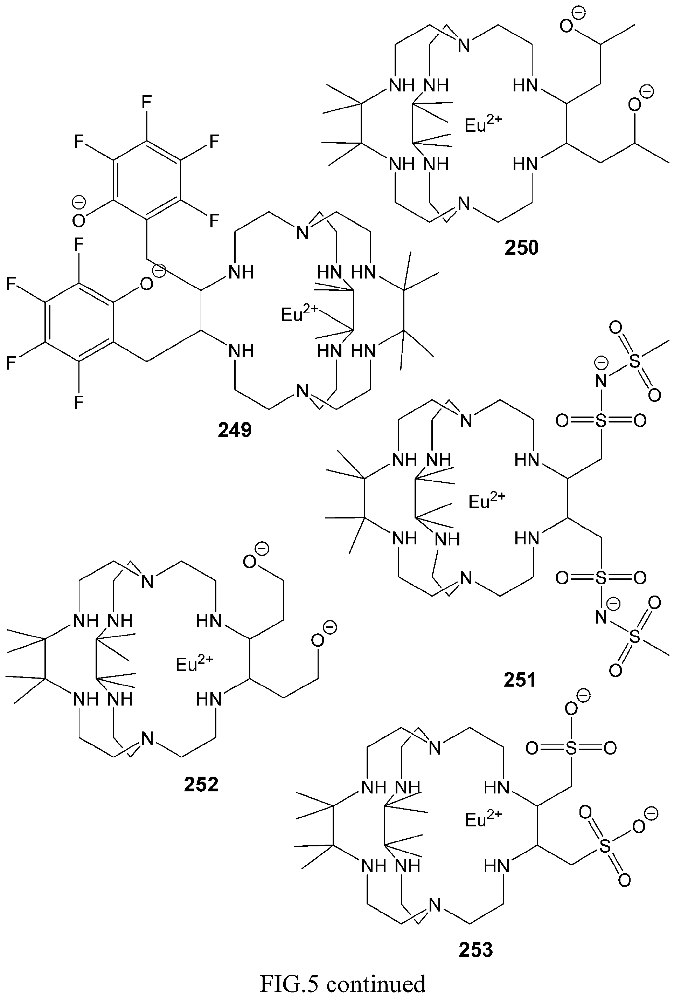

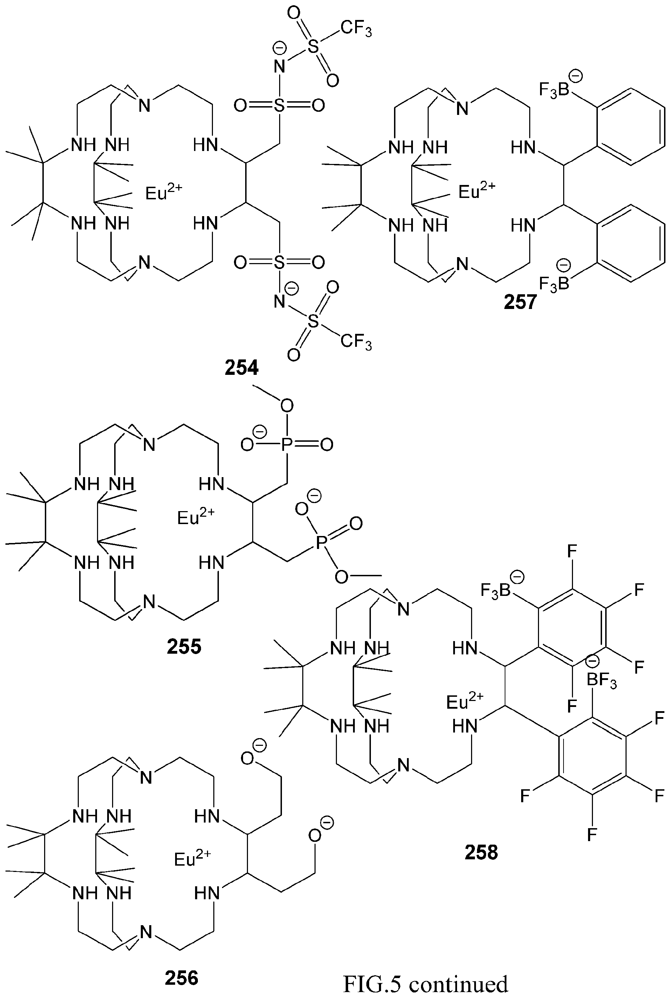

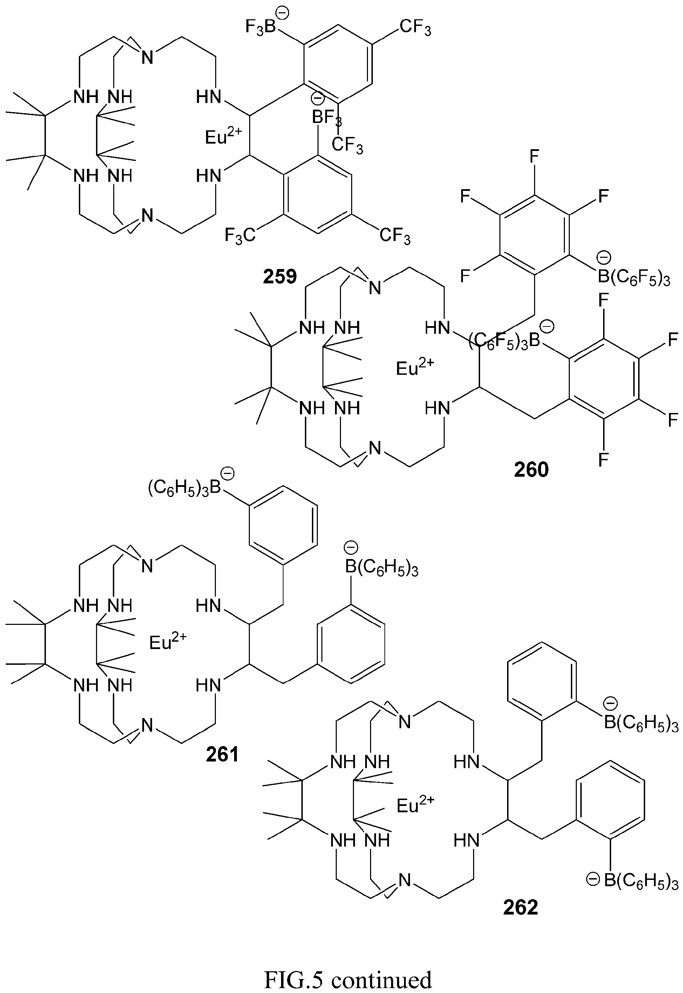

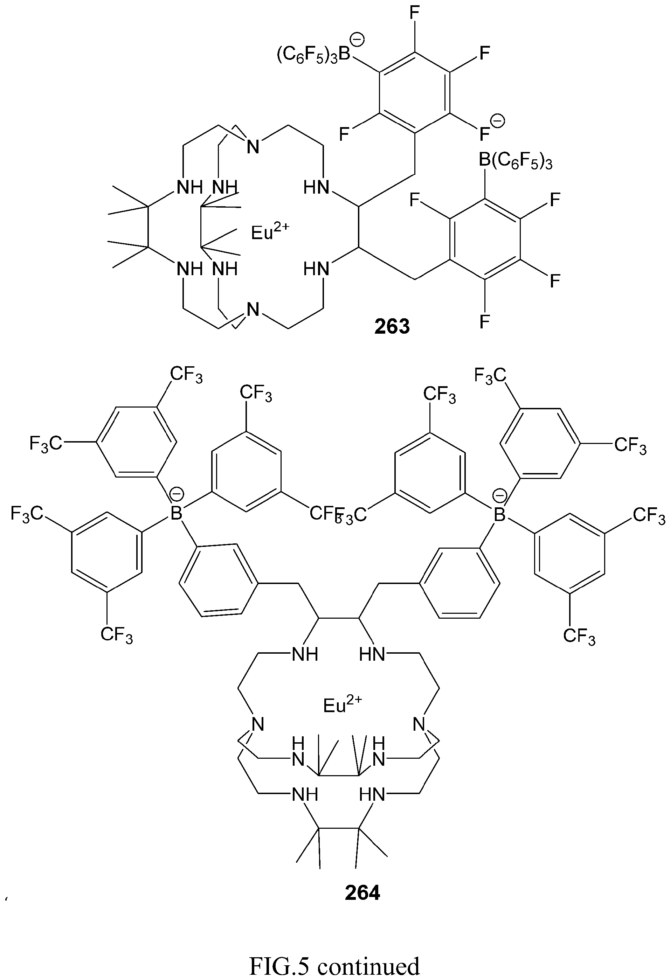

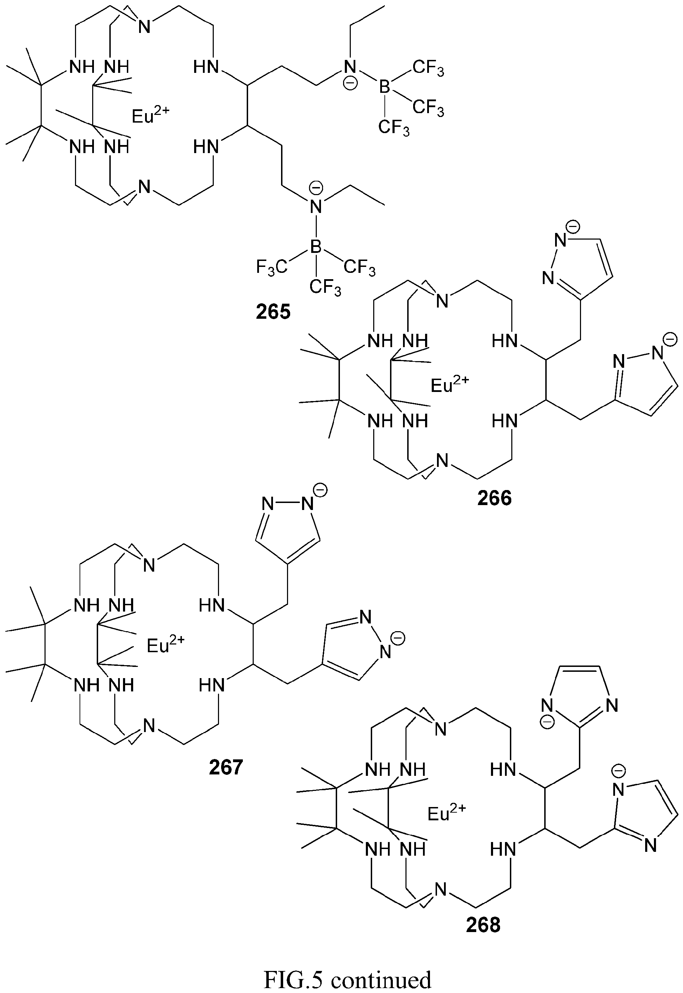

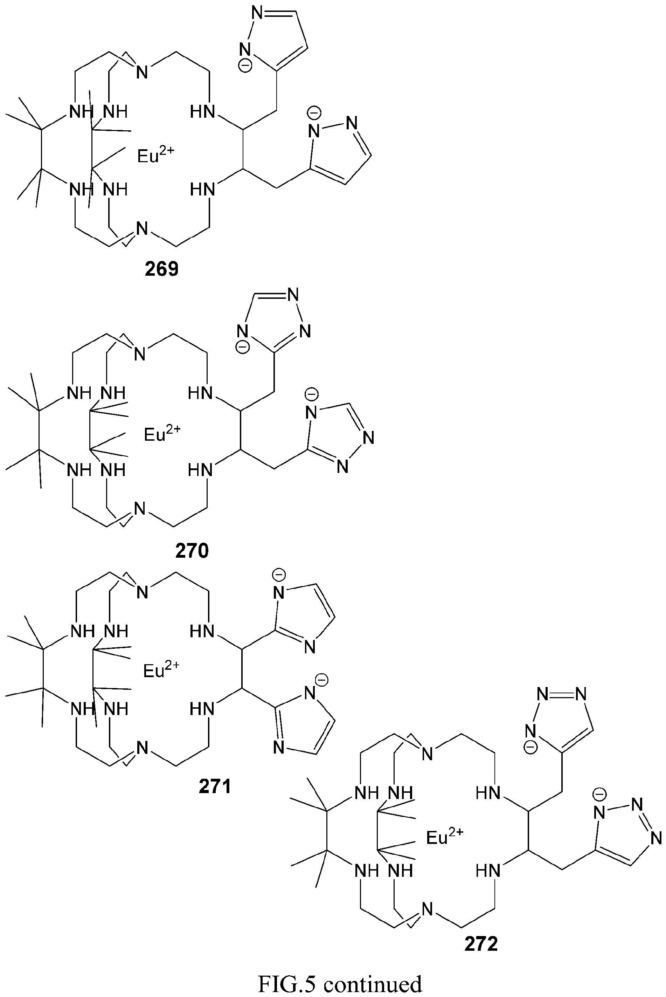

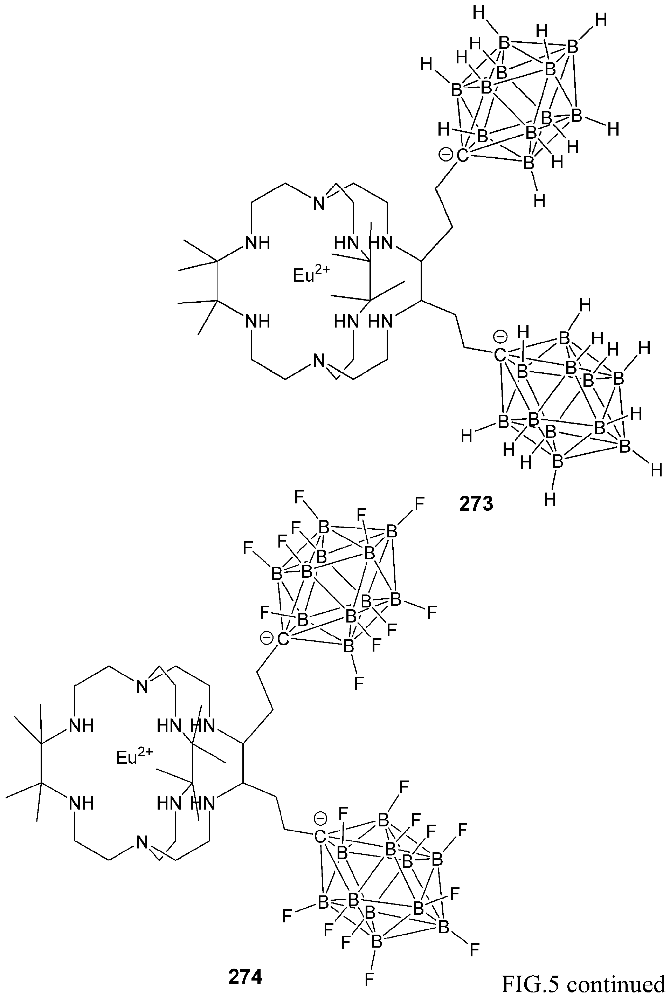

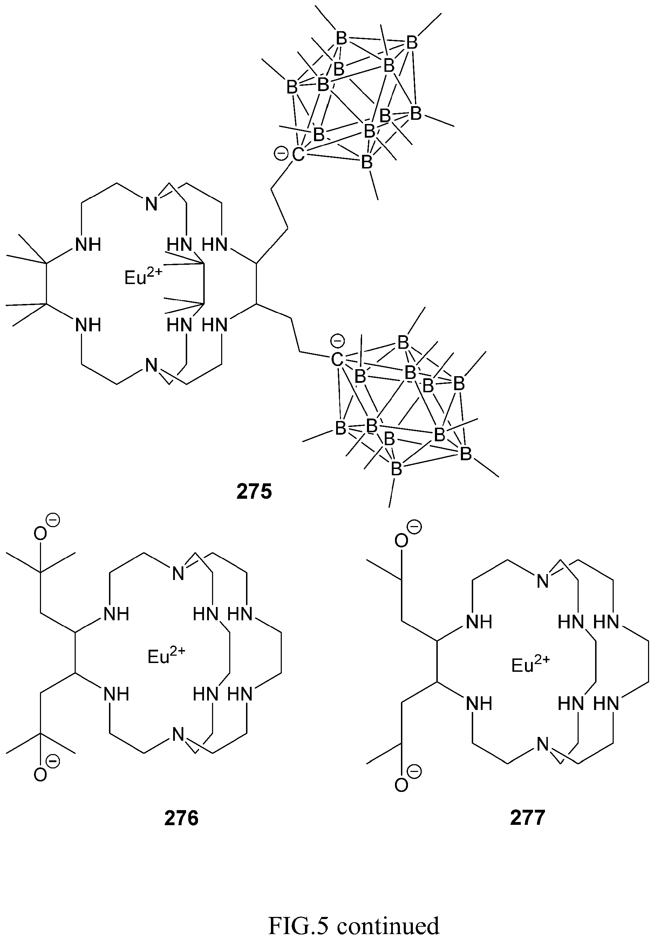

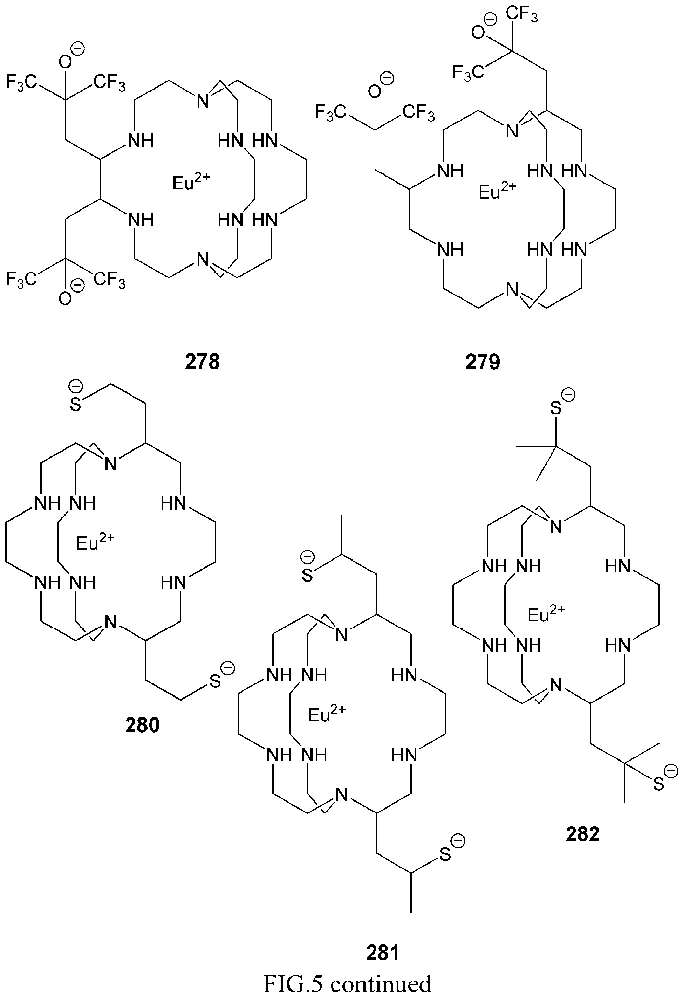

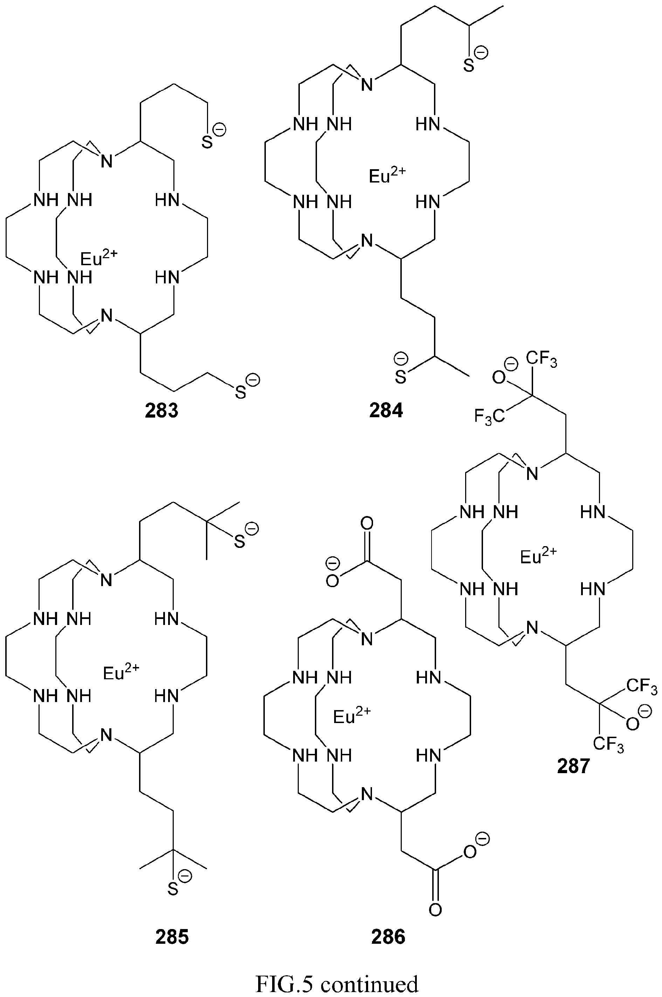

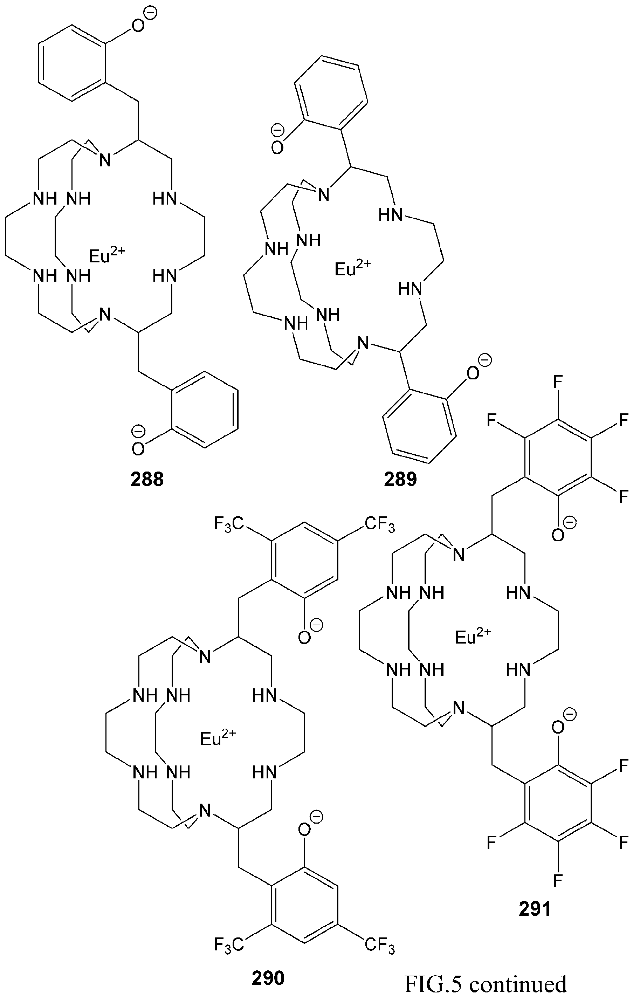

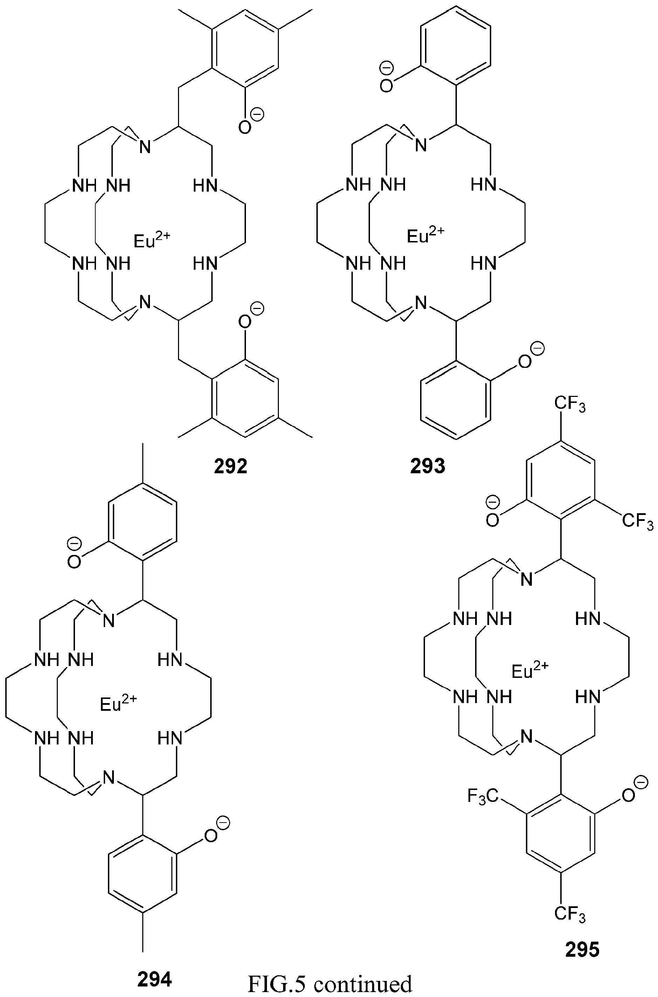

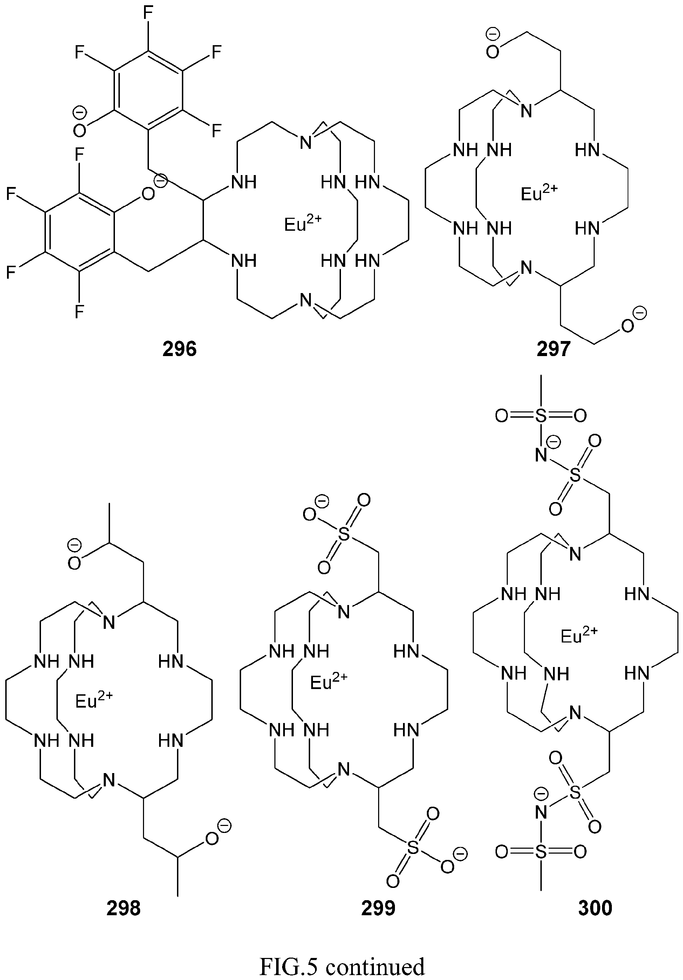

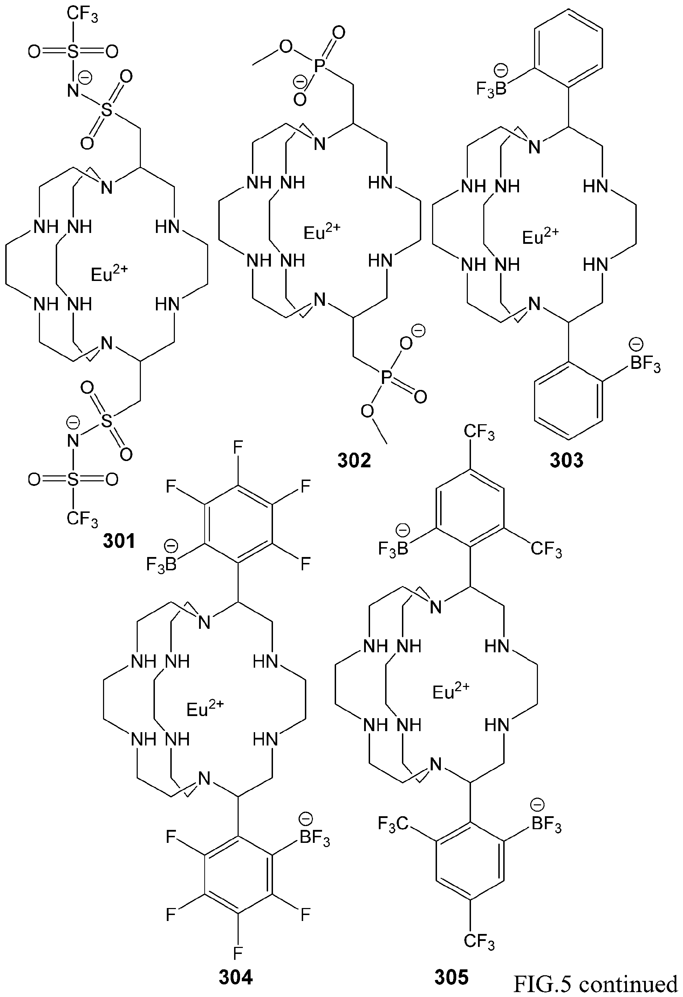

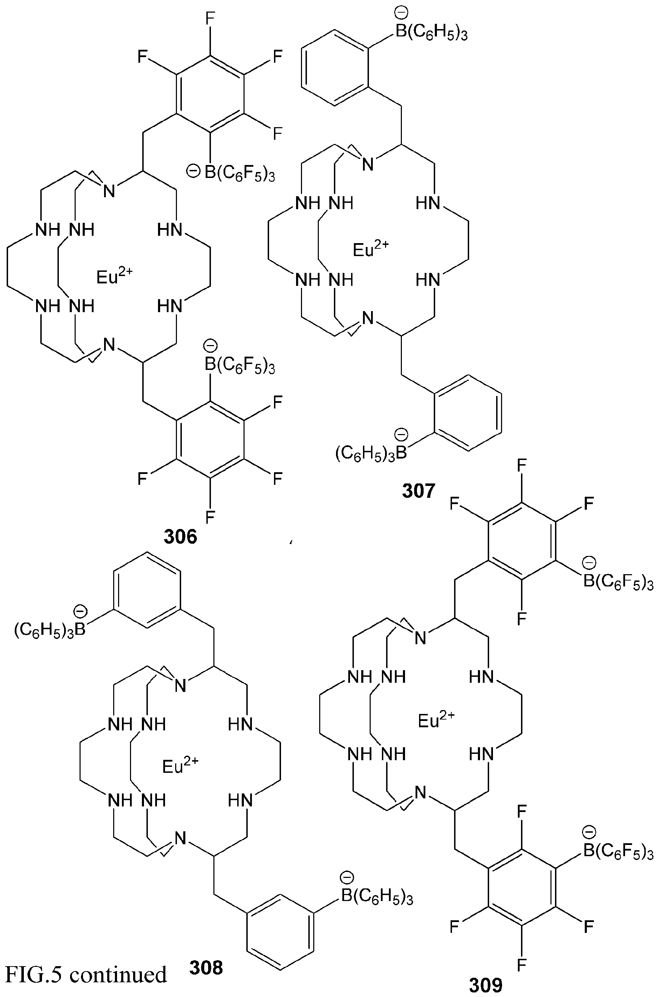

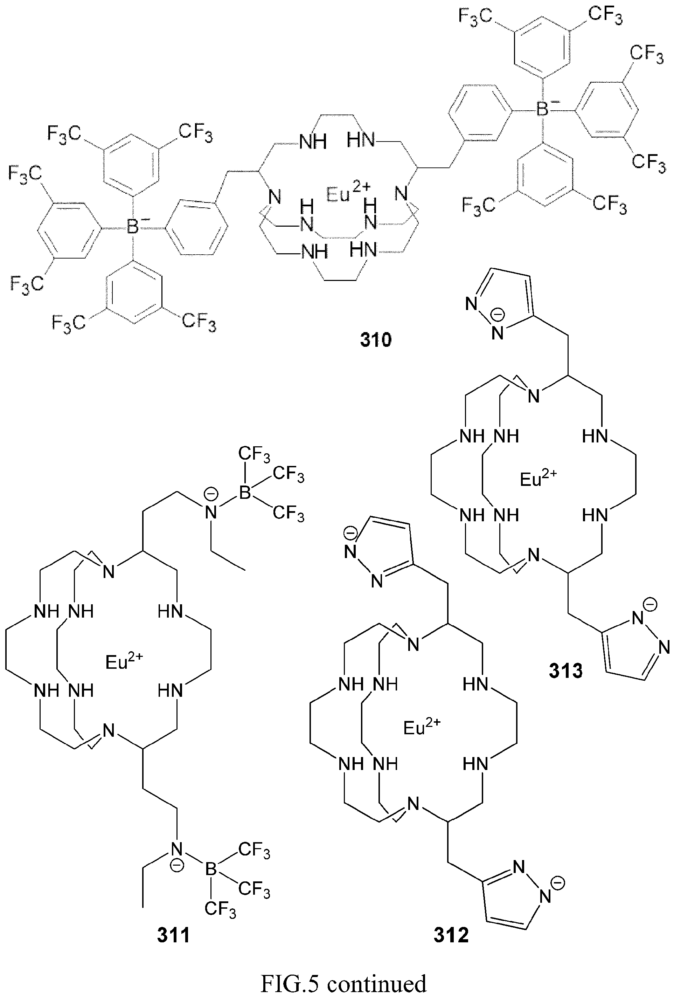

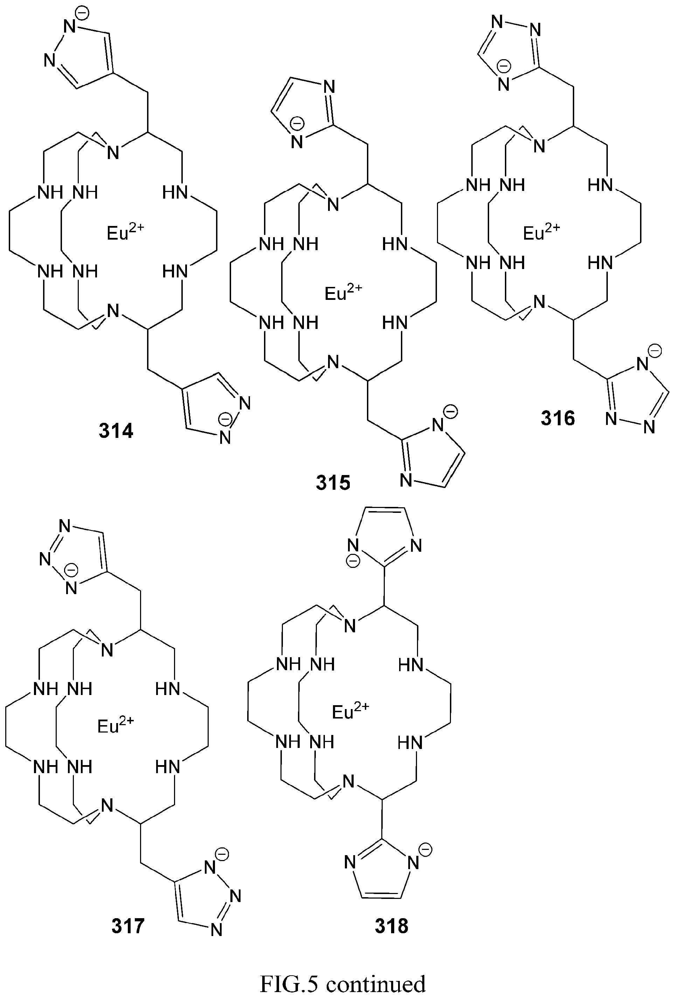

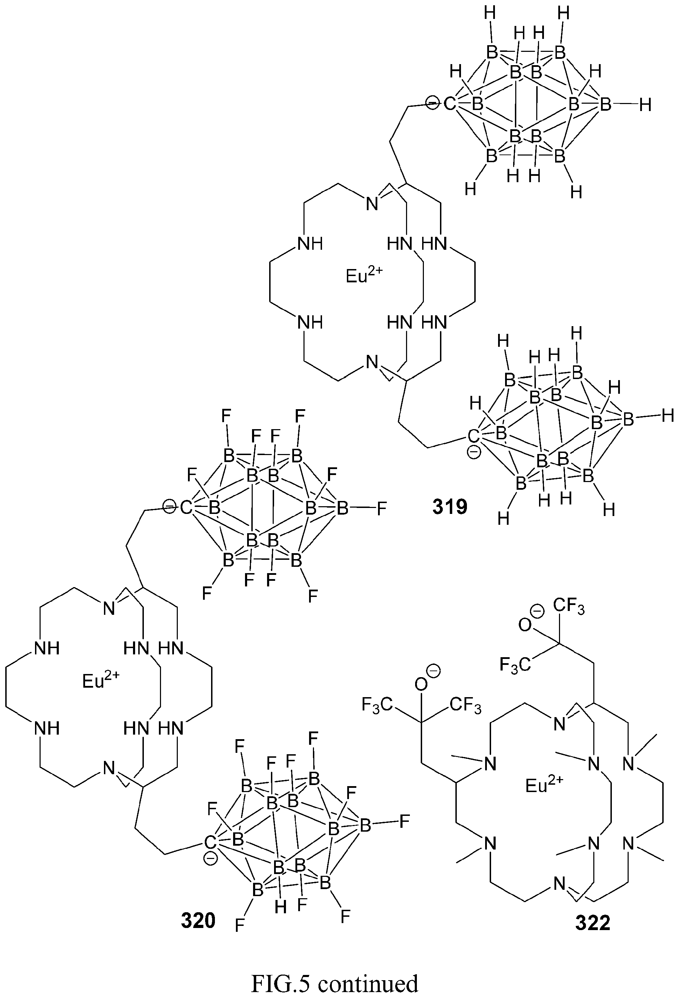

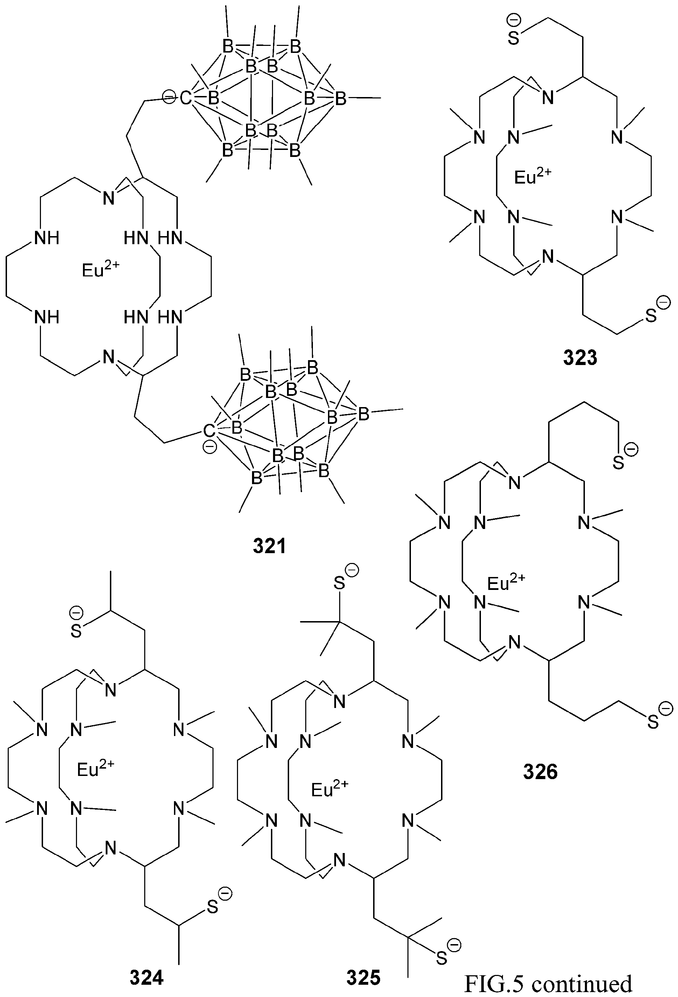

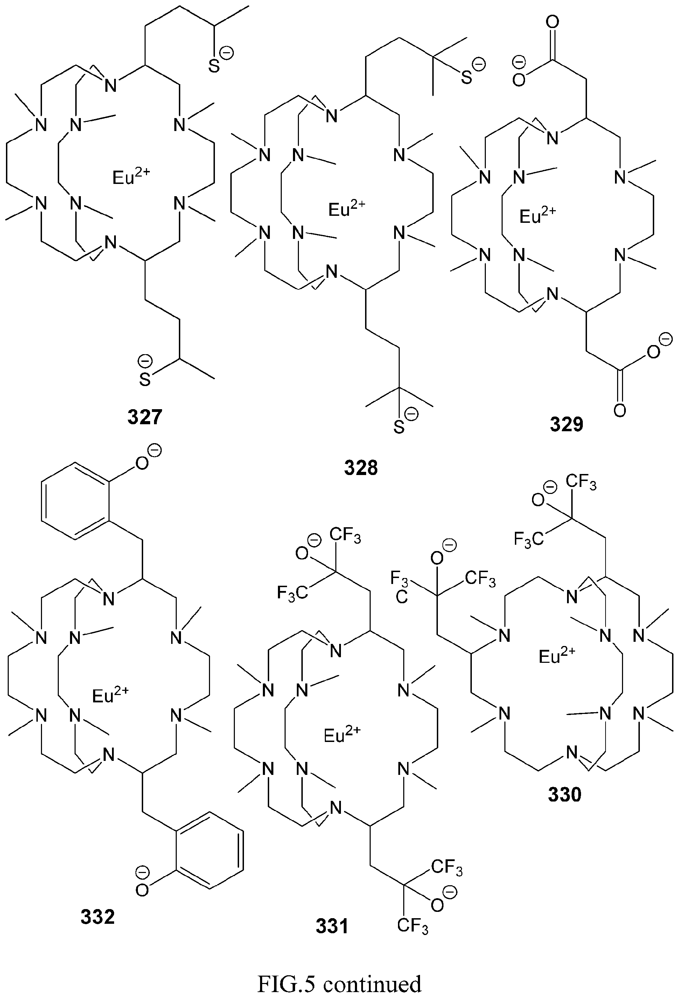

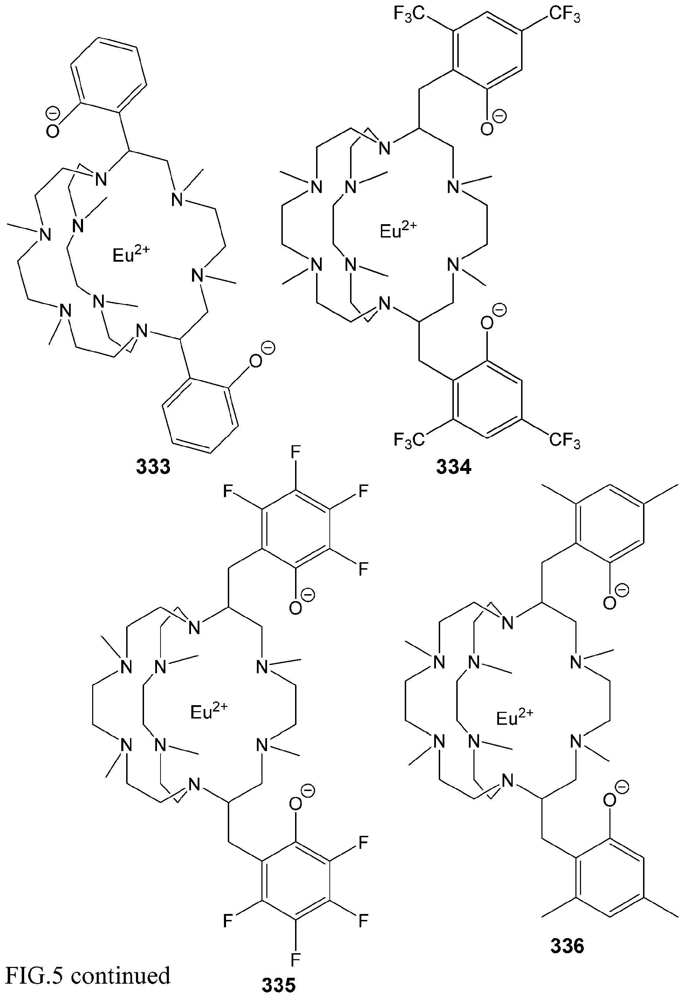

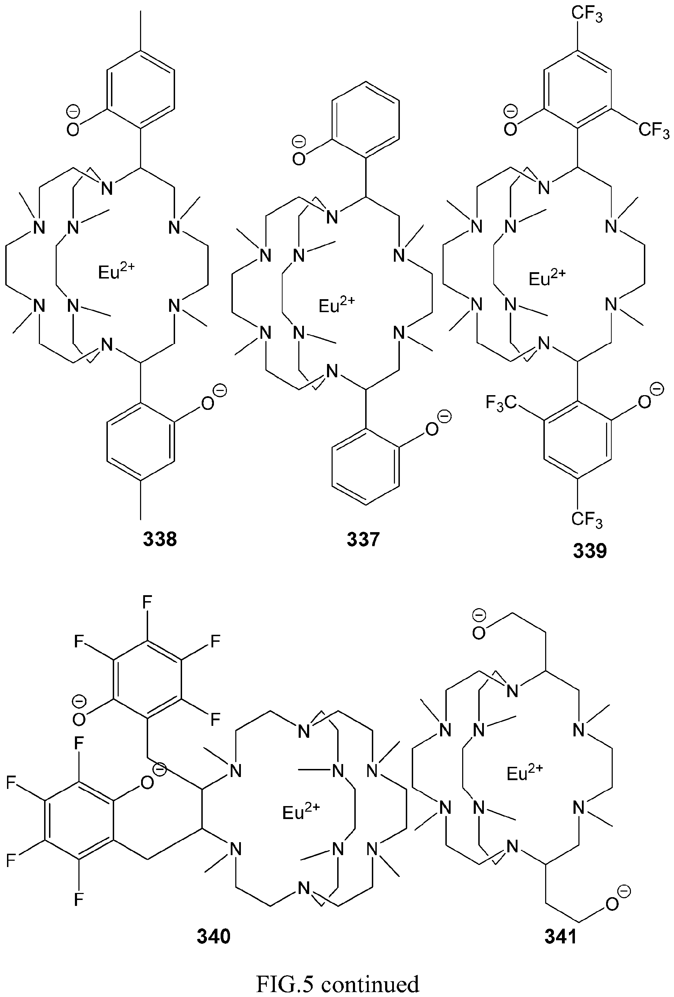

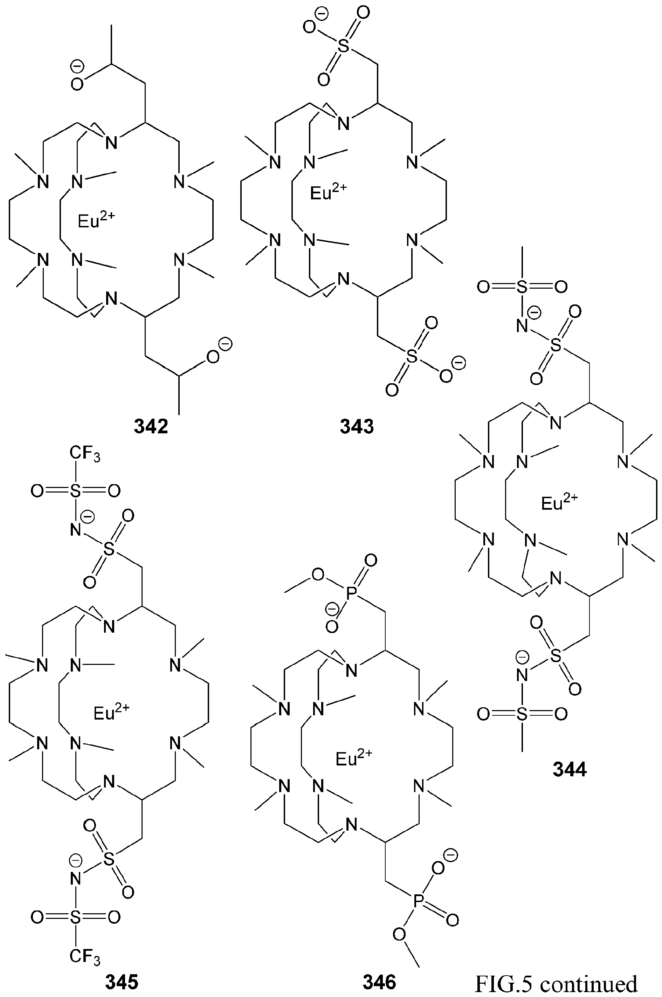

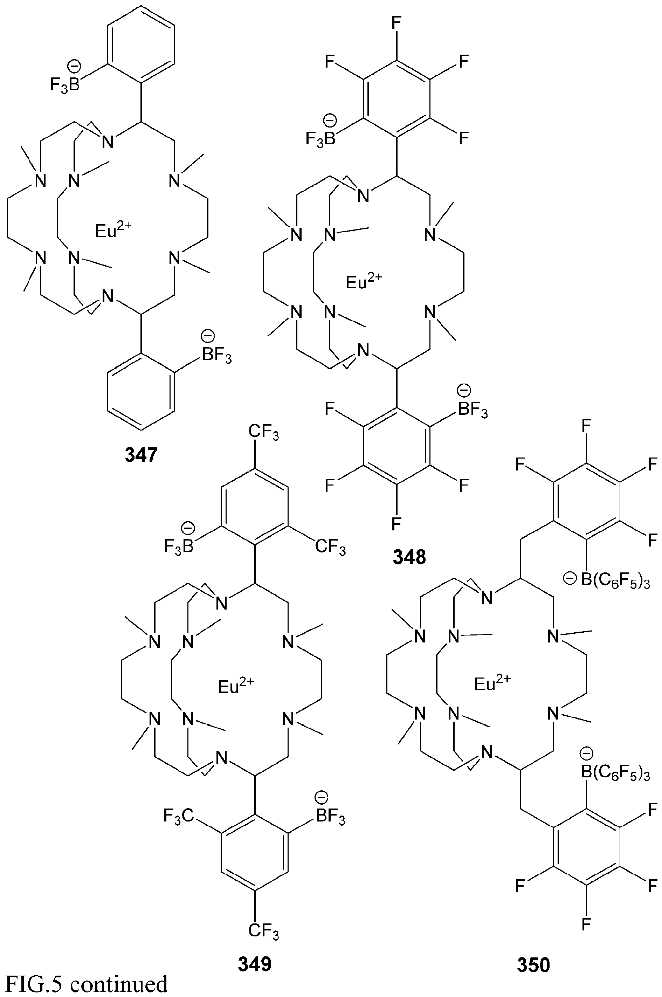

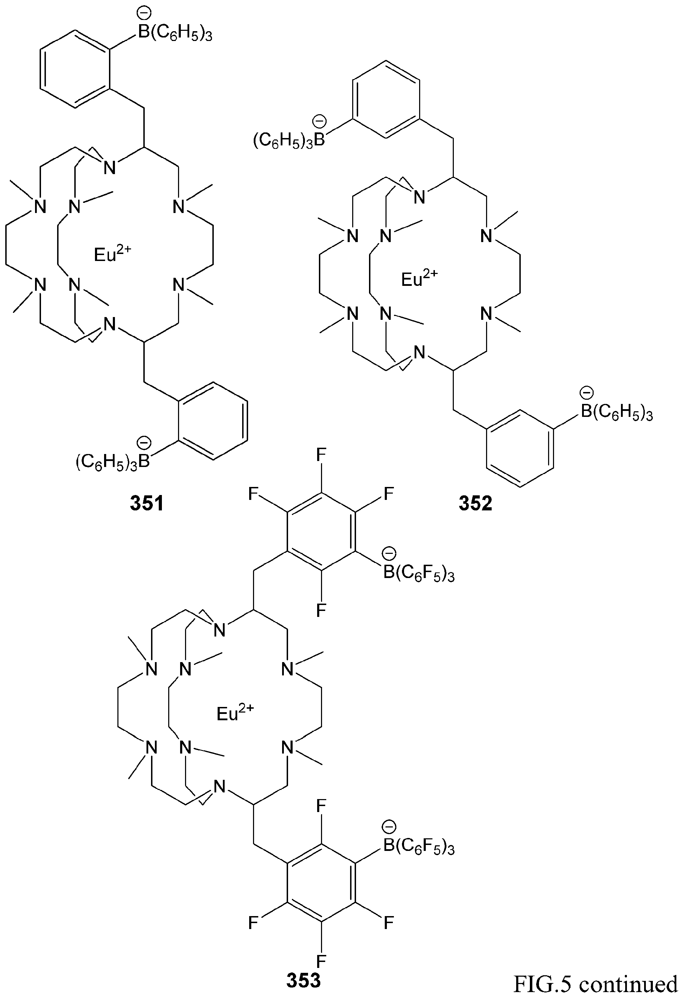

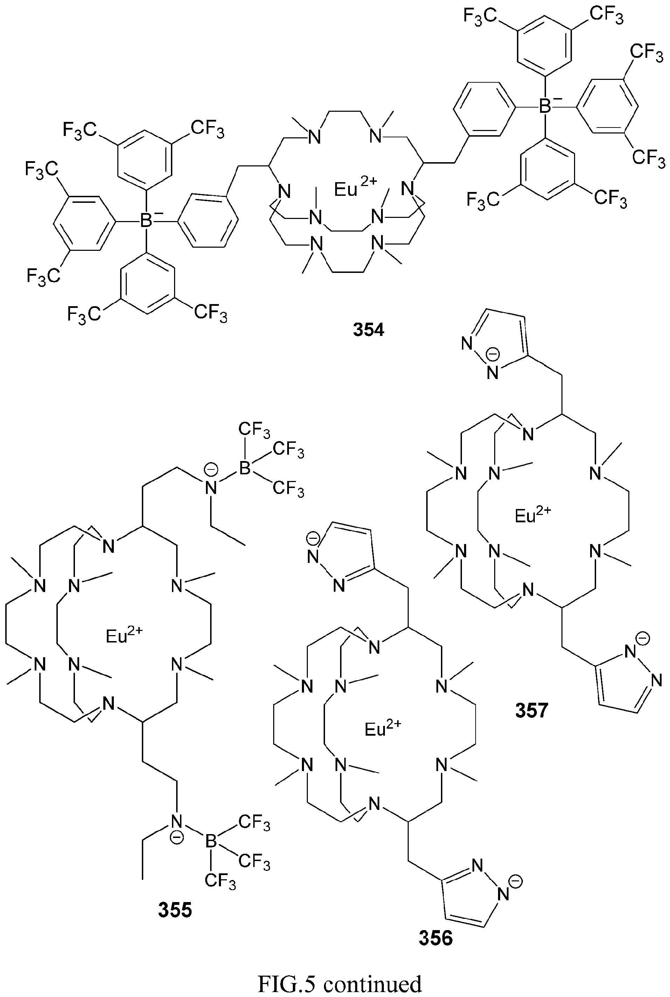

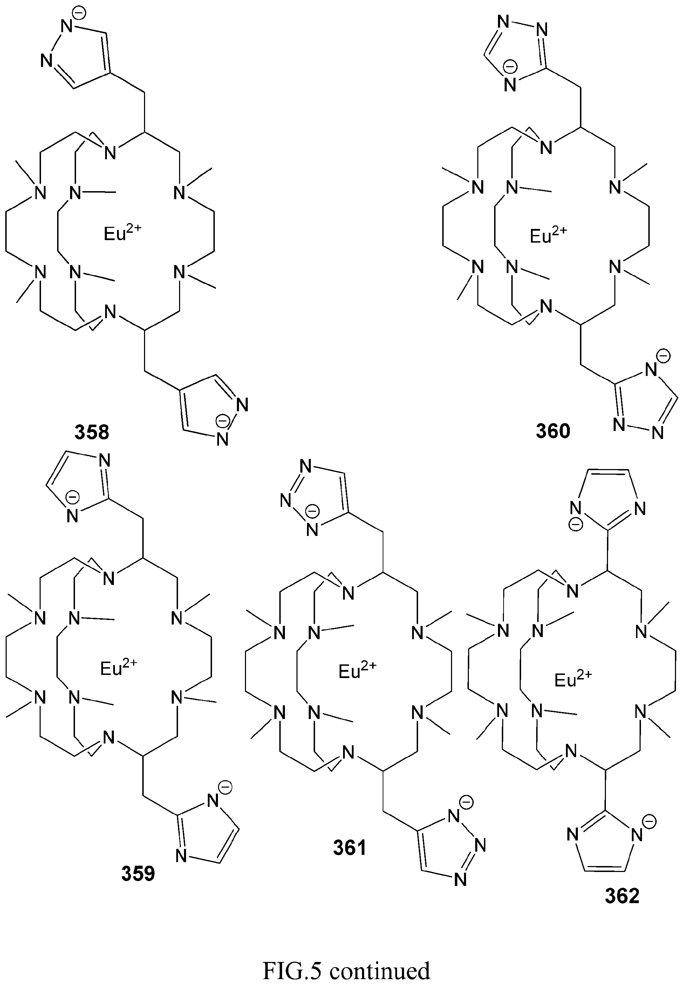

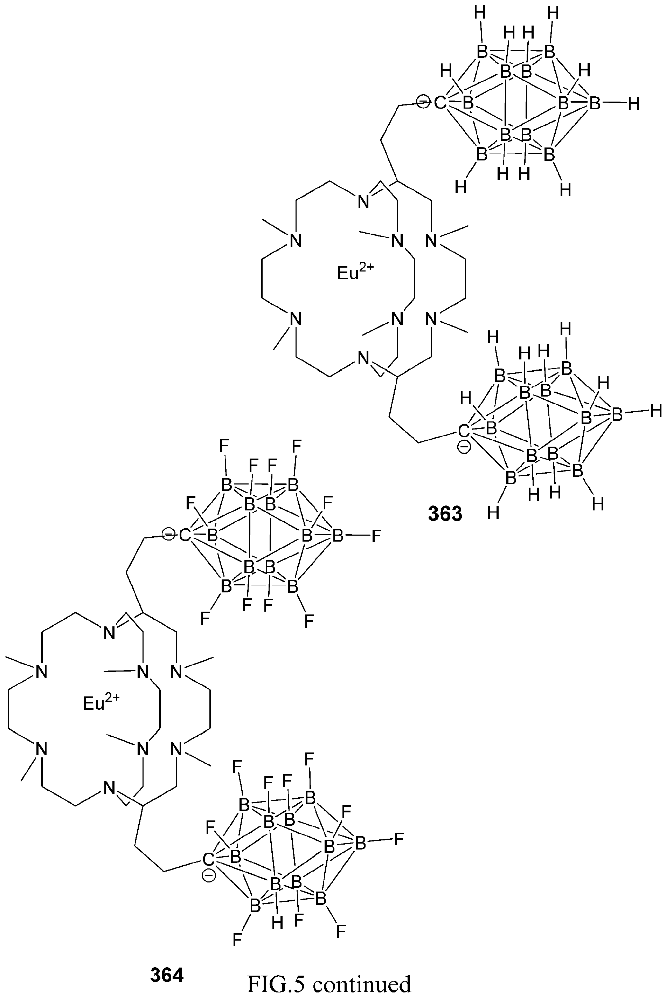

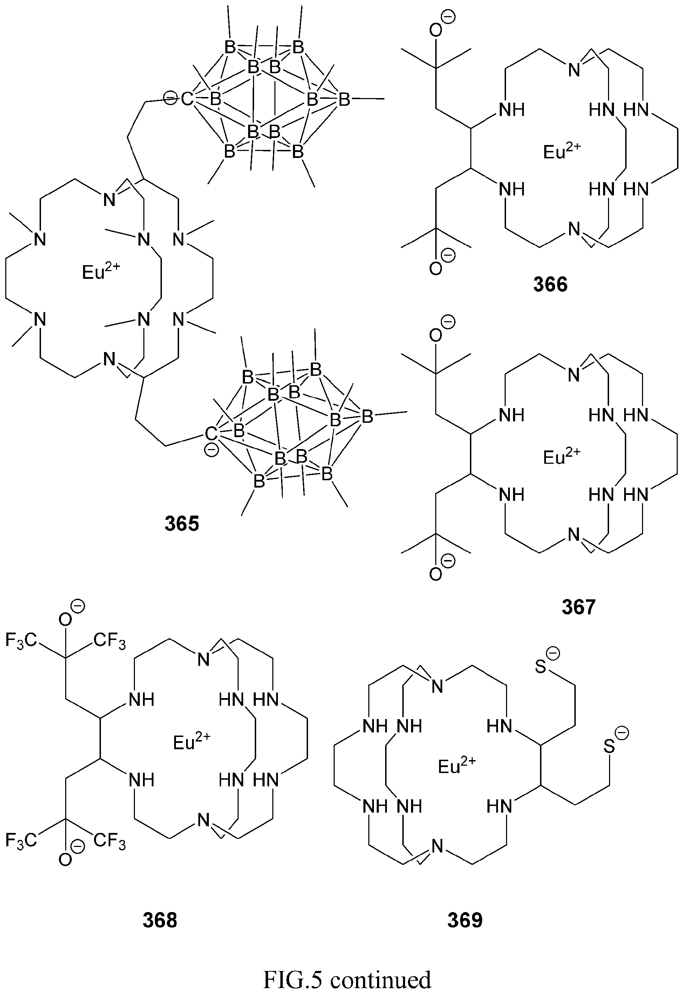

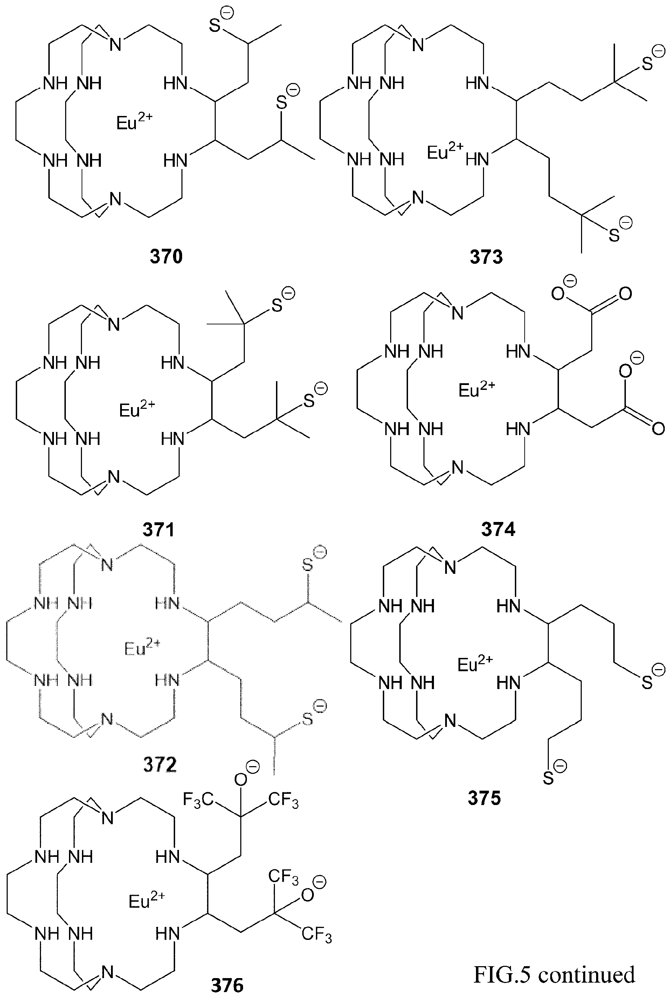

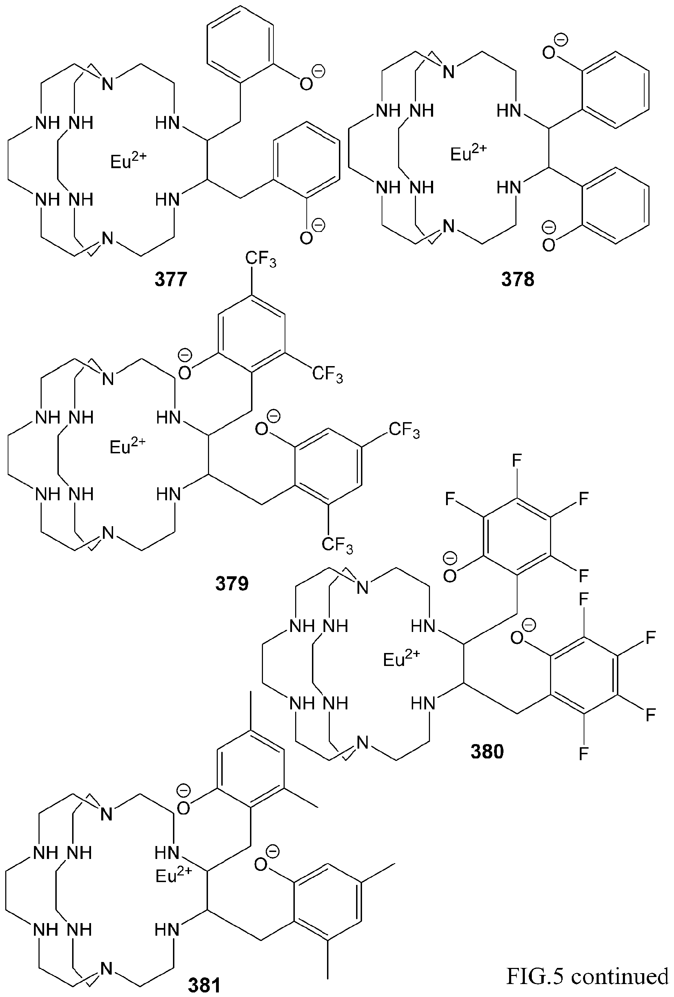

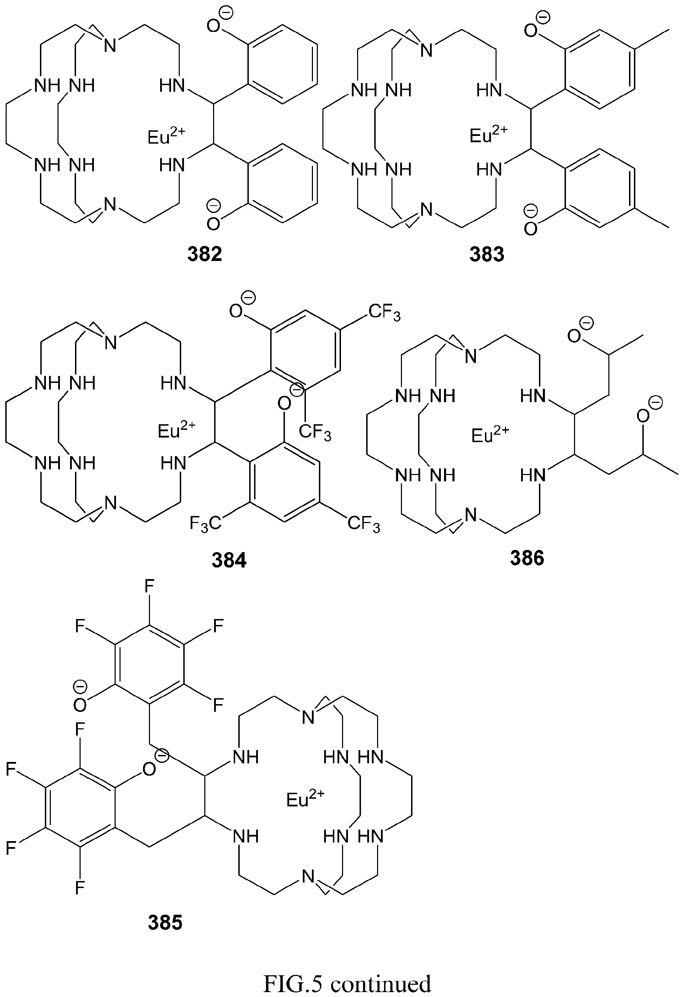

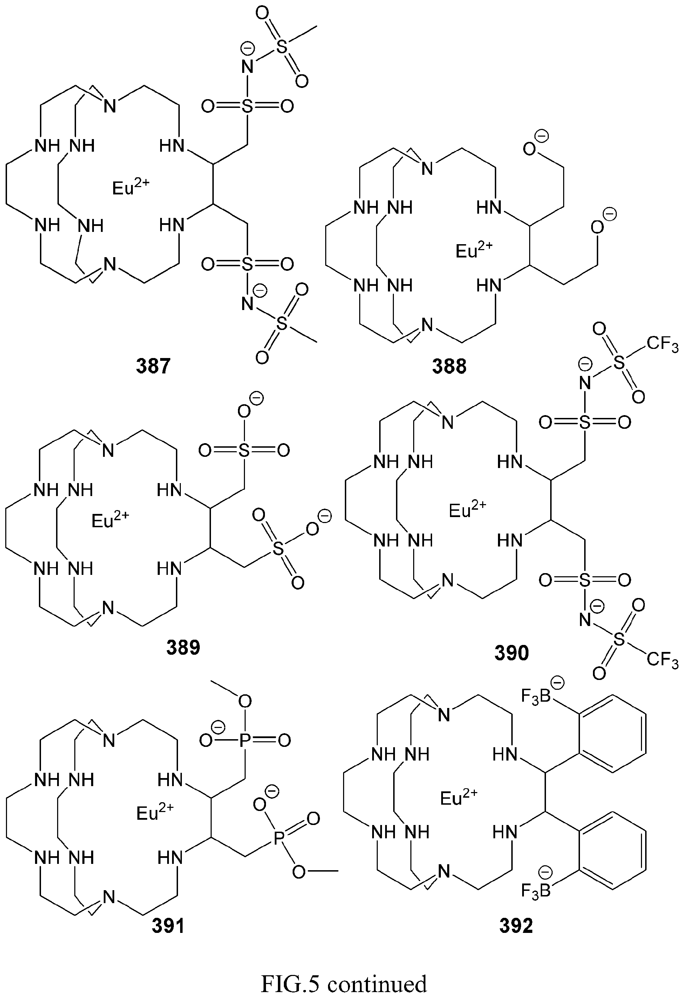

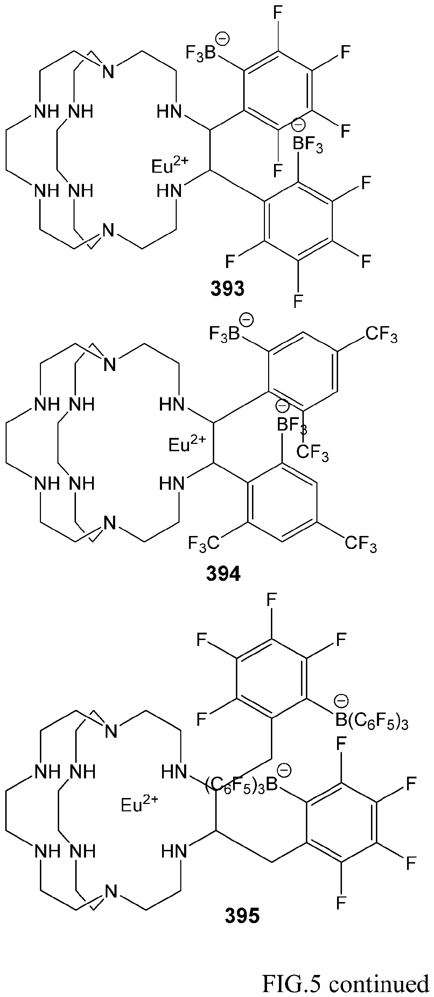

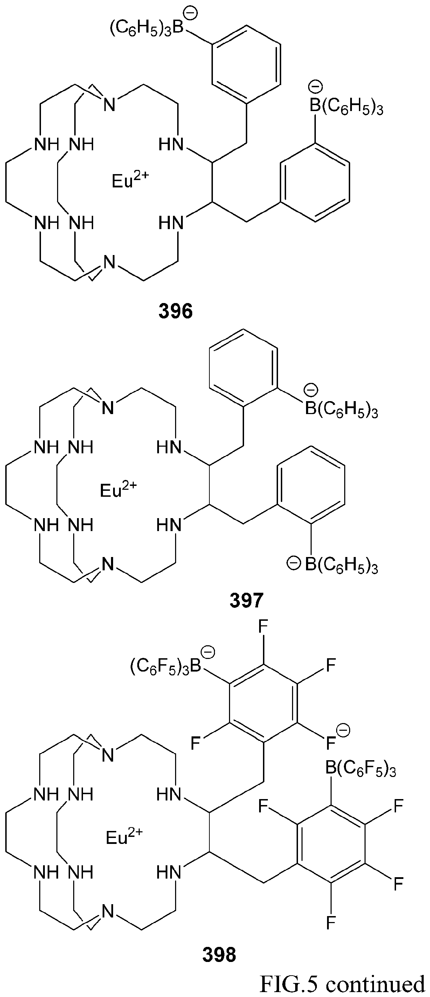

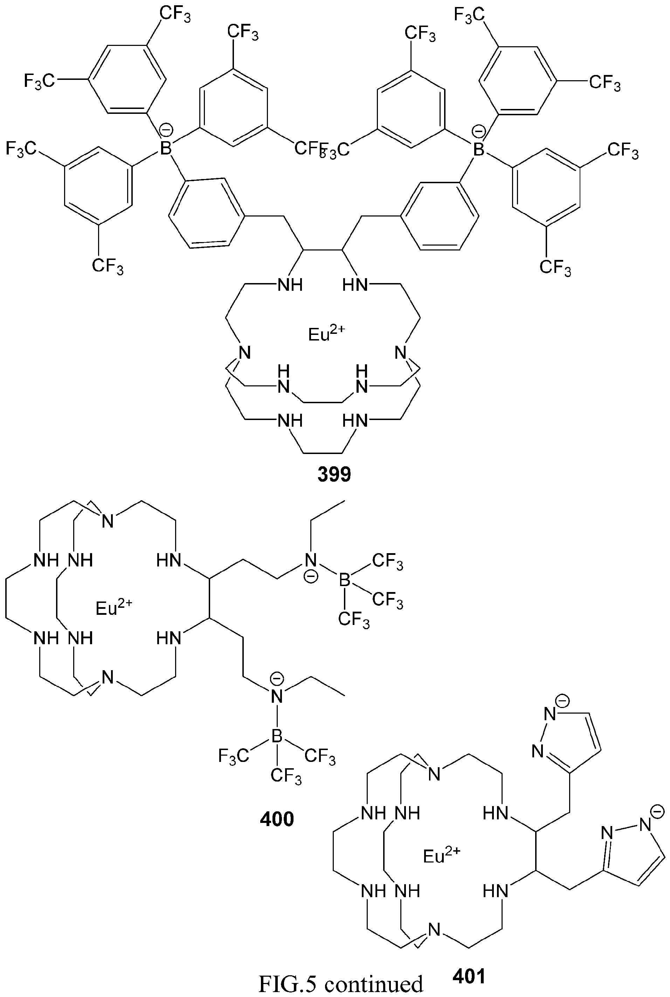

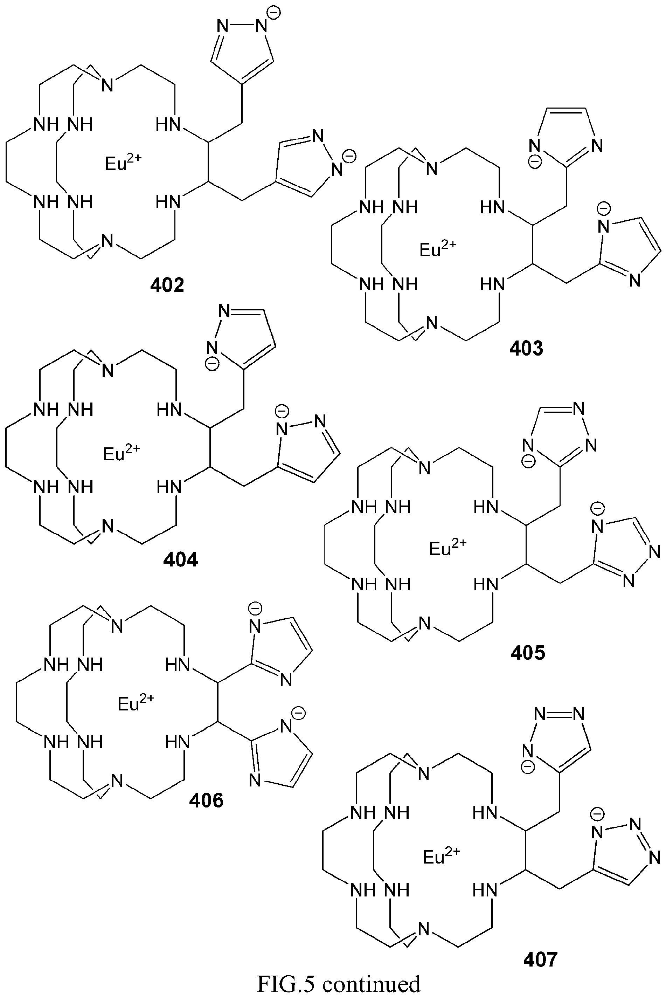

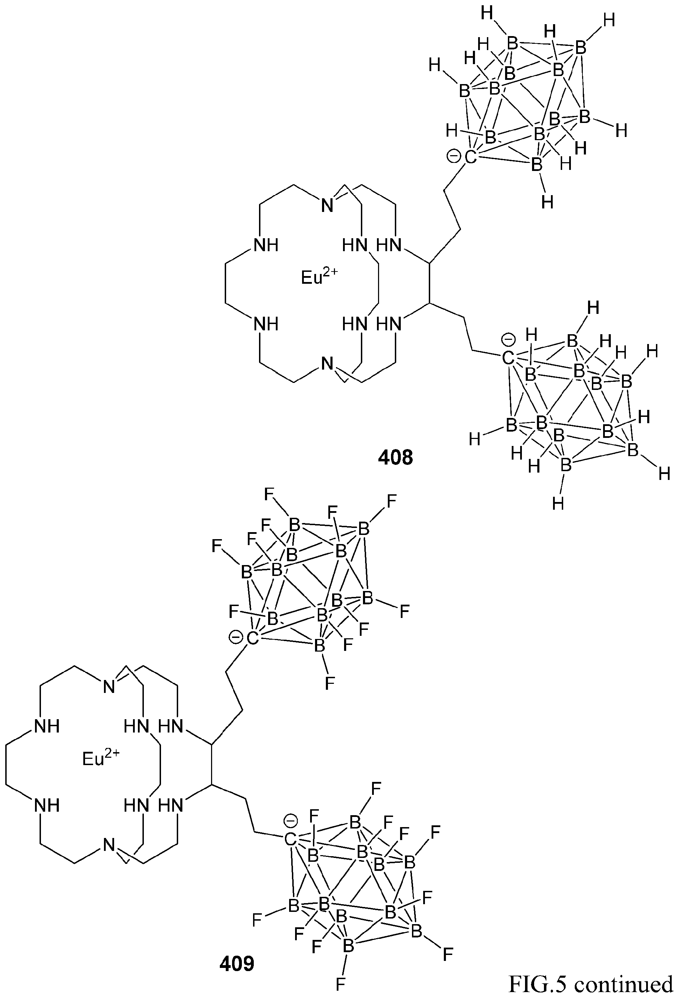

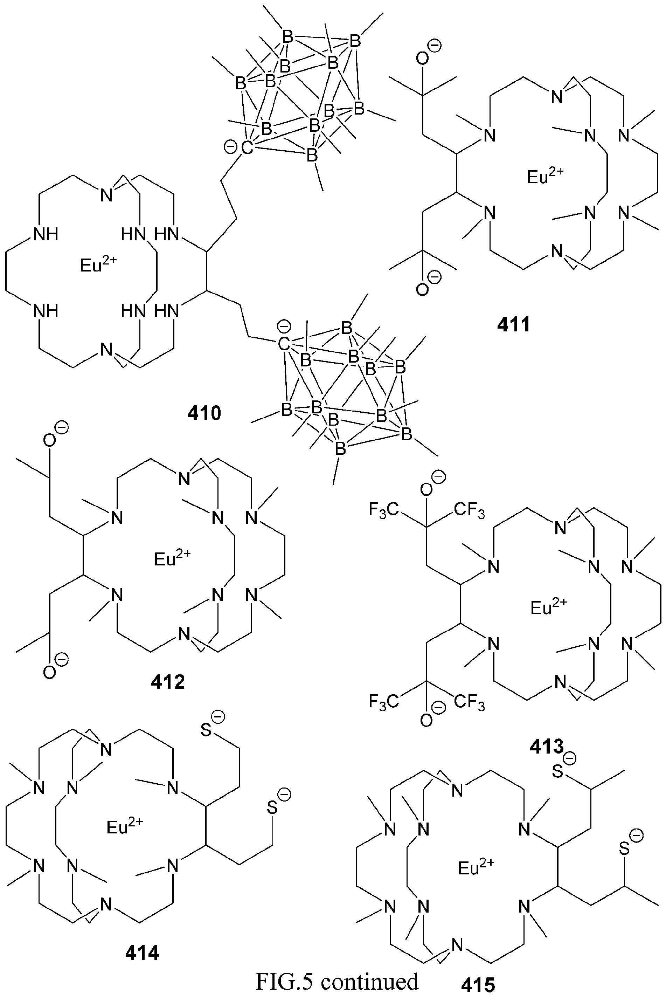

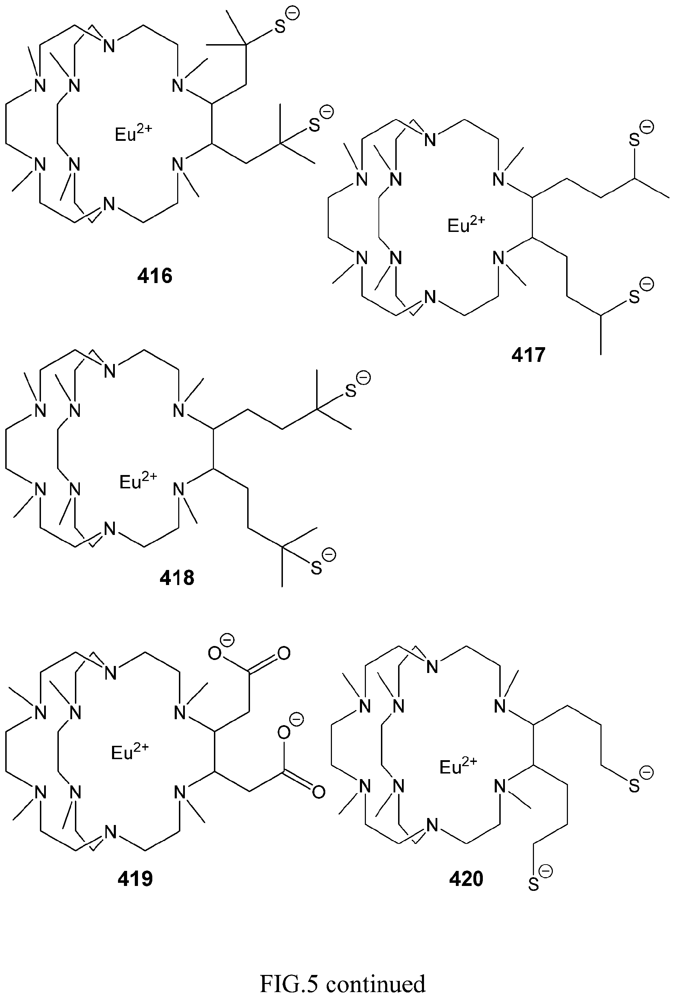

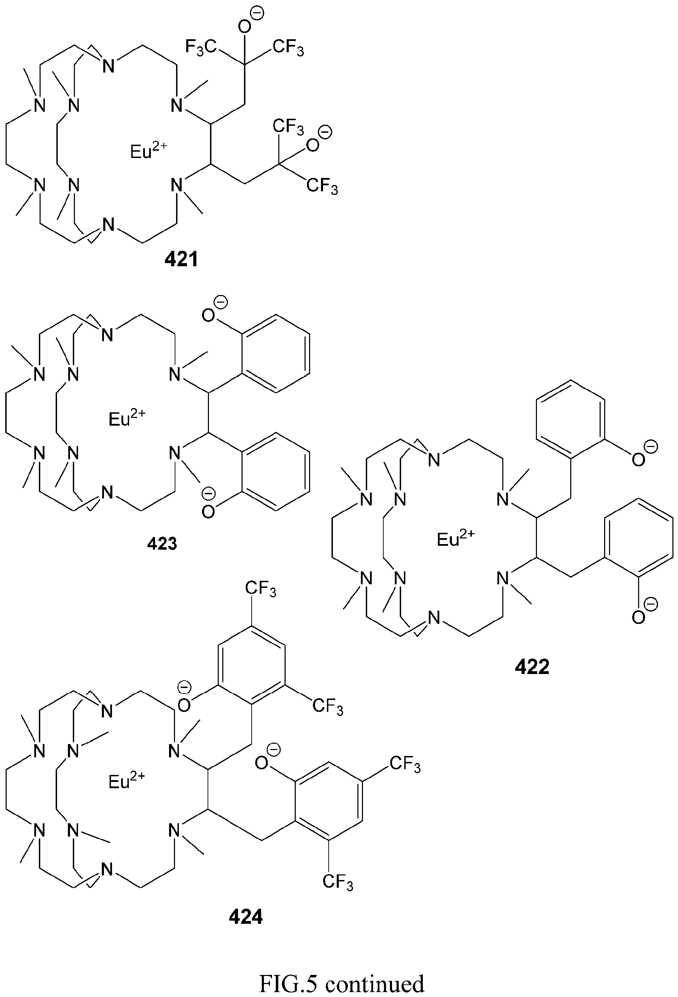

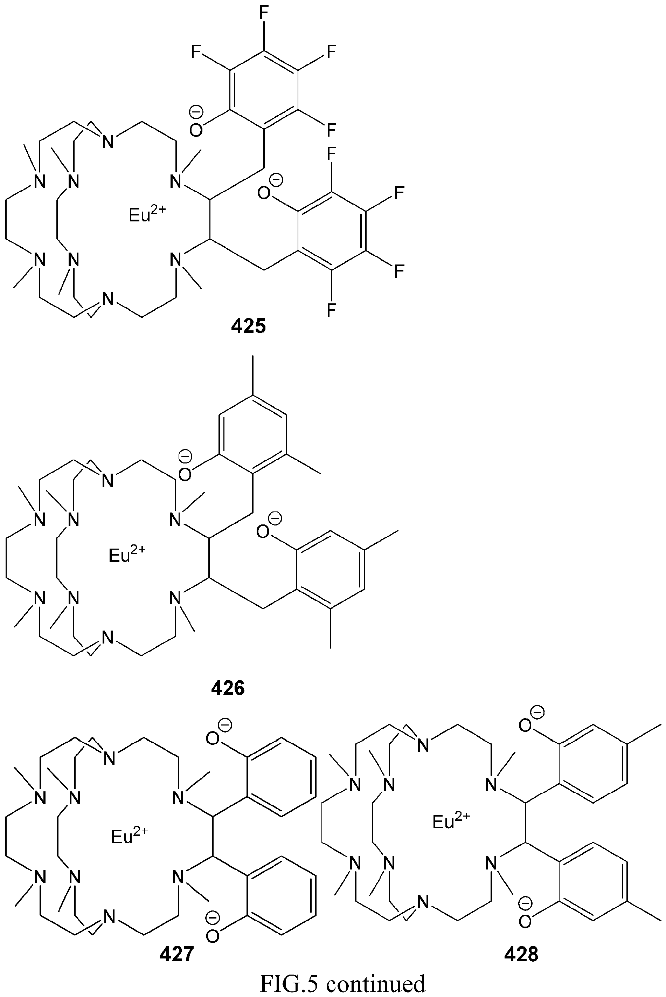

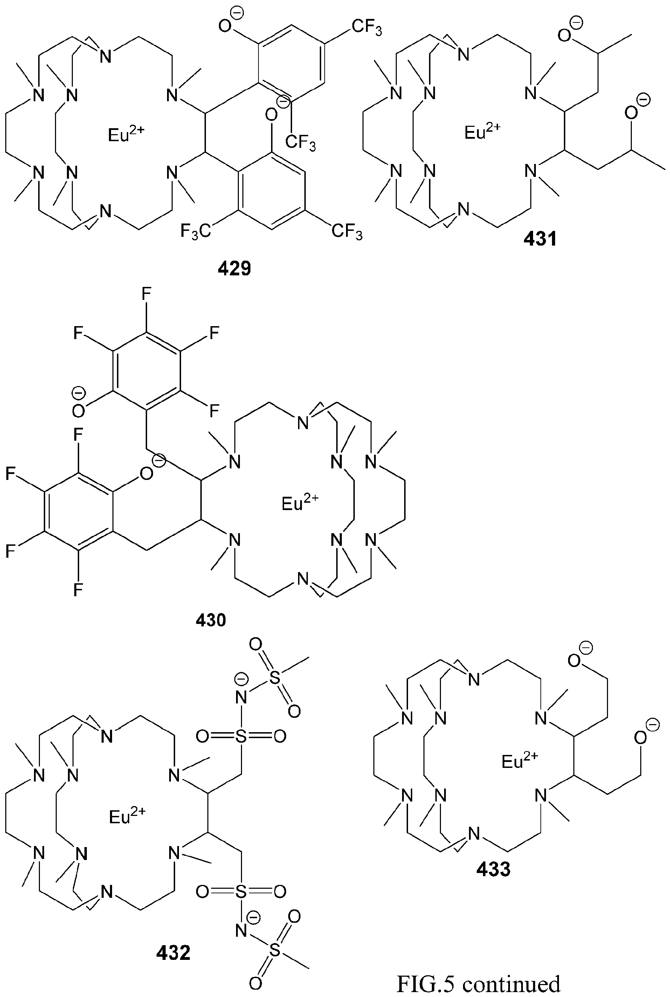

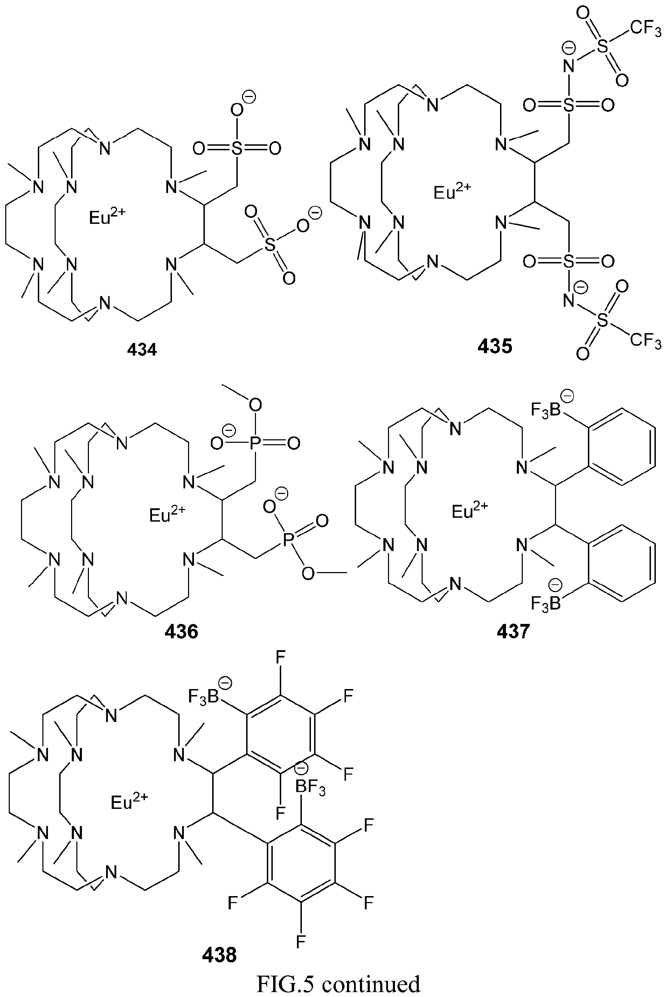

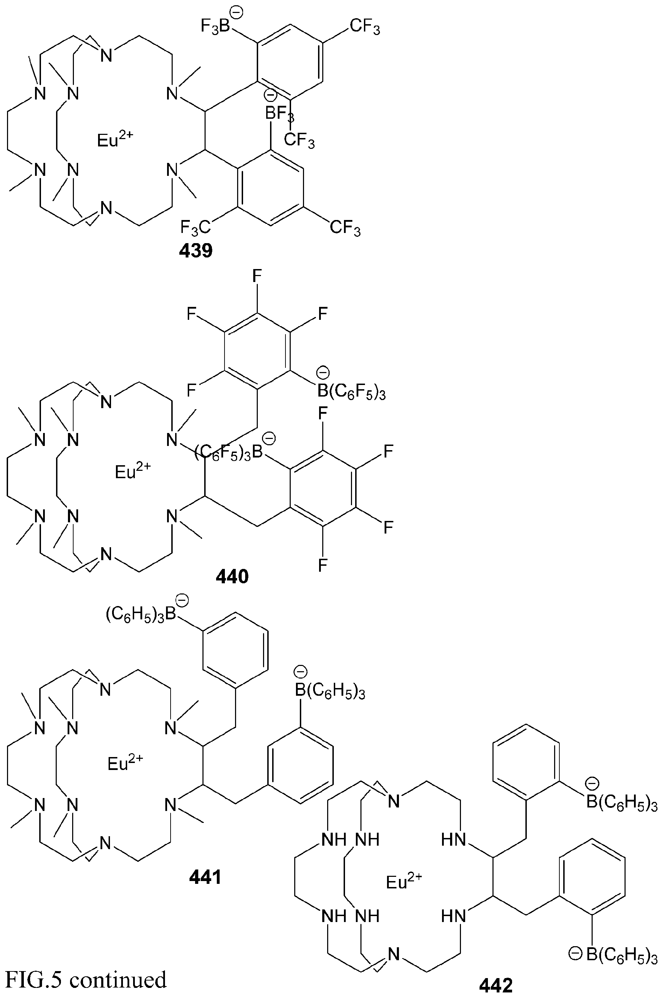

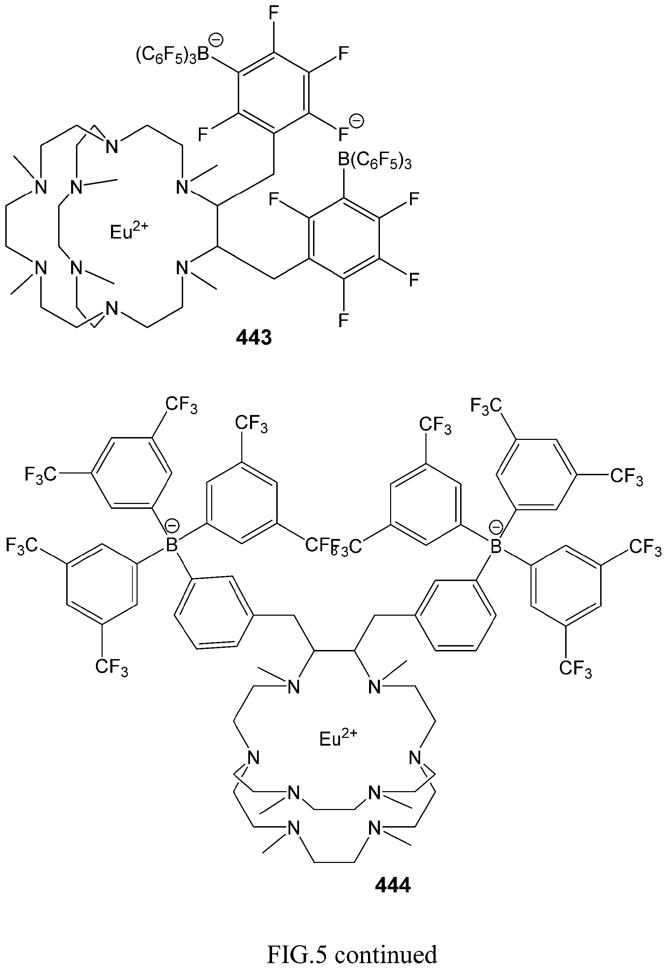

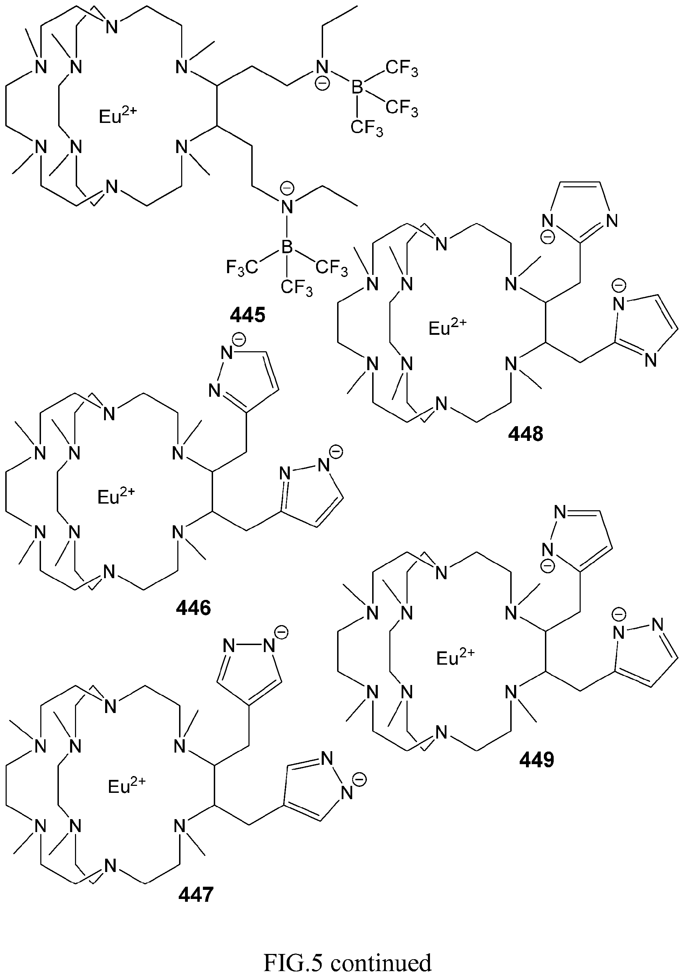

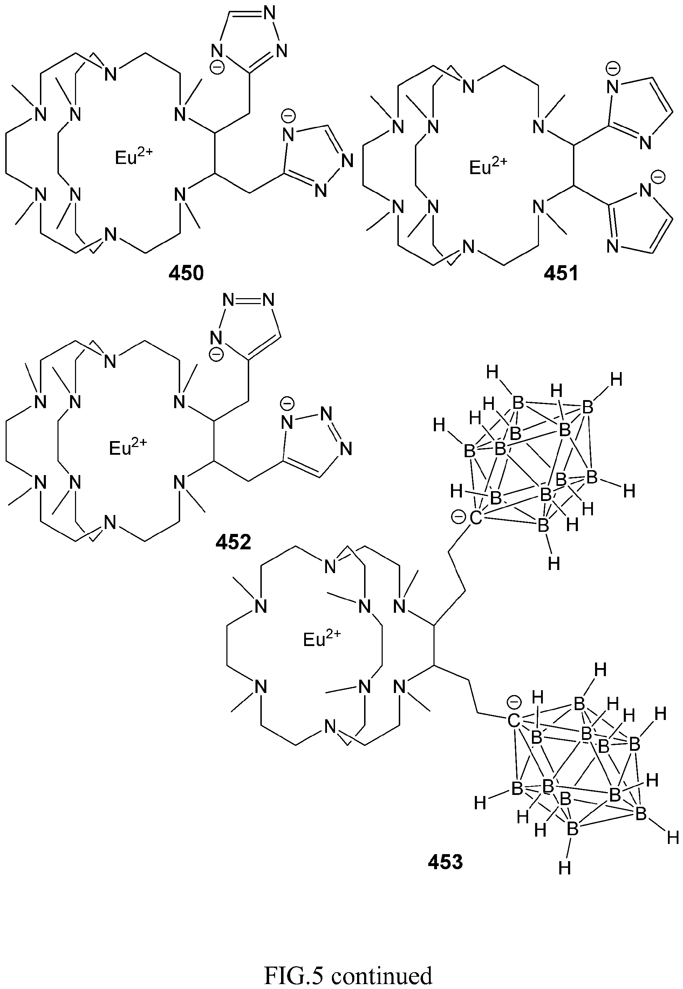

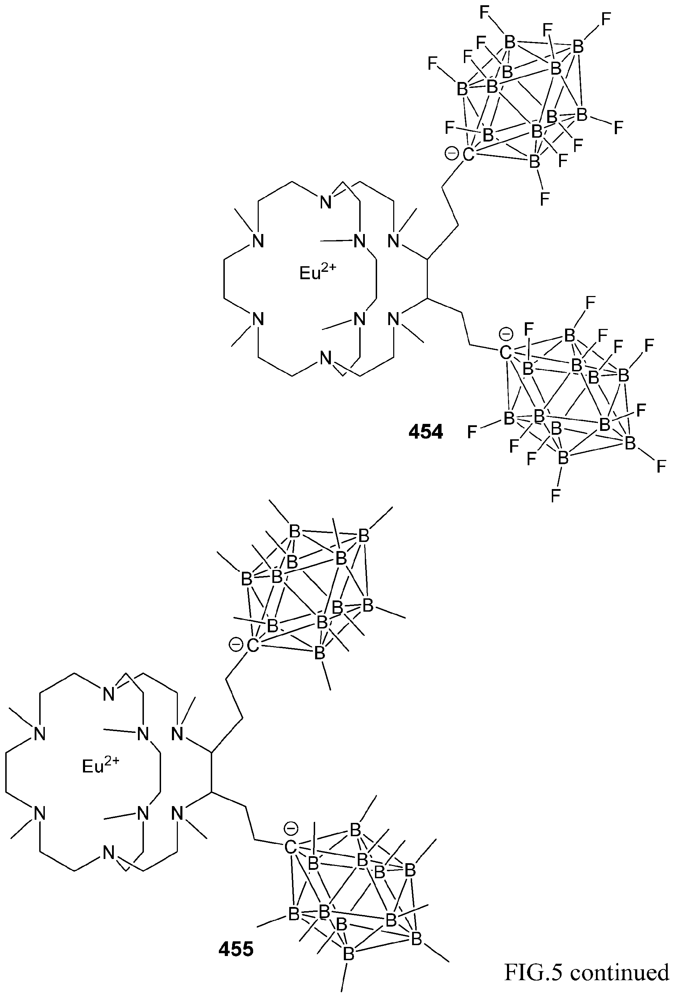

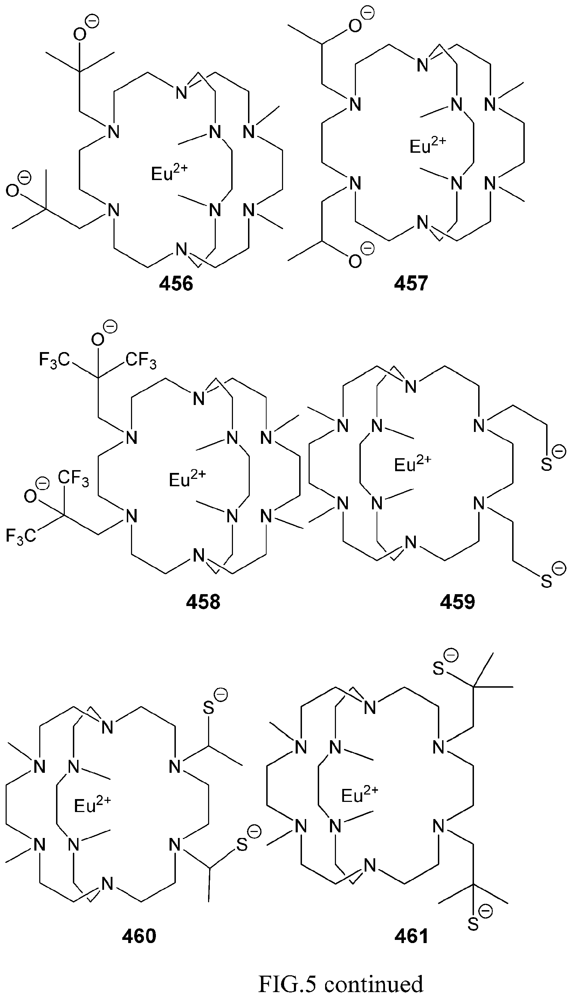

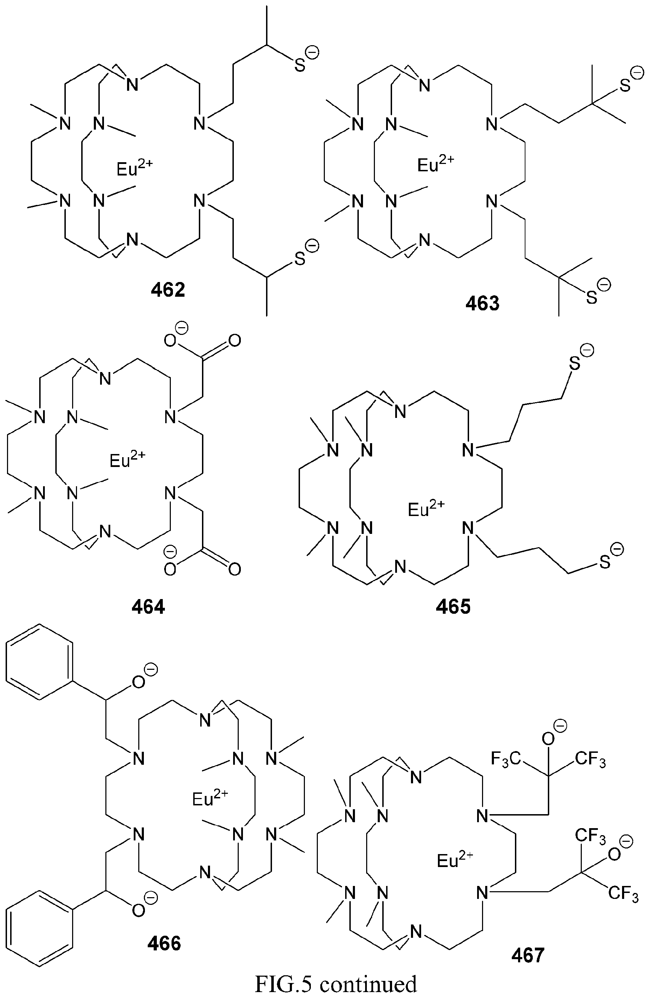

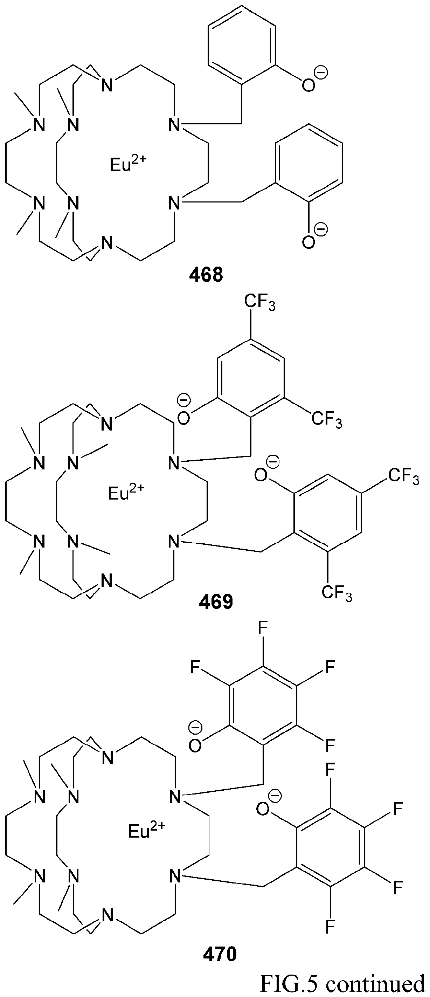

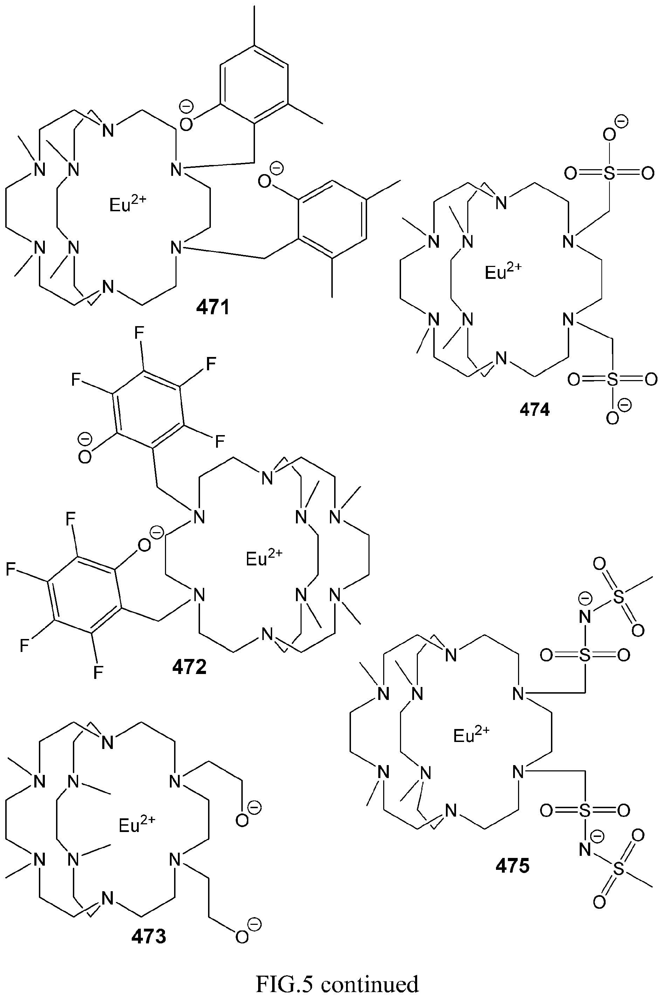

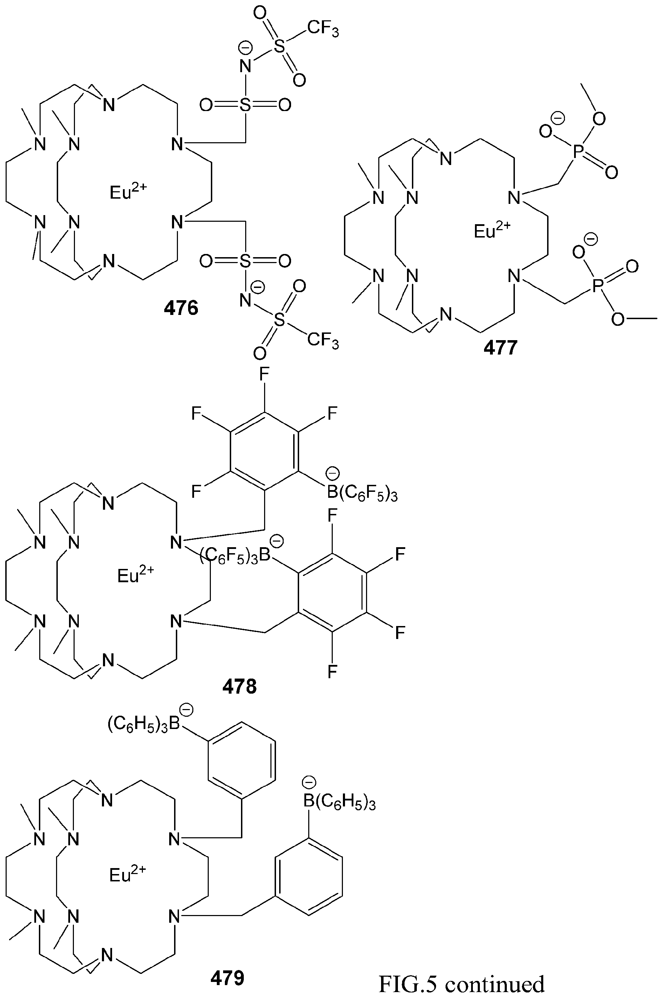

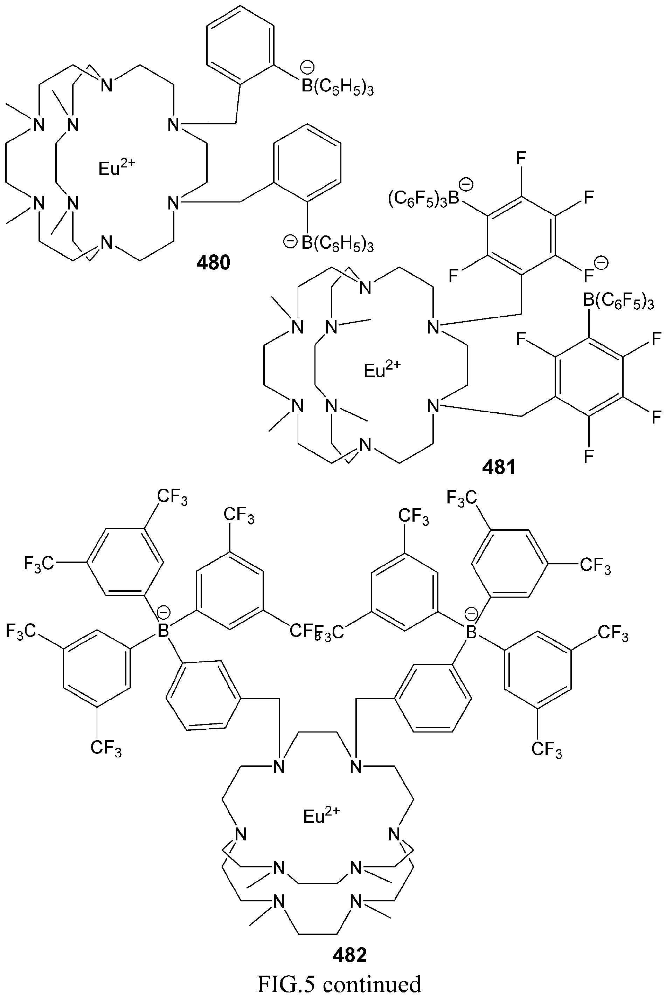

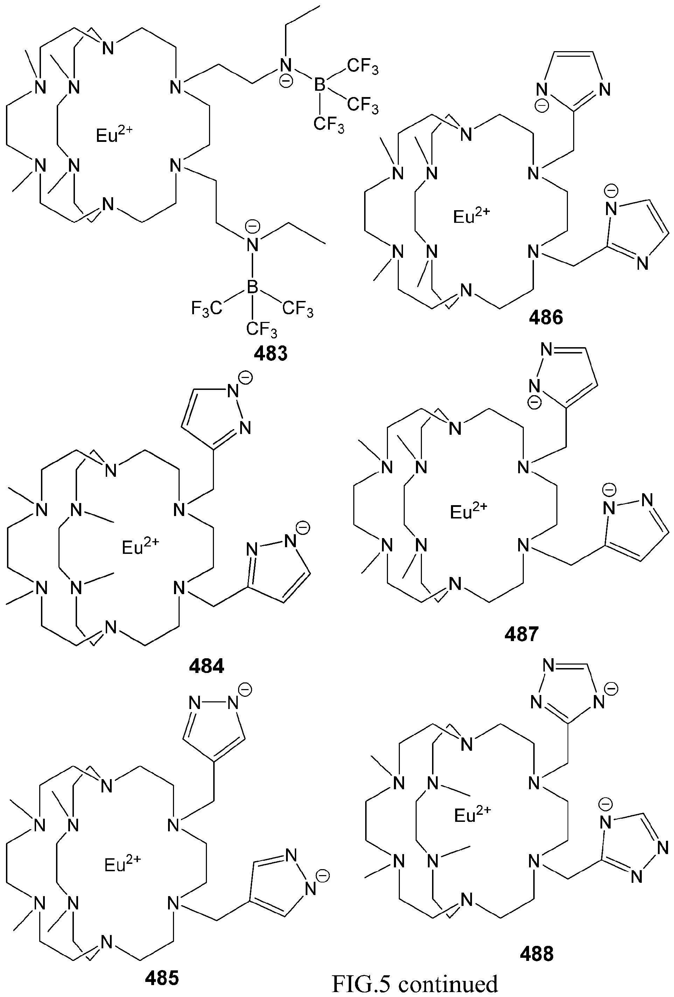

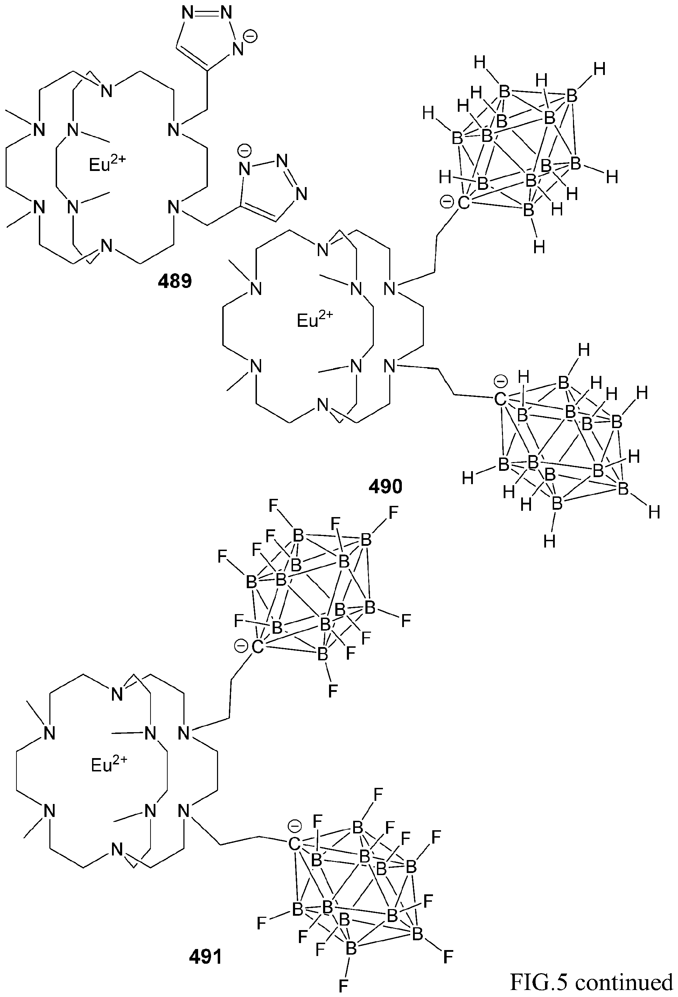

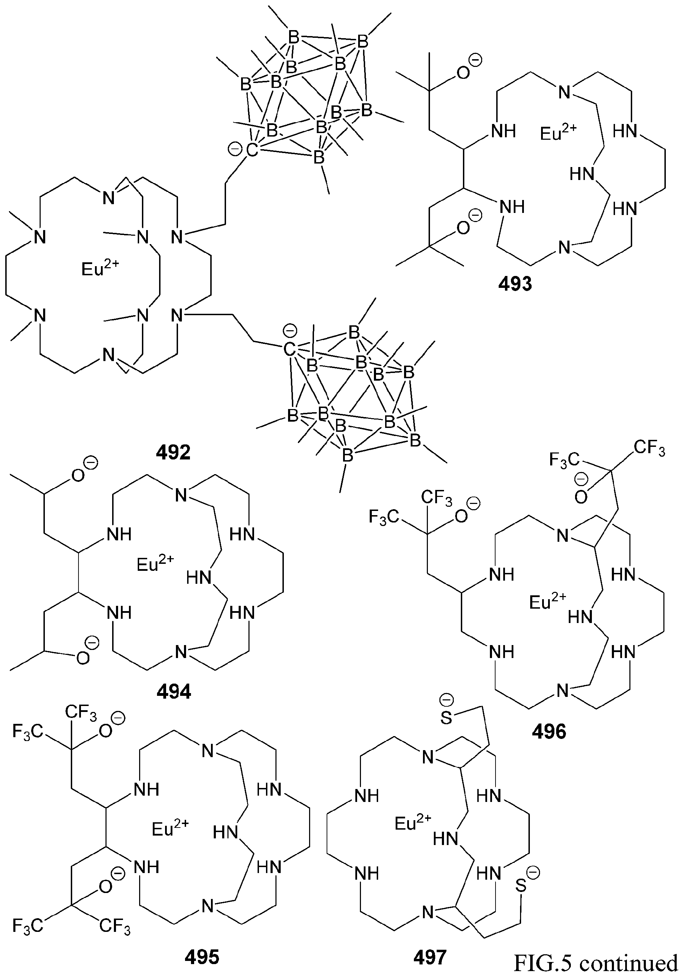

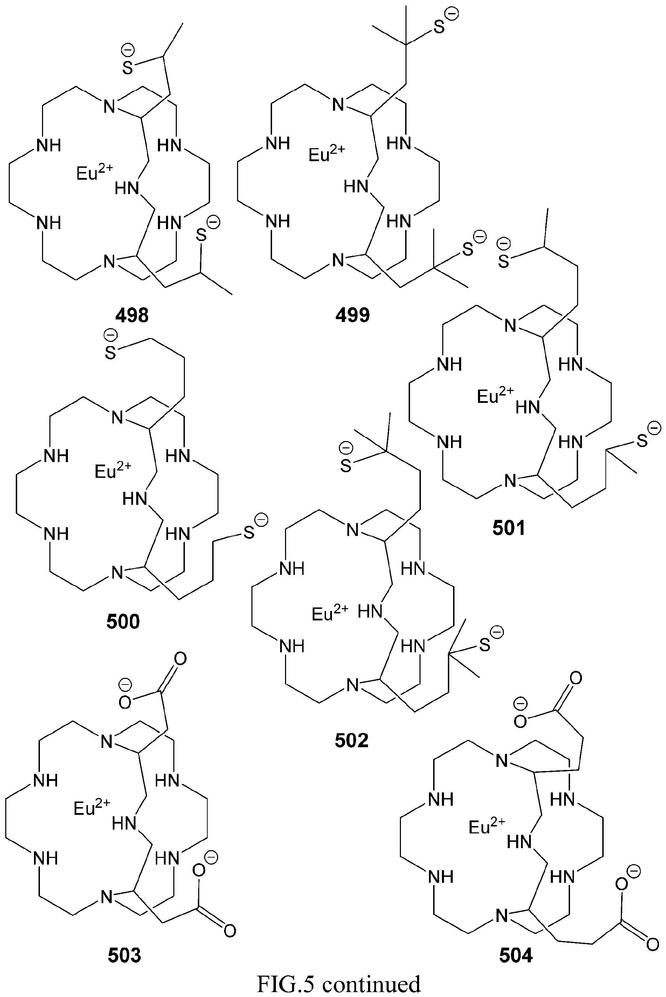

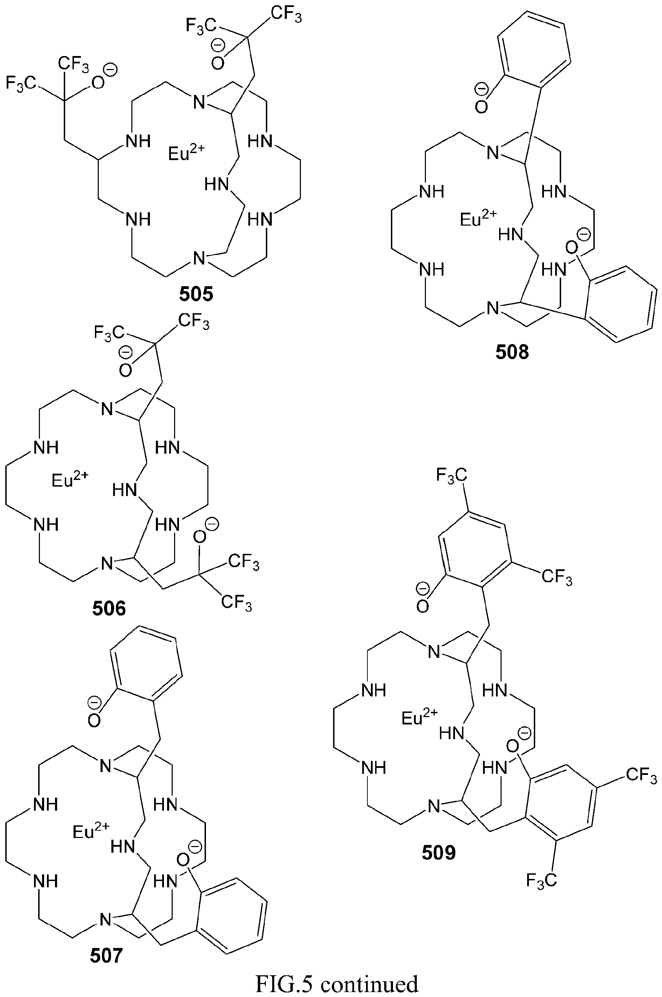

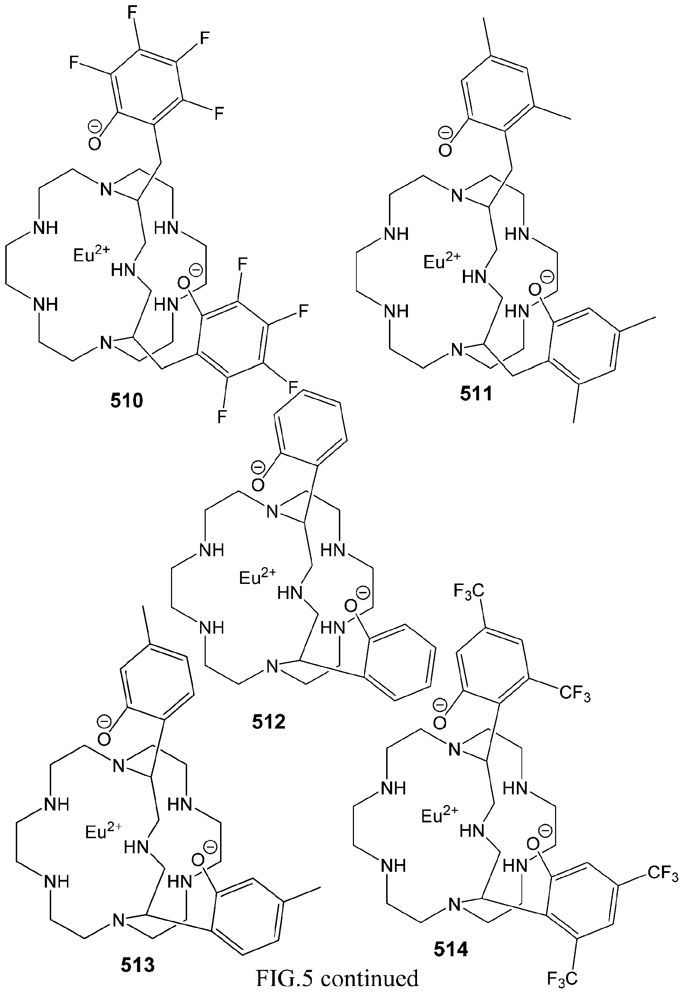

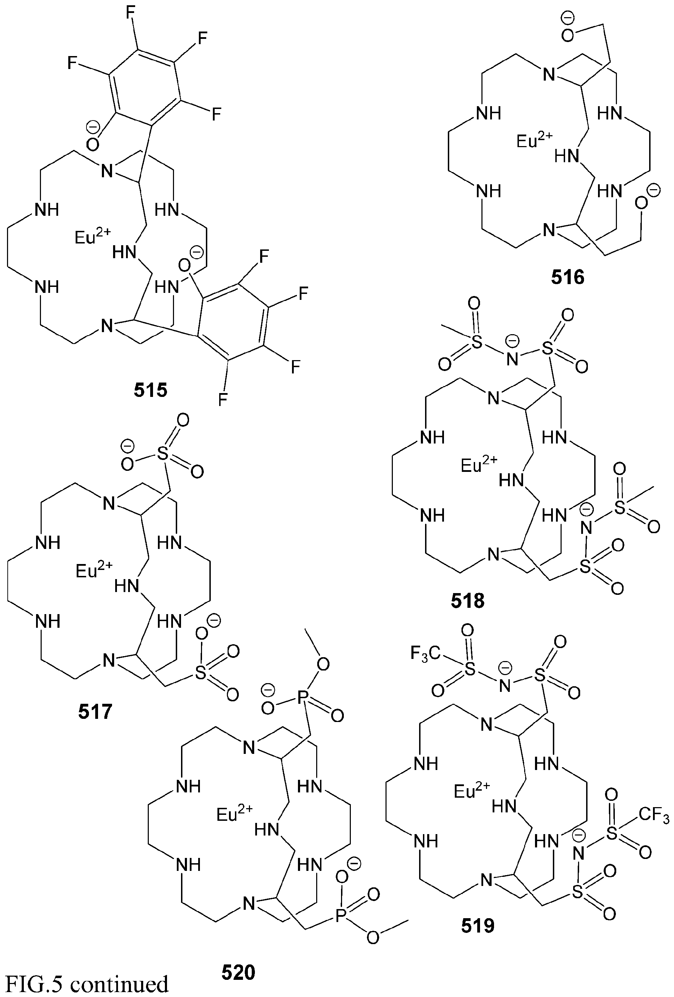

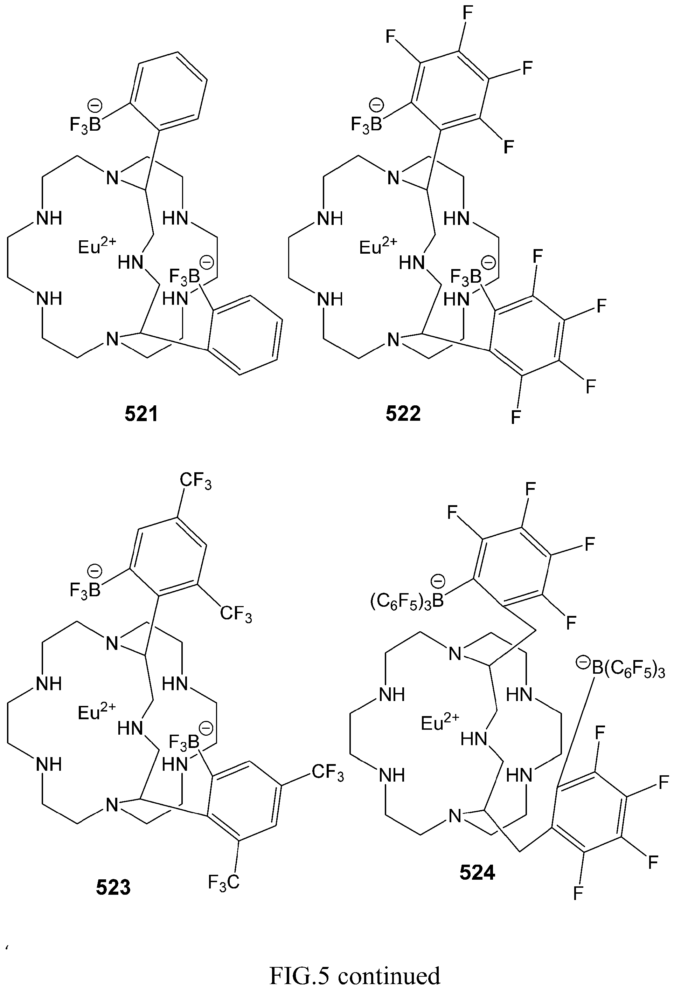

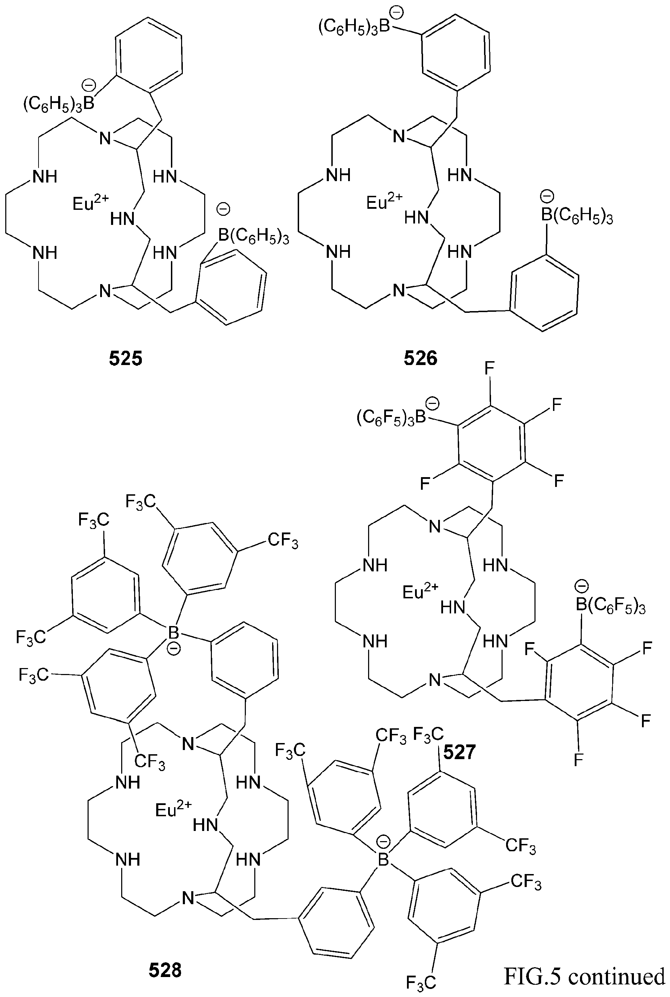

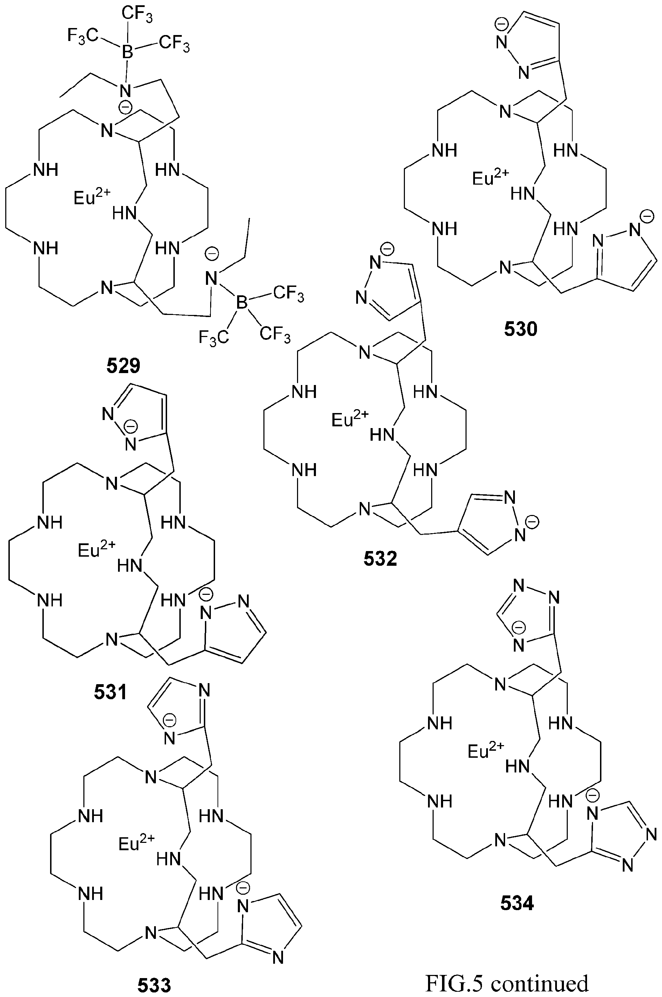

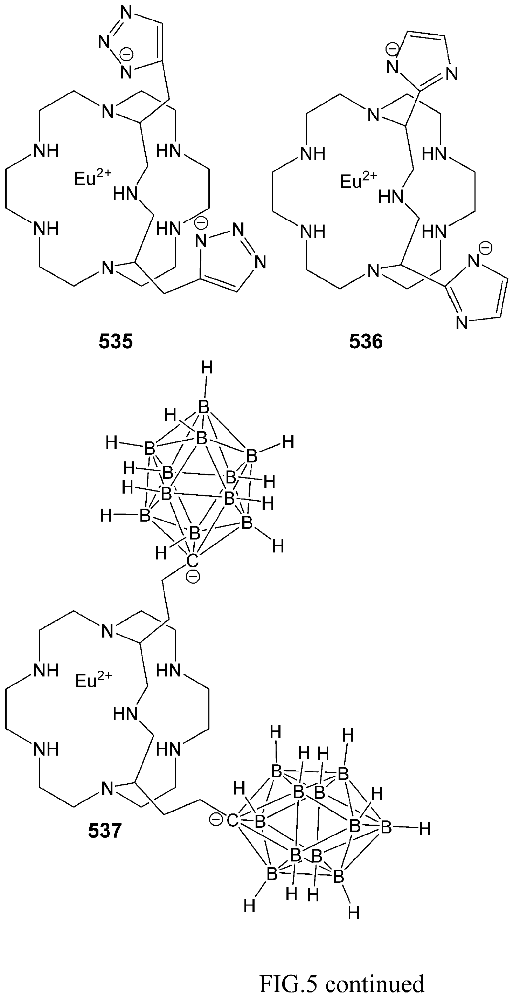

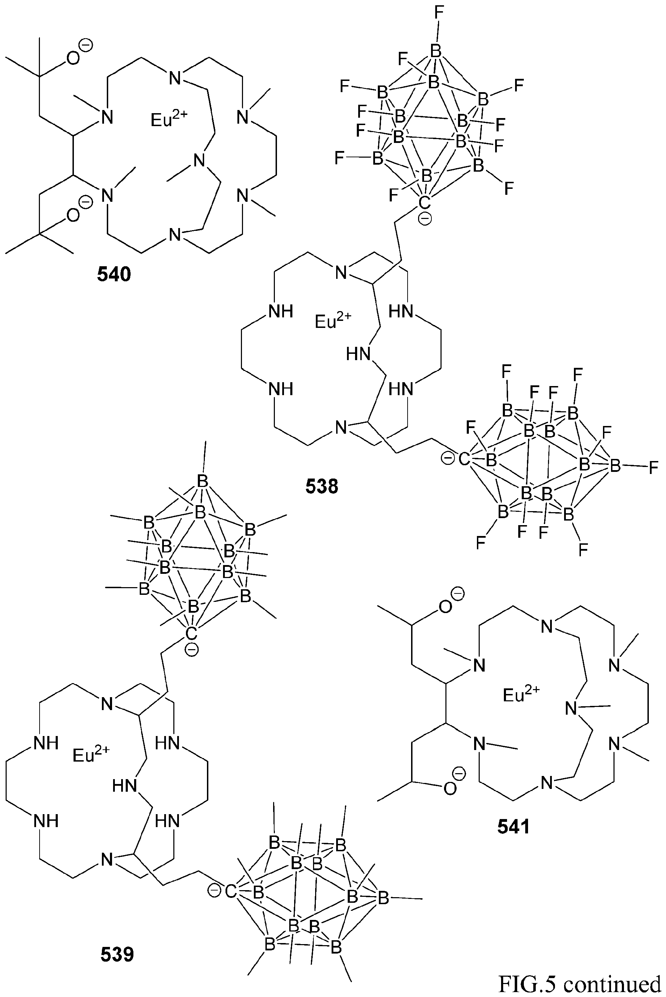

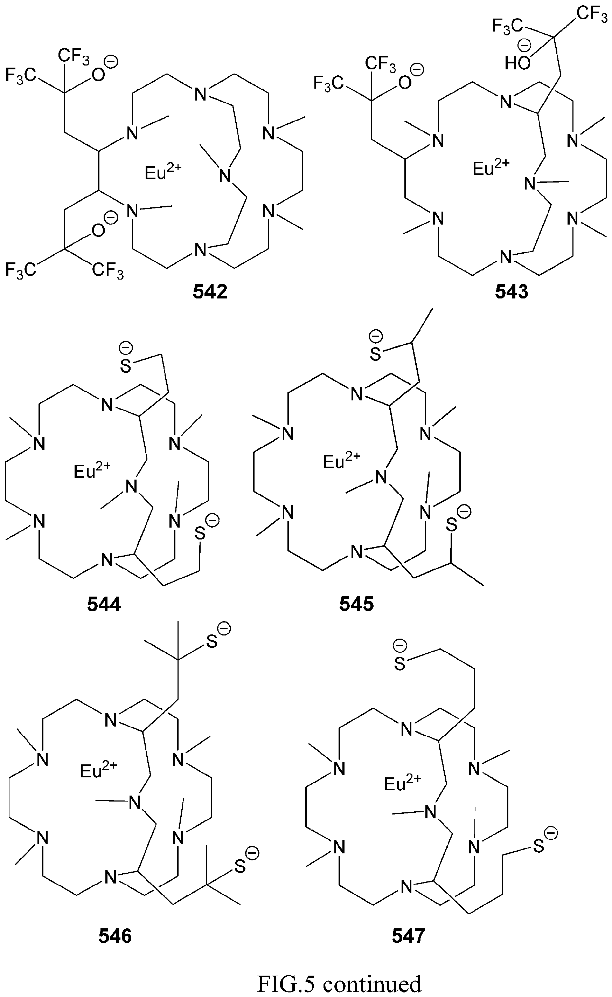

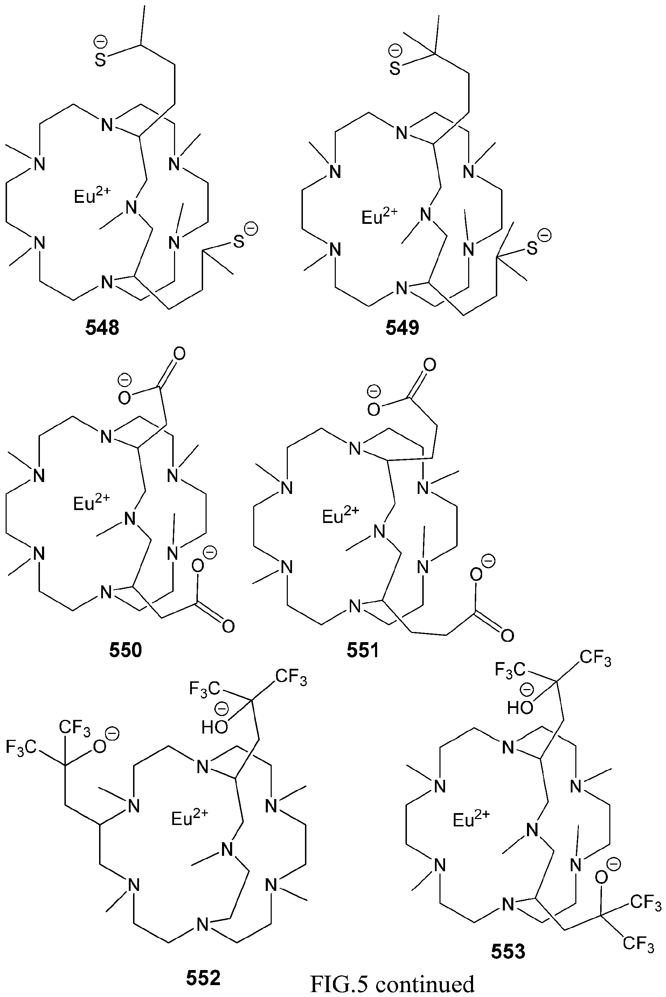

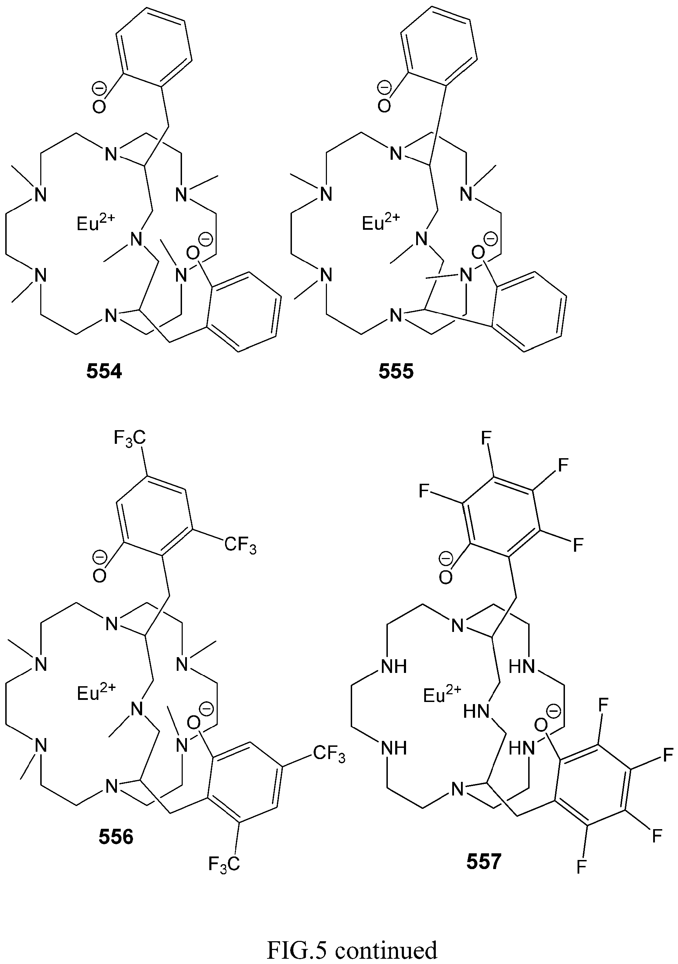

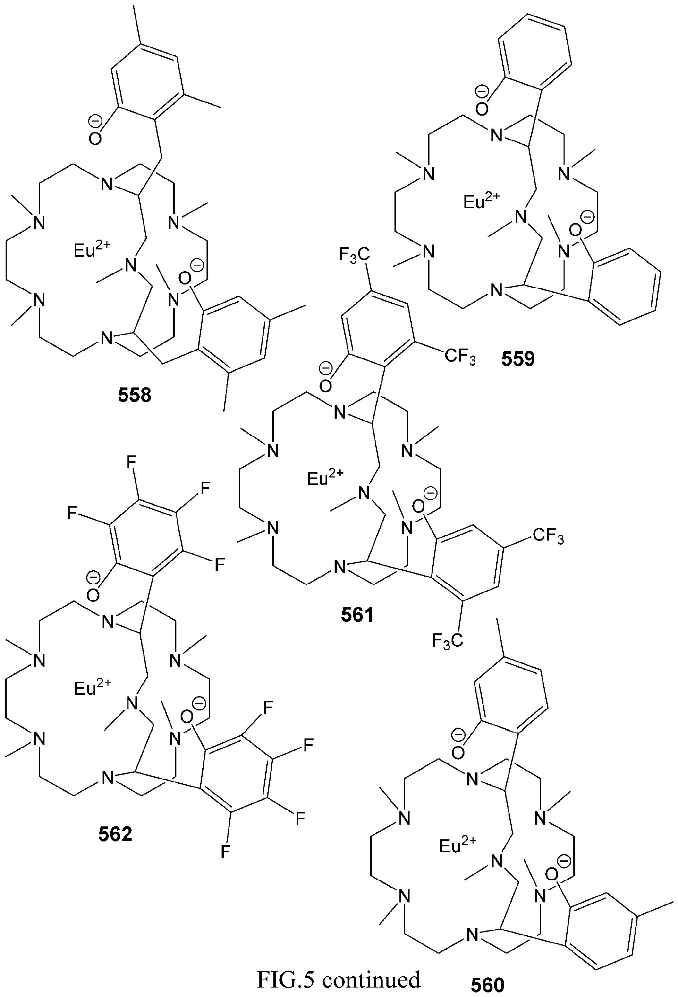

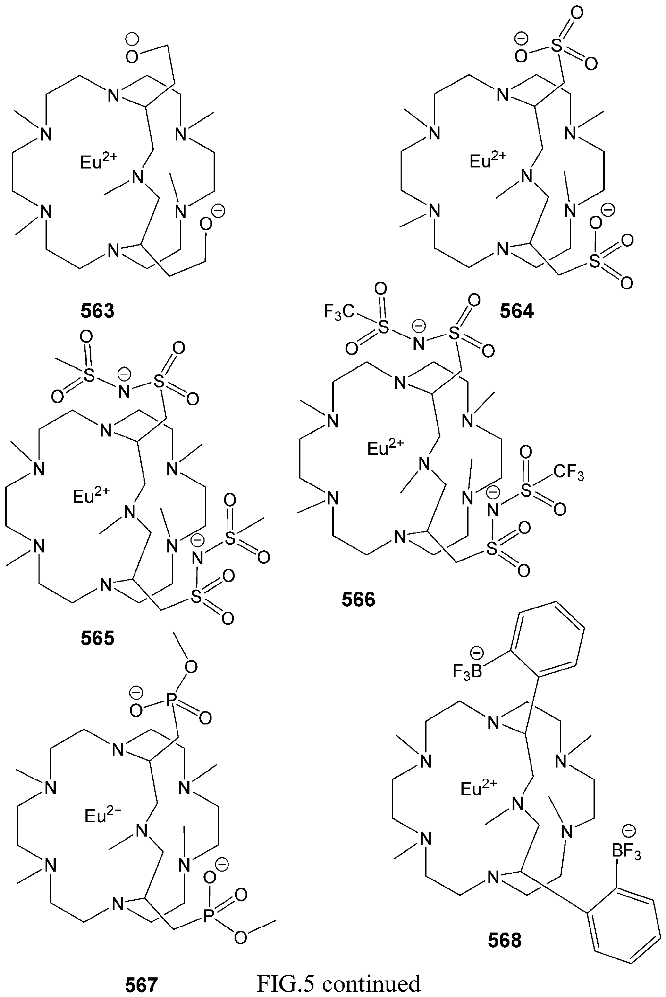

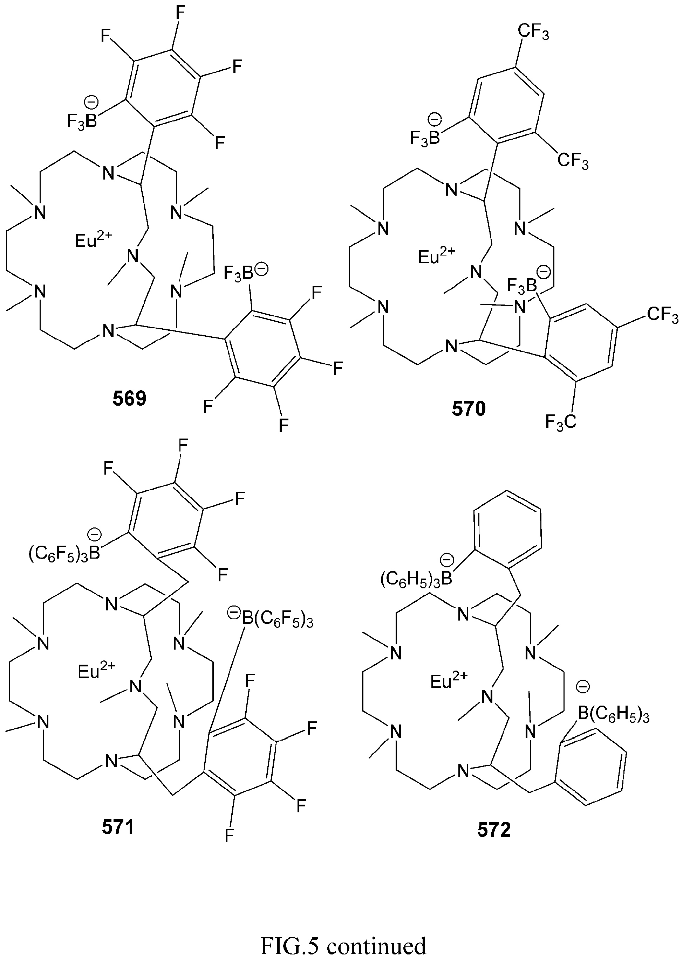

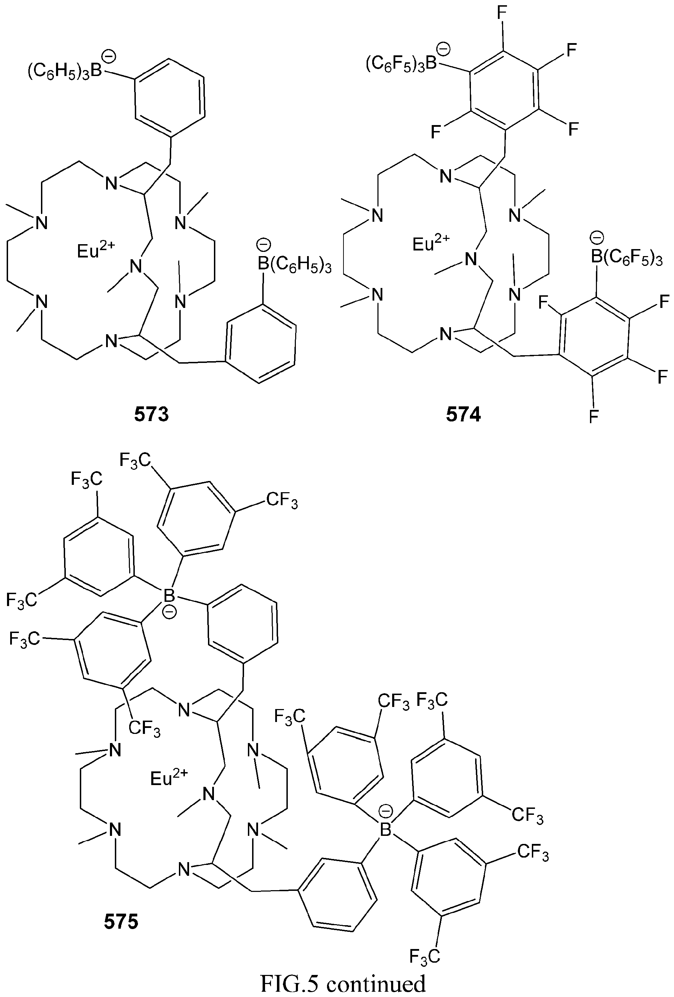

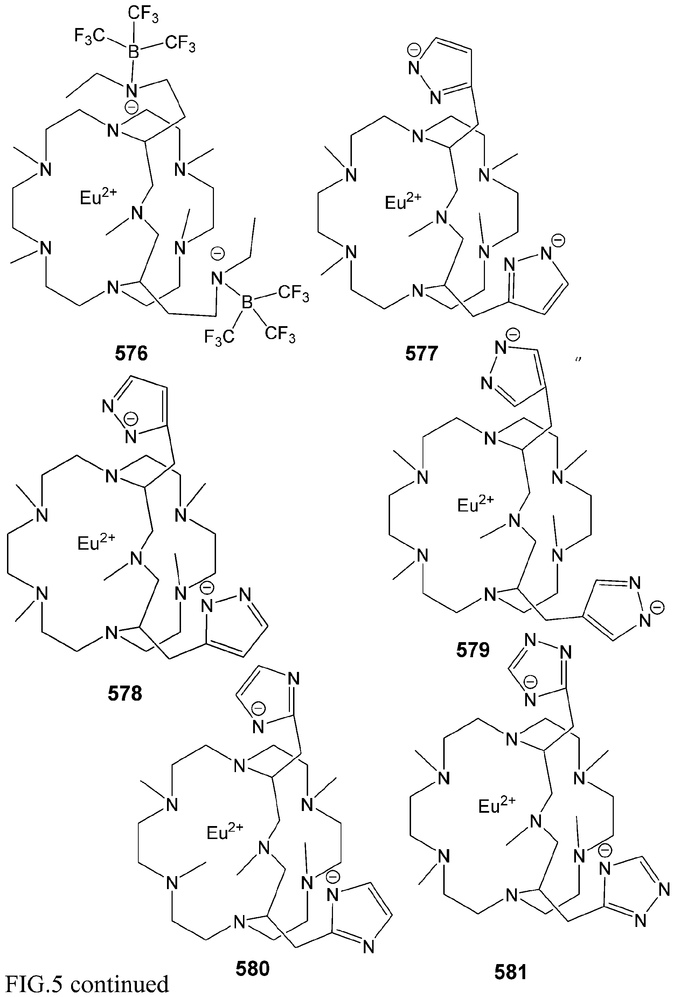

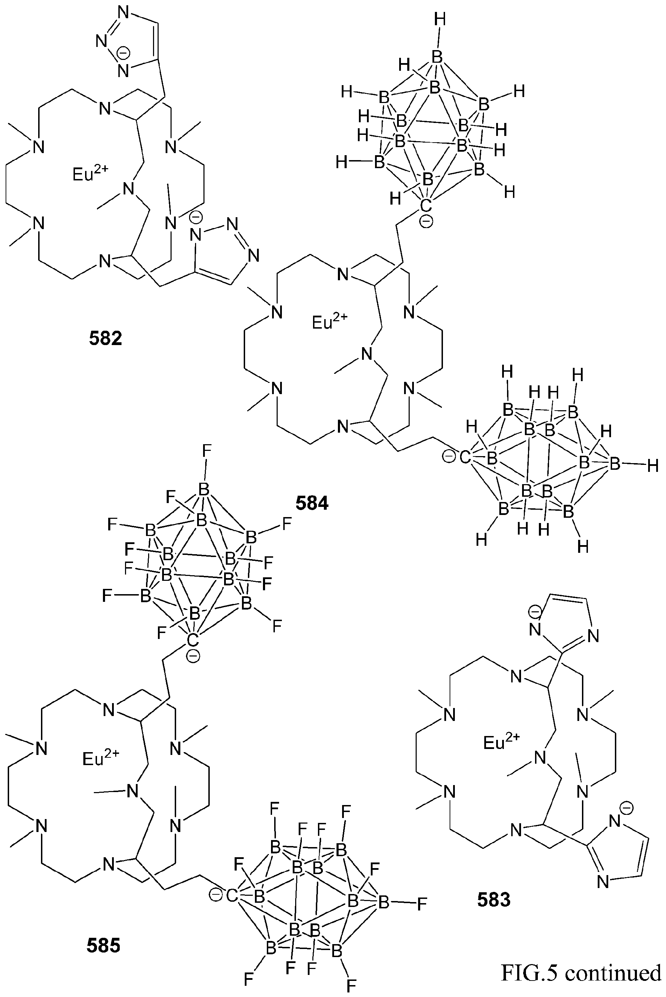

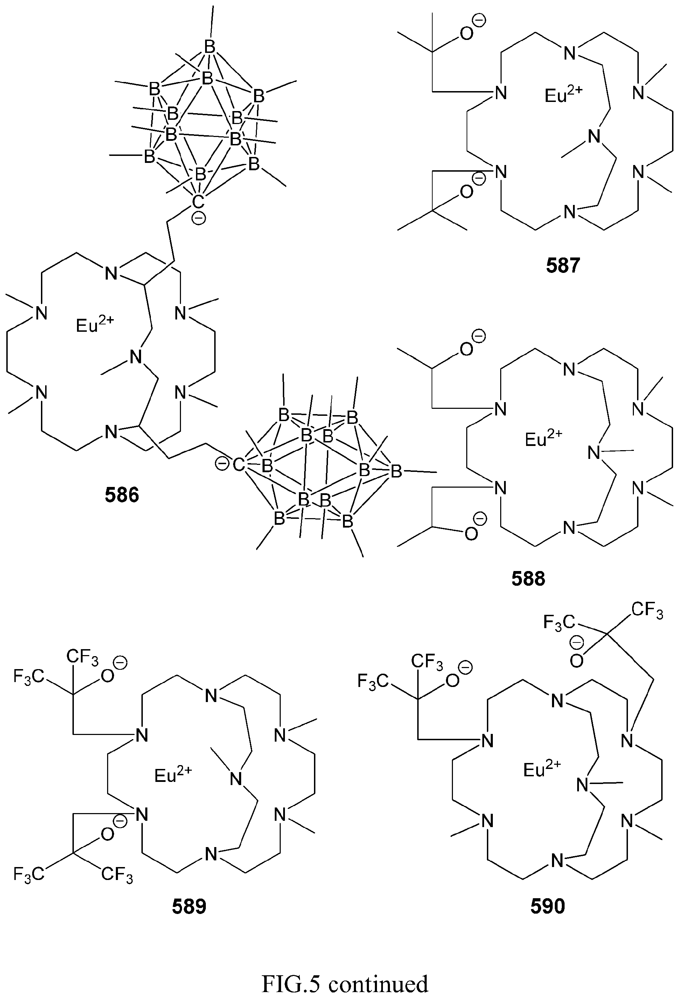

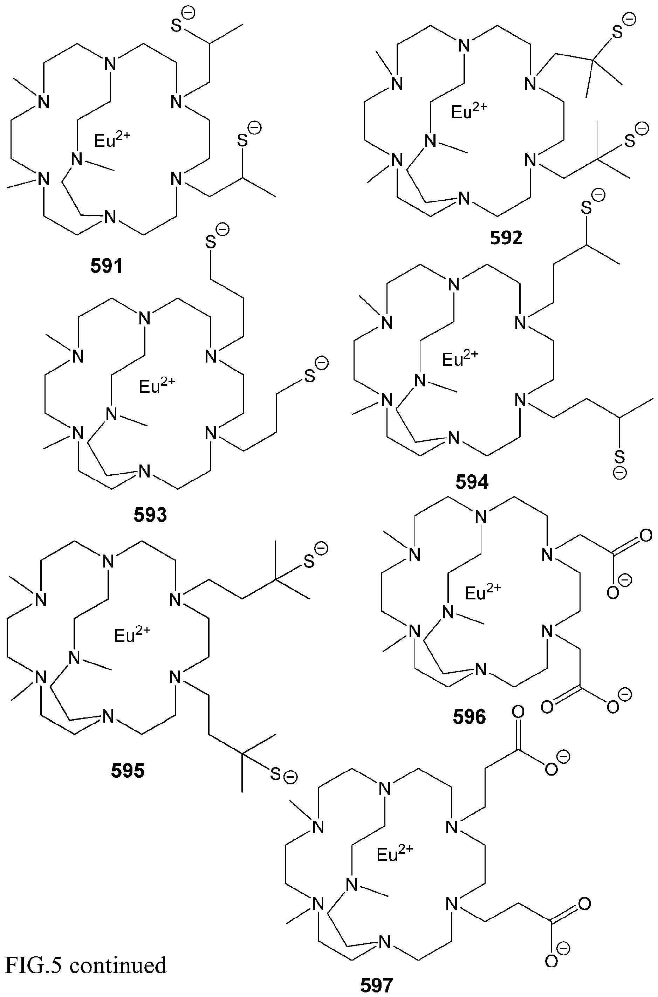

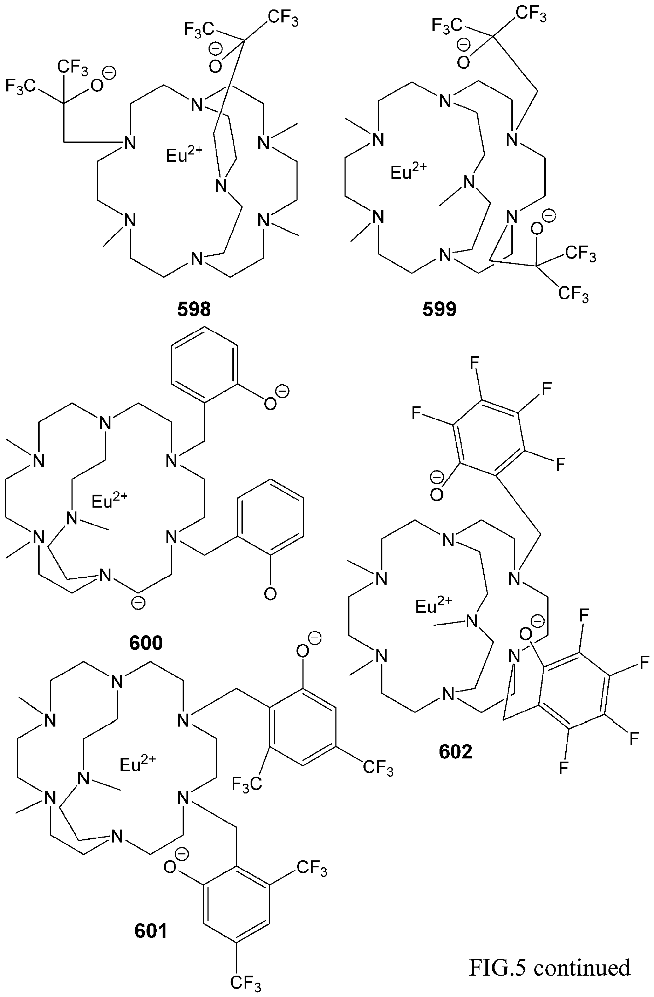

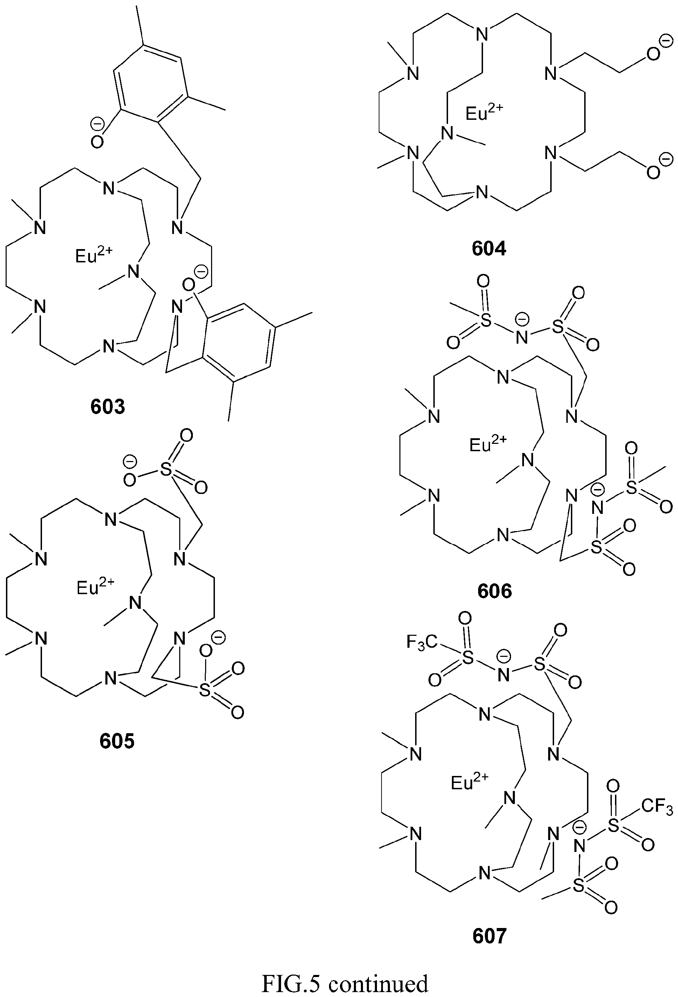

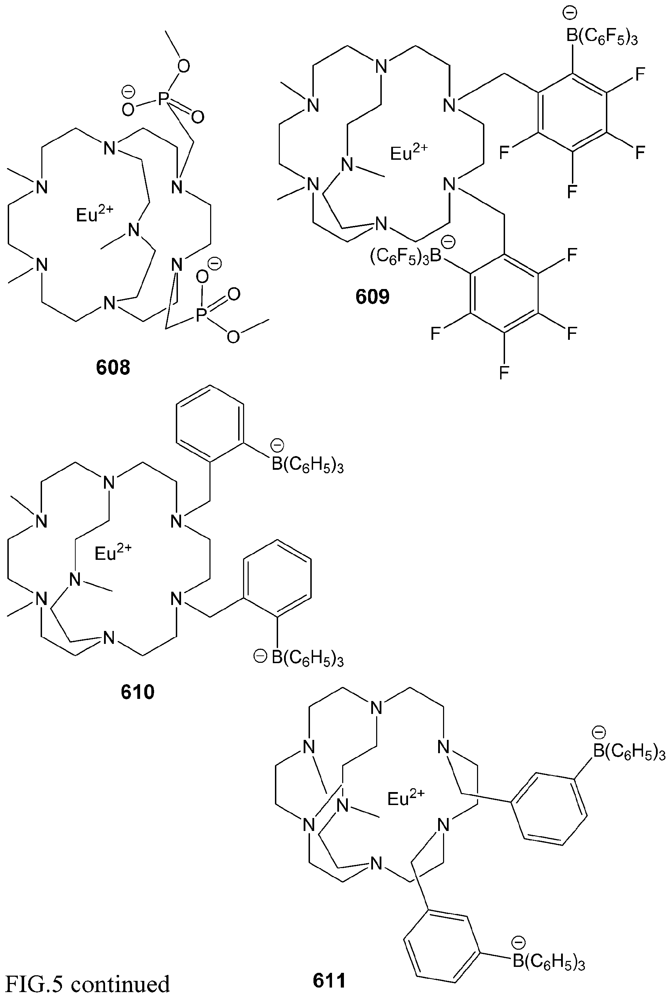

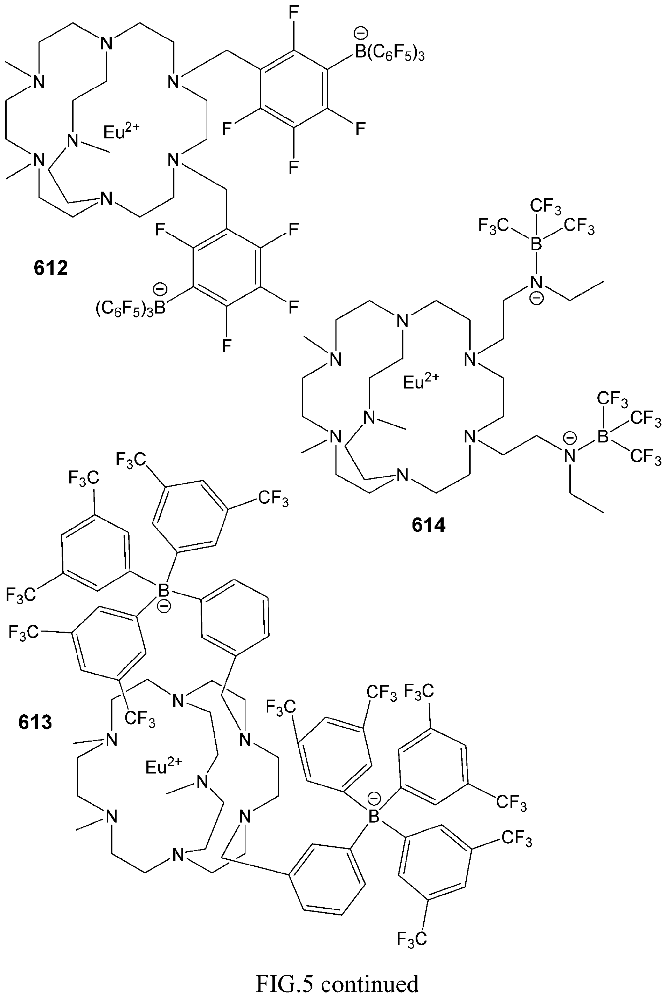

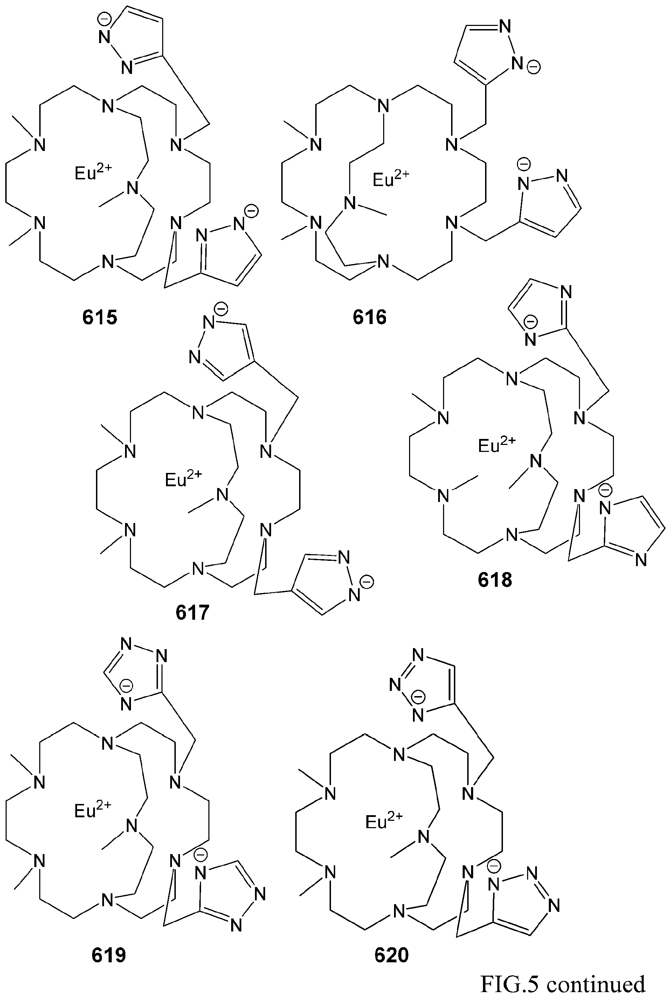

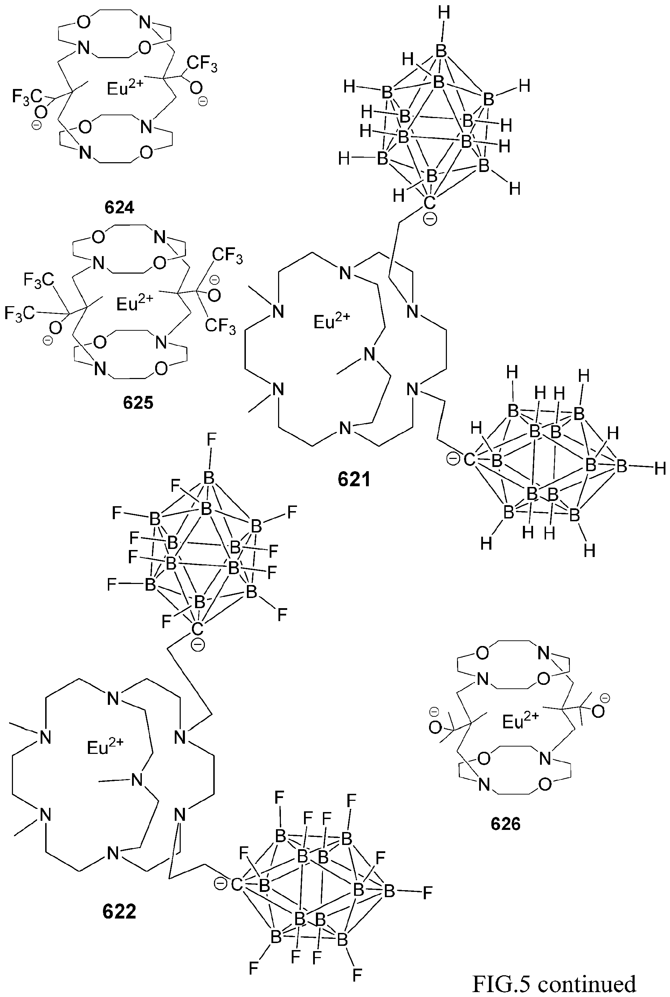

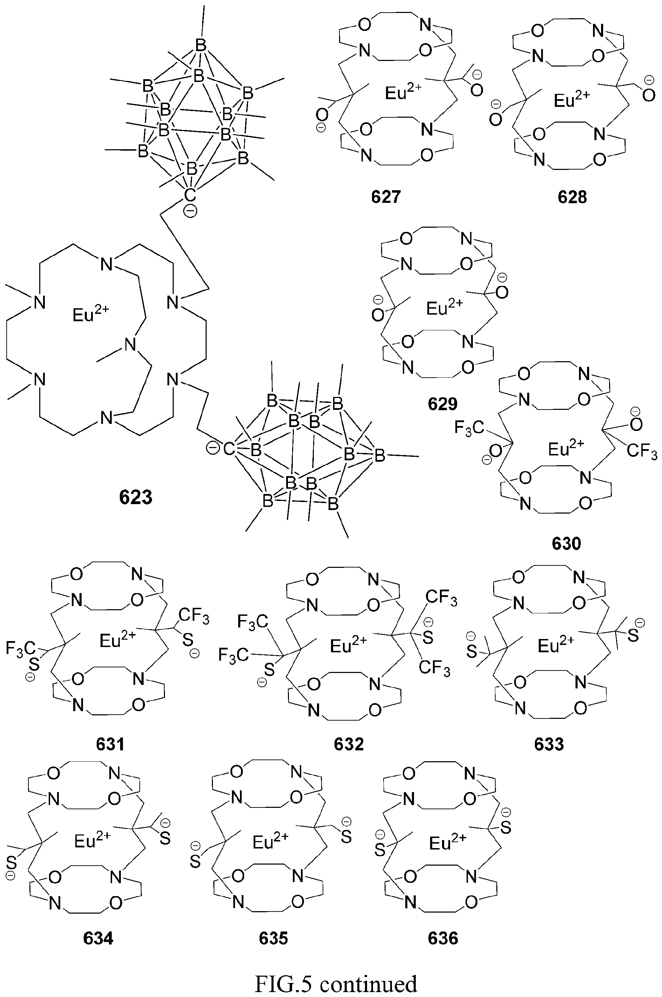

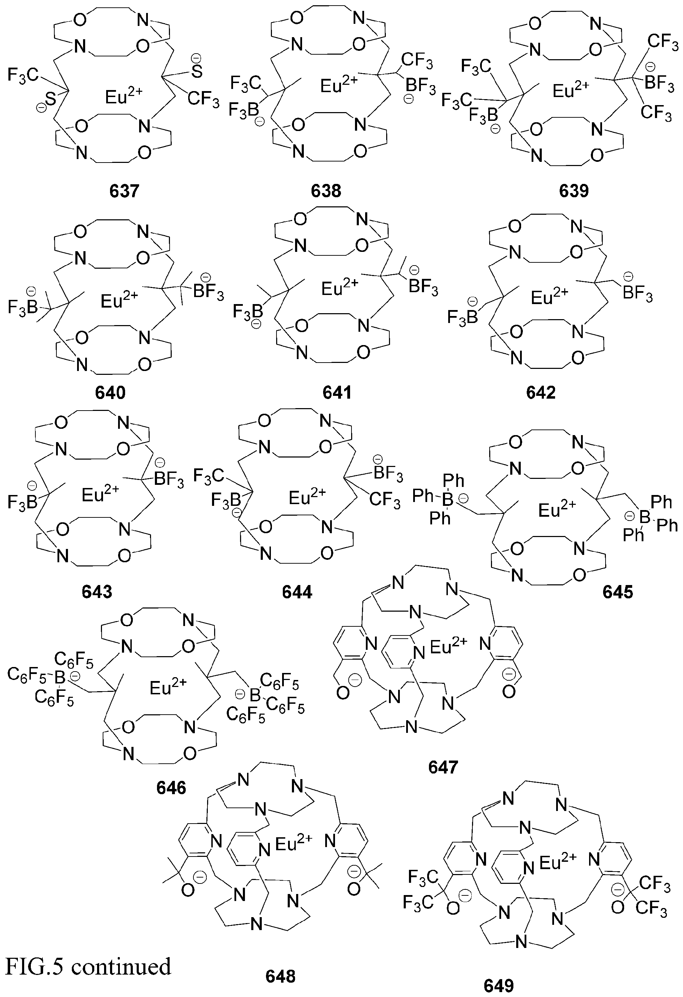

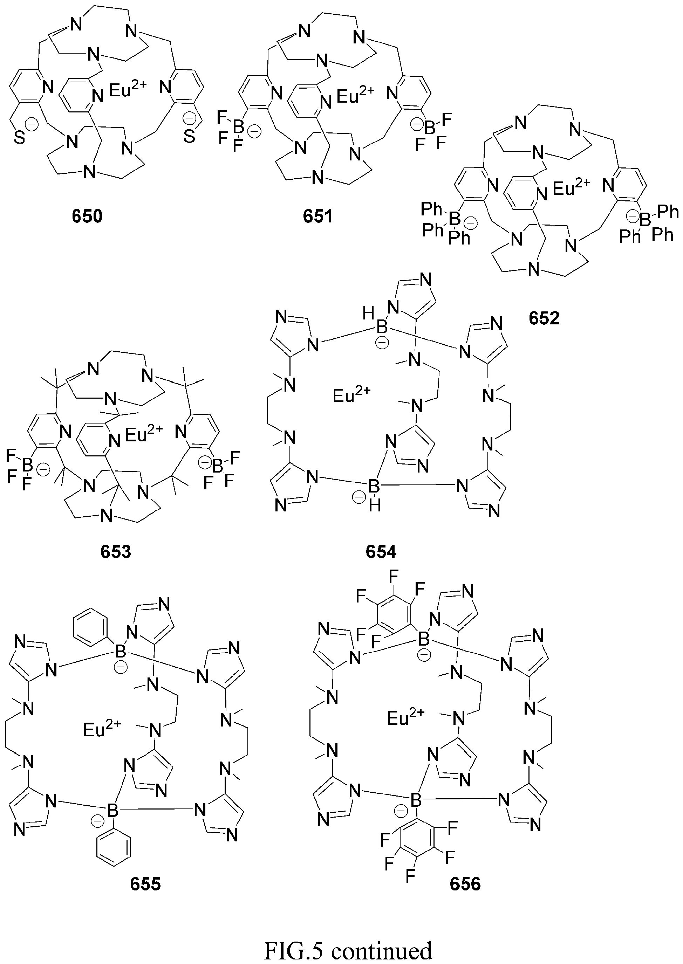

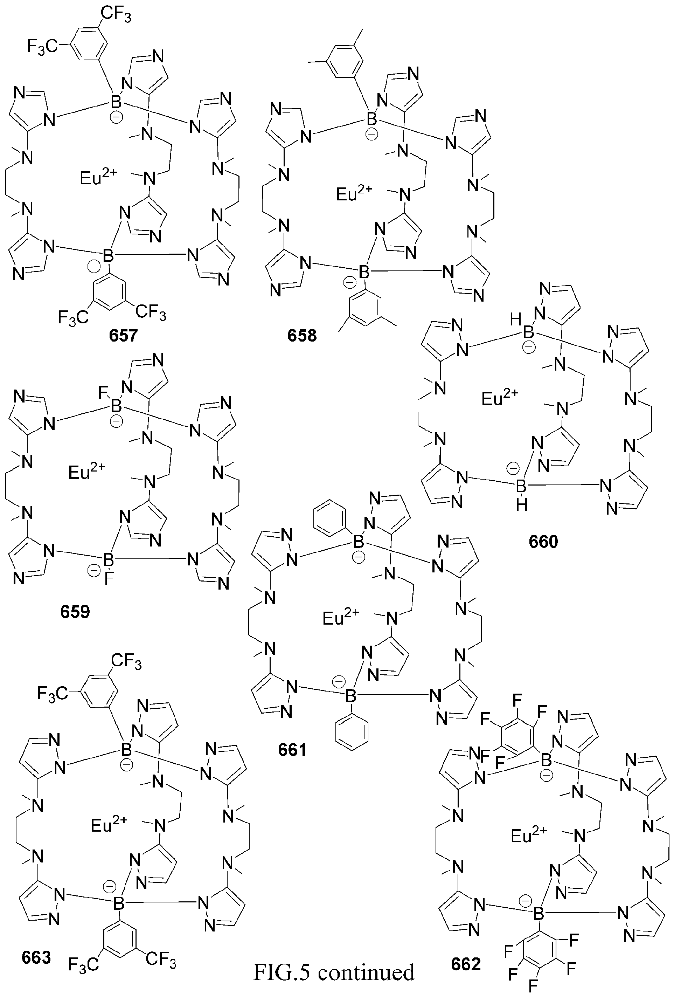

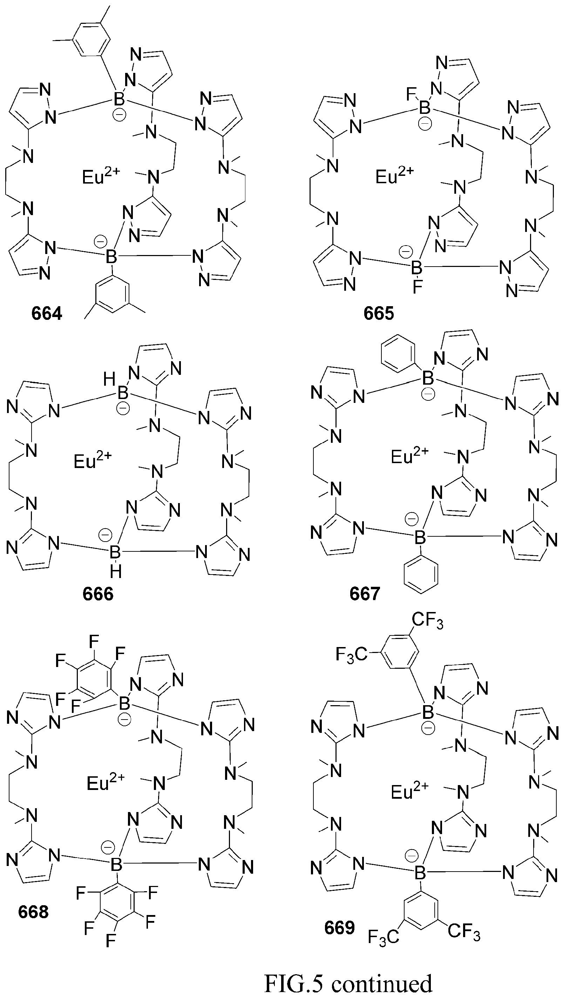

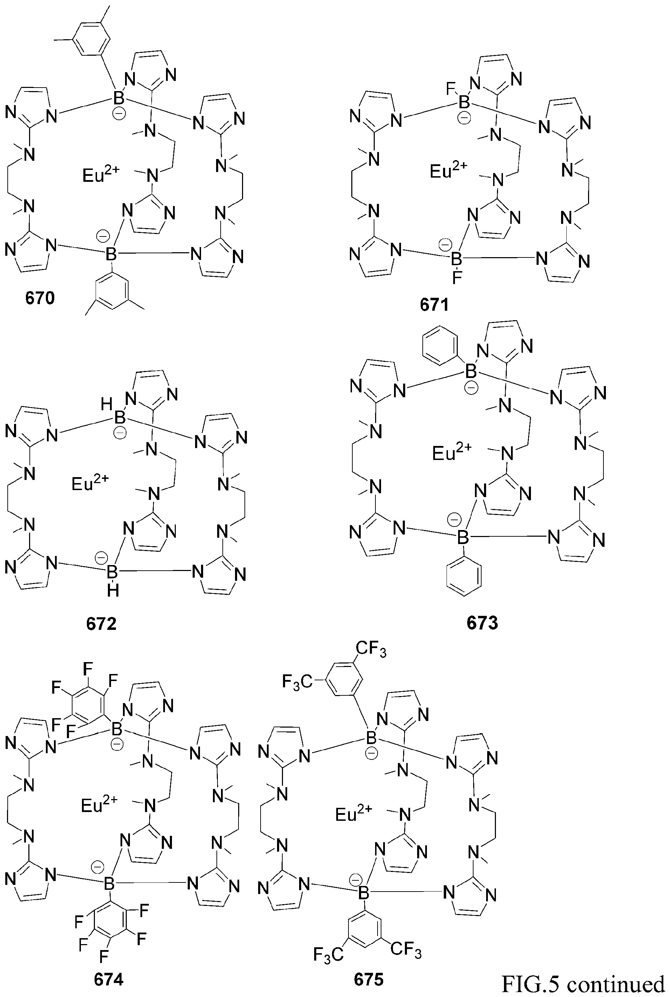

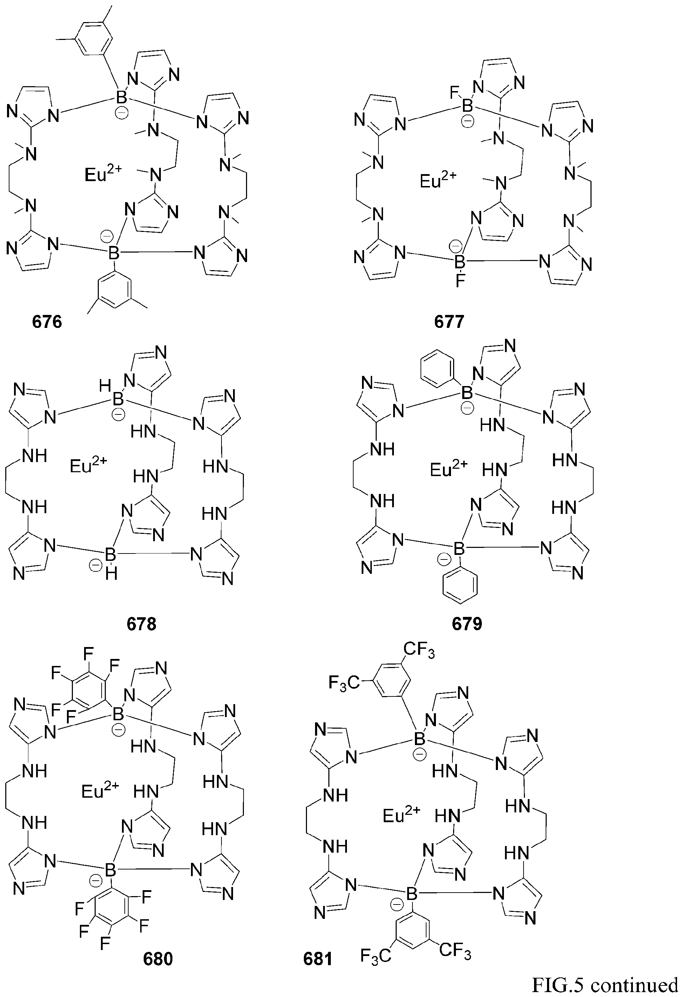

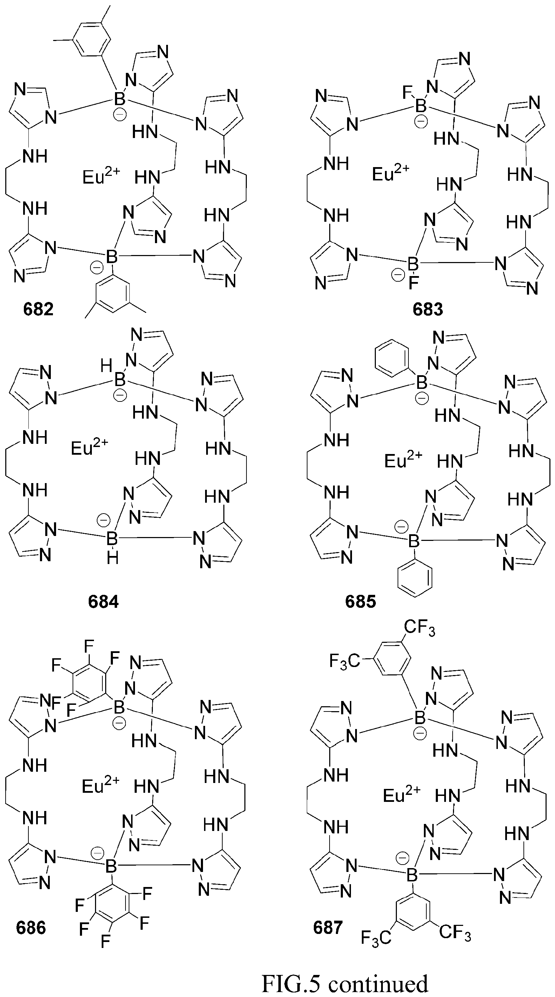

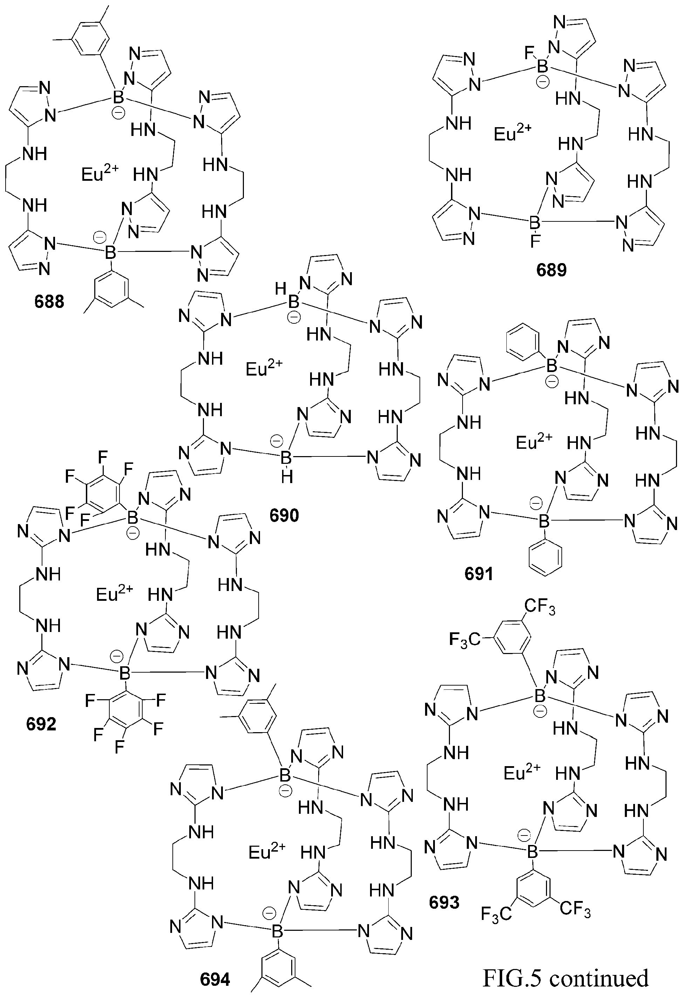

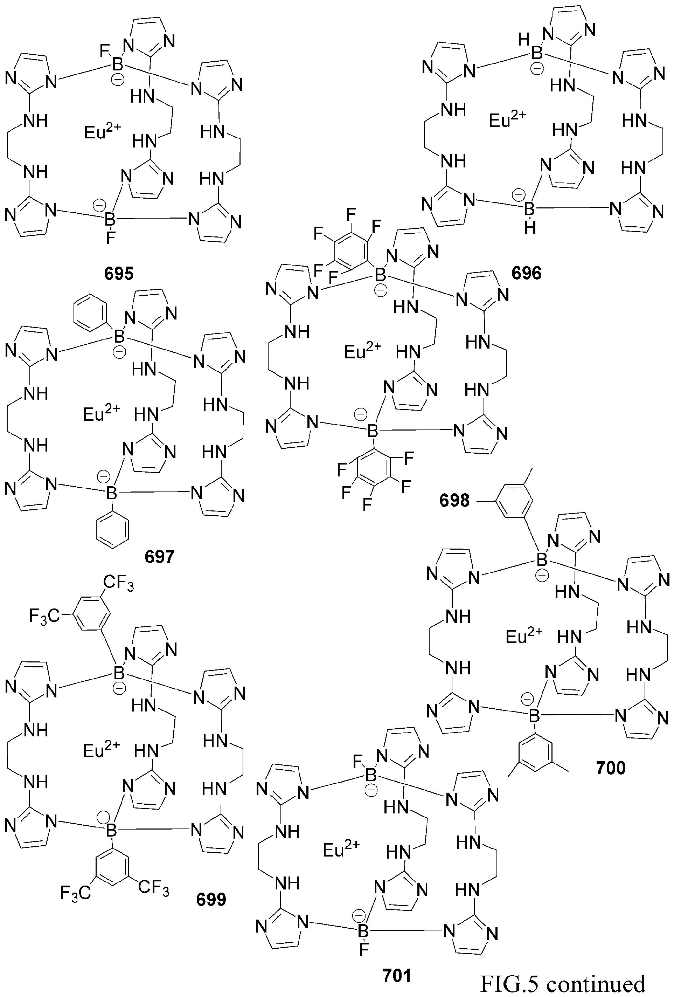

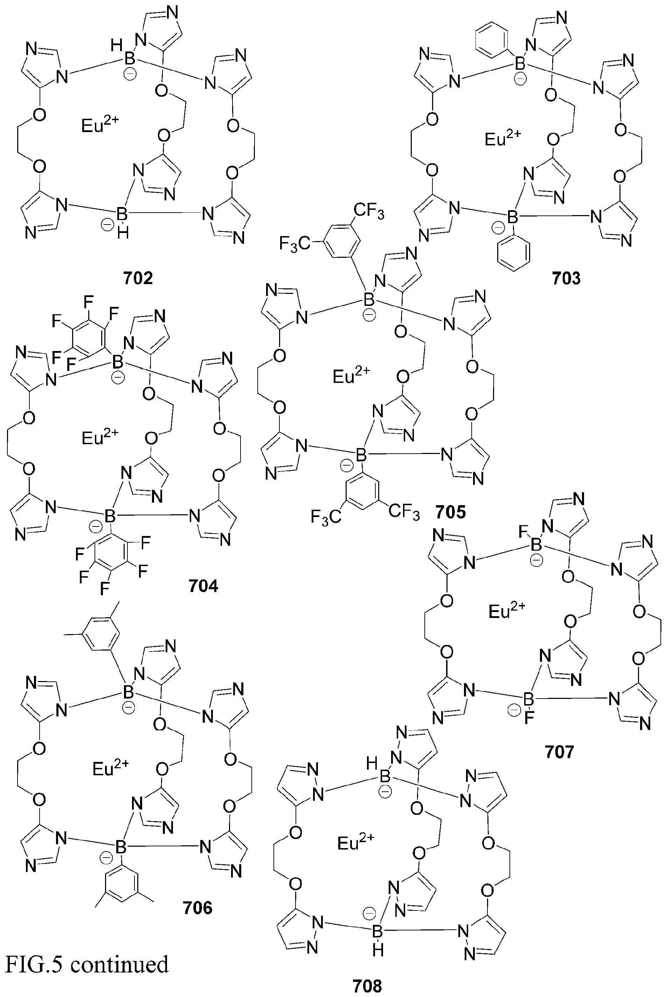

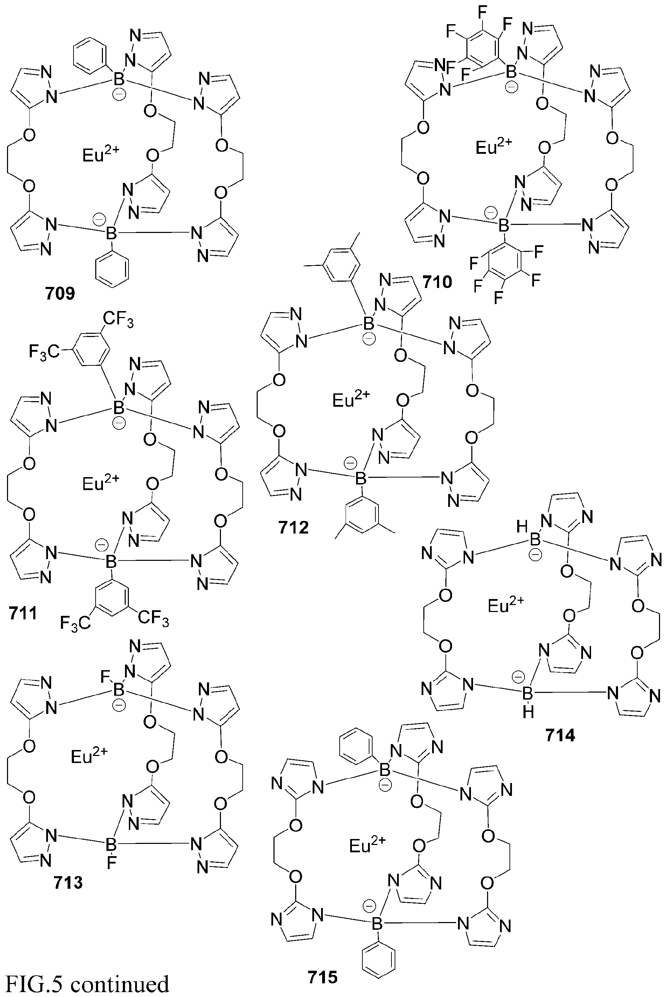

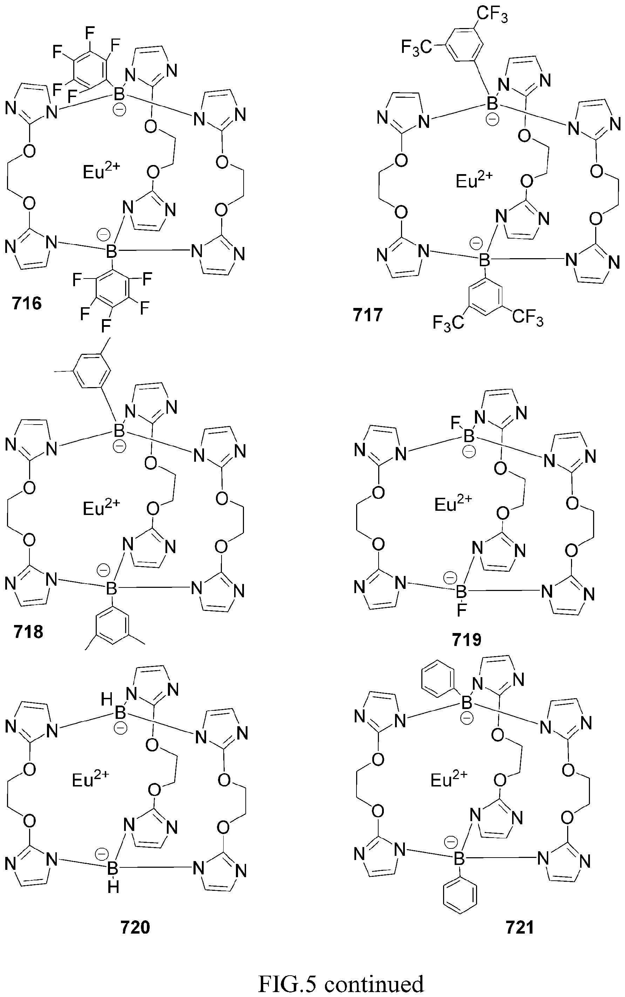

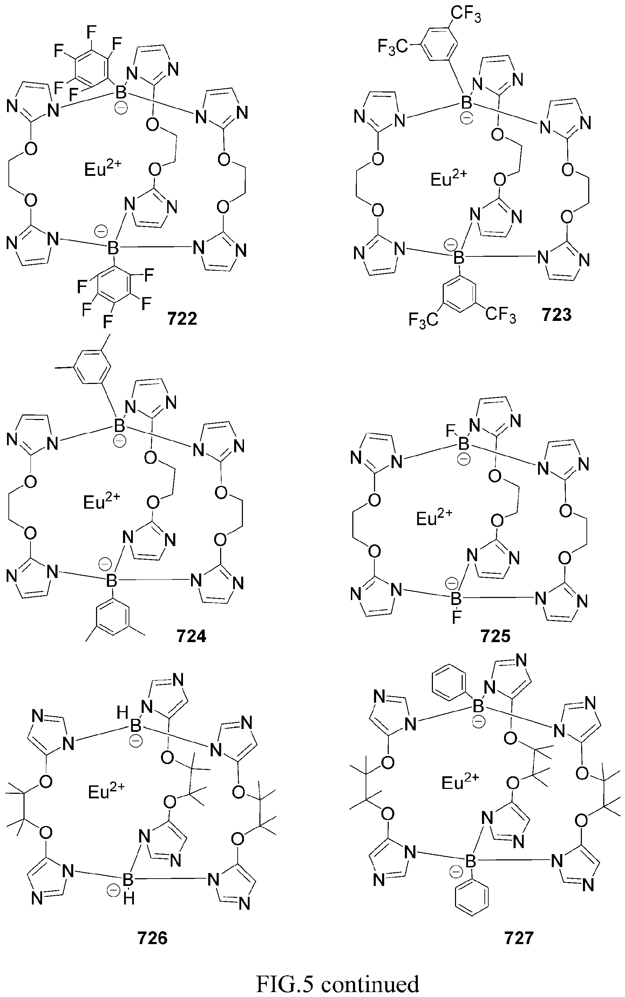

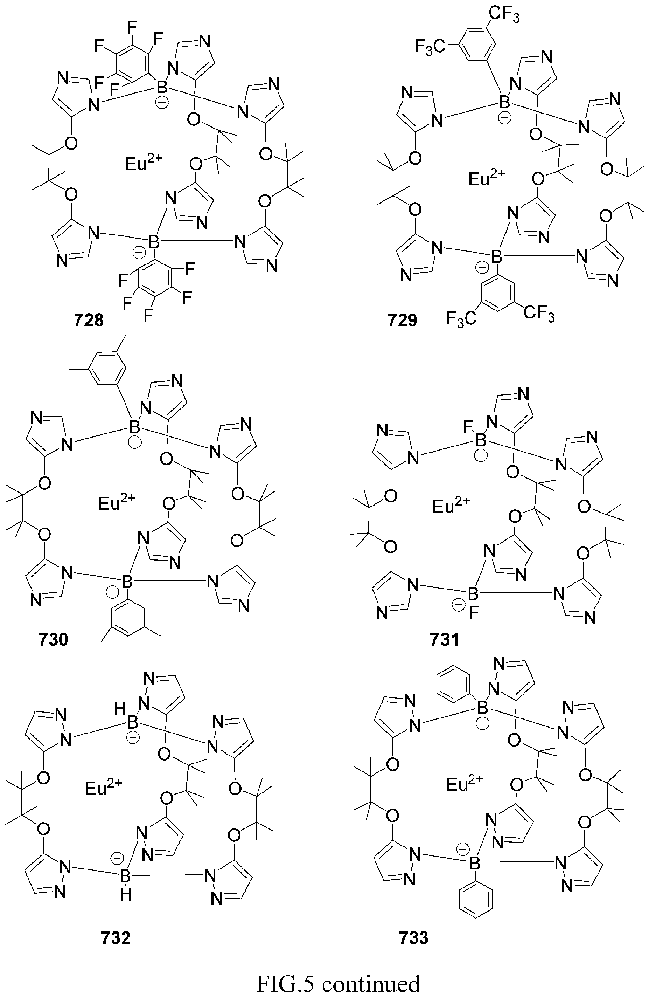

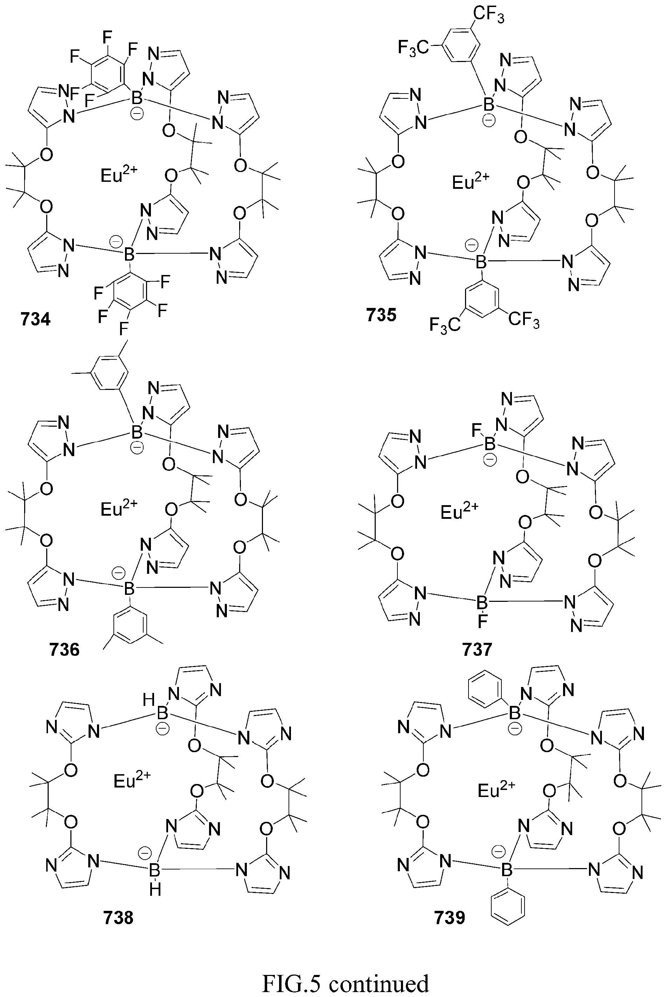

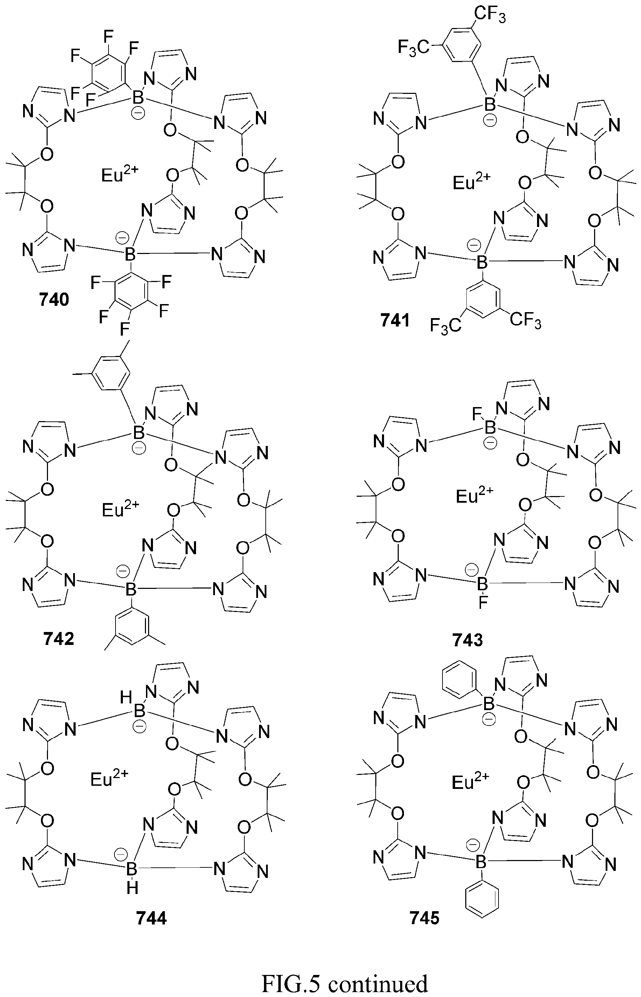

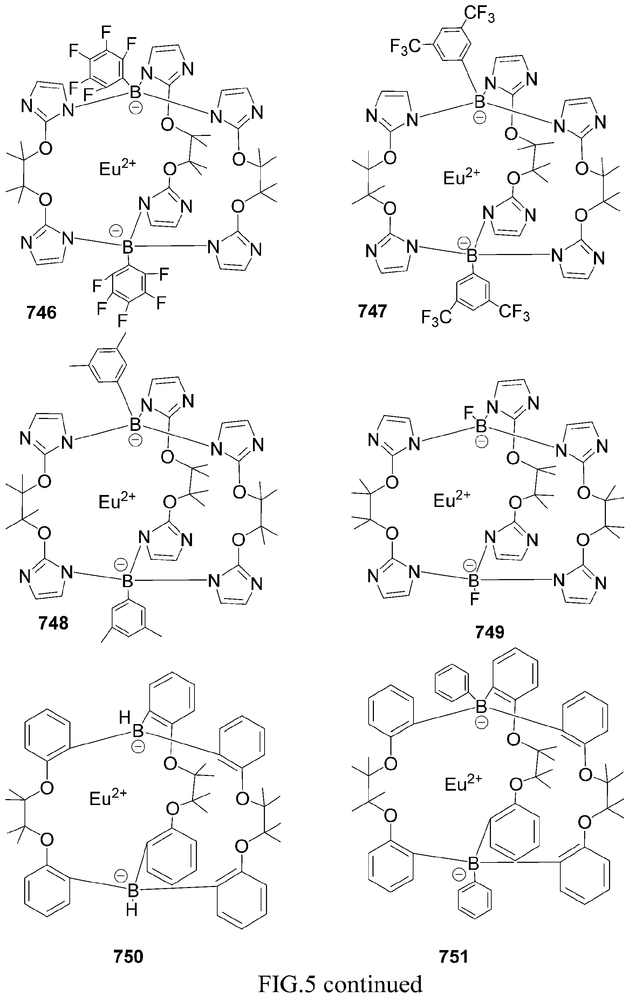

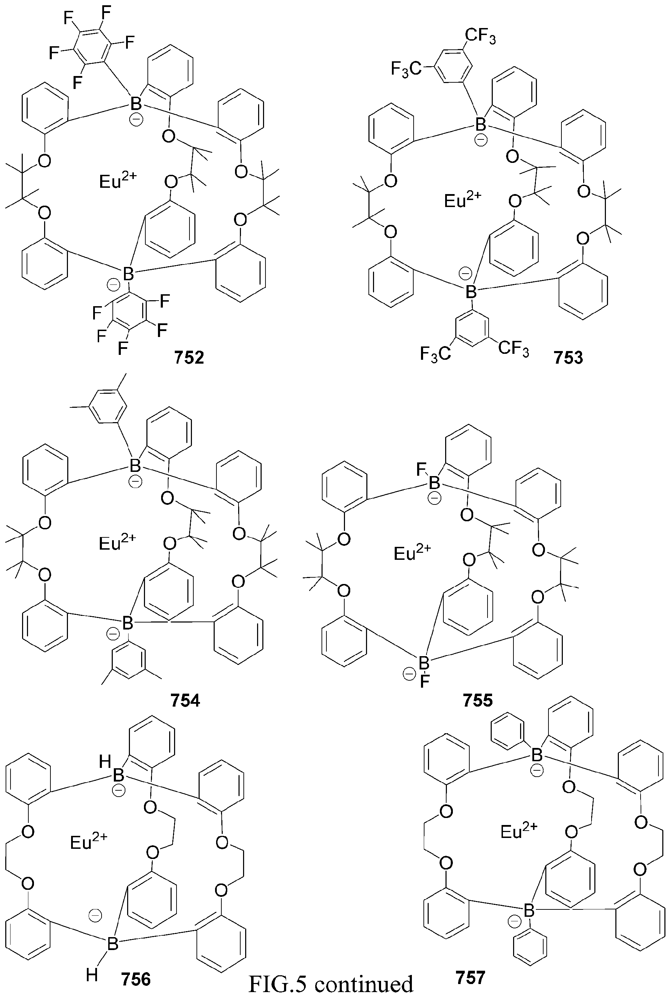

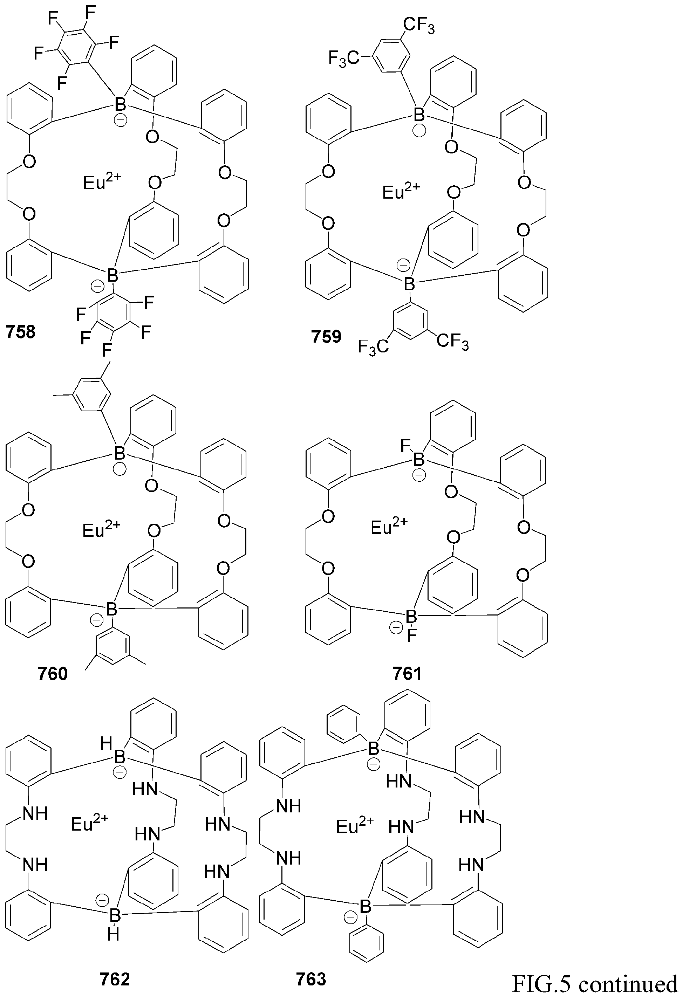

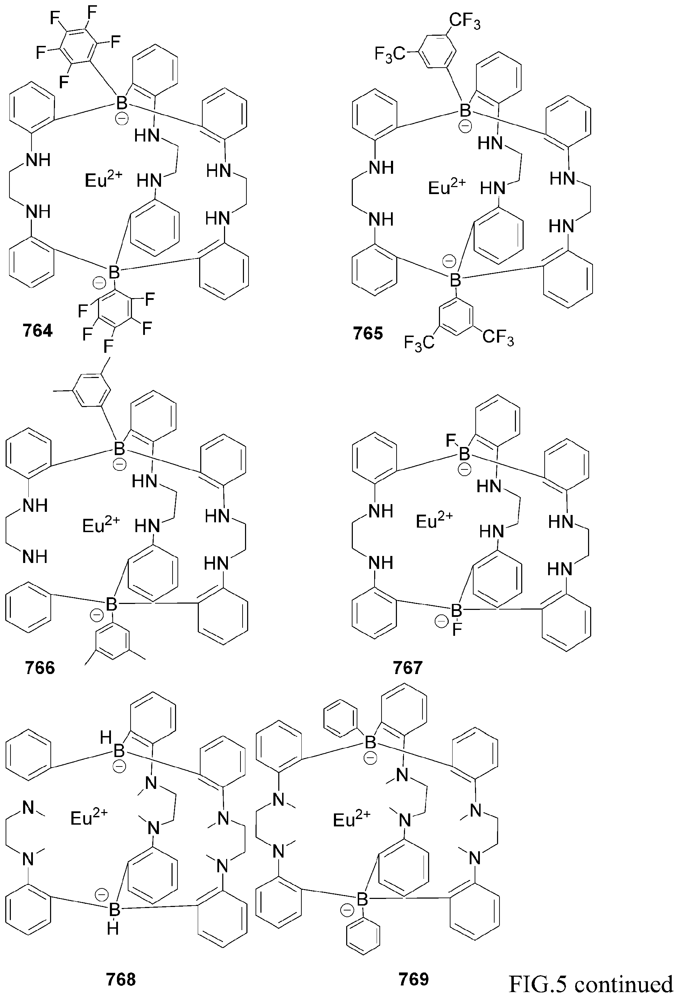

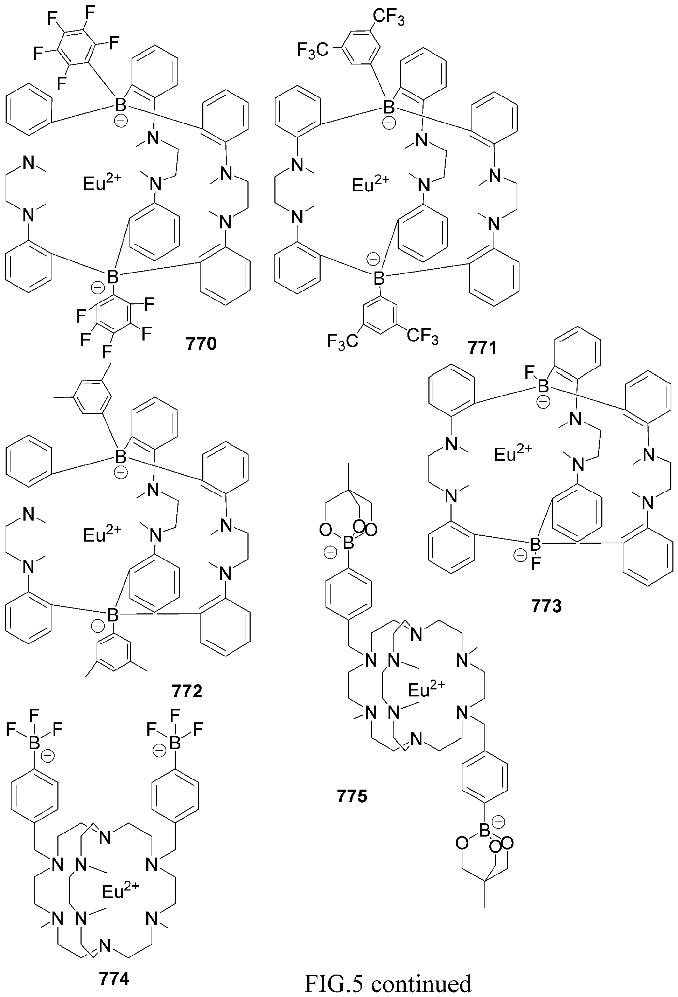

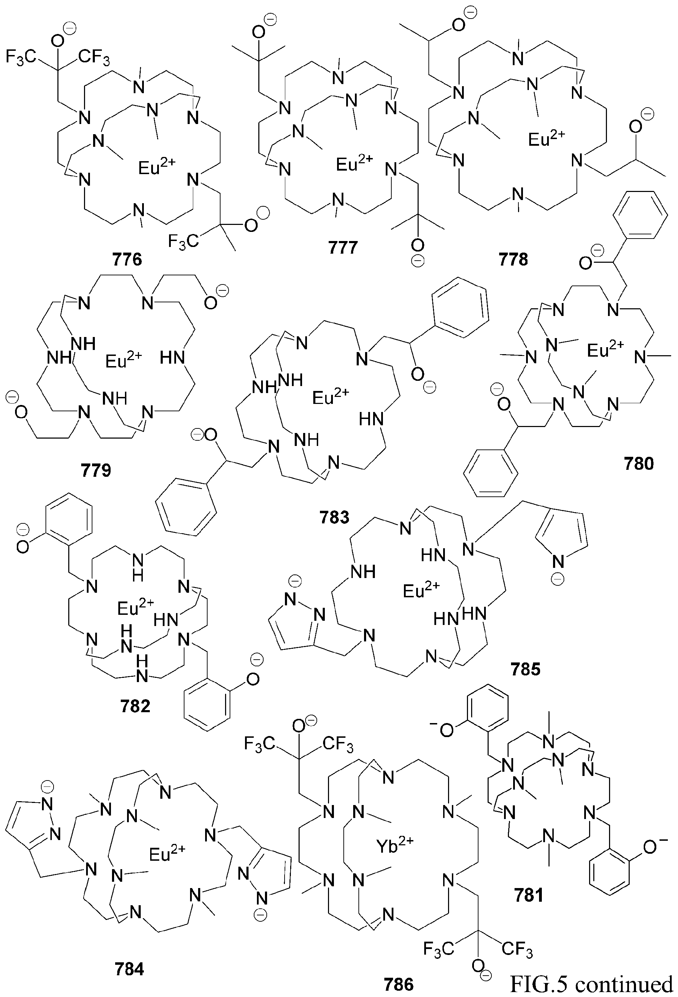

Preferably, the two monoanionic groups are covalently linked with different positions of the cyclic organic ligand. In a bicyclic or polycyclic organic ligand, the two monoanionic groups can be covalently linked with different branches of the bicyclic or polycyclic organic ligand, or they can be present in the same branch of the bicyclic or polycyclic ligand. Preferred examples of the monoanionic groups are shown in Figure 5.

It is preferred that the two monoanionic groups are covalently linked with different carbon or nitrogen atoms that are part of the bicyclic or polycyclic ring structure.

The two monoanionic groups, for example O- or B- or N- , are separated from each other by a sequence of at least two, preferably at least three, more preferably at least four, most preferably at least five intervening atoms. In this way, the monoanionic groups can compensate the charge of the divalent lanthanide, while the metal-organic coordination compound has a low polarity like quadropole and/or dipole polarity. Consequently, the metal-organic coordination compound has molecular character instead of salt character and has higher miscibility with matrices, can be dissolved in nonpolar solvents and can undergo sublimation or evaporation more easily since intermolecular interactions are reduced.

The cyclic organic ligand can be freely chosen from monocyclic, bicyclic or polycyclic ligands. Preferably, the number of ring atoms is chosen in a way to allow for the divalent lanthanide to be coordinated inside the cyclic organic ligand. Preferably, the divalent lanthanide is Europium or Ytterbium, most preferably Europium. Therefore, the cyclic organic ligand is preferably selected in a way that the divalent Europium can be contained inside the cyclic structure and does not undergo oxidation.

By providing a single molecular structure that avoids non covalently bound moieties, such as halogenids, as part of the metal-organic coordination compound, drift or diffusion of parts the compounds in an electrical field, e. g. in OLED applications, can be avoided, so that the stability is substantially increased. Solution processing of OLEDs is easier and film formation is easier due to the solubility in nonpolar solvents. Also sublimation is easier so that OLED devices can be prepared by sublimation of the metal-organic coordination compounds of the present invention.

The metal-organic coordination compound contains exactly two monoanionic groups covalently linked with a cyclic organic ligand so that the coordination compound is neutrally charged.

According to one embodiment of the invention, the coordination compound comprises at least one divalent lanthanide coordinated by a cyclic organic ligand having the formula 1-1 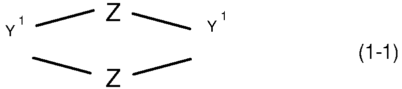
wherein Y¹ for each occurrence is independently a divalent group containing 1 to 30 atoms,
or formula 1-2 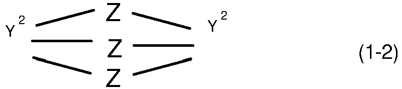
wherein Y² for each occurrence is independently a trivalent group containing 1 to 30 atoms,
Z for each occurrence is independently a divalent organic group, wherein Z has a shortest sequence of at least 3 atoms, preferably 3 to 5 atoms, between the two linkages with Y¹ or Y² in case Y¹ or Y² is a cyclic group connected with each Z via different ring atoms,
wherein Z has a shortest sequence of at least 6 atoms, preferably 6 to 8 atoms, in case Y¹ or Y² is connected with each Z via one atom,
wherein the cyclic organic ligand of formula 1-1 and 1-2 contains two monoanionic groups covalently linked with the cyclic organic ligand and being separated from each other by a sequence of at least two intervening atoms.

Preferably, each Y is independently selected from 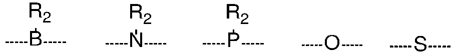 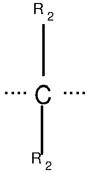 or each Y¹ is independently selected from 3 - to 15-membered, preferably 4- to 12-membered, cyclic organic groups that can contain 1 to 4 hetero atoms selected from N, B, P, O, S and is connected with each Z via a non-neighboring carbon or hetero atom, wherein R₂ is hydrogen or any covalently bound substituent being identical or different in each occurrence.

Preferably, each Y2 is independently selected from B, B-R₂⁻, N, P, C-R₂,
or each Y² is independently selected from 3- to 15-membered, preferably 6- to 12 membered cyclic organic groups that can contain one to four hetero atoms selected from N, B, P, O, S, and is connected with each Z via a non-neighboring carbon or hetero atom,
wherein R₂ is hydrogen or any covalently bound substituent being identical or different in each occurrence

Preferably, each Z contains at most one monoanionic group. In an alternative embodiment, one Z contains two monoanionic groups and the other Z, Y1 and Y2 do not contain monoanionic groups.

According to a further embodiment, Y1 or Y2 form the monoanionic groups.

According to one embodiment of the invention, the coordination compound comprises at least one divalent lanthanide coordinated by a cyclic organic ligand according to formula 3-1 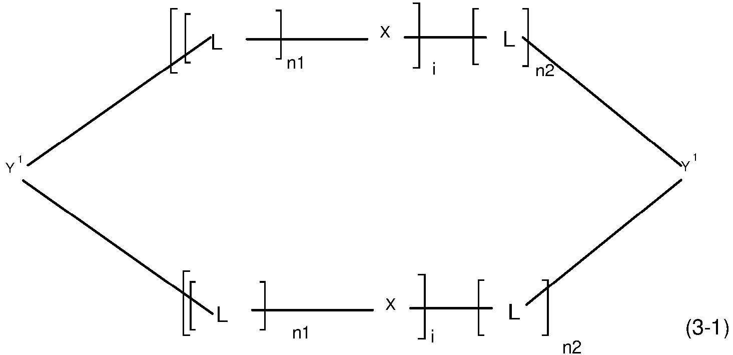 wherein
- Y¹ for each occurrence independently is a divalent organic group, preferably a divalent cyclic group that has two valences at different ring positions, or is 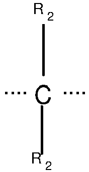 or according to formula 3-2 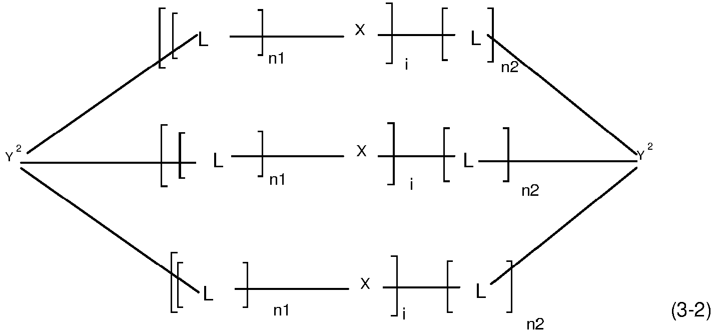 wherein
   - Y² for each occurrence independently is a trivalent organic group, preferably C-R₂ or a cyclic group that has three valences at different ring positions, or is B or B-R₂⁻ or N or P,
   - X is independently selected for each occurrence from the group of: 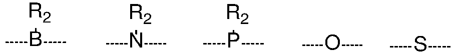
   - L for each occurrence independently is a divalent linear, branched or cyclic organic group that can be substituted and that is formed by removing two hydrogen atoms from an organic linear, branched or cyclic molecule that can be substituted, or is a divalent group -CR₁R₁-
   - wherein R₁ and R₂ are hydrogen or any covalently bound substituents being identical or different in each occurrence and
   - n1, n2, i independently are equal to 1, 2, 3 or 4,
   - wherein the cyclic organic ligand of formula 3-1 and 3-2 contain two monoanionic groups covalently linked with the cyclic organic ligand and being
separated from each other by a sequence of at least two intervening atoms. wherein in the divalent groups -CR₁R₁- the two R₁ can be covalently linked with each other, thereby forming a cyclic group, and in -CR₂R₂- the two R₂ can be covalently linked to form a cyclic structure.

Preferably, the compound of formula 3-2 has one or more of the following features:
- Y² is B or B-R₂⁻ or N, wherein preferably R₂ is alkyl, alkoxy, carboxy, aryl, aryloxy or F,
- all three structural elements 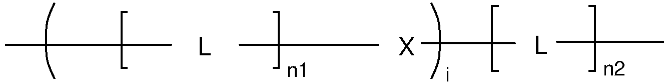 in the compound of formula 3-2 are identical, except for the presence of the monoanionic groups in one or two of the structural elements
- divalent cyclic organic groups L for each occurrence independently are divalent cyclic organic groups that can be substituted and that are formed by removing two hydrogen atoms from neighbouring carbon and/or nitrogen atoms in the ring of an organic cyclic molecule that can be substituted, n1 and n2 being 1,
- cyclic organic groups are carbocyclic or heterocyclic groups, the heteroatoms being selected from P, N, Si, O, S
- if in 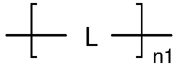 and/or 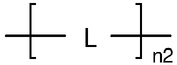 L is -CR¹R¹-, then n1 and/or n2 is 2, 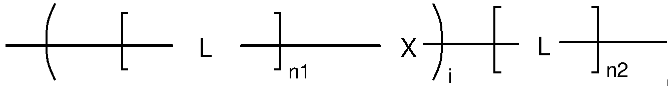 contains two groups X being 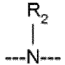 , wherein both R₂ together form a group 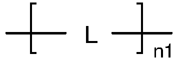 which is identical with group 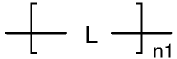 linking the two groups X,
- i is 2.

Preferably, the cyclic organic ligand has a structure according to formula 2a, 2c, 2e or 2f: 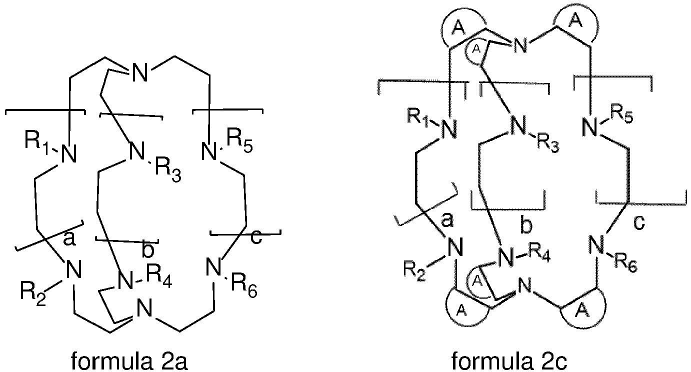 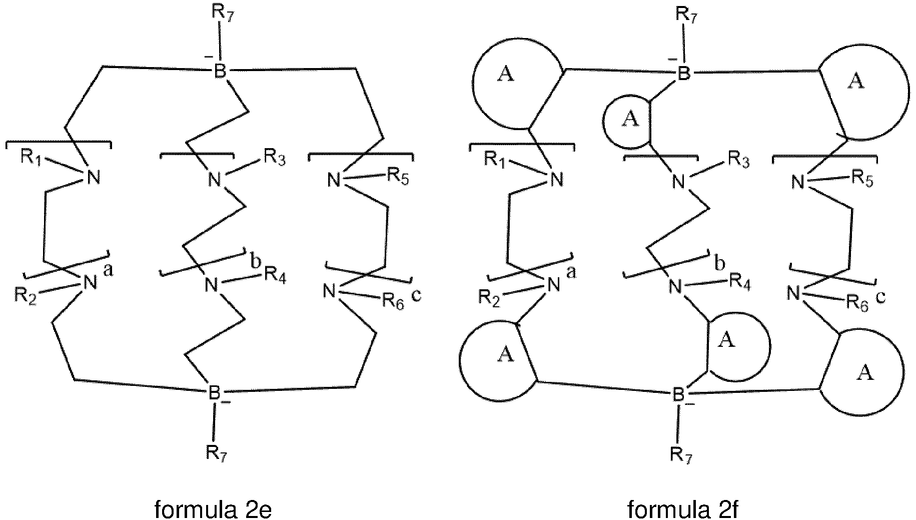 wherein in formula 2a and 2c,
R₁ or R₂ can be a monoanionic group,
or a ring carbon atom adjacent to N-R₁ or N-R₂ can be substituted by a monoanionic group,
R₃ or R₄ can be a monoanionic group, or a ring carbon atom adjacent to N-R₃ or N-R₄ can be substituted by a monoanionic group, or
R₅ or R₆ can be a monoanionic group, or a ring carbon atom adjacent to N-R₅ or N-R₆ can be substituted by a monoanionic group,
with a total of two monoanionic groups covalently linked with the cyclic organic ligand.

As an alternative, both anionic groups can be located in the same branch.

In these formulae, the monoanionic group preferably has the anionic charge in a β or γ position to the ring carbon or nitrogen atom to which the monoanionic group is covalently bound, or
according to a preferred embodiment, the monoanionic group has the structure -CH₂-CR'R'-X with X being a monoanionic atom selected from O⁻ and S⁻,
or has the structure 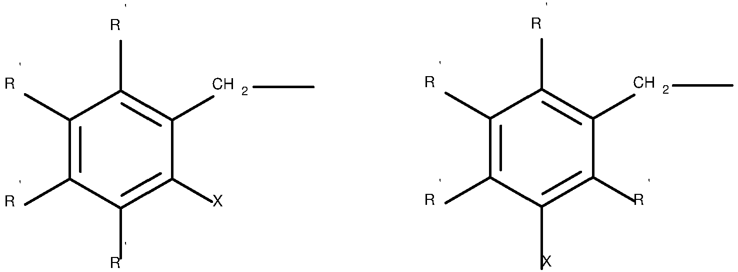
with X being a monoanionic atom selected from O⁻ and S⁻
with each R' independently being H, substituted or unsubstituted C₁₋₁₂-alkyl,
substituted or unsubstituted aryl, halogen, and wherein one R' of the monoanionic group can be NO₂, and wherein two groups R' can be covalently linked to form a cyclic group,
or is selected from the following structures 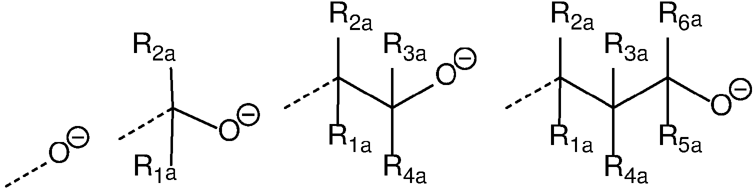 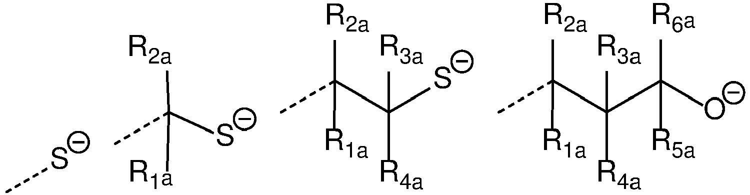 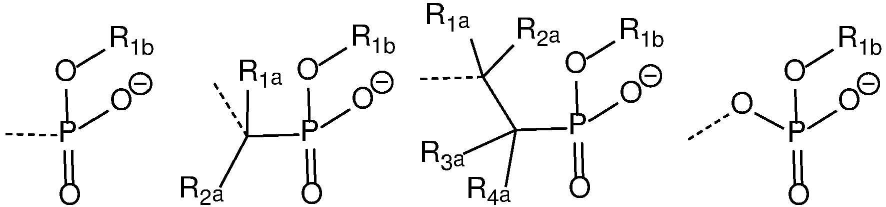 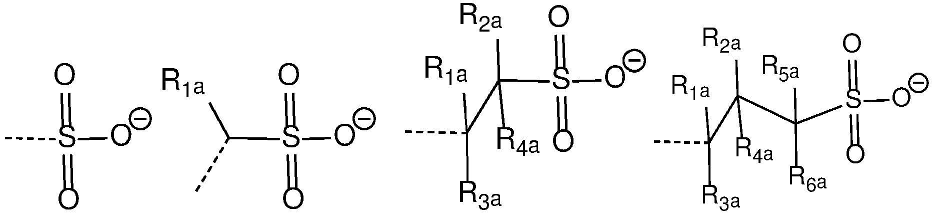 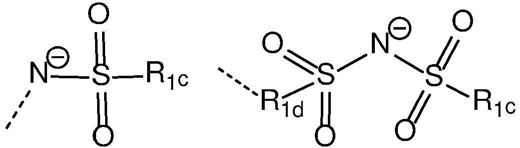 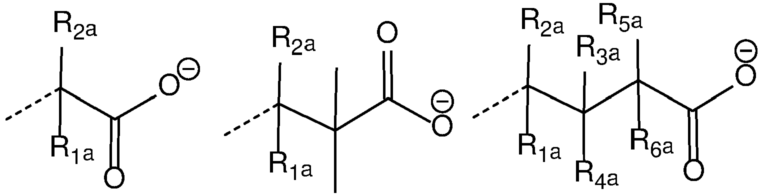 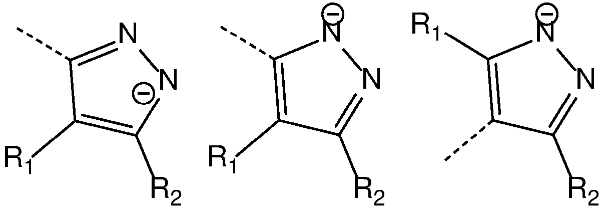 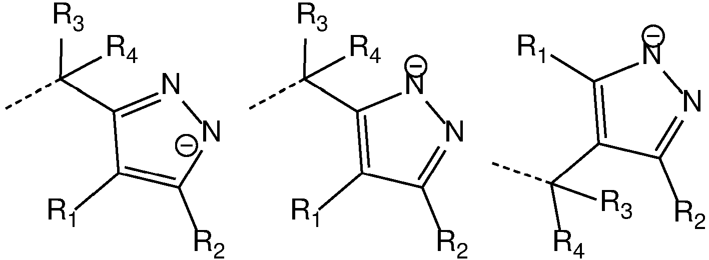 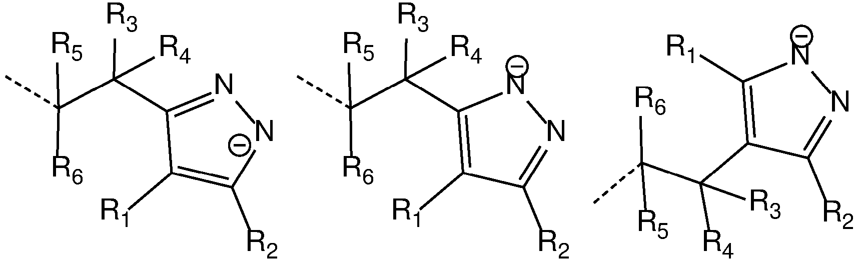 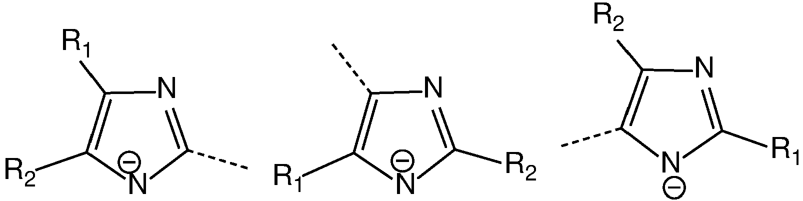 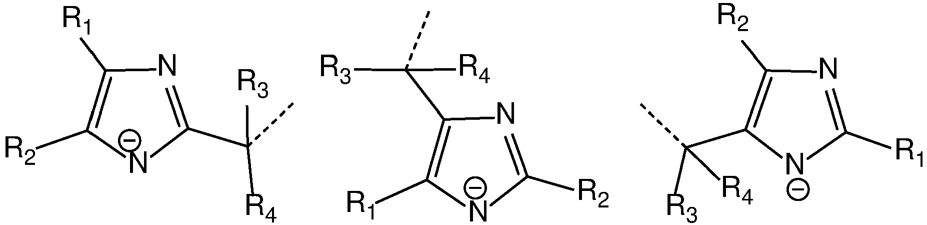 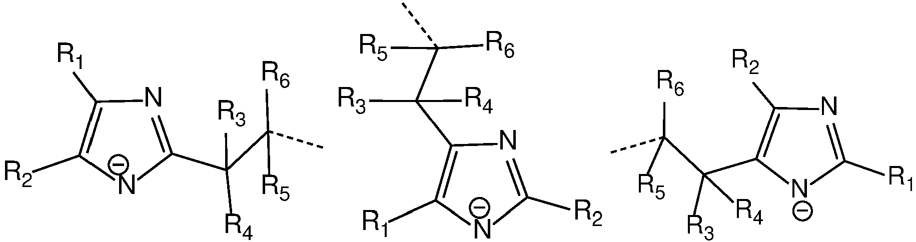 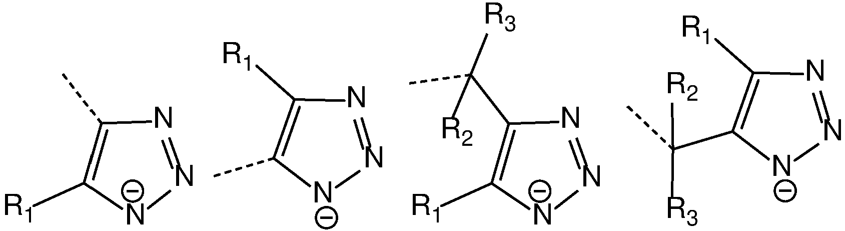 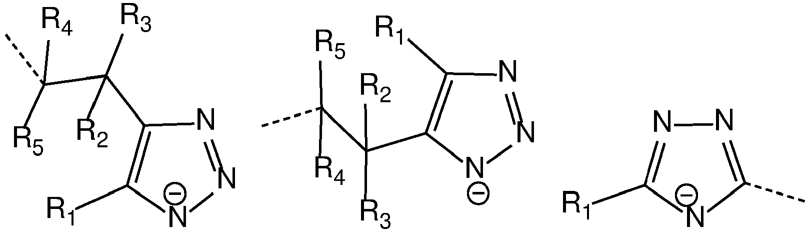 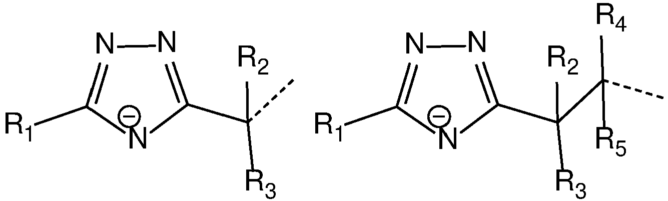 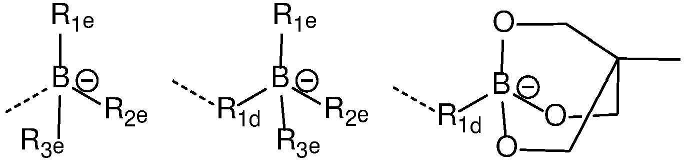 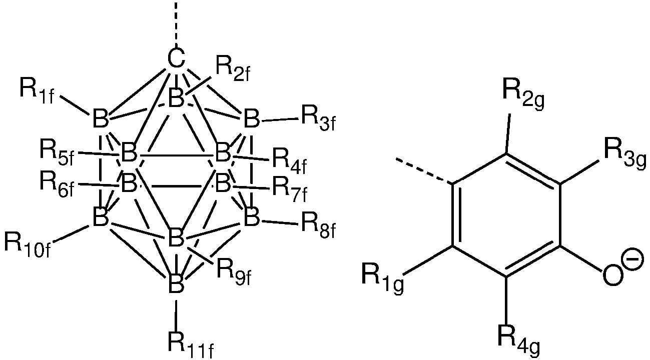 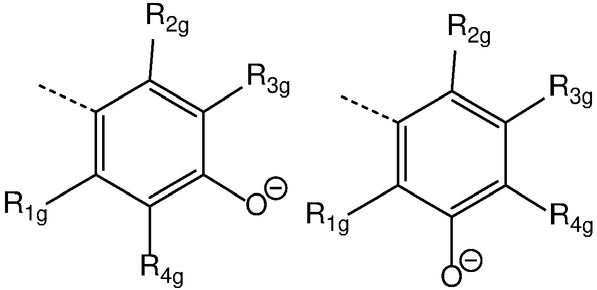 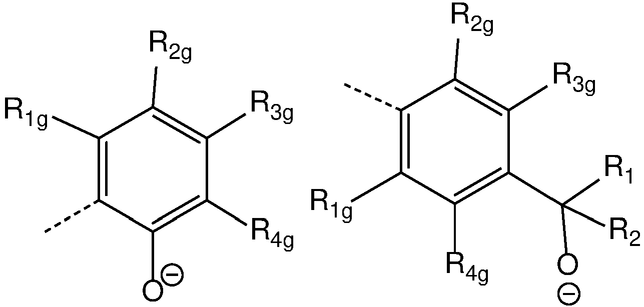 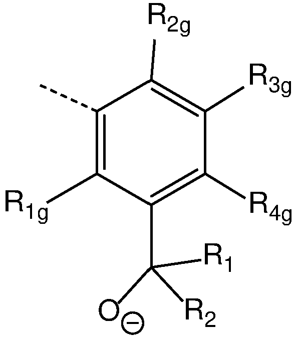 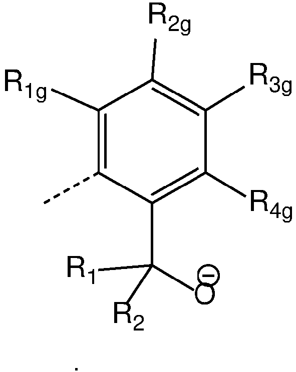 with
R₁ to R₆ independently being H or organyl,
R₁ₐ to R₆ₐ independently being H, CH₃, CF₃, CN, F or CₙH₂ₙ₊₁ with n being an integer of 1 to 5,
R_{1b} being CₙH₂ₙ₊₁ with n being an integer of 1 to 5,
R_{1c} being CH₃ or CF₃,
R_{1d} being a divalent organic fragment, preferably selected from alkylene, perfluoroalkylene, which optionally can be substituted,
R₁ₑ to R₃ₑ independently being a 5-membered heteroaryl group with up to 3 heteroatoms, selected from N, S, O, which optionally can be substituted,
or a 6-membered aryl or heteroaryl group which can contain up to 4 heteroatoms, selected from N, S, O, preferably from N, and which optionally can be substituted or a group selected from CF₃, F, H, OMe, OEt
R_{1f} to R_{11f} independently being H, Cl, Br, F, CH₃, CF₃
R_{1g} to R_{4g} independently being H, organyl, halogen, preferably F, CF₃, CN, OMe.

In preferred coordination compounds of the present invention, the cyclic organic ligand contains exactly two monoanionic groups and therefore is twice negatively charged and with a divalent lanthanide forms a complex of neutral charge.

In some embodiments, the cyclic organic ligand does not contain fluorine atoms or does not contain halogen atoms at all.

The object is furthermore achieved by a mixture comprising a second electrically neutral organic compound, and the coordination compound as defined above,
wherein the coordination compound is imbedded into the at least one second electrically neutral organic compound,
wherein the second organic compound has a triplet energy higher than 2.5 eV, preferably higher than 2.6 eV, more preferably higher than 2.7 eV and/or wherein the coordination compound has a higher hole affinity compared to the second organic compound.

The object is furthermore achieved by a compound comprising the coordination compound as defined above, and a polymer with a molecular weight Mn above 1000 g/mol, wherein the coordination compound is covalently attached to the polymer backbone.

The objects are furthermore achieved by a contrast enhancement medium for magnet resonance tomography (MRT), the contrast enhancement medium comprising the coordination compound as defined above.

The object is furthermore achieved by an organic electronic device comprising:
a first electrode;
a second electrode; and
an organic layer arranged such that it is electrically interposed between the first and second electrodes, wherein the organic layer comprises the coordination compound, the mixture or the compound as defined above.

Preferably, the organic electronic device is an optoelectronic device, the optoelectronic device being at least one of an organic light-emitting diode, an organic photo detector or a photovoltaic cell.

The object is furthermore achieved by a method of forming an organic device, the method comprising: Forming a layer of the coordination compound, the mixture or the compound as defined above, wherein the layer is deposited from a gas phase, in particular using an evaporation, carrier gas, and/or sublimation process, and/or by a solution-based process.

The metal-organic coordination compounds according to the present invention are preferably used in an organic electronic device, more preferably an organic light-emitting device, OLED.

For OLED applications it is preferred that the monoanionic groups are pointing inwardly in the cyclic organic ligand.

Without being restricted in any way by this theory, bulky anions may lead to the formation of blue light-emitting metal-organic coordination compounds, whereas small and hard anions like O- lead to green light-emitting metal-organic coordination compounds.

When two monoanionic groups are attached as side groups to the monocyclic, bicyclic or polycyclic organic ligand, often isomer mixtures of structural isomers are obtained. It is possible to obtain only one type of isomer or a high purity of one type of isomer by appropriate synthesis path routes or work-up sequences for cleaning the materials.

Structural isomers can contain the two monoanionic groups at different positions within one branch of a bicyclic or polycyclic organic ligand, or within different branches of a bicyclic or polycyclic ligand.

Figure 5 shows several examples of structural isomers of this type.

According to the present invention, bicyclic or polycyclic organic ligands are preferred over monocyclic ligands, most preferred are bicyclic ligands.

According to one embodiment of the invention, substituted phenyl groups are less preferred as anionic groups.

### Brief Description of the Drawings

In the drawings, like reference characters generally refer to the same parts throughout the different views. The drawings are not necessarily to scale, emphasis is instead generally being placed upon illustrating the principles of the invention. In some of the drawings, the monoanionic groups are not shown. If the monoanionic groups are shown in a particular chemical structures then usually only one structural isomer is depicted. While showing only one structural isomer, the compounds include or encompass all structural isomers of the shown structural isomer. For example, the structural isomers of the binding position of the monoanionic group inside the branch or inside the compound are included in the shown structure. In the following description, various aspects of the invention are described with reference to the following drawings, in which:
**FIG. 1A** to **FIG. 7A** illustrate various formulas of coordination compounds according to various aspects to which two monoanionic groups need to be covalently linked to form coordination compounds of the present invention;
**FIG. 8A** and **FIG. 8B** illustrate schematic cross sections of organic electronic devices according to various aspects;
**FIG.** 9 illustrates a flow diagram of a method for producing an organic electronic device according to various aspects; and
**FIG. 10** illustrates a flow diagram of a method for producing a coordination compound according to various aspects; and
**FIG. 11** illustrates a formula of a coordination compound according to various embodiments; and
**FIG. 12** illustrates a formula for producing a coordination compound according to various embodiments; and
**FIG. 13** and **FIG. 14** illustrate device characteristics of coordination compounds applied in OLED devices; and

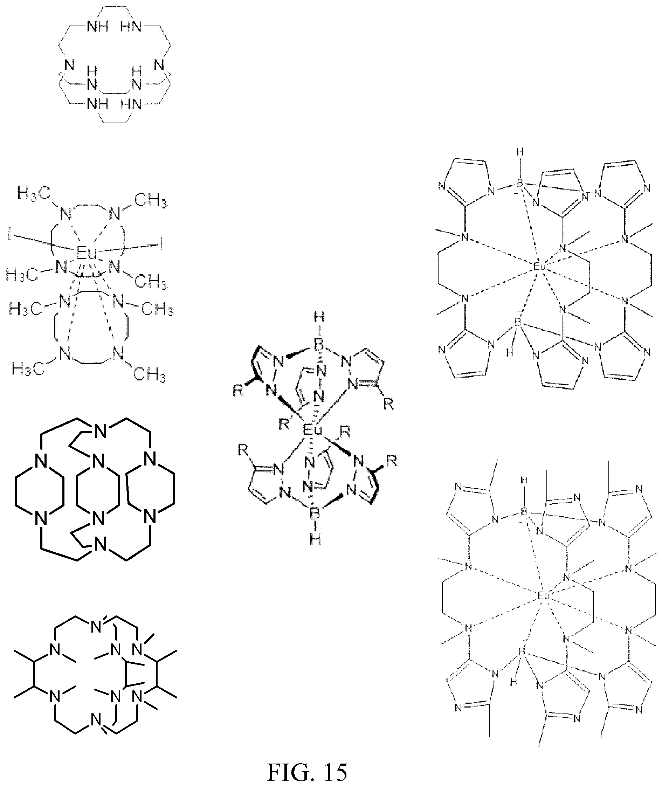

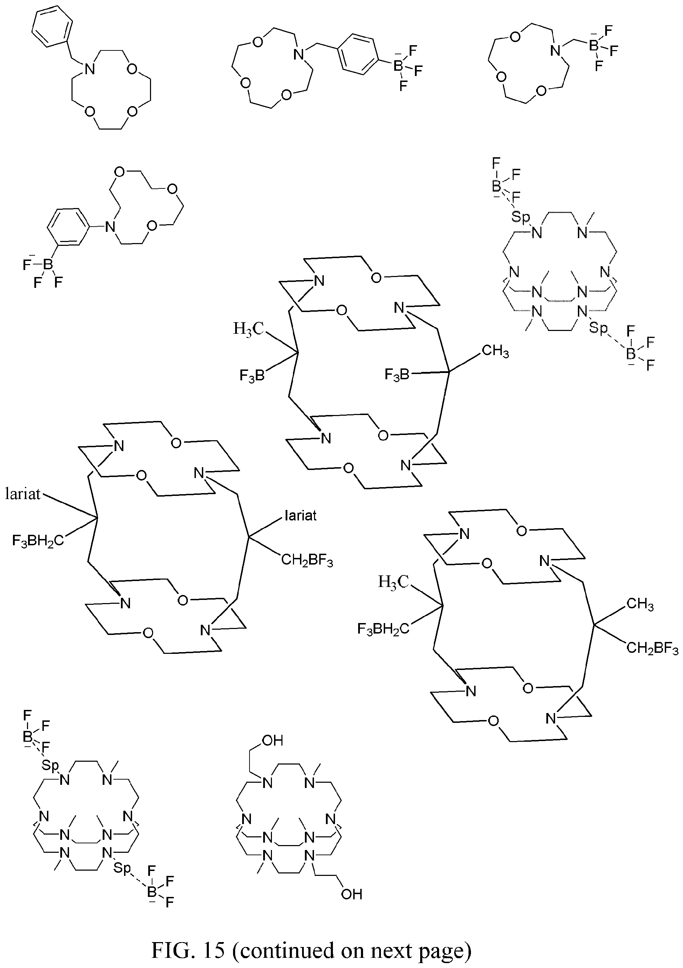

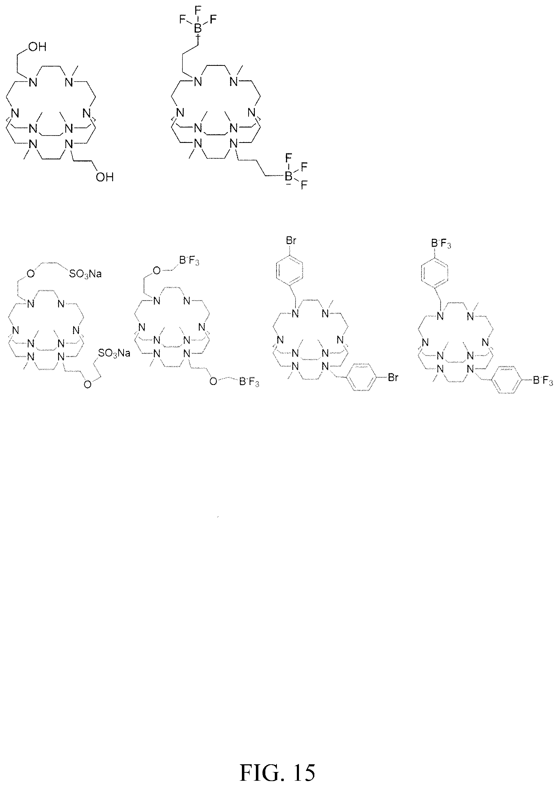

**FIG. 15** illustrates formulas of coordination compounds in examples of an organic electronic device.
**FIG. 16** shows preferred anionic ligands.

### Description

The following detailed description refers to the accompanying drawings that show, by way of illustration, specific details and aspects in which the invention may be practiced.

The word "exemplary" is used herein to mean "serving as an example, instance, or illustration". Any aspect or design described herein as "exemplary" is not necessarily to be construed as preferred or advantageous over other aspects or designs.

Various embodiments relate to metal organic compounds, including Yb(II) or Eu(II) coordinated with a macrocyclic organic ligand including a plurality of aliphatic amine groups and the applications of those compounds.

In this description, a coordination compound is taken to mean a compound where the central active metal is coordinated without a direct metal carbon bond.

In this description, an electroluminescent coordination compound is any material that is able to emit light upon electrical excitation, followed by recombination of electrons and holes. It shall be irrelevant in this context, whether the recombination of the electrons and holes takes place directly on the electroluminescent compound or first an excitation is formed on a different compound and subsequently transferred to the electroluminescent compound. Further, the electroluminescent coordination compound does not necessarily have to be used in an electronic device but, as example, may be used as a dye or a contrast enhancement medium for magnet resonance tomography.

The divalent lanthanide included in the coordination compound according to various embodiments may be any lanthanide cation that is twofold positively charged, e.g. Yb2+, Eu2+, and Sm2+, in particular Yb2+ and Eu2+.

The macrocyclic organic ligand according to various embodiments may be combined with actinides of divalent oxidation state. For example, Am2+ has a similar electronic configuration to Eu2+ and may therefore have similarly emission properties.

In this description, the arylene is a divalent organic fragment that is derived from an aromatic or heteroaromatic hydrocarbon by removing two hydrogen atoms from the aromatic or heteroaromatic hydrocarbon, preferably from different carbon and/or hetero atoms. One example is a (hetero) aromatic hydrocarbon that has had hydrogen atoms removed from two, preferably adjacent, hydrogen-bearing atoms (in case of aromatic hydrocarbon two carbon atoms, in case of heteroaromatic hydrocarbons two atoms selected from carbon and heteroatoms). An aromatic hydrocarbon or arene (or sometimes aryl hydrocarbon) is a hydrocarbon with sigma bonds and delocalized pi electrons between carbon atoms forming a circle. An alkylene is a divalent organic fragment that is derived from an alkyl group by removing one additional carbon atom.

The terms "halo," "halogen," and "halide" are used interchangeably and refer to fluorine, chlorine, bromine, and iodine.

The term "acyl" refers to a substituted carbonyl radical (C(O)-Rs).

The term "ester" refers to a substituted oxycarbonyl (-O-C(O)-Rs or - C(O)-O-Rs) radical.

The term "ether" refers to an -ORs radical.

The terms "sulfanyl" or "thio-ether" are used interchangeably and refer to a -SRs radical.

The term "sulfinyl" refers to a -S(O)-Rs radical.

The term "sulfonyl" refers to a -SO₂ -Rs radical.

The term "phosphino" refers to a -P(R_{S})₃ radical, wherein each Rs can be same or different.

The term "silyl" refers to a -Si(Rs)₃ radical, wherein each Rs can be same or different.

In each of the above, Rs can be hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, and combination thereof. Preferred Rs are selected from the group consisting of alkyl, cycloalkyl, aryl, heteroaryl, and combination thereof.

The term "alkyl" refers to and includes both straight and branched chain alkyl radicals. Preferred alkyl groups are those containing from one to fifteen, preferably one to ten, more preferably one to five carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl, and the like. Additionally, the alkyl group is optionally substituted, e.g. by halogen or cycloalkyl.

The term "cycloalkyl" refers to and includes monocyclic, polycyclic, and spiro alkyl radicals. Preferred cycloalkyl groups are those containing 3 to 12, preferably 3 to 8, more preferably 3 to 6 ring carbon atoms and includes cyclopropyl, cyclopentyl, cyclohexyl, bicyclo[3.1.1]heptyl, spiro[4.5]decyl, spiro[5.5]undecyl, adamantyl, and the like. Additionally, the cycloalkyl group is optionally substituted, e.g. by halogen, alkyl or heteroalkyl.

The terms "heteroalkyl" or "heterocycloalkyl" refer to an alkyl or a cycloalkyl radical, respectively, having at least one carbon atom replaced by a heteroatom. Preferably the at least one heteroatom is selected from O, S, N, P, B, Si and Se, more preferably O, S or N. Preferably 1 to 5, more preferably 1 to 3, most preferably 1 or 2 heteroatoms are present in the radical. The radical can be covalently linked with the remainder of the molecule via a carbon or heteroatom (e.g. N). Additionally, the heteroalkyl or heterocycloalkyl group is optionally substituted as indicated for alkyl and cycloalkyl.

The term "alkenyl" refers to and includes both straight and branched chain alkene radicals. Alkenyl groups are essentially alkyl groups with more than one carbon atom that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one, preferably 1 to 5, more preferably 1 to 3, most preferably 1 or 2 carbon atoms replaced by a heteroatom. Preferably, the at least one heteroatom is selected from O, S, N, P, B, Si, and Se, more preferably O, S, or N. Preferred alkenyl/cycloalkenyl/heteroalkenyl groups are those containing two/three/one to fifteen carbon atoms. Additionally, the alkenyl, cycloalkenyl, or heteroalkenyl group is optionally substituted, as indicated above.

The terms "aralkyl" or "arylalkyl" are used interchangeably and refer to an alkyl group that is substituted with an aryl group. Additionally, the aralkyl group is optionally substituted, as indicated for alkyl and aryl.

The term "heterocyclic group" refers to and includes aromatic and non-aromatic cyclic radicals containing at least one, preferably 1 to 5, more preferably 1 to 3, most preferably 1 or 2 heteroatom. Preferably the at least one heteroatom is selected from O, S, N, P, B, Si, and Se, more preferably O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Further preferred heterocyclic groups are carbazole groups and dibenzofuran groups. Additionally, the heterocyclic group may be optionally substituted, e.g. by halogen, alkyl or aryl. The heterocyclic group can be covalently linked with the remainder of the molecule via carbon and/or heteroatoms, preferably one carbon or nitrogen atom.

The term "aryl" refers to and includes both single-ring aromatic hydrocarbyl groups and polycyclic aromatic ring systems. The polycyclic rings may have two or more rings in which two carbons are common to two adjoining rings (the rings are "fused") or wherein one carbon is common to two adjoining rings (e.g. biphenyl) wherein at least one of the rings is an aromatic hydrocarbyl group, e.g., the other rings can be cycloalkyls, cycloalkenyls, aryl, heterocycles, and/or heteroaryls. Preferred aryl groups are those containing six to thirty carbon atoms, preferably six to twenty carbon atoms, more preferably six to twelve carbon atoms. Suitable aryl groups include phenyl, biphenyl, triphenyl, triphenylene, tetraphenylene, naphthalene, anthracene, phenalene, phenanthrene, fluorene, pyrene, chrysene, perylene, radialene and azulene. Most preferred is phenyl, that can be substituted by non-aromatic groups. Especially preferred is an aryl group having six carbons. Additionally, the aryl group is optionally substituted, e.g. by halogen, alkyl, heteroalkyl.

The term "heteroaryl" refers to and includes both single-ring aromatic groups and polycyclic aromatic ring systems that include at least one heteroatom. The heteroatoms include, but are not limited to and are preferably selected from O, S, N, P, B, Si, and Se. In many instances, O, S, or N are the preferred heteroatoms. Hetero-single ring aromatic systems are preferably single rings with 5 or 6 ring atoms, and the ring can have from one to five/six, preferably 1 to 3, more preferably 1 or 2 heteroatoms. The hetero-polycyclic ring systems can have two or more rings in which two atoms are common to two adjoining rings (the rings are "fused") or wherein one carbon is common to two adjoining rings (e.g. bipyridine) wherein at least one of the rings is a heteroaryl, e.g., the other rings can be cycloalkyls, cycloalkenyls, aryl, heterocycles, and/or heteroaryls. The hetero-polycyclic aromatic ring systems can have from 1 to 5, preferably 1 to 3, more preferably 1 or 2 heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzofuran, dibenzoselenophene, carbazole, imidazole, pyridine, triazine, 1,2-azaborine, 1,3-azaborine, 1,4-azaborine, borazine, and aza-analogs thereof, from which one hydrogen atom has been removed from a hydrogen-bearing carbon or heteroatom to form the covalent link to the remainder of the molecule. Additionally, the heteroaryl group is optionally substituted, e.g. by halogen, alkyl or aryl.

Of the aryl and heteroaryl groups listed above, the groups derived from benzene, furan, dibenzofuran, dibenzoselenophene, carbazole, imidazole, pyridine, pyrazine, pyrimidine, triazine, and the respective aza-analogs of each thereof are of particular interest.

The alkyl, cycloalkyl, heteroalkyl, heterocycloalkyl, alkenyl, cycloalkenyl, heteroalkenyl, alkynyl, aralkyl, heterocyclic group, aryl, and heteroaryl groups or residues, as used herein, are independently unsubstituted, or independently substituted, with one or more (general) substituents, preferably the substituents mentioned above.

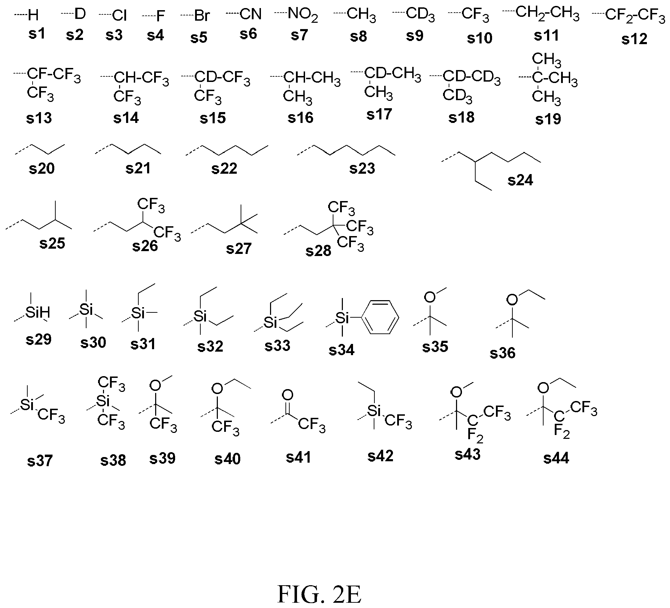

Preferably, the (general) substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof with the number of carbon atoms and heteroatoms as defined above for the respective term. Furthermore, one or two substituents can be selected from polymer chains which can be covalently linked with the remainder of the molecule by a suitable organic spacer. Therefore, the cyclic organic ligand can be covalently linked with a polymer chain or a polymer backbone. Fig. 2E shows possible substituents.

In some instances, the preferred general substituents are selected from the group consisting of deuterium, fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof with the number of carbon atoms and heteroatoms as defined above for the respective term.

In some instances, the preferred general substituents are selected from the group consisting of deuterium, fluorine, alkyl, cycloalkyl, alkoxy, aryloxy, amino, silyl, aryl, heteroaryl, sulfanyl, and combinations thereof with the number of carbon atoms and heteroatoms as defined above for the respective term.

In yet other instances, the more preferred general substituents are selected from the group consisting of deuterium, fluorine, alkyl, cycloalkyl, aryl, heteroaryl, and combinations thereof with the number of carbon atoms and heteroatoms as defined above for the respective term.

The terms "substituted" and "substitution" refer to a substituent other than H that is bonded to the relevant position, e.g., a carbon or nitrogen. For example, when R1 represents mono-substitution, then one R1 must be other than H (i.e., a substitution). Similarly, when R1 represents di-substitution, then two of R1 must be other than H. Similarly, when R1 represents no substitution, R1, for example, can be a hydrogen for available valencies of straight or branched chain or ring atoms, as in carbon atoms for benzene and the nitrogen atom in pyrrole, or simply represents nothing for ring atoms with fully filled valencies, e.g., the nitrogen atom in pyridine. The maximum number of substitutions possible in a straight or branched chain or ring structure will depend on the total number of available valencies in the ring atoms or number of hydrogen atoms that can be replaced. All residues and substituents are selected in a way that a chemically stable and accessible chemical group results.

As used herein, "combinations thereof" indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include up to forty atoms that are not hydrogen or deuterium, or those that include up to thirty atoms that are not hydrogen or deuterium counted for all substituents of a given molecule, or for the respective molecule in total. In many instances, a preferred combination of substituent groups will include up to twenty atoms that are not hydrogen or deuterium, counted for all substituents of a given molecule.

The "aza" designation in the fragments described herein, i.e. aza-cryptate, etc. means that one or more carbon atom or (other) heteroatom of a parent compound is replaced by a nitrogen atom, without any limitation. For example, in a crown ether -O- is replaced by -NH- to give the respective aza compound. One of ordinary skill in the art can readily envision other nitrogen analogs of the aza-derivatives, and all such analogs are intended to be encompassed by the terms as set forth herein.

As used herein, "deuterium" refers to an isotope of hydrogen. Deuterated compounds can be readily prepared using methods known in the art.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

In some instances, a pair of adjacent or non-adjacent substituents or residues can be optionally joined (i.e. covalently linked with each other) or fused into a ring. The preferred ring formed therewith is a five-, six-, or seven-membered carbocyclic or heterocyclic ring, including both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2, 2' positions in a biphenyl, or 1, 8 position in a naphthalene, or 2,3-positions in a phenyl, or 1,2-positions in a piperidine, as long as they can form a stable fused ring system.

Preferably, the divalent Lanthanide is Europium or Ytterbium.

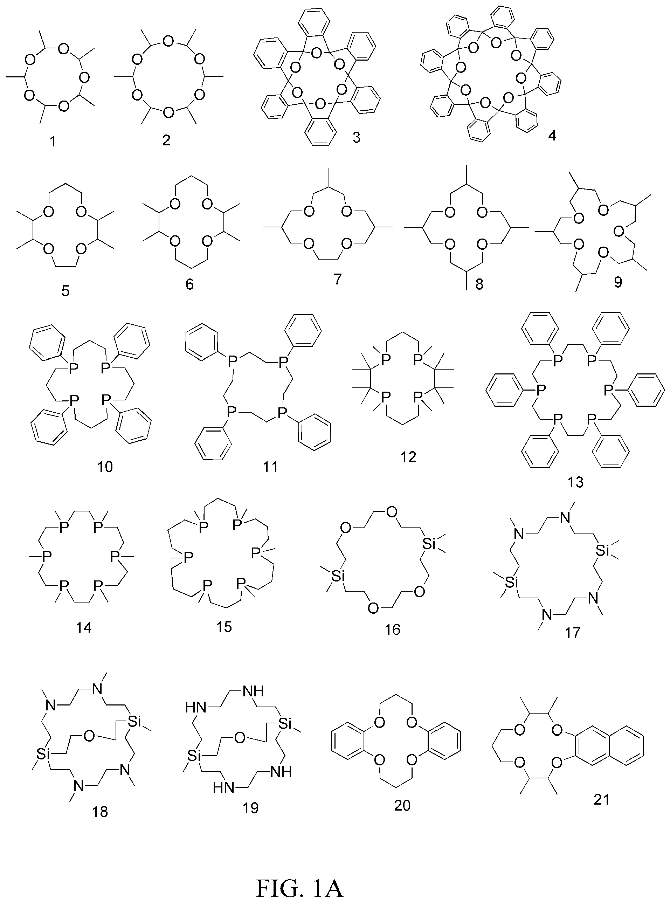

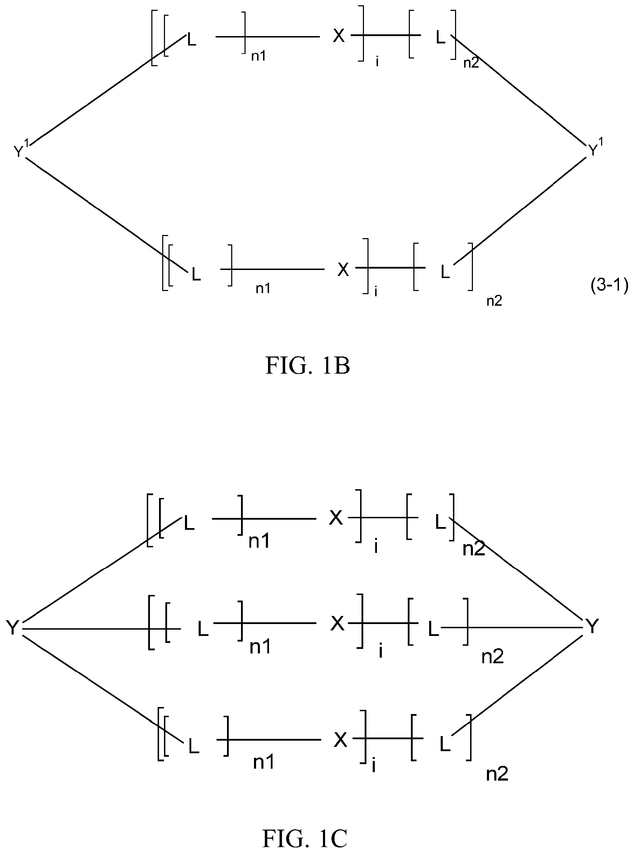

In various embodiments, the coordination compound may contain any organic ligand according to FIG. 1A. **FIG. 1A** illustrates examples 1 to 21 of the organic ligand according to various embodiments without limitation. FIG 1B and 1C illustrate organic ligands according to the present invention.

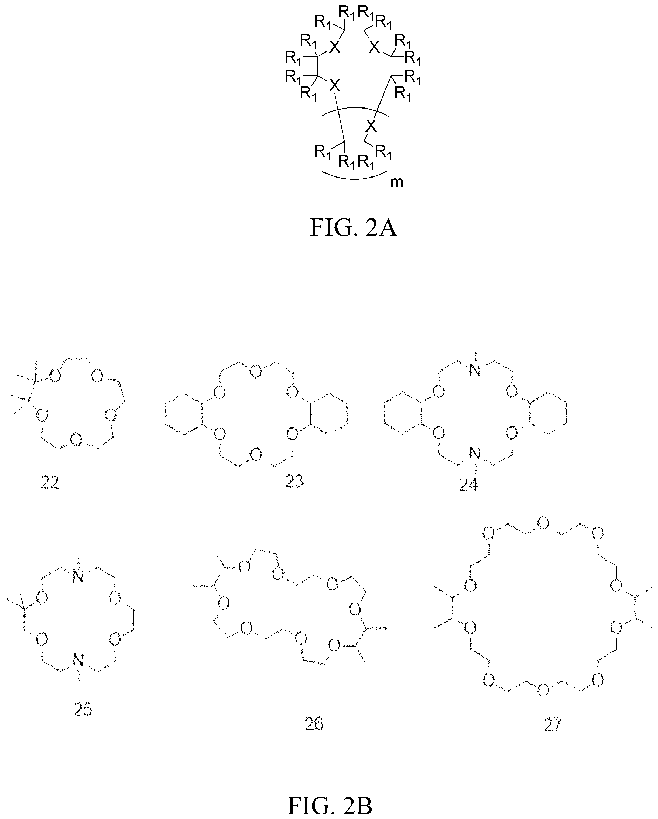

In various embodiments, the coordination compound may include organic ligands described by the generic formula illustrated in **FIG. 2A****.** Here, m preferably is an integer from 1 to 15, X is independently in each occurrence O or N-R₂, and R₁ and R₂ may be the same or different in each occurrence. Specific examples according to FIG. 2A, which shall, however, not limit the full scope of the possible set of materials, are compounds 22 to 27 illustrated in **FIG. 2B****.**

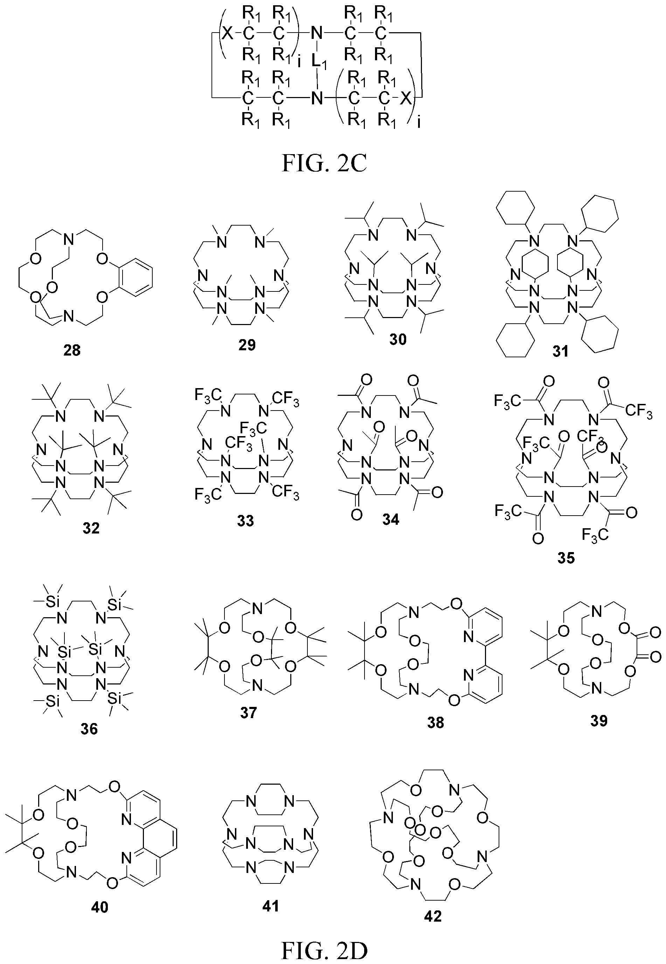

In various preferred embodiments, the coordination compounds include cyclic organic ligands which are polycyclic ligands also known as cryptands. Examples are described by generic formula illustrated in **FIG. 2C****.**

Here, L₁ represents the linker, formed from two R₂ substituents, connected to each other at any position. Specific examples according to the formula illustrated in FIG. 2C, which shall not limit the scope, are compounds 28 to 42 illustrated in **FIG. 2D****.**

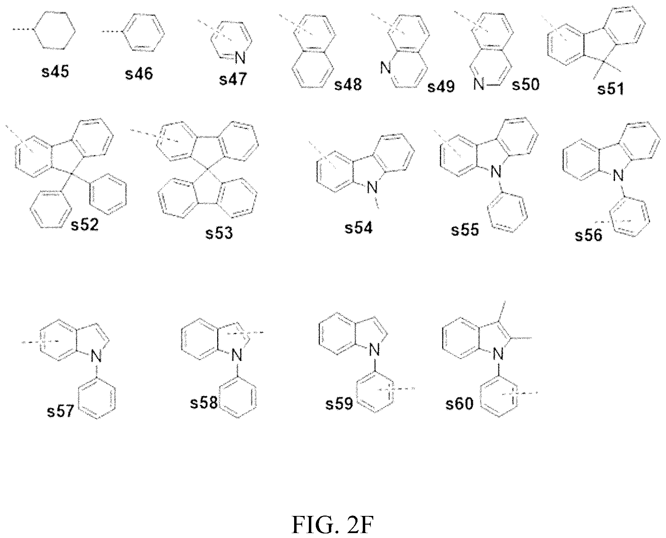

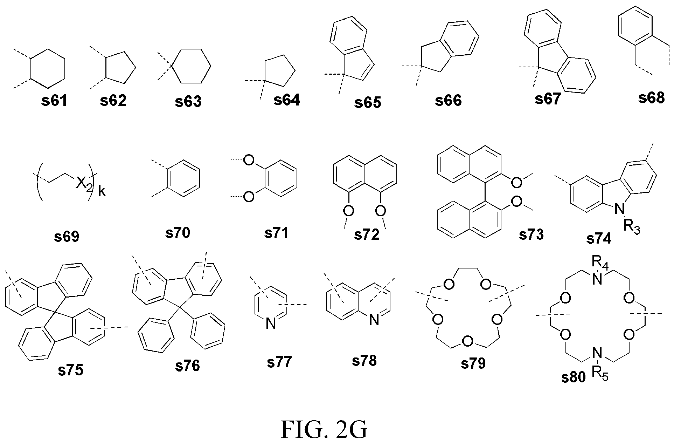

The coordination compound according to various embodiments may contain a ligand according to formula (1-1), (1-2), (3-1) or (3-2) with a plurality of substituents, which are not equal to hydrogen. Preferably 0 to 18, more preferably 0 to 12 or 0 to 6 of these substituents can be present, for example 1 to 18, 1 to 12 or 1 to 6 substituents. Those substituents may be any chemical fragment that can be covalently attached in accordance to the formula (1-1), (1-2), (3-1) or (3-2) . Examples of substituents s1 to s80 are illustrated in **FIG. 2E****,** **FIG. 2F** and **FIG. 2G****.**

Here, dashed lines may show the preferred covalent attachment to the macrocyclic ligand backbone according to the formula (1-1), (1-2), (3-1) or (3-2). In various embodiments, the substituents may form any cyclic bridges with each other, and examples, which however shall not limit the scope, are illustrated in FIG. 2F and FIG. 2G.

In FIG. 2F and FIG. 2G, a dashed line may represent the connection of the shown molecular fragment to the macrocyclic backbone of formula (1-1), (1-2), (3-1) or (3-2). Furthermore, X₂ may be selected from O or N-R₆ and k may be an integer between 4 and 20 and R₃ to R₆ are selected independently in each occurrence from hydrogen, deuterium, in various embodiments substituted C1-C10 linear or branched alkyl, perfluorinated alkyl, partially fluorinated alkyl, in various embodiments substituted aryl, in various embodiments substituted heteroaryl, perfluorinated aryl, partially fluorinated aryl, in various embodiments substituted cycloalkyl, in various embodiments substituted alkenyl, in various embodiments substituted alkynyl.

In various embodiments, the substituents include an anionic group.

In various embodiments, the organic ligand illustrated in FIG. 1A to C may be configured to include two singly charged anions.

A light emitting chemical cell may be an embodiment of an OLED in this description. Drift of charged species within a device including the organic electroluminescent coordination compound according to the formula illustrated in FIG. 1A or B can be avoided according to the present invention. By making the metal-organic coordination compound neutrally charged, a drift in the electrical field of an OLED can be prevented, leading to an improved stability of the OLED device. For applications that require fast response times, for example flat panel displays, ion drift may lead to time dependent OLED characteristics, which may be difficult to control, and may not be desirable as such. The coordination compounds of the present invention do not show this type of ion drift. The choice of the covalently linked anions may depend strongly on the application. Even without anion drift, a coordination compound according to various embodiments may exhibit an exceptionally large dipole moment. By including the two mono-anionic groups in the coordination compound by covalently linking them with a cyclic organic ligand, an unwanted polarity (quadrupole or dipole) can be reduced. The emitter material preferably has a molecular instead of salt-like character and can be easily mixed with neutral substantially organic matrices. For forming OLEDs, the improved solubility in nonpolar solvent and the better sublimation behavior can be advantageous.

Preferably, as shown in FIG. 1B and C, in the metal-organic coordination compound, in which the coordination compound comprises at least one divalent lanthanide coordinated by a cyclic organic ligand, the cyclic organic ligand has the formula 3-1 or 3-2: 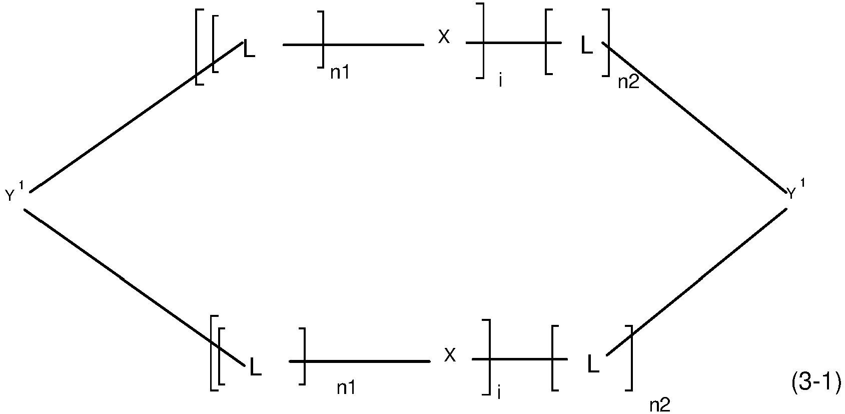 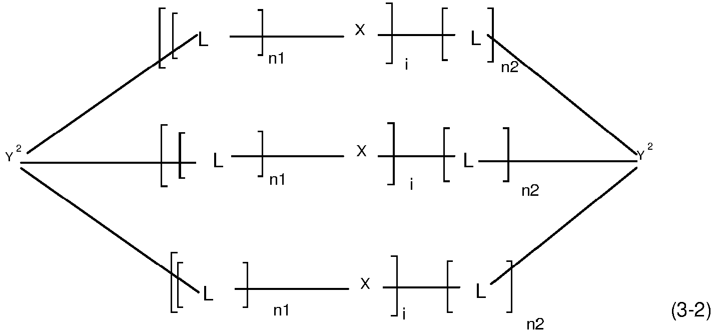 wherein
- Y² for each occurrence independently is a trivalent organic group, preferably C-R₂ or a cyclic group that has three valences at different ring positions, or is B or B-R₂⁻ or N or P,
- X is independently selected for each occurrence from the group of: 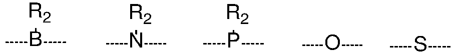
- L for each occurrence independently is a divalent linear, branched or cyclic organic group that can be substituted and that is formed by removing two hydrogen atoms from an organic linear, branched or cyclic molecule that can be substituted, or is a divalent group -CR₁R₁-
- wherein R₁ and R₂ are hydrogen or any covalently bound substituents being identical or different in each occurrence and
- n1, n2, i independently are equal to 1, 2, 3 or 4,
- wherein the cyclic organic ligand of formula 3-1 and 3-2 contains two monoanionic groups covalently linked with the cyclic organic ligand and being separated from each other by a sequence of at least two intervening atoms.
wherein in the divalent groups -CR₁R₁- the two R₁ can be covalently linked with each other, thereby forming a cyclic group, and in -CR₂R₂- the two R₂ can be covalently linked to form a cyclic structure.

Preferably L for each occurrence independently is a divalent cyclic organic group that can be substitued and that is formed by removing two hydrogen atoms from neighboring carbon and/or nitrogen atoms in the ring.

Preferably, n1 and n2 are 1 or 2, more preferably 1, so that the sum of n1+n2 is 2, 3, or 4, more preferably 2 or 3, specifically 2.

Preferably, the compound of formula 3-2 has one or more of the following features:
- Y² is B or B-R₂⁻ with R₂ being alkyl, alkoxy, carboxy, aryl, aryloxy or F, or B-H⁻ or N,
- all three structural elements 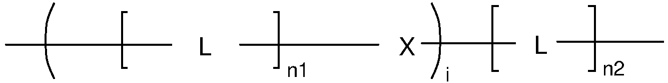 in the compound of formula 3-2 are identical, except for the presence of the monoanionic groups in one or two of the structural elements,
- divalent cyclic organic groups L for each occurance independently are divalent cyclic organic groups that can be substituted and that are formed by removing two hydrogen atoms from neighbouring carbon and/or nitrogen atoms in the ring of an organic cyclic molecule that can be substituted, n1 and n2 being 1,
- cyclic organic groups are carbocyclic or heterocyclic groups, the heteroatoms being selected from P, N, Si, O, S
- if in 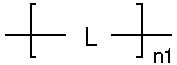 and/or 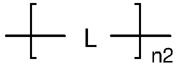 L is -CR¹R¹-, then n1 and/or n2 are 2, 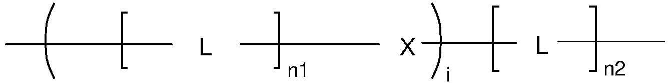 contains two groups X being 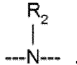 wherein both R₂ together form a group 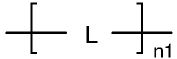 which is identical with group 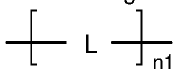 linking the two groups X,
- i is 2.

Therein, any number of the above features can be combined to form a preferred subset of compounds of formula 3. For example, 2, 3 or 4 of the features can be combined to give a more preferred compound of formula 3, which has to include two monoanionic groups.

This way, oxidation of the atypical divalent into the common trivalent oxidation state is prevented for the lanthanide.

Thus, a metal organic coordination compound is provided that is highly stabilized against oxidation such that processing at ambient conditions becomes possible, but at the same time refraining from using soft coordinating ligands such that the beneficial intra-metallic optical transitions of the central metal ion are not shifted into the red spectral region. Illustratively, this is achieved by creating a hard, nitrogen-based coordination sphere for the central divalent ion, and simultaneously preventing oxidation using a plurality of bulky aliphatic substitutions.

Thus, problems according to the related art associated with delocalized excitations are elegantly circumvented using atomic emitters, where the excitation resides substantially on a single atom, giving rise to an atomic excited state. Thus, chemical degradation of the emitting atom itself by excitation is avoided. In various embodiments, the single atom is sufficiently heavy such that both, spin 0 and spin 1 excitations contribute to light emission by means of the heavy metal effect.

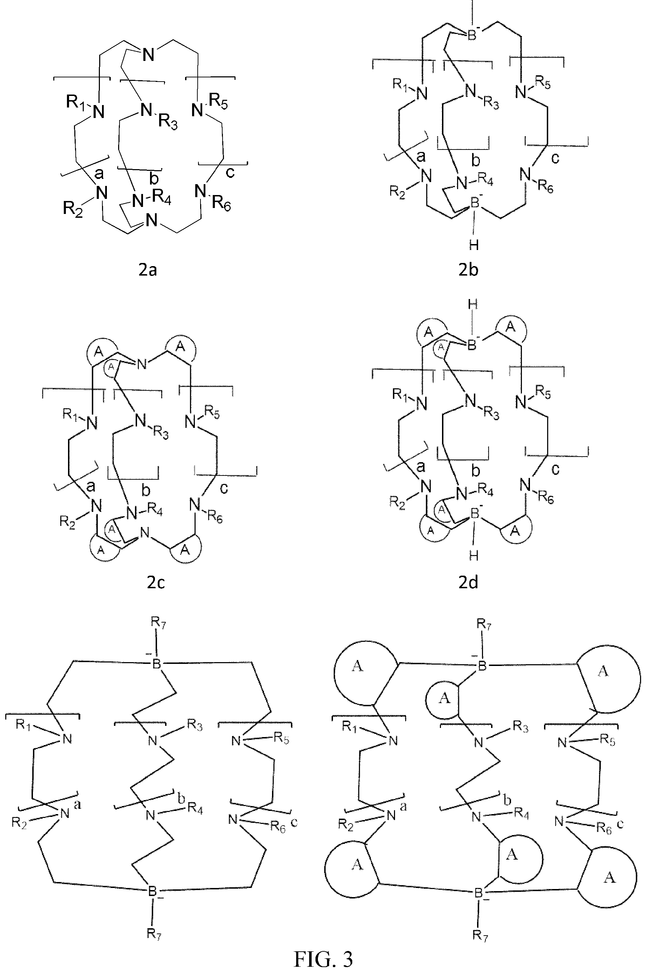

Preferably, the coordination compound includes Eu²⁺ or Yb²⁺ and cyclic organic ligands that coordinate to Eu²⁺ or Yb²⁺. In other words, Ytterbium or Europium in oxidation state +2 preferably are coordinated by an organic ligand represented by the formula illustrated in FIG. 3. Thus, **FIG. 3** illustrates, preferably in formula 2a, an embodiment of the cyclic organic ligand illustrated in FIG. 1A-FIG. 2K according to the present invention. Preferably, the cyclic organic ligand of formula 1 has a structure according to formula 2a, 2c, 2e or 2f, as shown in FIG. 3: 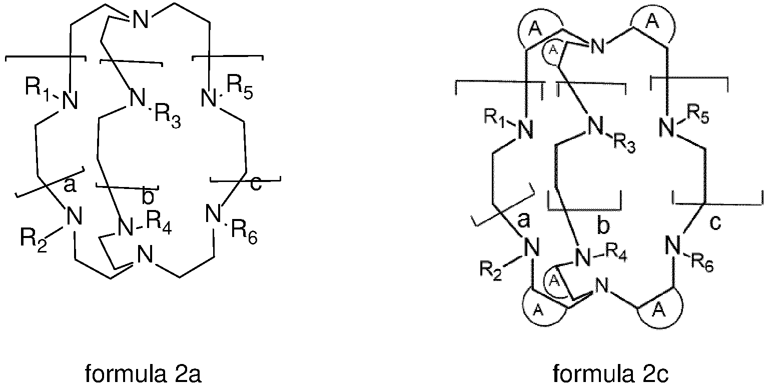 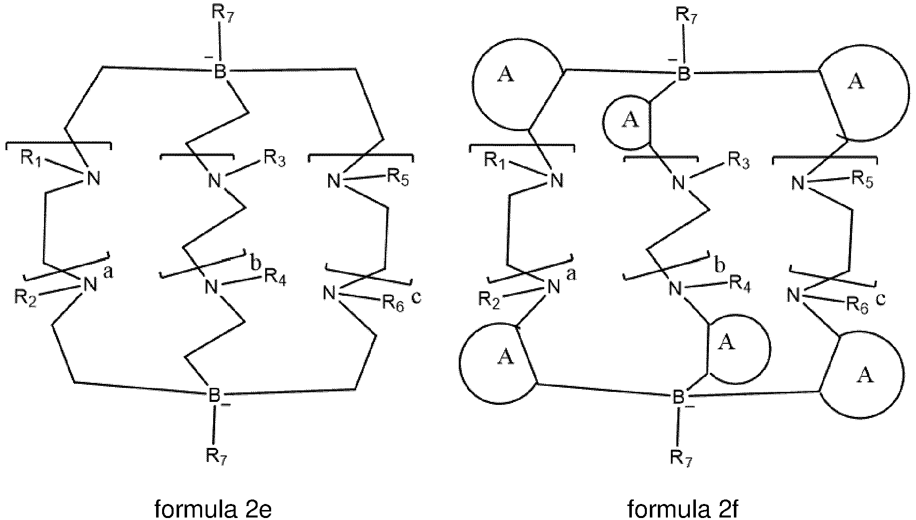 wherein
- R₁, R₂, R₃, R₄, R₅, R₆ in formula 2a and 2c independently in each occurrence represent hydrogen or a substituent, and R₁, R₂, R₃, R₄, R₅, R₆, R₇ in formula 2e, 2f independently in each occurrence represent hydrogen or a substituent,
- **a, b, c** are each independently an integer of 0 or more,
- 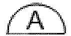 independently in each occurrence represents a divalent cyclic organic group, wherein one or more of the ring-forming carbon atoms can be substituted or can be part of a cyclic group,
wherein the cyclic organic ligand of formula 2a and 2c contains two monoanionic groups covalently linked with the cyclic organic ligand and being separated from each other by a sequence of at least two intervening atoms.

Preferably, the monoanionic group has the anionic charge in a β- or γ-position to the ring carbon or nitrogen atom to which the monoanionic group is covalently bound, or wherein in formula 2a and 2c,
R₁ or R₂ can be a monoanionic group,
or a ring carbon atom adjacent to N-R₁ or N-R₂ can be substituted by a monoanionic group,
R₃ or R₄ can be a monoanionic group, or a ring carbon atom adjacent to N-R₃ or N-R₄ can be substituted by a monoanionic group, or
R₅ or R₆ can be a monoanionic group, or a ring carbon atom adjacent to N-R₅ or N-R₆ can be substituted by a monoanionic group,
with a total of two monoanionic groups covalently linked with the cyclic organic ligand.

For example, R₁, R₂, R₃, R₄, R₅, R₆ in formula 2a independently in each occurrence represent an organyl group, comprising at least one carbon atom, preferably at least one additional atom different from hydrogen, more preferably at least two carbon atoms, and R₁, R₂, R₃, R₄, R₅, R₆, R₇ in formula 2b, 2c, 2d, 2e, 2f independently in each occurrence represent hydrogen or an organyl group, comprising at least one carbon atom
- **a, b, c** are each independently an integer of 0 or more.
- 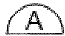 independently in each occurrence represents a divalent cyclic organic group.

For example, alternatively R₁, R₂, R₃, R₄, R₅, R₆ independently in each occurrence can represent an organoheteryl group. Further, a, b, c are each independently an integer of 0 or more. As example, a, b and c are each equal to 1.

Thus, the term (electroluminescent) coordination compound may be describing molecules, including metal cations that may be bound to a charged ligand represented by the formula illustrated in FIG. 3.

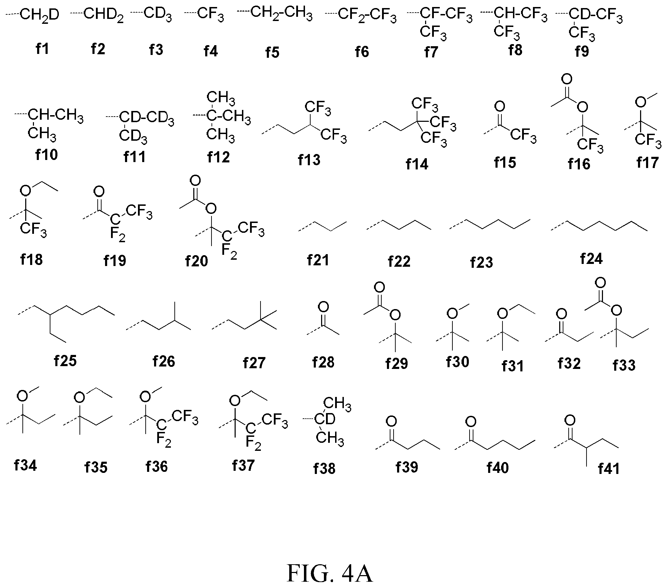

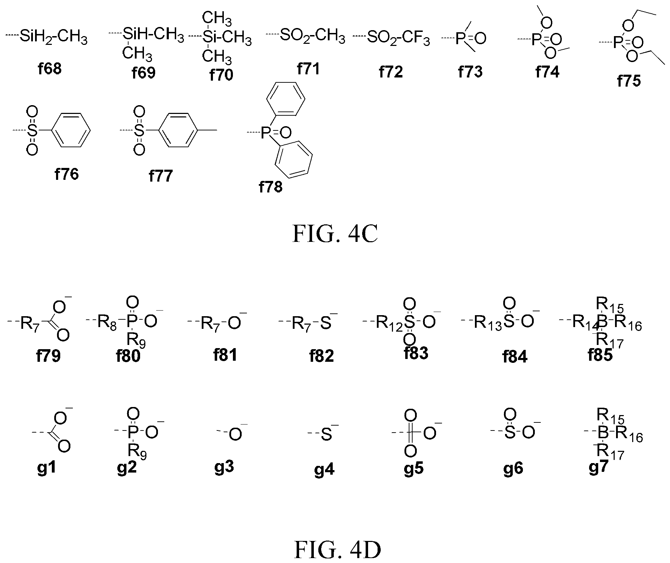

The term organoheteryl group as used herein defines an univalent group including one or more carbon atoms and one or more heteroatoms, which will be thus organic, but which has its free valence at an atom other than carbon. FIG. 4C illustrates the examples of organoheteryl groups f68 to f78, wherein a dashed line may represent preferred connection points. In other words, **FIG. 4A to FIG. 4C** illustrate chemical formulas of R₁, R₂, R₃, R₄, R₅, R₆ of the cyclic organic ligand illustrated in FIG. 3, preferably formula 2a. R₁, R₂, R₃, R₄, R₅, R₆ may be in each occurrence independently selected from the group of f1 to f78.

The term organyl group as used herein defines an organic substituent group, regardless of functional type and constitution, having one free valence at a carbon atom and thus containing at least one carbon atom. Preferably, it contains at least one additional atom, which is different from hydrogen.

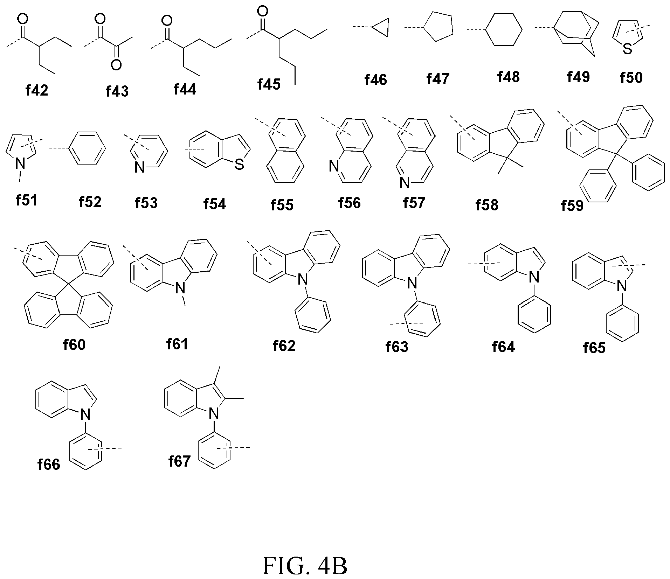

The organyl group preferably includes one to 25 carbon atoms, more preferably the organyl group includes 2 to 25 carbon atoms, more preferably the organyl group includes 3 to 25 carbon atoms. **FIG. 4A** and **FIG. 4B** illustrate examples of organyl group f1 to f67, wherein a dashed line may represent preferred connection points.

The value of a, b, c in the formula illustrated in FIG. 3, preferably formula 2a, is preferably an integer of from 0 to 5, preferably 0 to 3, more preferably 0 to 1. In one embodiment of the invention, the organyl or organoheteryl group includes a charged group, providing a negative monoanionic charge to the ligand. Examples of charged groups are presented in **FIG. 4D****.** Here, a dashed line represents preferred connection points. R₇, R₈, R₁₀, R₁₁, R₁₂, R₁₃, R₁₄ represent divalent groups formed by removing two hydrogen atoms from (preferably substituted) alkanes, or (preferably substituted) arenes or (preferably substituted) heteroarenes and the free valencies of which are not engaged in a double bond. For example, the coordination compound comprises two monoanionic groups, which are covalently bound to the organic ligand, wherein the negatively charged anion is at least one selected from the group of g1 to g7 and f79 to f85, wherein g1 to g7 and f79 to f85 are 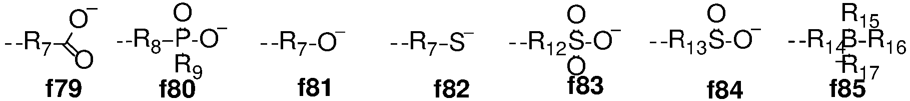 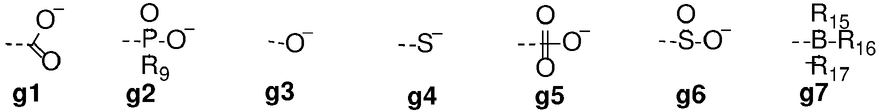 wherein
- a dashed line represents a connection point with the organic ligand. R₇, R₈, R₁₂, R₁₃, R₁₄ represent the divalent groups formed by removing of two hydrogen atoms from, preferably substituted, alkanes, or, preferably substituted, arenes or, preferably substituted, heteroarenes and the free valencies of which are not engaged in a double bond,
- R₉, R₁₅, R₁₆, R₁₇ represent a monovalent group formed by removing of one hydrogen atom from, preferably substituted, alkanes, or, preferably substituted, arenes, or, preferably substituted, heteroarenes.

The term "substituted" as used herein means that a hydrogen atom, attached to the parent structure is replaced by a non-hydrogen atom or by a group of atoms forming a chemical group.

**Fig 5** shows several metal-organic coordination compounds according to the present invention. The compounds are exemplary for the concept of the present invention wherein two monoanionic groups are covalently linked with a cyclic organic ligand.

The compounds shown have a polycyclic group to which the two monoanionic groups are covalently linked.

It is to be understood that the monoanionic groups can be freely exchanged between the different bicyclic or polycyclic structures. Furthermore, the cyclic organic ligand can contain further substituents, specifically organyl groups, organoheteryl groups, aryl groups, heteroaryl groups, heterocyclic groups, alkenyl groups, heteroalkyl groups, hetero cycloalkyl groups, cycloalkyl groups, alkyl groups or combinations thereof. Furthermore, halogen substituents may be present in the different compounds.

Preferably, the cyclic organic ligand is a bicyclic or polycyclic organic ligand. This bicyclic or polycyclic organic ligand is preferably selected from the bicyclic or polycyclic groups shown in structures 4, 112, 230, 327, 477, 509, 549, 624, 647, 654, 660, 672, 678, 684, 690, 702, 708, 714, 756, 762.

In any of these shown structures, one or more substituents can be added or removed. Furthermore, covalently linked monoanionic groups can be exchanged by other monoanionic groups. Furthermore, the position at which the monoanionic groups are linked with the bicyclic or polycyclic structure can be changed. Therefore, the bicyclic or polycyclic organic core is preferred with being bound to the specific compound depicted.

For example, in structure 762, the hydrogens shown can be replaced by substituents. Furthermore, one or more hydrogen atoms of the carbocyclic groups can be exchanged by substituents.

In the structure 509, for example, the position at which the two monoanionic groups are covalently linked can be changed within the cyclic residue. Furthermore, substituents may replace the shown hydrogen atoms or hydrogen atoms attached to carbon atoms. The monoanionic groups can be freely chosen from all possible covalently linked monoanionic groups, for example as shown in structures 510, 511 ff.

The above-listed structures are exemplary for the core bicyclic or polycyclic structure, to which the two monoanionic groups are covalently linked, and not limited to the specific compound shown. For example, in structure 4, the two substituents on the left hand side bearing the monoanionic groups can be positioned in different locations of the cyclic structure and can be replaced by other monoanionic groups. Further substituents can be present.

On the other hand, structure 230 shows methyl substituents on ring carbon atoms. These methyl substituents can be replaced by hydrogen or other substituents, and the two shown monoanionic groups can be attached to different positions of the cyclic organic ligand.

Therefore, the present invention will encompass any possible combination of bicyclic or polycyclic ring structures, monoanionic groups and substituents shown in Figure 5.

According to one embodiment, the coordination components according to the present invention only contain the bicyclic or polycyclic structure shown in any of the formulae in combination with the monoanionic groups shown in any of the formulae, specifically the bicyclic or polycyclic structures 4, 112, 230, 327, 477, 509, 549, 624, 647, 654, 660, 672, 678, 684, 690, 702, 708, 714, 756, 762.

If the monoanionic group is part of the ring structures as for example in structure 654, the location of these monoanionic groups cannot be changed, and there cannot be any additional monoanionic groups.

According to one embodiment of the invention, therefore the bicyclic or polycyclic group or core of the cyclic organic ligand is selected from one of those shown in Figure 5 and specifically the above-mentioned structures 4, 112, 230, 327, 477, 509, 549, 624, 647, 654, 660, 672, 678, 684, 690, 702, 708, 714, 756, 762. This bicyclic or polycyclic group or core can be substituted and bears the two monoanionic groups.

Furthermore, the monoanionic groups covalently linked with a cyclic organic ligand are preferably chosen from those shown in the structures of Figure 5, more preferably those of the above-mentioned structures 4, 112, 230, 327, 477, 509, 549, 624, 647, 654, 660, 672, 678, 684, 690, 702, 708, 714, 756, 762.

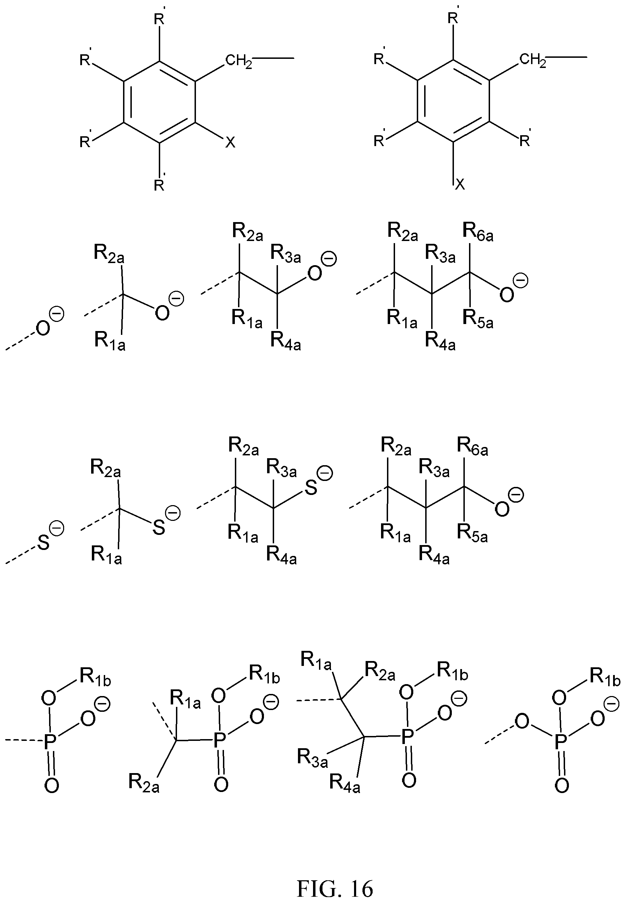

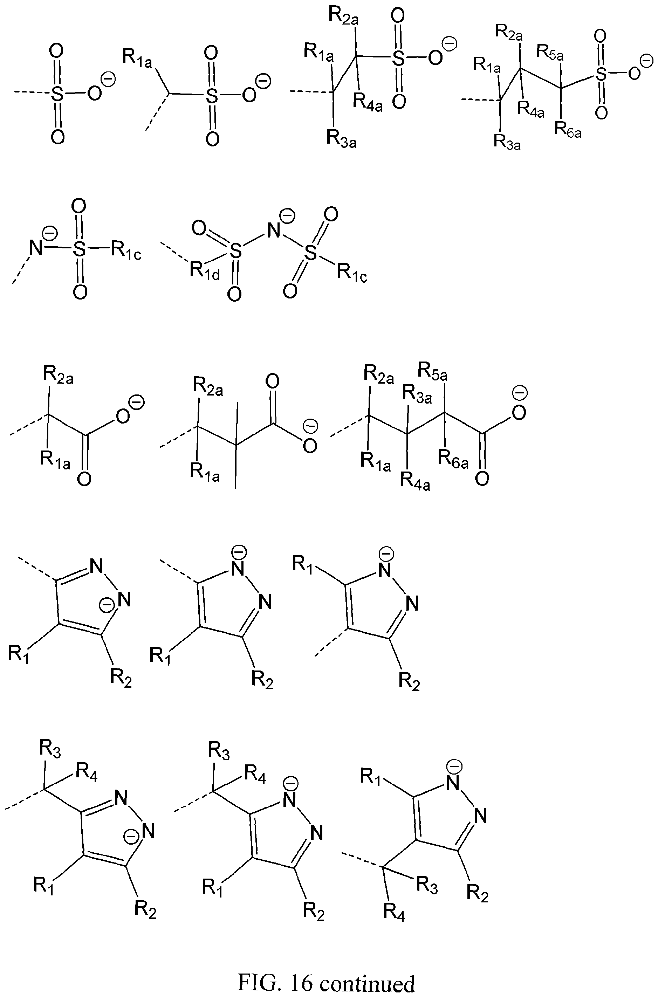

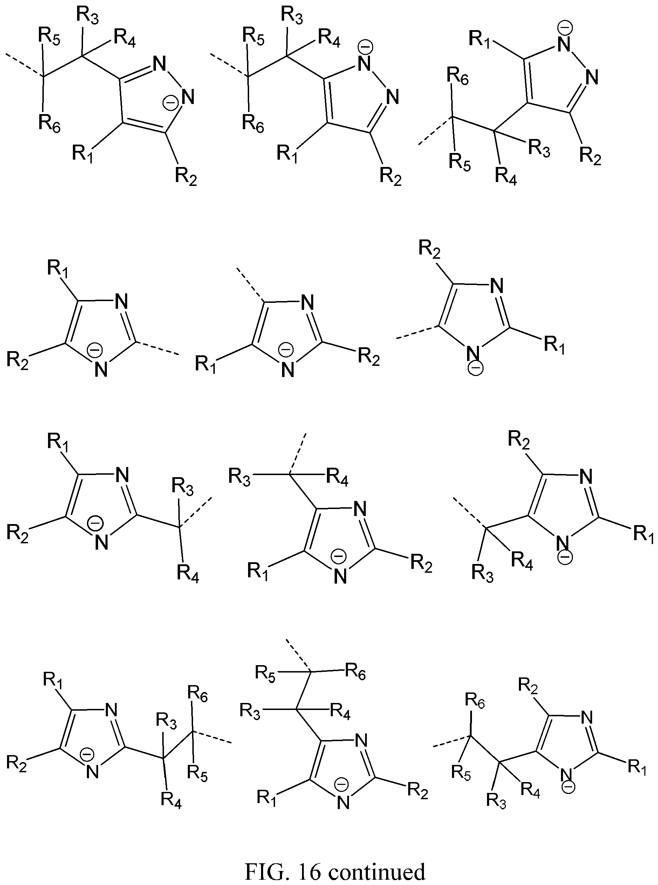

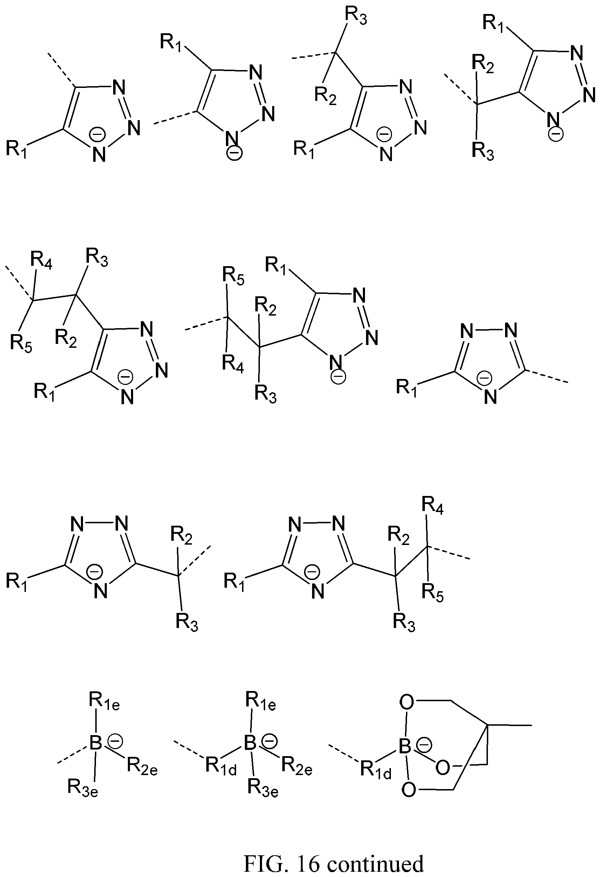

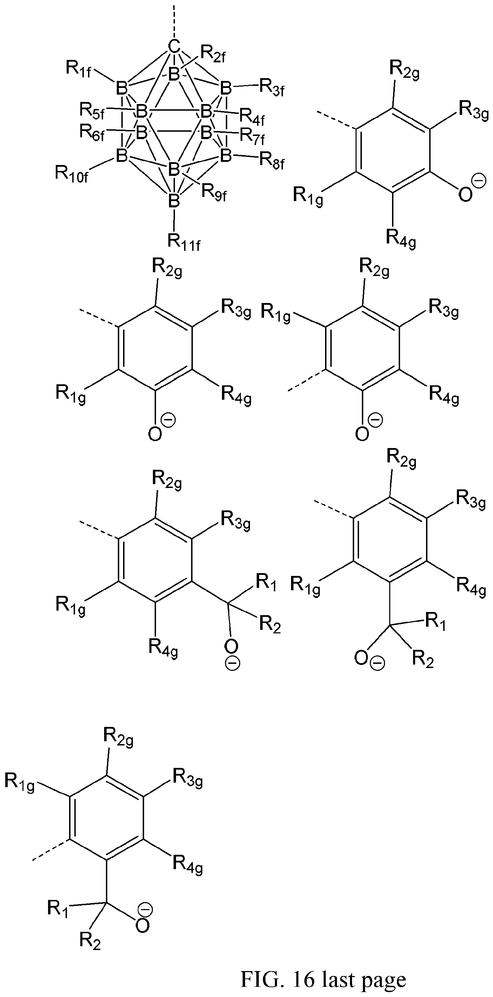

Preferred structures of the monoanionic group furthermore are shown in Figure 16. The dashed line shows the position in which the monoanionic groups are covalently linked with a cyclic organic ligand. The dashed line can be a single bond or a spacer group, for example a C1-12-alkylene spacer group, more preferably C1-6-alkylene spacer group. Thus, the cyclic organic ligand and the shown monoanionic groups can be separated by a covalent bond or 1, 2, 3, 4, 5 or 6 carbon atoms in a preferred embodiment.

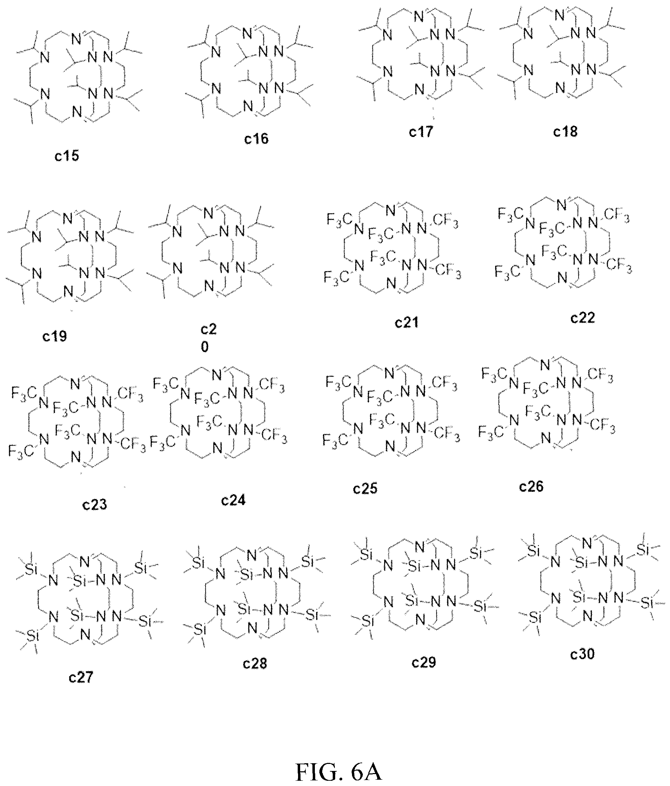

These covalently linked monoanionic groups can for example be linked with the cyclic groups shown in Figures 1A, 1B, 1C, 2A, 2B, 2C, 2D, the core structures or preferred core structures shown in Figure 5 and specifically in above-mentioned structures 4, 112, 230, 327, 477, 509, 549, 624, 647, 654, 660, 672, 678, 684, 690, 702, 708, 714, 756, 762 as outlined above, the structure shown in Figure 6A an 6B, 11 and 15.

Furthermore, these preferred monoanionic groups, more preferably those of Fig. 16, can be combined with the following cyclic organic ligands as shown in the following structures. According to another embodiment, any covalently linked monoanionic group can be present in the following structures. 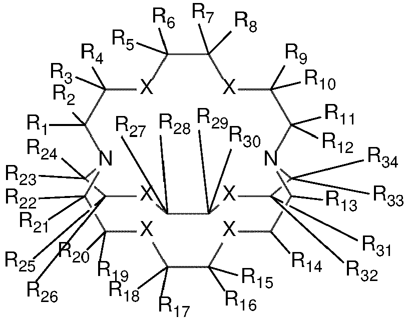 wherein
X in each position independently is O or S, preferably O,
two of R₁ to R₃₄ represent a monoanionic group and the remaining R₁ to R₃₄ independently represent hydrogen or organyl groups. 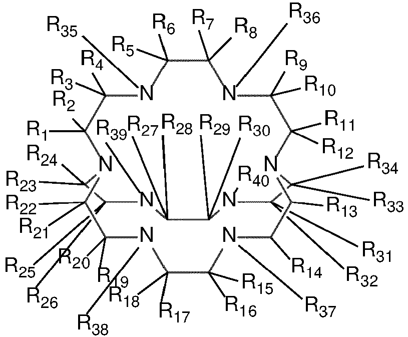
wherein two of R₁ to R₄₀ represent a monoanionic group, and the remaining R₁ to R₄₀ independently represent hydrogen or organyl groups. 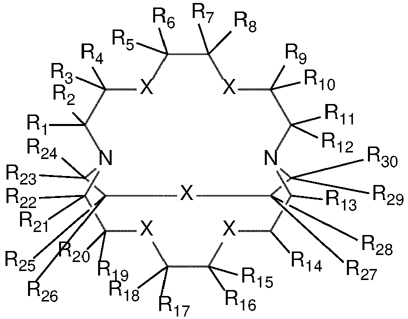
wherein X in each position independently is O or S, preferably O,
two of R₁ to R₃₀ represent a monoanionic group, and the remaining R₁ to R₃₀ independently represent hydrogen or organyl groups 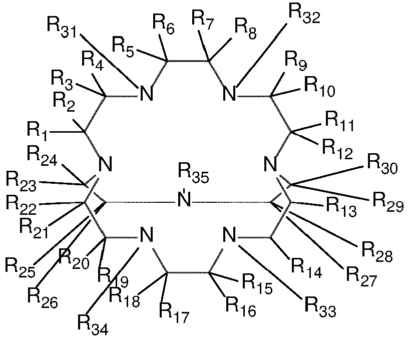
wherein R₁ to R₃₅ are as defined above for R₁ to R₃₀. 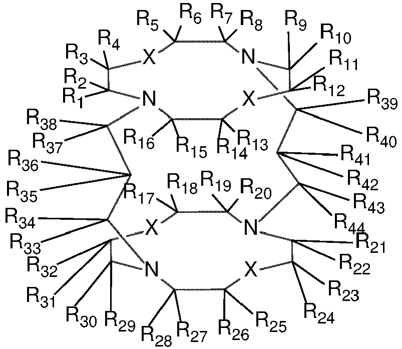
wherein R₁ to R₄₄ are as defined above for R₁ to R₃₀. 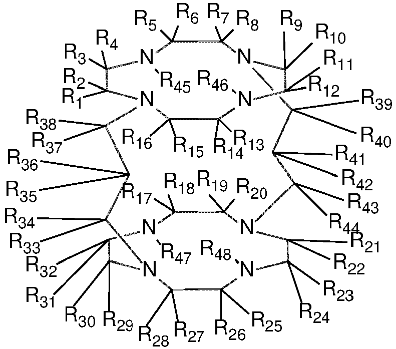
wherein R₁ to R₄₈ are as defined above for R₁ to R₃₀. 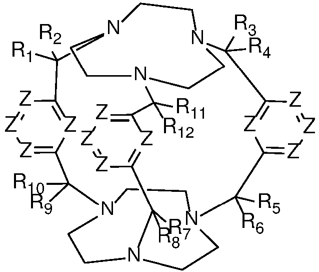
wherein Z in each position is independently selected from N, C-R₁₃,
R₁ to R₁₃ are as defined above for R₁ to R₃₀. 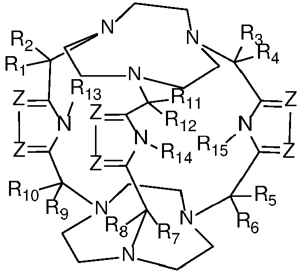
wherein Z in each position is independently selected from N, C-R₁₆,
R₁ to R₁₆ are as defined above for R₁ to R₃₀. 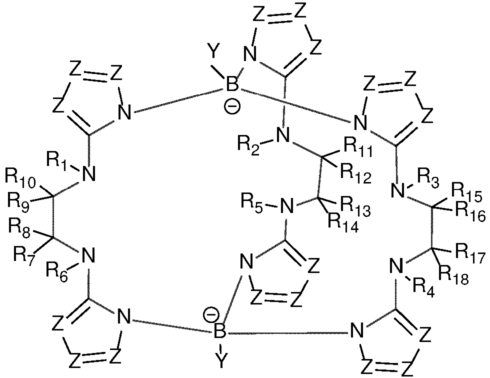
wherein Z in each position is independently selected from N or CH
Y in each position is independently selected from H, F, -OR₁₉ aryl or heteroaryl which aryl and heteroaryl can optionally be substituted,
R₁ to R₁₉ organyl (but not ionic)
wherein Z, Y, R₁ to R₁₉ do not contain ionic groups. 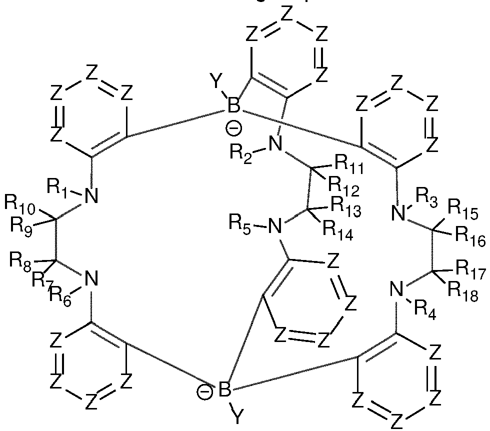
wherein Z in each position is independently selected from N or CH
Y in each position is independently selected from H, F, O-R₁₉, aryl or heteroaryl (both optionally substituted)
R₁ to R₁₉ organyl (but not ionic),
wherein Z, Y, R₁ to R₁₉ do not contain ionic groups.

The position of the two monoanionic groups can be freely chosen between the shown substituents.

Furthermore, preferably 0 to 16, more preferably 0 to 12, most preferably 0 to 6 of the shown groups represent substituents and thus are different from hydrogen.

The last two cyclic organic ligands have monoanionic charge at the boron atoms. Therefore, no additional monoanionic group is present.

Most preferred is a combination of the cyclic organic ligands shown with the monoanionic groups shown in Figure 16 with the above-mentioned definitions of the residues.

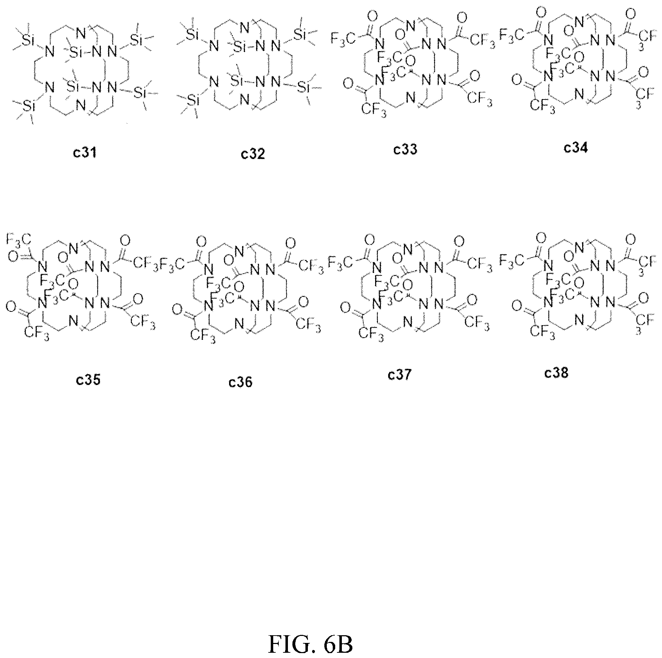

In various embodiments, the cyclic organic ligand may be selected from the group of c15 to c44 illustrated in **FIG. 6A** and **FIG. 6B****,** while the two monoanionic groups are covalently linked with the shown cyclic organic ligands.

In various embodiments, the coordination compound according to the invention may be used in a mixture with at least one second electrically neutral or charged organic compound. Preferable for application in electroluminescent devices, the second organic compound may have a triplet energy higher than 2.5 eV,more preferably higher than 2.6 eV and even more preferably higher than 2.7 eV to ensure that the excitation is confined at the preferably blue light emitting coordination compound according to the invention. Alternatively or in addition, the coordination compound may have a higher hole affinity compared to the second organic compound.

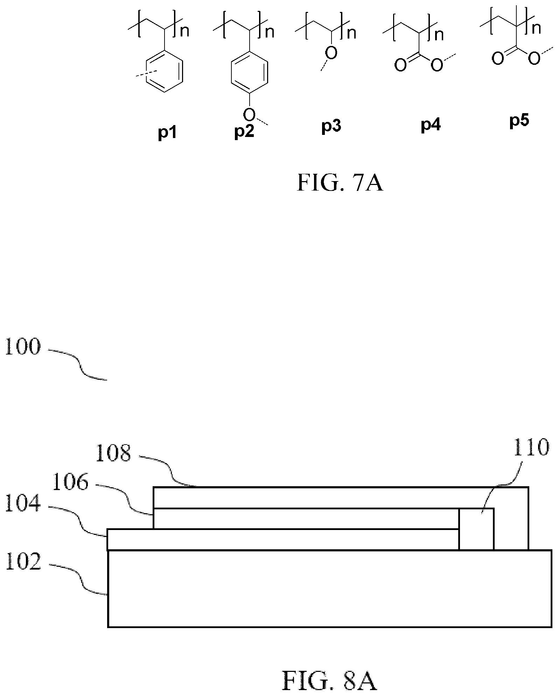

In various embodiments, a compound may include the coordination compound according to any of the described or illustrated embodiments and a polymer with a molecular weight Mn above 1000 g/mol. The coordination compound may be covalently attached to the polymer backbone. The polymer molecule may be an auxiliary organic molecule. In various embodiments, any of the side groups R₁, R₂ of the formula (3-1) or (3-2) may be linked to another organic molecule, such as a polymer. Some repeat units of polymers that may bind to the coordination compound of formula (3-1) or (3-2) via R₁ or R₂ are illustrated in **FIG. 7A** as p1 to p5. Here, a dashed line may represent a preferred connection to R₁ or R₂. It is, however, understood that those are specific examples for illustration purpose only.

In various embodiments, a contrast enhancement medium for magnet resonance tomography (MRT) may include the coordination compound according to the invention e.g. as a pure compound, in a mixture or a compound as described before. For MRT preference is given for Europium over Ytterbium. The divalent Europium and Ytterbium coordination compound according to various embodiments shows an unmatched stability in solution, e.g. including aqueous solution and even to slightly basic or acidic environment and even at elevated temperatures. Therefore, the coordination compound can be applied in biological specimens, such as inside the blood circulation system. Because of its half-filled f-orbital, divalent Europium has one of the highest local ionic magnetic moments (J=7/2). This moment may interact with surrounding water molecules leading to paramagnetic exchange, making it useful as contrast enhancing agent in magnetic resonance imaging (MRT).

In various embodiments, the coordination compound according to various embodiments may be dispersed into a matrix of suitable optical properties, such as for example a high transparency in certain desired parts of the visible spectrum. If this matrix is brought in optical contact to a light source emitting at sufficiently short wavelength, this light may be absorbed by the coordination compound and reemitted at substantially longer wavelength. A suitable light source may for example be a light emitting diode emitting light at wavelength substantially shorter than 430 nm, which may be reemitted by the coordination compound at wavelength longer than 430 nm. The matrix may have any physical dimensions. It may for example be a thin layer of 10 nm to 10,000 nm. The matrix may as well be of granular form or in form of small particles of average diameter between 10 nm and 100,000 nm. For use in optical devices, in the latter cases, the matrix may be applied inside another host material for support.

The combination of unique paramagnetic and optical properties with the ease of processing makes the coordination compound according to various embodiments highly attractive for application in organic electronic devices. The solubility of the metal-organic coordination compound in nonpolar solvent is increased, and sublimation and evaporation are improved. The compound has preferably molecular instead of salt-like character and can be easily mixed with organic matrices. In this description an organic electronic device may be any device or structure including substantially organic layers or subunits, where an electro-magnetic stimulus may be converted into an electrical field or current or vice versa, or where an input electrical current or electrical field may be used to modulate an output electrical current or electrical field.

In one embodiment, the organic electronic device including the coordination compound according to various embodiments may be used as semiconducting organic material in an organic field-effect transistor or an organic thin-film transistor.

In another embodiment the organic electronic device converts external static or dynamic magnetic fields into proportional, measurable electrical signals and thus becomes a magnet field sensor.

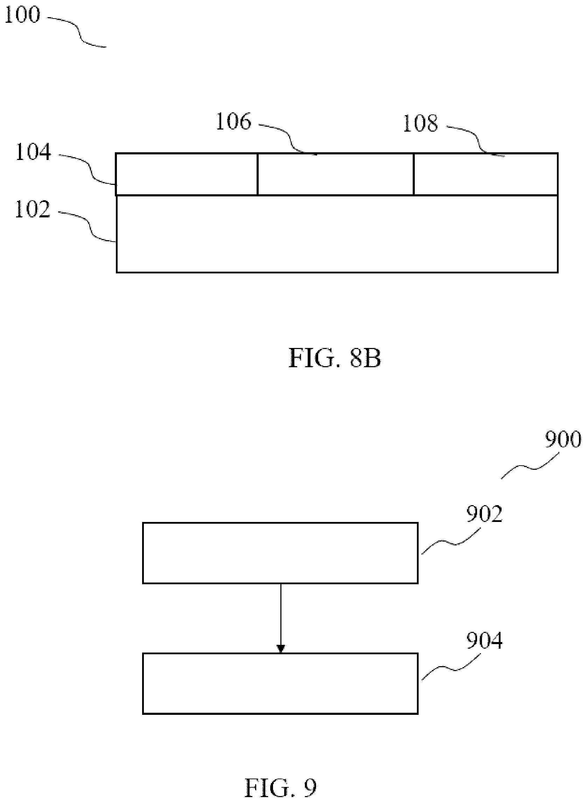

**FIG. 8A** and **FIG. 8B** illustrate embodiments of an organic electronic device 100 configured as optically active device. The organic electronic device may include a first electrode 104, e.g. on a substrate 102 or as the substrate; a second electrode 108; and an organic layer 106 arranged such that it is electrically interposed between the first and second electrodes 104, 108. The first and second electrodes 104, 108 may be electrically insulated from each other by an insulating structure 110, e.g. a resin or polyimide. The first and second electrodes 104, 108 may be stacked over each other (FIG. 8A) or may be arranged in a common plane (FIG. 8B).

The organic layer 106 may include the coordination compound according to any of the described or illustrated embodiments, e.g. as a pure compound, in a mixture or in a compound as described before.

In various embodiments, the organic electronic device 100 may be an optoelectronic device, the optoelectronic device being at least one of an organic light emitting diode (OLED), an organic photodetector, or a photovoltaic cell. That is, photons from an external electromagnetic field may be absorbed in the organic layer 106 and converted into current by means of an electrical field between the first and second electrodes 104, 108. Such a device would be a photodiode (oPD) and its primarily use may be to sense external light. It would be an organic photovoltaic (OPV) device, if the primarily use is to convert light into current.

In various embodiments the paramagnetic moments of the coordination compounds including divalent Europium may be aligned to exhibit a macroscopic magnetic moment. This macroscopic moment of the organic layer 106 may be employed as part of a magneto-optic or magneto-electric sensor. It may as well be used as part of a touchscreen function of a flat panel display. It may as well be used to build spintronic devices. The alignment of the paramagnetic coordination compound according to various embodiments may be achieved with any suitable technique, for example, but not limited to, techniques that align the coordination compound during, or after processing. For example, the coordination compound may be aligned after processing using a strong external electro-magnetic, or static magnetic field. In conjunction with the application of this external magnetic field, the alignment may be improved or permanently frozen-in by heating the organic layer 106 to be aligned. Hereby the heating may proceed above the glass-transition temperature or beyond the melting temperature of the coordination compound or parts or the whole organic layer 106. Alternatively, the paramagnetic coordination compound may be aligned in-situ during processing of the layer including the paramagnetic coordination compound, for example, but not limited to, by application of a static or dynamic external magnetic field during the processing from either solution or gas phase. In this context, the macroscopic magnetic moment may as well be formed inhomogeneously over the area of the device by applying suitable external magnetic sources during processing.

The organic layer 106 is arranged electrically between the first and second electrodes 104, 108 such that an electronic current may flow from the first electrode 104 through the organic layer 106 to the second electrode 108 and vice versa during operation, e.g. in light emission applications. Alternatively, in photoelectric applications, a charge carrier pair may be formed in the organic layer 106 and charge carriers of the charge carrier pair may be transported to the first and second electrodes 104, 108 respectively. In other words, in light emission applications, upon application of sufficient voltage, holes and electrons are injected from the anode and the cathode, respectively, and drift towards the organic layer 106, where charges of opposite sign recombine to form a short-lived localized excited state. The short-lived excited state may relax to the ground state thereby giving rise to light emission. The first and second electrodes 104, 108 may be substantially unstructured layers, e.g. for general lighting applications, or may be structured, e.g. for light emitting diodes or transistors for pixels in a display application.

The organic electronic device 100 may be configured to emit substantially monochromatic light such as red, green, blue, or polychromatic light such as white. The light may be emitted through the first electrode 104 (bottom emitter), through the second electrode 108 (top emitter), or through first and second electrodes 104, 108 (bidirectional emitter). The light may as well substantially be emitted in a direction parallel to the organic layer 106 using suitable opaque electrodes 104, 108. In such a layout lasing may be achieved, and the device may be an organic laser, which, in this description, may be considered as a specific type of electroluminescent devices. The coordination compounds according to various embodiments may have excellent emission properties, including a narrow, deep blue emission spectrum with short excited state lifetime. Given its high atomic weight, the optical transitions of divalent Ytterbium and Europium may be as well widely indifferent to excitation with either spin 1 or spin 0 electron-hole pairs. In other words, they will harvest singlet and triplet excitations, giving high light emission efficiency. As such the coordination compounds according to various embodiments may be ideally suited for application in organic electroluminescent devices, such as organic light emitting diodes (OLED). In this description, an electroluminescent device may be any device including an organic layer disposed between and electrically connected to an anode 104/108 and a cathode 108/104. Upon application of sufficient voltage, holes and electrons may be injected from the anode 104/108 and the cathode 108/104, respectively, and drift towards the organic layer 106, where charges of opposite sign recombine to form a short-lived localized excited state. The short-lived excited state may relax to the ground state thereby giving rise to light emission. Relaxation pathways without light emission, such as thermal relaxation, may be possible too, but may be considered undesirable, as they lower the conversion efficiency of current into light of the device.

Further layers may be formed and in electrical connection between the first and second electrodes 104, 108, e.g. configured for charge carrier (electron or hole) injection, configured for charge carrier transport, configured for charge carrier blockage or configured for charge generation. The recombination of electrons and holes may as well happen at the interface of the organic layer 106, which contains the coordination compound according to the invention and an adjacent layer, such an electron blocking layer. Further optically functional layers, e.g. a further electroluminescent material and/or a wavelength conversion material may be formed electrically between the first and second electrodes 104, 108 and in the optical path of the organic layer 106, e.g. on top of the second electrode 108 and/or on the opposite side of the substrate 102. In addition, encapsulation structures may be formed encapsulating the electrically active area, e.g. the area in which an electrical current flows, and may be configured to reduce or avoid an intrusion of oxygen and/or water into the electrically active area. Further optically functional layers, e.g. an antireflection coating, a waveguide structure and/or an optical decoupling layer may be formed within the optical light path of the organic layer 106.

As example, hole or electron blocking layers may be used to optimize the individual hole and electron currents through the organic electronic device 100. This may be known to those skilled in the art as charge balance in order to optimize efficiency and operational stability. In various embodiments, dedicated hole or electron charge transport layers may be present in the organic electronic device 100 to space the emission region from the first and second electrodes 104, 108.

Examples of hole transport materials include known materials such as fluorene and derivatives thereof, aromatic amine and derivatives thereof, carbazole derivatives, and polyparaphenylene derivatives. Examples of electron transport materials include oxadiazole derivatives, triazine derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and its derivative, diphenoquinone derivatives, and metal complexes of 8-hydroxyquinoline and its derivatives and various derivatives of silanes.

In various embodiments, those charge transport layers may include electrical dopant molecules or metals, or may be in contact to charge injection layers. Any of those auxiliary layers may be fully organic or may include inorganic functional moieties. For example, charge transport layers may be made of the class of Perovskite materials.

The coordination compound as illustrated or described in any one of the embodiments may be used as a pure organic emitting layer 106 of any thickness in the range of 0.1 nm and 100 nm, preferably in the range of 0.1 and 10 nm.

The organic layer 106 may in various embodiments include charge transport materials to improve charge transport into and through the organic layer 106. Charge transport materials may be any material that is able to transport either holes or electrons or both types of charges. In particular a charge transport material may be any aryl, or heteroaryl organic compound or any metal complex or any mixture thereof. Examples of materials present in the light emitting layer 106 include carbazole derivatives, including carbazole-phophine oxide materials, oxadiazole derivatives, triazine derivatives, silane derivatives and any other material or combination of materials with sufficiently high triplet level that may transport charges or otherwise benefit the device performance. The volume percentage of the coordination compound as a function of the combined charge transport materials may be between 0.5 and 99.5 vol% in the organic layer 106.

In various embodiments, the oxidation potential of the coordination compound of the organic layer 106 may be higher compared to all charge transport materials present in the organic layer 106. A wide variety of techniques on how to measure oxidation potentials have been published in the literature. However, for the purpose of various embodiments, the particular technique of how to measure the oxidation potential is not essential, e.g. only the relative order between the coordination compound and the charge transport materials may be of importance. Oxidation potentials may for example be deducted using quantum mechanical computing techniques based on density functional theory and experimental techniques are very well known in the art, e.g. see R.J.Cox, Photographic Sensitivity, Academic Press, 1973, Chapter 15.

The organic layer 106 may contain any other organic or inorganic material in a range of 0.1 to 99.9 vol% that are not intended to transport charges. For example. The organic layer 106 may include polymers (in a mixture or as a compound) may be added to improve film quality and prevent crystallization. Other materials may be added to evenly space the coordination compound inside the organic layer 106.

In color conversion materials, the divalent lanthanides according to various embodiments may allow the ease of processing using vacuum based techniques. Thus, an application of divalent Europium or Ytterbium in organic electronic devices, such as organic photovoltaic (OPV), organic light emitting diodes (OLED), organic sensors, organic memory or organic sensors may be of advantage. In other words, the evaporation temperature of the compounds including the divalent lanthanides may be sufficiently low to allow for thermal vacuum processing techniques to be used. Reduced evaporation temperatures can be achieved according to the invention by using the metal-organic coordination compounds as here the ionic character of the compounds is strongly suppressed, if compared to a similar organic salts without covalently attached monoanionic groups. Consequently, the evaporation temperature is reduced and incorporation into organic electronic devices becomes possible using state-of-the-art vacuum deposition techniques.

The excitation of the light emitting coordination compound may be energetically confined. This way, high efficiencies may be achieved. Thus, confinement layers may be formed adjacent to the organic layer 106, wherein the confinement layers may have a triplet energy T₁ higher than 1.8 eV, e.g. higher than 2.3eV, e.g. higher than 2.7 eV. Similarly, any material of the organic layer 106 (other than the electroluminescent compound) may have a triplet energy T₁ higher than 1.8 eV, e.g. higher than 2.3eV, e.g. higher than 2.7 eV.

In various embodiments, the organic electronic device 100 includes two or more sub units each including at least one light emitting layer. The subunits may be stacked over each other physically separated and electrically connected by a charge generation layer or, alternatively, may be arranged side by side. The subunits may be subpixels of a pixel in a display or general lighting application. The light emitted by the subunits may be mixed to generate a light of a predetermined color. Each subunit may emit light of the same or a different color. The overall light emitted by such organic electronic device 100 may contain a narrow spectral region, such as blue, or may contain a wide spectral region such as white, or a combination thereof. The coordination compound illustrated or described in any one of the embodiments may or may not be present in all subunits of the organic electronic device 100.

In various embodiments, the light emitted by the organic electronic device 100 may be in optical contact to at least one optically active layer, including any optically active materials such as organic molecules or quantum dots. The optically active layer may be a spectral filter element, which may absorb part of the light emitted by the organic electronic device 100. In another embodiment, the optically active layer may absorb at least part of the light emitted by the organic electronic device 100 and may reemit it at longer wavelength (wavelength conversion).

As example, the organic layer 106 may be configured to emit light substantially at wavelengths shorter than 500 nm and the optically active layer may be configured to substantially reemit light at wavelengths longer than 500 nm. The optically active layer may be placed in between the anode and cathode of the organic electronic device 100 or outside of it. The optically active layer may as well be part of the organic layer 106.

The organic electronic device 100 may be configured as a large area OLED device used for illumination, signage, or as a backlight. Alternatively, the organic electronic device 100 may include a plurality of OLEDs arranged in a pixilated layout (plurality of OLED pixels) that are individually connected electrically, e.g. for flat panel display applications. Here, individual pixels may have the capability of emitting light of substantially narrow spectral portions; especially of red, green, and blue. The coordination compound may or may not be present in any of the individual pixels.

In another embodiment, the individual pixels may be configured to emit white light. Red, green, and blue spectral portions are generated by using suitable filter elements in optical contact with the pixelated OLEDs.

In another embodiment, the OLED pixels emit blue light and the red and green spectral portions may be generated by using a suitable color conversion element in optical contact with the OLED pixels.

In various embodiments, the organic electronic device 100 includes an anode 104; a cathode 108; and an organic layer 106 disposed between the anode 104 and the cathode 108. The organic layer 106 includes an electroluminescent coordination compound according to various embodiments. The coordination compound includes at least one divalent lanthanide coordinated by a cyclic organic ligand according to the present invention.

The divalent Lanthanide may be Europium or Ytterbium. The organic ligand according to the invention is electrically neutral. The coordination compound includes two monoanionic groups in covalently linked form. The coordination compound may be imbedded into at least one second electrically neutral organic compound. The second organic compound may have a triplet energy higher than 2.5eV, more preferably higher than 2.6eV and even more preferably higher than 2.7eV. The coordination compound may have a higher hole affinity compared to the second organic compound.

An organic electronic device 100 according to various embodiments may be fabricated using a wide range of commonly used techniques, including, but not limited to, deposition of all or some layer, from gas phase vacuum deposition, solution phase, or gas phase using a carrier gas method.

In various embodiments, deposition via the gas phase in vacuum may be used, whereby the coordination compound may either undergo sublimation or evaporation. The coordination compounds according to the invention show an improved sublimation/evaporation yield. The transfer into the gas phase may be improved by using a carrier gas technology, whereby an inert gas that may not be deposited into the organic layer may be helping the sublimation or evaporation of the coordination compound to be deposited. During the deposition process from gas phase, the coordination compound may as well be co-deposited with one or more material to fabricate any desired mixed layers.

**FIG. 9** illustrates a flow diagram of a method of forming an organic device, the method may include forming 900 of a layer of the coordination compound according to any of the described or illustrated embodiments as a pure compound or in a mixture or linked to a polymer as described before. The layer may be deposited from a gas phase, in particular using an evaporation and/or sublimation process, and/or by a solution-based process. The forming 900 of the layer may include forming 902 a first layer including the organic ligand and forming 904 a second layer directly in contact with the first layer, wherein the second layer includes a divalent lanthanide salt. The second layer may be deposited by a solution-based or thermal vacuum based process.

In this embodiment, the coordination compound according to various embodiments may be formed in-situ using gas phase deposition techniques. Here, the organic ligand of the invention according to the formula illustrated in FIG. 1B or C but excluding the divalent lanthanide metal, may be first (902) deposited onto a suitable substrate or other organic layer thereby forming a seed layer. Any suitable technique may be used to fabricate this seed layer. This seed layer including the organic ligand may include any other material; preferred may be organic charge transport materials, for example, suitable to achieve hole transport. Preferred may be in various embodiments inert organic or inorganic materials that aid the layer formation, improve its thermal stability, or improve the distribution of the organic ligand within this seed layer. In the next deposition step (904) and sequential to forming (902) the seed layer including the organic ligand according to the formula illustrated in FIG. 1B or C but excluding the divalent lanthanide metal, a molecular salt including a divalent lanthanide may be evaporated. The divalent lanthanide salt may be any charge neutral compound including a lanthanide in its divalent oxidation state and one or two suitable anions. The divalent lanthanide salt may as well be simultaneously co-deposited with one or more material to fabricate mixed layers. At the interface of the seed layer including the organic ligand, according to the formula illustrated in FIG. 1B or C, and because of its high thermal activation energy, the divalent lanthanide salt may interact with the organic ligand to form the coordination compound according to various embodiments in-situ. Alternatively, the lanthanide salt may be processed first and the ligand according to FIG. 1B or C is processed in a second step. Alternatively, to use a molecular salt, a lanthanide metal vapor may be used to form the coordination compound according to various embodiments in-situ. In the latter case, an oxidation of the lanthanide takes place. This reaction may be improved by the presence of suitable electron acceptor units in the seed layer or by using the neutral acid version of the ligand illustrated in FIG. 1B or C. Suitable electron acceptors present in the seed layer may be the reduced, i.e. charge neutral p-dopants.

It is preferred to use the metal-organic coordination compound as it is using gas phase deposition techniques. In this way, there is no need to employ divalent lanthanide salts in which the anions have to be removed afterwards. Since the metal-organic coordination compounds according to the present invention contain the two monoanionic groups covalently linked with a cyclic organic ligand, both the divalent lanthanide and the cyclic organic ligand can be gas phase deposited at the same time.

Another preferred technique to fabricate layers including the coordination compound according to various embodiments may be deposition from a liquid phase using a mixture or a single organic solvent, whereby the coordination compound according to various embodiments may be dissolved or forms a suspension within the organic solvent; in this description may be referred to as the ink. The ink using this deposition process, may include a wide variety of other materials apart from the coordination compound according to various embodiments to allow fabrication of mixed layers from solution. Additives within the ink may for example, but may not be limited to, be organic or inorganic materials capable of transporting charges, materials that improve the film formation, materials that improve the distribution of the coordination compound within a host material, organic or inorganic materials that improve the efficiency of the device, e.g. by reducing the refractive index. The deposition from solution may not be limited to any specific technique. Examples of the deposition from solution include spin coating, casting, dip coating, gravure coating, bar coating, roll coating, spray coating, screen printing, flexographic printing, offset printing, inkjet printing. The improved solubility of the coordination compounds according to the present invention in non-polar solvents eases the deposition from solution and makes it advantageous.

Various post processing techniques may be applied to improve the performance or stability of the organic electronic device. In one embodiment, some or all layers of the organic electronic device include functional groups capable of chemically crosslinking upon thermal or optical exposure thereby forming larger covalently bound molecules with improved physical properties.

The coordination compounds according to various embodiments exhibit a high paramagnetic moment, e.g. in case the divalent lanthanide is Europium. The paramagnetic moments of the coordination compound according to various embodiments may be aligned to exhibit a macroscopic magnetic moment. This macroscopic moment of the (organic) layer (106) may be employed as part of a magneto-optic or magneto-electric sensor integrated into the organic electronic device of the invention. It may as well be used as part of a touchscreen function of an organic electronic device flat panel display. It may as well be used to build opto-electronic light emitting spintronic devices.

The alignment of the paramagnetic coordination compound according to various embodiments may be achieved with any suitable technique, for example, but not limited to, techniques that align the coordination compound during, or after processing. For example, the coordination compound may be aligned after processing using a strong external electro-magnetic, or static magnetic field. In conjunction with the application of this external magnetic field, the alignment may be improved or permanently frozen-in by heating the organic layer to be aligned. Hereby the heating may proceed above the glass-transition temperature or beyond the melting temperature of the coordination compound or parts or the whole organic layer (106).

Alternatively, the paramagnetic coordination compound may be aligned in-situ during processing of the layer including the paramagnetic coordination compound, for example, but not limited to, by application of a static or dynamic external magnetic field during the processing from either solution or gas phase. In this context, the macroscopic magnetic moment may as well be formed inhomogeneously over the area of the organic electronic device by applying suitable external magnetic sources during processing.

According to another embodiment of the invention, by covalently linking the two monoanionic groups with a cyclic organic ligand, an unwanted polarity (quadrupole and/or dipole) can be reduced, which makes the compounds specifically suitable for OLED applications as they better blend into the organic environment of the OLED device.

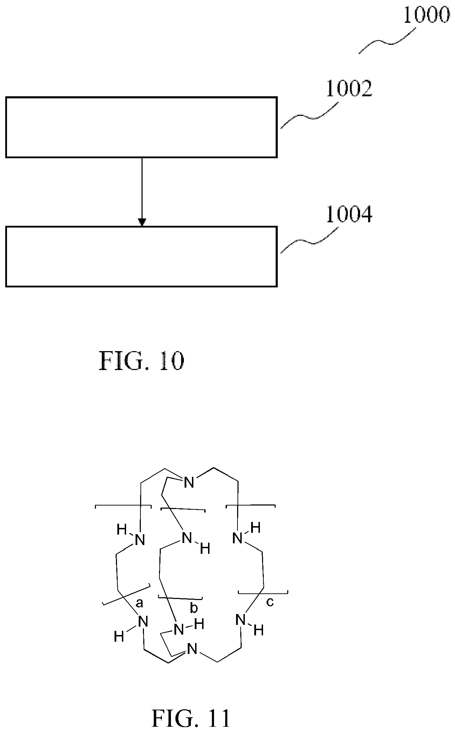

**FIG. 10** illustrates a flow diagram of a method to synthesize the coordination compound according to any of the described or illustrated embodiments. The method 1000 may include a reaction of a divalent lanthanide salt and an organic ligand according to any one of FIG. 1 to FIG. 4 at pressure greater than or equal to about 1 bar. The method 1000 may include that the divalent lanthanide salt is coordinated with an organic precursor 1002 and subsequently at least one of the groups R1, R2, R3, R4, R5, R6 is attached to the organic precursor 1004.

In various embodiments, the coordination compound may be formed in-situ using deposition from solution. Here, the organic ligand of the present invention including or excluding the lanthanide metal, may be first deposited onto a suitable substrate or other organic layer thereby forming a seed layer. Any suitable technique may be used to fabricate this seed layer. This seed layer including the organic ligand may include any other material. Preferred may be organic charge transport materials, for example suitable to achieve hole transport for organic electronic devices. Preferred may be further additional inert organic or inorganic materials that aid the layer formation or improve its thermal stability or improve the distribution of the organic ligand within this seed layer. In a next deposition step and sequential to forming the seed layer including the organic ligand according to the invention excluding the metal, a layer including a molecular salt including a divalent lanthanide may be fabricated using a solution process. The divalent lanthanide salt may be any charge neutral compound including a lanthanide in an oxidation state 2+ and one or two suitable anions. The anions may as well be covalently bound to a suitable polymer. The ink including the divalent lanthanide salt may as well include one or more additive to fabricate mixed layers. These additives may be organic or inorganic materials to aid charge transport, may be organic or inorganic materials that improve the efficiency of the device, or may be any material that improves the film formation. At the interface of the seed layer including the organic ligand according to the invention, the solubilized divalent lanthanide metal will interact with the organic ligand to form the coordination compound according to various embodiments in-situ.

Alternatively, the process of first forming the layer containing the ligand and secondly processing the lanthanide salt may be inverted.

Preferably, the metal-organic coordination compound is used directly.

In various embodiments, the method of forming an organic electronic device includes forming an anode; forming a cathode; and forming an organic layer disposed between the anode and the cathode. The organic layer may be formed to include an electroluminescent coordination compound. The coordination compound may include at least one divalent lanthanide coordinated by a cyclic organic ligand according to the invention

In the coordination compounds according to various embodiments, substituents larger than -CH3 group may be attached to the nitrogen atoms of an azo-cryptand. This way, a needed chemical stability to Eu2+ and Yb2+ coordination compounds is provided. The application of such coordination compounds may be useful for an improvement of a luminescence efficiency and a lifetime of the electroluminescent device.

There is a wide range of possibilities to synthesize the coordination compounds according to various embodiments. Some qualitative schemes are briefly discussed below for the purpose of illustration only.

In one qualitative scheme, the organic ligand according to the invention may be synthesized first. In a next step a suitable salt including the divalent Europium or Ytterbium may be added. By choosing suitable conditions, the coordination compound according to various embodiments may be formed and may precipitate. If bulky ligands are present in the compounds, the coordination compounds according to various embodiments may exhibit a very high kinetic barrier against reaction of the metal cation with the environment.

Preferred may be ligands according to the formula illustrated in **FIG.** 11. Here, a, b and c may be independently an integer of 0 or more. The monoanionic ligands are not shown.

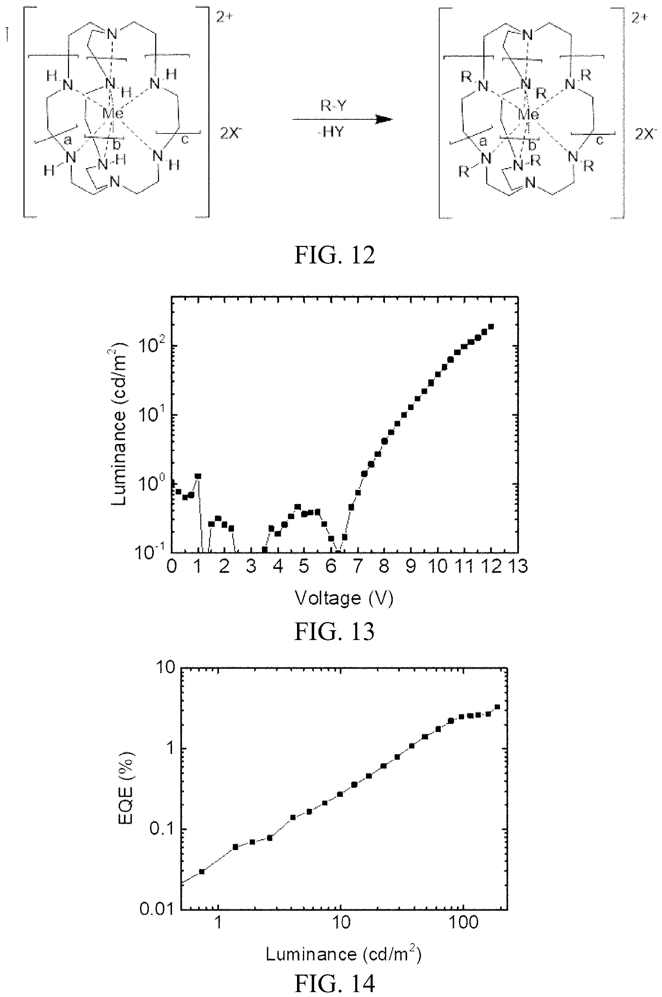

In another embodiment, the synthesis of coordination compound could be done according to the schema illustrated in **FIG.** 12. Here, Me may be Eu²⁺ or Yb²⁺ and a dashed line may represent the coordination bond of the ligand to the central metal ion; a, b, c may be integer of 0 or more, X - may represent the single charged anion, R-Y may represent the compound, which may be able to react with secondary amines.

The (electroluminescent) coordination compound according to various embodiments may thus be extremely stable and as such ideally suited for a large variety of processing methods and applications.

For one or more aspects, at least one of the components set forth in one or more of the preceding figures may be configured to perform one or more operations, techniques, processes, and/or methods as set forth in the example section below.

### EXAMPLES

The examples set forth herein are illustrative and not exhaustive.

Any of the examples may be combined with any other example (or combination of examples), unless explicitly stated otherwise. The foregoing description of one or more implementations provides illustration and description, but is not intended to be exhaustive or to limit the scope of aspects to the precise form disclosed. Modifications and variations are possible in light of the above teachings or may be acquired from practice of various aspects. Any of the above-described examples may be combined with any other example (or combination of examples), unless explicitly stated otherwise.

### Experimental

### Synthesis

The following exemplary procedures are, unless indicated otherwise, carried out under a protective gas atmosphere in oven dried glassware. The solvents and starting reagents can be purchased from any commercial source, such as Merck or Acros. Ligands, used for the synthesis of the coordination compound types without attached monoanionic groups were synthesized according to the reaction scheme:

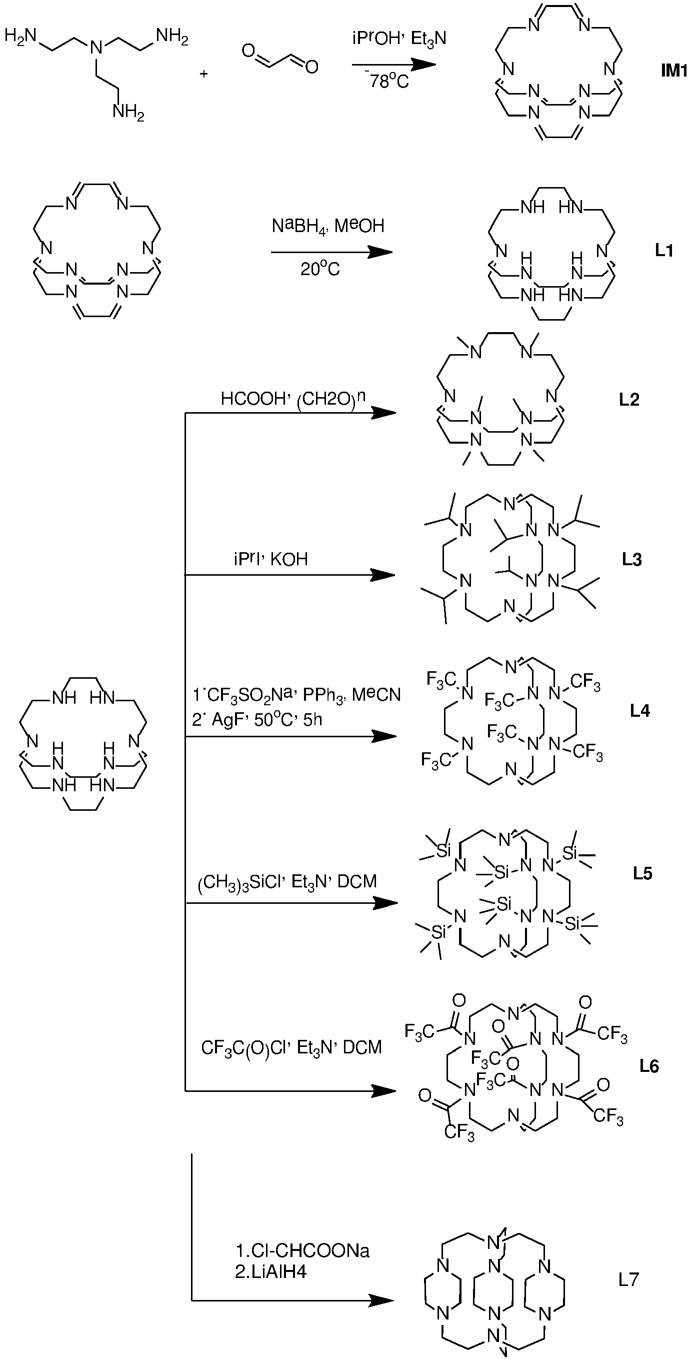

### Synthesis of IM1

The synthesis was done using modified procedure of Redko as mentioned above. Tren (9.73g, 66.6mmol), Et3N (25ml) and i-PrOH PrOH (500ml) were placed in a 1L three-neck round-bottom flask equipped with an addition funnel and mechanical stirrer. The resulting solution was cooled to -78°C (dry ice -*i*-PrOH bath), and a solution of aq. glyoxal (40%, 14.5g, 0.1mol) in i-PrOH (250ml) was then added at a rate of 2 drops/sec with vigorous stirring. Subsequently, the reaction mixture was allowed to warm to room temperature. Next, the solvent was removed on a rotary evaporator yielding a brown solid, which was extracted 5 times with toluene (1500ml in total). Combined extracts were concentrated on rotary evaporator to the volume of about 50ml, precipitated white solid was separated by suction filtration, washed with cold toluene (50ml) and hexane (50ml), dried in vacuum yielding 7.77g (65%) of white solid.
1H NMR (CDCl3): δ = 2.7 (br. 12H), 3.51 (br. 12H), 7.7 (s, 6H)
MS: m/z = 358(M⁺)

### Synthesis of L1

2L round-bottom flask was charged with 5g (14 mmol) of IM1 and 120ml methanol. To the resulting solution, 10 g (0.26 mol) of NaBH₄ was added within 3h in 5 portions. After the addition was complete, the reaction solution was stirred at 25 °C overnight. 100ml of deionized water was added to the reaction mixture with stirring, the solvents were removed under reduced pressure yielding a colorless oil. Residue was stirred with 50ml of deionized water, the white solid was separated by filtration and dried in vacuum at 60°C. Yield: 4.8g (92%)
H-NMR (CDCl₃): δ = 2.50 (t, 12 H,); 2.74 (t, 12 H); 2.77 (s, 12 H).
MS: m/z = 371 (M⁺).

### Synthesis of compound L2

To a solution of L1 (2.50 g, 6.7 mmol) in 90% formic acid (100 ml), a large excess of paraformaldehyde (12.5 g) was added. The mixture was heated under vigorous stirring. After complete dissolution, the solution was refluxed for 3 days under Argon and then evaporated. H20 (200 ml) was added to the residue, the solution was basified to pH 10 by addition of KOH and extracted with CHCl₃ (3 x 150 mL). The extract was evaporated and the obtained yellow oil dissolved with an aq. HCl. The solution was washed with toluene, then basified to pH10 by addition of KOH and extracted with hexane (3x100ml). The combined extract was washed with brine (20ml), dried over anhydrous Na₂SO₄, filtered and evaporated to the dryness yielding yellowish solid (1.56g, 51%)
H-NMR (CDCl₃): δ = 2.29 (s, 18 H,); 2.56 (m, 24 H); 2.72 (s, 12 H).
MS: m/z = 455 (M⁺).

### Synthesis of compound L3

1L three-neck round-bottom flask, equipped with magnetic stirring bar, Soxhlet extractor loaded with 10g of anhydrous MgSO4, and reflux condenser was charged with anhydrous toluene (500ml), IM2 (2.50 g, 6.7 mmol) and isopropyl iodide (6eq. 40.5mmol, 6.88g) and the mixture was heated under reflux for 2h. A solid potassium hydroxide (6eq., 40.5mmol, 2.27g) was added and the resulting mixture was heated under reflux for 20h. The mixture was filtered and extracted 5 times with 2M solution of HCl (100ml). The combined extracts were washed with toluene (50ml), then basified to pH10 by addition of KOH and extracted with hexane (3x100ml). The combined extract was washed with brine (20ml), dried over anhydrous Na₂SO₄, filtered and evaporated to the dryness yielding crude product, which was further purified by column chromatography. Yield: 2.5g (59%)
H-NMR (CDCl₃): δ = 0.92 (d, 36 H); 2.52 (m, 36 H); 2.79 (m, 6 H)MS: m/z = 623 (M⁺).

### Synthesis of compound L4

In a nitrogen-filled glovebox, a 10 mL oven-dried reaction vessel was charged with CF3SO2Na (7.5 mmol, 1.17 g, 1.5 equiv.) and PPh3 (15 mmol, 3.93 g, 3 equiv.), then a solution of IM2 (1.8 g, 5 mmol)
in MeCN (25 mL) was added. The resulting solution was stirred at room temperature for 1h. After that, AgF (2.25 mmol, 286 mg, 4.5 equiv) was added. The resulting mixture was further stirred at 50 °C for 5 h. After cooling to room temperature, the volatiles were removed under vacuum and the residue was purified by column chromatography to give the title compound. Yield: 2.51g (83%)
H-NMR (CDCl₃): δ = 2.29 (s, 18 H,); 2.56 (m, 24 H); 2.72 (s, 12 H)
19F-NMR: (CDCl3) δ = -68.10

### Synthesis of compound L5

A clean and dry 250ml 3-necked round bottom flask, equipped with a magnetic stir bar, reflux condenser fitted with a nitrogen inlet and thermometer may be charged with L1 (7.41, 20mmol). The flask may be sealed with a septum, an atmosphere may be replaced by nitrogen. Anhydrous trimethylamine (132mmol, 6.6eq, 13.3g) may be added through a septum with a syringe, followed by an addition of anhydrous dichloromethane (100ml) via double tipped cannula. The septum may be replaced by an addition funnel, which was charged with a solution of chlorotrimethylsilane (CTMS) (132mmol, 6.6eq., 14.3g), dissolved in 50 ml of anhydrous dichloromethane. The reaction mixture may be cooled down to -10 °C and the solution of CTMS was added dropwise with a rate, which allows to keep the temperature of the reaction mixture below 0 °C. After the addition may be finished, the cooling bath may be removed and the mixture may be stirred at room temperature (3 h) and under reflux (6h) in order to complete the reaction. The mixture was cooled using ice bath and quenched by addition of water. Organic layer may be separated, washed with water (3x 20ml), brine (1x20ml), dried over sodium sulfate and evaporated to dryness yielding a crude product, which was purified by column chromatography (SiO₂, ethylacetate:methanol 50:1). Yield: 11.7 (73%), white powder.
H-NMR (CDCl₃): δ = 0.08 (s, 54H); 2.47 (m, 12H); 2.65(m, 24 H)

### Synthesis of compound L6

A clean and dry 100ml 3-necked round bottom flask, equipped with a magnetic stir bar, reflux condenser fitted with a nitrogen inlet and thermometer was charged with L1 (3.7g, 10mmol). The flask was sealed with a septum, the atmosphere replaced by nitrogen. Anhydrous trimethylamine (70mmol, 7eq, 7.08g) was added through a septum with a syringe, followed by an addition of anhydrous dichloromethane (50ml) via double tipped cannula. The septum was replaced by an addition funnel, which was charged with a solution of trifluoroacetic anhydride (66mmol, 6.6eq., 13.88g), dissolved in 25 ml of anhydrous dichloromethane. The reaction mixture was cooled down to -10 °C and the solution of anhydride was added dropwise with a rate, which allows to keep the temperature of the reaction mixture below 0 °C. After the addition was complete, the cooling bath was removed and the mixture stirred at room temperature (3 h) in order to complete the reaction. The mixture was cooled using ice bath and quenched by addition of water. Organic layer was separated, washed with water (3x 20ml), brine (1x20ml), dried over sodium sulfate and evaporated to dryness yielding a crude product, which was purified by column chromatography (SiO₂, ethylacetate:methanol 50:1). Yield: 5.5g (58%), off-white powder.
H-NMR (CDCl₃): δ = 2.62 (m, 12 H); 3.3 (m, 24 H)
19F-NMR: (CDCl3) δ = -76.30

### Synthesis of compound L7

A mixture of 3,7 g of L1 (10 mmol) and 4.66 g of ClCH₂COONa (40 mmol, 33% excess) was refluxed in 50 mL of 1-butanol in a 100 mL round-bottom flask for 12 h in a nitrogen atmosphere. The butanol was then evaporated in N₂ stream, the resulting mixture was redissolved in 50 mL of 2-propanol and filtered and the 2-propanol was evaporated to yield a crude oily mixture of polyamides. After addition of 2.0 g of LiAlH₄ and 50 mL of fresh THF to the mixture, it was refluxed for 12 h in a nitrogen atmosphere. After the mixture was cooled, excess LiAlH₄ was quenched by dropwise addition of 10 mL of saturated aqueous NaOH and 20 mL of 2-propanol. Precipitated aluminium salts were separated by filtration, filtrate evaporated to dryness and a crude product was purified by column chromatography (SiO_{2,} ethylacetate:methanol 10:1 to 1:1). Yield 2.8g (53%), pale yellow oil, which crystalizes upon storage.
H-NMR (CDCl3): δ =2.43 (b, 10H), 2.72 (b, 38 H)
ESI-MS:449 [M+H]⁺, 471[M+Na]⁺

### Synthetic procedures for coordination compounds according to the present invention

All solvents and chemicals were purchased from Merck, Acros or Fischer and used without any additional purification, unless stated otherwise.

All ligands were obtained as a mixture of constitutional isomers and used without its separation, unless stated otherwise.

Starting compound were prepared using published procedures (Table 1)

**Table 1. Starting compounds prepared according to published procedures**

| | | | |
|---|---|---|---|
| 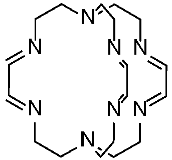 | (4E,6E,13E,15E,21E,23E)-1,4,7,10,13,16,21,24-octaazabicyclo[8.8.8]hexacosa-4,6,13,15,21,23-hexaene | | Prepared according to known procedure (Redko et al. SYNTHESIS 2006, 5, 0759-0761) |
| 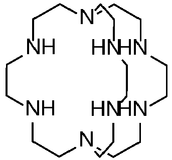 | 1,4,7,10,13,16,21,24-octaazabicyclo[8.8.8]hexacosane | | Prepared according to known procedure (Redko et al. SYNTHESIS 2006, 5, 0759-0761) |
| 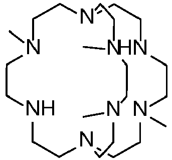 | 4,7,13,21-tetramethyl-1,4,7,10,13,16,21,24-octaazabicyclo[8.8.8]hexacosane | | Prepared according to known procedure (Farrell et al. Dalton Trans., 2006, 3204-3211) |
| 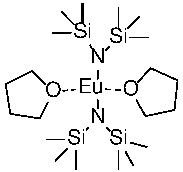 | bis(bis(trimethylsilyl)amino)-bis-tetrahydrofurane-europium (II) | - | Prepared analogous to known procedure (Tilley et al. J. Am. Chem. Soc., 104, 13, 1982) |

### 1. General synthetic procedures for ligands

### 1.1. Reductive methylation

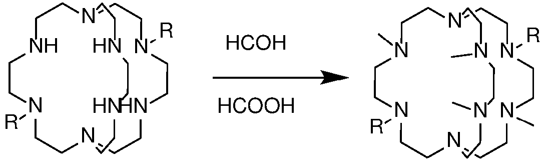

The starting compound, containing secondary amine groups, 13.8mmol (1eq) was treated with 746 mmol (54 eq.) of formaldehyde (37% aqueous solution) and 1.81 mol (55 eq.) of formic acid under reflux for 64 h. The reaction was cooled to room temperature and neutralized by an addition of solid NaHCO3 (60-70eq.). The mixture was diluted with MeOH and the remaining solid was removed by filtration, a filter cake is washed with methanol. Combined filtrate was concentrated, the solid residue extracted with 300 ml propan-2-ol. The extract was again concentrated and the residue extracted with 300 ml dichloromethane. The liquid phase was concentrated in vacuum to give a yellow oil, which was purified by column chromatography to give a mixture of the three product isomers.

### 1.2 Reductive alkylation

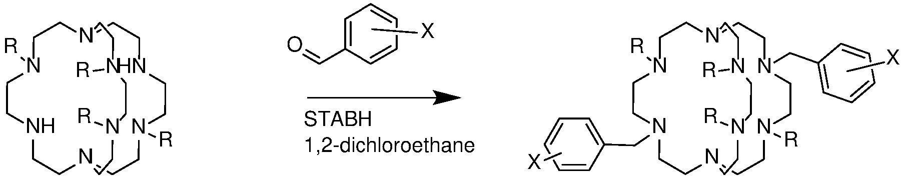

R= H, Me
X= Br, -OH

5.4 mmol (1.0 eq.) of octaamine was dissolved in 35 ml of 1,2-dichloroethane. A solution of 10.8 mmol (2.0 eq.) of corresponding aldehyde in 35 ml of 1,2-dichloroethane) was slowly added to this mixture. An addition of 15 mmol (2.78 eq.) of sodium triacetoxyborohydride (STABH) and 15 mmol (2.78 eq.) of glacial acetic acid followed. The mixture was stirred at room temperature under Argon overnight. Reaction was quenched by an addition of dry NaHCO3 powder, the solid was separated by filtration and washed 3 times with chloroform on the filter. Combined filtrate was evaporated to dryness under reduced pressure. The crude product was purified by flash chromatography.

Compounds, prepared using this general procedure:

| #Nr. | Structure/Name | | Yield, % | NMR (solvents) | ESI-MS |
|---|---|---|---|---|---|
| 1 | 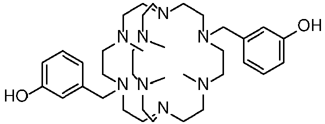 3,3'-((7,16,21,24-tetramethyl-1,4,7,10,13,16,21,24-octaazabicyclo[8.8.8]hexacosane-4,13-diyl)bis(methylene))diphenol. | | 57 | | 583.5 (M+H)⁺ |
| 2 | 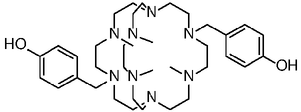 4,4'-((7,16,21,24-tetramethyl-1,4,7,10,13,16,21,24-octaazabicyclo[8.8.8]hexacosane-4,13-diyl)bis(methylene))diphenol. | | 60 | | 583.5 (M+H)⁺ |
| 3 | 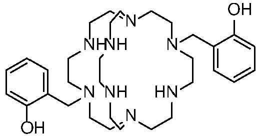 2,2'-((1,4,7,10,13,16,21,24-octaazabicyclo[8.8.8]hexacosane-4,13-diyl)bis(methylene))diphenol. | | 46 | **¹H NMR** (300 MHz, D₃COD): δ [ppm] = 7.14 (m, 4H, Ar), 6.78 (m, 4H, Ar), 3.34 (m, 4H, CH₂Ar), 2.32 - 2.74 (m, 36 H) | 583.5 (M+H)⁺ |
| 4 | 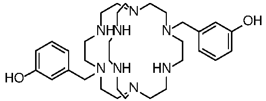 3,3'-((1,4,7,10,13,16,21,24-octaazabicyclo[8.8.8]hexacosane-4,13-diyl)bis(methylene))diphenol. | | 62 | | 583.5 (M+H)⁺ |
| 5 | 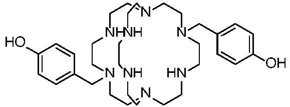 4,4'-((1,4,7,10,13,16,21,24-octaazabicyclo[8.8.8]hexacosane-4,13-diyl)bis(methylene))diphenol. | | 53 | | 583.5 (M+H)⁺ |
| 6 | 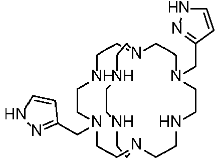 4,13-bis((1H-pyrazol-3-yl)methyl)-1,4,7,10,13,16,21,24-octaazabicyclo[8.8.8]hexacosane | | 10 | | 587.4 (M+H)⁺ |
| 7 | 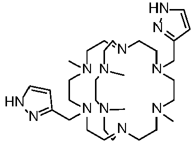 4,13-bis((1H-pyrazol-3-yl)methyl)-7,16,21,24-tetramethyl-1,4,7,10,13,16,21,24-octaazabicyclo[8.8.8]hexacosane | | 15 | | 587.4 (M+H)⁺ |

### 1.3 Alkylation with oxiranes

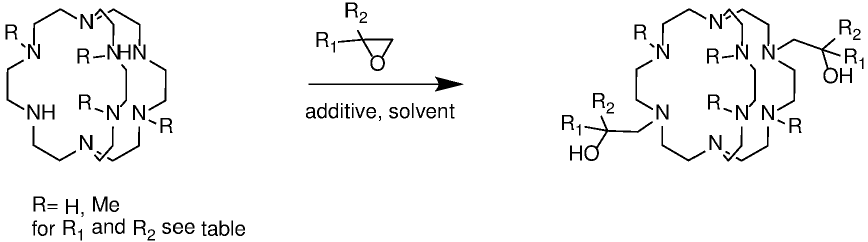

### Method a:

13.8 mmol (1.0 eq.) of octaamine was dissolved in 100 ml THF. The reaction mixture was cooled in an ice bath, and a solution of 27.7 mmol (2.0 eq.) oxirane in 100 ml THF is added dropwise. The reaction was allowed to warm to room temperature while being stirred for 48 h. The solvent was removed and the residue dried in vacuum at 60 °C yielding a crude product, which was in some occasions methylated using general reductive methylation procedure.*

In some cases, separation of the obtained constitutional isomers was achieved by crystallization out of a proper mixture of solvents, e.g. 4.2 mmol (3.3 g) of the isomer mixture XYZ was dissolved in 10 ml of CH2Cl2 and diluted with the same amount of hexane. The mixture was refrigerated at -20 °C for 48 h. The crystalline product was removed by filtration, washed with 2 ml hexane and dried in vacuum to give 1.1 mmol (0.9 g) of one pure isomer. The supernatant mixture was concentrated and dried in vacuum to give 3.1 mmol (2.4 g) of mixture of the remaining isomers. The constitution was not be determined unambiguously. **

### Method b:

Octaamine (5,4 mmol, 1,0 eq.) was placed into a reactor, melted and degassed in vacuum at 150° C. The reactor was filled with N2 gas. The amine was cooled down to RT and 15 ml of oxygen-free anhydrous acetonitrile was added to dissolve the solid. A solution of oxirane (10,8 mmol, 2.0 eq.) in 5 ml of oxygen-free anhydrous acetonitrile was dropwise added, followed by an optional addition of lithium tetrafluoroborate (10,79 mmol, 2 eq.) in 10 ml anhydrous oxygen-free acetonitrile. The mixture was stirred for 5 days under reflux, then evaporated to dryness. The solid residue was extracted with toluene, extracts were combined and filtered. Evaporation of the solvent under reduced pressure yielded a crude product, which was in some occasions methylated using general methylation procedure

Compounds, prepared using this general procedure:

| #Nr. | Structure/Name | Method /additive | Yield, % | NMR (solvents) | ESI-MS |
|---|---|---|---|---|---|
| | 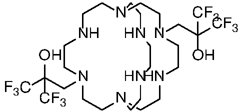 2,2'-((1,4,7,10,13,16,21,24-octaazabicyclo[8.8.8]hexacosane-4,13-diyl)bis(methylene))bis(1,1,1,3,3,3-hexafluoropropan-2-ol) | a | 90 | | 731 (M+H)⁺ |
| | 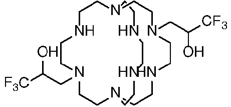 3,3'-(1,4,7,10,13,16,21,24-octaazabicyclo[8.8.8]hexacosane-4,13-diyl)bis(1,1,1-trifluoropropan-2-ol) | a | 82 | | 595 (M+H)⁺ |
| | 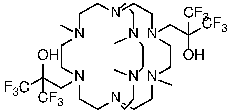 2,2'-((7,16,21,24-tetramethyl-1,4,7,10,13,16,21,24-octaazabicyclo[8.8.8]hexacosane-4,13-diyl)bis(methylene))bis(1,1,1,3,3,3-hexafluoropropan-2-ol) | a*,** | 22 % - pure (82 % - mixture) | **¹H NMR** (300 MHz, CDCl₃): δ [ppm] = 10.28 (br.s., 2 H, *OH*), 2.93 - 2.99 (s, 4 H, N-C*H*₂-CR₃), 2.36 - 2.91 (m, 36 H, C*H₂*), 2.26 (s, 12 H, C*H₃*) **¹⁹F NMR** (282 MHz, CDCl₃) = - 77.7 (s, C*F₃*) | 787 (M+H)⁺ |
| | 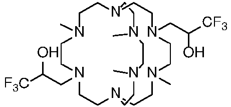 | a*,** | | | 651.5 (M+H)⁺ |
| | 3,3'-(7,16,21,24-tetramethyl-1,4,7,10,13,16,21,24-octaazabicyclo[8.8.8]hexacosane-4,13-diyl)bis(1,1,1-trifluoropropan-2-ol) | | | | |
| | 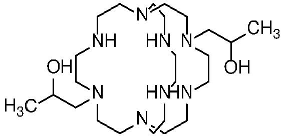 1,1'-(1,4,7,10,13,16,21,24-octaazabicyclo[8.8.8]hexacosane-4,13-diyl)bis(propan-2-ol) | b (with additive) | | | 487.4 (M+H)⁺ |
| | 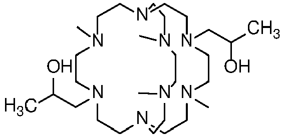 1,1'-(7,16,21,24-tetramethyl-1,4,7,10,13,16,21,24-octaazabicyclo[8.8.8]hexacosane-4,13-diyl)bis(propan-2-ol) | b* (with additive), ** | | | 543.5 (M+H)⁺ |
| | 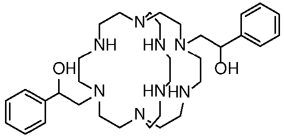 2,2'-(1,4,7,10,13,16,21,24-octaazabicyclo[8.8.8]hexacosane-4,13-diyl)bis(1-phenylethan-1-ol) | b (without additive) | | | 611.5 (M+H)⁺ |
| | 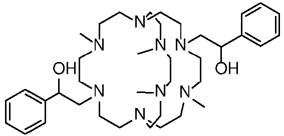 2,2'-(7,16,21,24-tetramethyl-1,4,7,10,13,16,21,24-octaazabicyclo[8.8.8]hexacosane-4,13-diyl)bis(1-phenylethan-1-ol) | b* (without additive), ** | | | 667,5 (M+H)⁺ |

### 1.4 Alkylation with alkylhalides

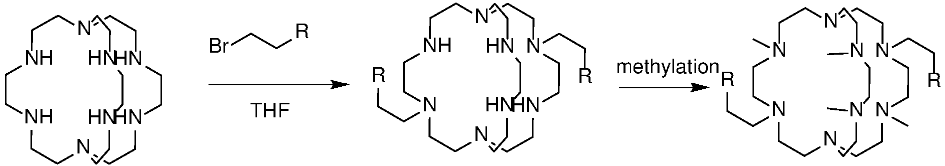

To a solution of 5g of 13.49mmol of 1,4,7,10,13,16,21,24-octaazabicyclo[8.8.8]hexacosane in 100ml of anhydrous THF, a solution of corresponding alkylhalide (29.68mmol, 2.2eq.) is added in one portion. Reaction is stirred under reflux for 12h, precipitated cryptand salt of hydrogen halides is separated by filtration, washed with THF, and re-dispersed in THF. After addition of 5eq (ca. 5ml) of 35% ammonia solution, two phases are formed. Organic phase is separated, aqueous phase is extracted with chloroform. Combined organic layers are evaporated to dryness yielding a crude product, which was used without further purification or in some occasions methylated using general methylation procedure.*

Compounds, prepared using this general procedure:

| #Nr. | Structure/Name | | Yield, % | NMR (solvents) | ESI-MS |
|---|---|---|---|---|---|
| | 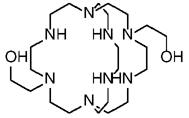 2,2'-(1,4,7,10,13,16,21,24-octaazabicyclo[8.8.8]hexacosane-4,13-diyl)bis(ethan-1-ol) | | | | 459.4 (M+H)⁺ |
| | 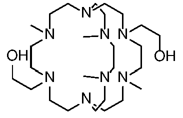 2,2'-(7,16,21,24-tetramethyl-1,4,7,10,13,16,21,24-octaazabicyclo[8.8.8]hexacosane-4,13-diyl)bis(ethan-1-ol) | * | | | 515.5 (M+H)⁺ |
| | 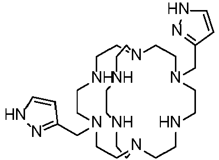 4,13-bis((1H-pyrazol-3-yl)methyl)-1,4,7,10,13,16,21,24-octaazabicyclo[8.8.8]hexacosane | | | | 531.4 (M+H)⁺ |
| | 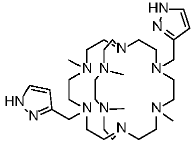 4,13-bis((1H-pyrazol-3-yl)methyl)-7,16,21,24-tetramethyl-1,4,7,10,13,16,21,24-octaazabicyclo[8.8.8]hexacosane | * | | | 587.4 (M+H)⁺ |

### 2. Synthesis of ligands with boron based anionic groups

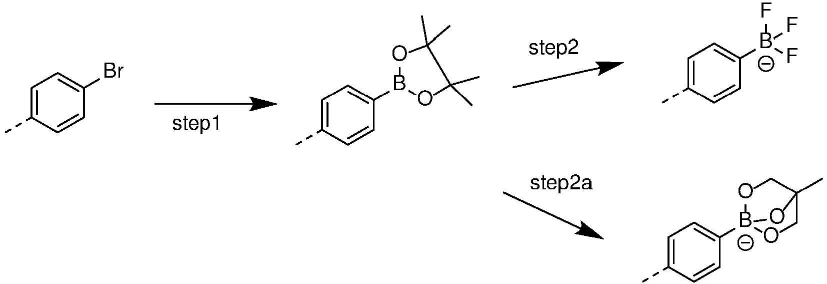

### Step1: 4,7,13,21-tetramethyl-16,24-bis(4-(4,4,5,5-tetramethyl-1,3,2-dioxaborolan-2-yl)benzyl)-1,4,7,10,13,16,21,24-octaazabicyclo[8.8.8]hexacosane

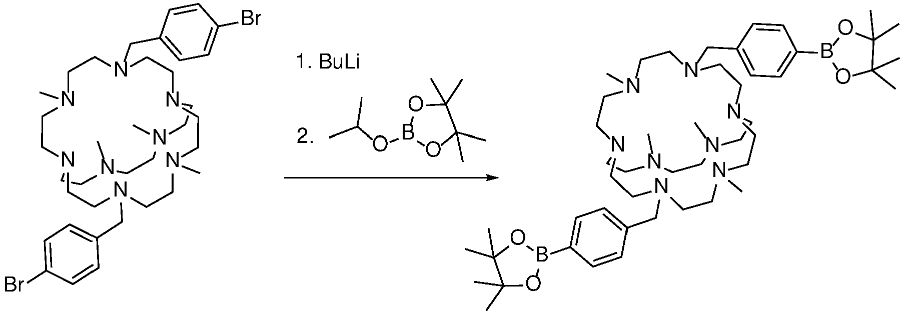

Into a single neck round bottom flask, purged with Ar, 1.4 mmol (1.0 eq.) of octaamine and dry THF (25 mL) were added. A solution of n-butyllithium (2.8 mmol, 2.0 eq., 2.5 M solution in hexane) was added dropwise at -78 °C and the mixture was stirred for one hour. 3.5 mmol (2.5 eq.) of 2-isopropoxy-4,4,5,5-tetramethyl-1,3,2-dioxaborolane was added dropwise at -78 °C and the mixture was stirred for 30 minutes before being very slowly allowed to room temperature and afterwards stirred overnight. The reaction was then quenched by the addition of water and the product was extracted with chloroform. The extract was dried over MgSO4 and the solvent was removed under reduced pressure.
Yield: 92 %; **¹H NMR** (300 MHz, CDCl₃): δ [ppm] = 7.68 (d, 4H, Ar), 7.26 (d, 4H, Ar), 3.58 (m, 4H, CH₂Ar), 2.33 - 2.76 (m, 36 H), 2.07 - 2.27 (m, 12 H); **¹¹B NMR** (300 MHz, CDCl₃): δ [ppm] = 33.9; **MS (ESI)**: m/z = 859.7 (M+H)

The same procedure was used for the synthesis of following prepared compounds:

| Nr. | Structure/Name | Yield, % | NMR (solvents) | ESI-MS | |
|---|---|---|---|---|---|
| 1. | 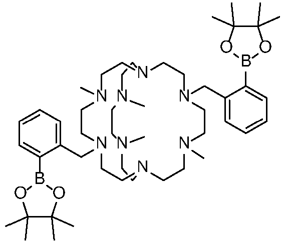 4,7,13,21-tetramethyl-16,24-bis(2-(4,4,5,5-tetramethyl-1,3,2-dioxaborolan-2-yl)benzyl)-1,4,7,10,13,16,21,24-octaaza bicyclo [8.8.8] hexacosa ne | | | 859.7 (M+H)⁺ | |
| 2 | 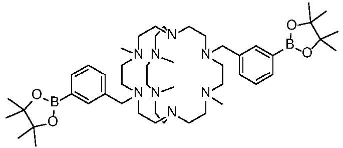 4,7,13,21-tetramethyl-16,24-bis(3-(4,4,5,5-tetramethyl-1,3,2-dioxaborolan-2-yl)benzyl)-1,4,7,10,13,16,21,24-octaaza bicyclo [8.8.8] hexacosa ne | | | 859.7 (M+H)⁺ | |

### Step2: Tetrabutylammonium (((7,16,21,24-tetramethyl-1,4,7,10,13,16,21,24-octaazabicyclo[8.8.8]hexacosane-4,13-diyl)bis(methylene))bis(4,1-phenylene))bis(tri-fluoroborate)

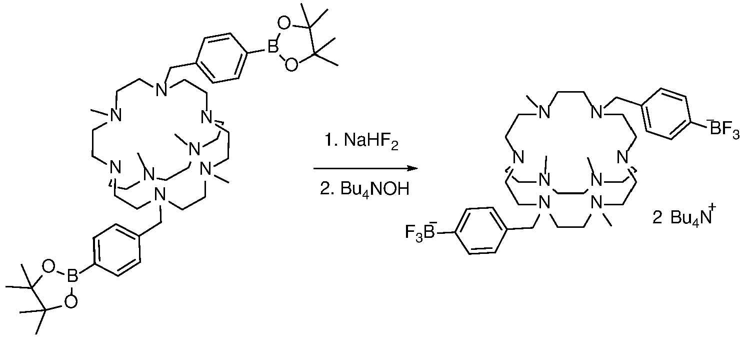

The reaction was carried out under ambient conditions. Into a single neck round bottom flask 2.6 mmol (1.0 eq.) of borolan and MeOH (24 mL) were added. 30 ml water solution of 52.4 mmol (20 eq.) NaHF2 was added dropwise by vigorously stirring and the mixture was stirred very intensively overnight. Water solution of Bu4NOH was then added till pH = 8.5. The product was extracted with chloroform. The extract was dried over MgSO4 and the solvent was removed under reduced pressure.
Yield: 84 %; **¹H NMR** (300 MHz, CDCl₃): δ [ppm] = 7.43 (d, 4H, Ar), 7.04 (d, 4H, Ar), 3.44 (m, 4H, CH₂Ar), 2.99 (m, 8H, Bu₄N), 2.34 - 2.76 (m, 36 H), 2.09 - 2.25 (m, 12 H), 1.41 (m, 8H, Bu₄N), 1.26 (m, 8H, Bu₄N), 0.87 (t, 12H, Bu₄N); **¹¹B NMR** (300 MHz, CDCl₃): δ [ppm] = 33.9; **MS (ESI)**: m/z = 370.3 (A²⁻); 242.3 (K⁺)

The same procedure was used for the synthesis of following compounds:

| Nr. | Name | Yield, % | NMR (solvents) | ESI-MS |
|---|---|---|---|---|
| 1. | 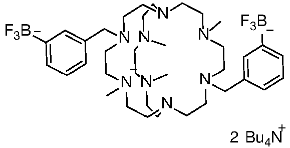 Tetrabutylammonium (((7,16,21,24-tetramethyl-1,4,7,10,13,16,21,24-octaazabicyclo[8.8.8]hexacosane-4,13-diyl)bis(methylene))bis(3,1-phenylene))bis(trifluoroborate) | | | 370.3 (A²⁻) |
| 2 | 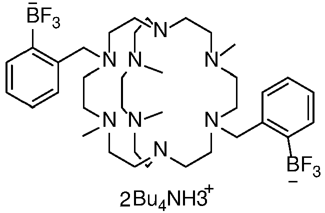 Tetrabutylammonium (((7,16,21,24-tetramethyl-1,4,7,10,13,16,21,24-octaazabicyclo[8.8.8]hexacosane-4,13-diyl)bis(methylene))bis(2,1-phenylene))bis(trifluoroborate) | | | 370.3 (A²⁻) |

### Step2a: Lithium(I) 1,1'-(4,4'-(13,16,21-trimethyl-1,4,7,10,13,16,21,24-octaazabicyclo[8.8.8]hexacosane-4,24-diyl)bis(methylene)bis(4,1 phenylene))bis(4 methyl 2,6,7 trioxa 1 boranuidabicyclo[2.2.2]octane).

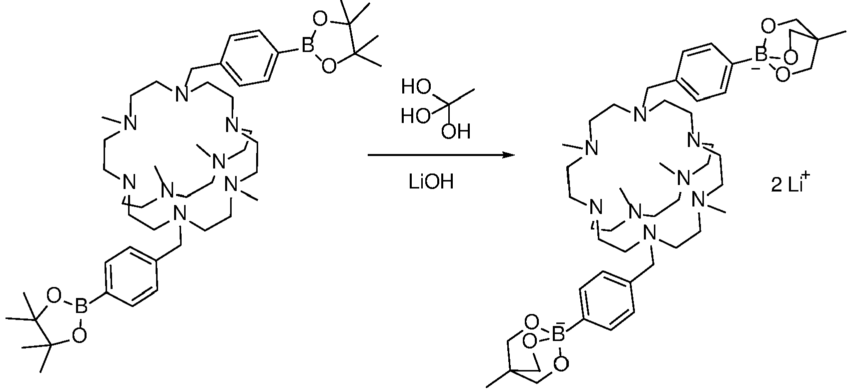

Into a single neck round bottom flask equipped with a Dean-Stark receiver, 1.7 mmol (1.0 eq.) of borolan, 1.7 mmol (1.0 eq.) of 1,1,1-tris(hydroxymethyl)ethane, 1.7 mmol (1.0 eq.) of LiOH and dry toluene (100 mL) were added. The mixture was boiled 5 hours. Precipitate was filtered, washed with toluene and cyclohexane.

Yield: 82 %; **¹H NMR** (300 MHz, DMSO): δ [ppm] = 7.65 (d, 4H, Ar), 7.21 (d, 4H, Ar), 3.65 (m, 6H), 3.52 (m,4H, CH₂Ar), 2.32 - 2.74 (m, 36 H), 2.07 - 2.27 (m, 12 H), 0.66 (s, 3 H); **MS (ESI)**: 430.3 (A²⁻).

The same procedure was used for the synthesis of following compounds:

| Nr. | Name | Yield, % | NMR (solvents) | ESI-MS |
|---|---|---|---|---|
| 1. | 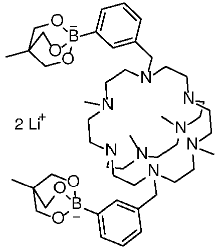 Lithium(I) 1,1'-(4,4'-(13,16,21-trimethyl-1,4,7,10,13,16,21,24-octaazabicyclo[8.8.8]hexacosane-4,24-diyl)bis(methylene)bis(3,1-phenylene))bis(4-methyl-2,6,7-trioxa-1-boranuidabicyclo[2.2.2]octane) | | | 430.3 (A²⁻) |
| 2 | 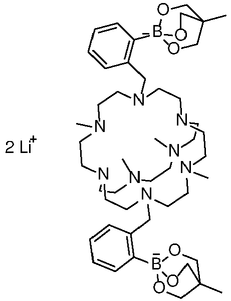 Lithium(I) 1,1'-(4,4'-(13,16,21-trimethyl-1,4,7,10,13,16,21,24-octaazabicyclo[8.8.8]hexacosane-4,24-diyl)bis(methylene)bis(2,1-phenylene))bis(4-methyl-2,6,7-trioxa-1-boranuidabicyclo[2.2.2]octane) | | | 430.3 (A²⁻) |

### 3. Synthesis of the emitters

### 3.1 Via cation exchange reaction (Method E)

To a solution of alkali metal or tetrabutylammonium salt of corresponding ligand (1,5mmol, 1eq) in THF (10ml), a solution of Europium Iodide (II) (1.425mmol, 0.95 eq.) in 10ml of THF was added dropwise. Solvent is evaporated and residue is extracted with chloroform. Combined extracts were evaporated to dryness yielding a crude product as sticky solid. Upon tritration of the crude mixture with hexane, a white suspension is formed. The product is separated by filtration and dried in vacuum.

### 3.2. Via reaction with bis(bis(trimethylsilyl)amino)-bis-tetrahydrofurane-europium (II) (Method F)

To a solution of 0.8 mmol (1.0 eq.) ligand, bearing acidic protons, in THF (15 ml), a solution of 0.76 mmol (0.95 eq.) of bis(bis(trimethylsilyl)amino)-bis-tetrahydrofurane-europium (II) in 15 ml THF is added dropwise. The resulting mixture was stirred for 1 h at room temperature. The volatiles were removed in vacuum. Residue is dissolved in an appropriate solvent (THF or dichloromethane) and precipitated with hexane. Product is separated by filtration and dried in vacuum.

### 3.3 Via anion exchange reaction (Method G)

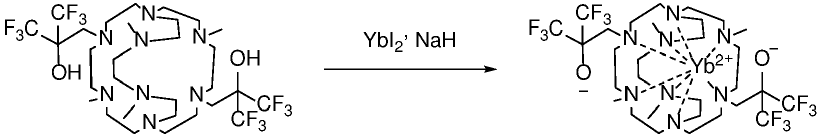

To a solution of 0.25 mmol (1.0 eq.) ytterbium (II) iodide in 5 ml THF was added dropwise a solution of 0.25 mmol (1.0 eq.) of the ligand in 6 ml THF. The resulting mixture was stirred for 10 min at room temperature and treated with 0.50 mmol (2.0 eq.) sodium hydride. Stirring was continued for 30 min. The mixture was filtered and the volatiles removed in vacuum. The residue is recrystallized from THF/hexane, filtered and dried in vacuum.

| Nr | Structure | Code | Yield (%)/ method | MS | Elementar analysis (calculated/found) | Emission λmax, nm (solvent) | PLQY, % | LT, µs |
|---|---|---|---|---|---|---|---|---|
| 1 | 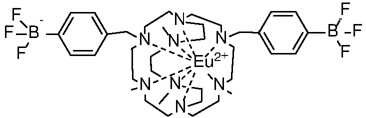 | 774 | 37/E | | C, 48.45; H, 6.78; N, 12.56 / | 464 (MeOH) | 1.7 | 1.04 |
| 2 | 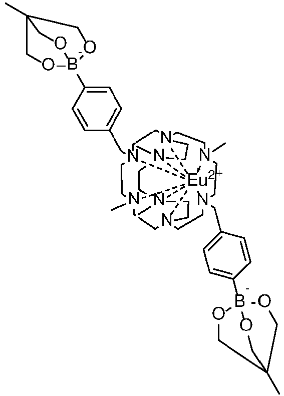 | 775 | 20/E | | C, 54.55; H, 7.76; N, 11.06 / | 448 (acetonitrile) | 22.5 | 1.2 |
| 3 | 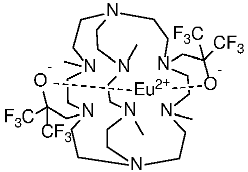 | 776 | 95/E | | C, 38.47; H, 5.60; N, 11.96 / | 527 (THF) | 40 | 0.72 |
| | | | | | | 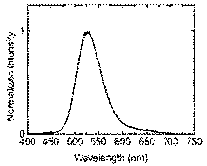 | | |
| 4 | 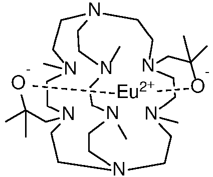 | 777 | | | C, 49.99; H, 8.95; N, 15.54 / | -- | -- | -- |
| | 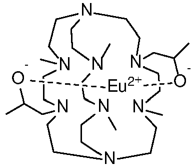 | 778 | 80/E | | C, 48.54; H, 8.73; N, 16.17 / | 480 (THF) | -- | 0.67 |
| 5 | | | | | | 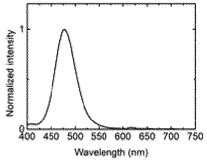 | | |
| 6 | 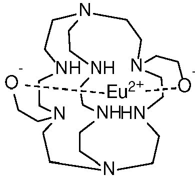 | 779 | 30/E | | C, 43.41; H, 7.95; N, 18.41/ | -- | -- | -- |
| 7 | 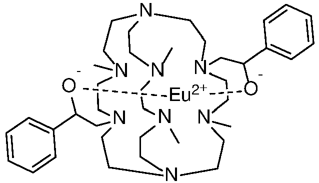 | 780 | | | C, 55.87; H, 7.90; N, 13.72 / | -- | -- | -- |
| 8 | 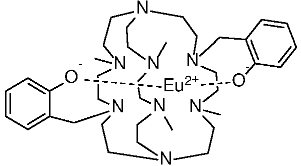 | 781 | | | C, 54.81; H, 7.67; N, 14.20 / | -- | -- | -- |
| 9 | 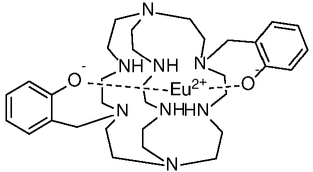 | 782 | | | C, 52.45; H, 7.15; N, 15.29/ | -- | -- | -- |
| 10 | 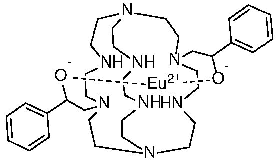 | 783 | | | C, 53.67; H, 7.42; N, 14.73/ | -- | -- | -- |
| 11 | 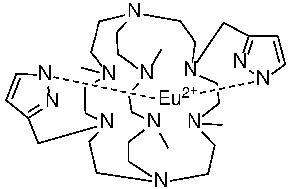 | 784 | | | C, 48.90; H, 7.66; N, 22.81/ | -- | -- | -- |
| 12 | 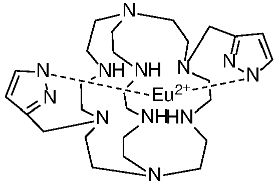 | 785 | | | C, 45.88; H, 7.11; N, 24.69/ | -- | -- | -- |
| 13 | 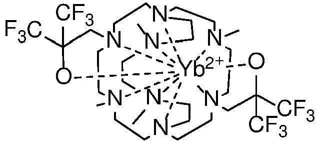 | 786 | 92/G | 961.3 (M+H)⁺ | C, 37.62; H, 5.47; N, 11.70/ | 450, 573 (THF) | | |
| | | | | | | 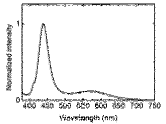 | | |

### General procedures for the synthesis of the coordination compounds

### Experimental part

### Devices.

Representative embodiments of an organic electronic device according to various embodiments will now be described, including a detailed description of the fabrication process of the organic electronic device. Yet, it may be understood that neither the specific techniques for fabrication of the device, nor the specific device layout, nor the specific compounds are intended to limit the scope.

### Material definitions:

| | |
|---|---|
| ITO: | Indium tin oxide |
| MoO3: | Molybdenum oxide |
| NPB: | N,N'-Di(1-naphthyl)-N,N'-diphenyl-(1,1'-biphenyl)-4,4'-diamine |
| TAPC: | 1,1-Bis[(di-4-tolylamino)phenyl]cyclohexane |
| TPBI: | 2,2',2-(1,3,5-Benzinetriyl)-tris(1-phenyl-1-H-benzimidazole) |
| Cs: | Cesium |
| Al: | Aluminium |

NPB, TAPC, TPBI were purchased from Lumtec and were used as-received.

### Device preparation:

The organic electronic device was prepared with the following layer sequence:
ITO / MoO3 (1 nm) / NPB (50 nm) / TAPC (10 nm) / TAPC:**Compound 3** (7 wt%) (20 nm) / TPBI (50 nm) / Cs / Al

The organic electronic device was fabricated on a 1"x1" size glass substrate, precoated with a transparent 90 nm thick ITO anode. The substrate was cleaned with various solvents in an ultrasonic bath and subsequently exposed to UV oxygen plasma for 10 min.

Next, the substrate was transferred in a sealed transferring container to a thermal evaporation chamber operating at around 10⁻⁷ mbar pressure. Here, MoO3 (1 nm), NPB (50 nm), TAPC (10 nm) were evaporated subsequently. The evaporation rate during processing was measured by a quartz crystal microbalance. Then, a 20 nm film of TAPC doped with above compound 3 was co-evaporated, ensuring a 7 wt% doping concentration by controlling the evaporation rates of each material respectively. Afterwards the evaporation continued with a 50 nm film of TPBi, followed by a thin Cs interlayer. The device was finished by evaporating a 100 nm film of cathode Al. The Al deposition was carried out using a structured shadow mask, such that the resulting overlap of pre-structured ITO, organic layers and Al gave an active area of 6.25 mm². Subsequently, the device was transferred to a nitrogen atmosphere glovebox and encapsulated by gluing an additional encapsulating glass substrate on top to prevent oxygen- and water-induced degradation. The electrical and electroluminescent properties of the device were measured using a Keithley 2400 source meter and by placing the device in an Ulbricht sphere with a calibrated Si photodiode and OceanOptics spectrometer.

The device showed turn-on at 6 V and reached 100 cd/m² luminance values at 11 V.

Resulting LV and EQE curves are presented in **FIG 13 and FIG 14****, respectively.**

FIG 13 show the Voltage-luminance characteristic of DEVICE. FIG 14 show the EQE-characteristic of DEVICE.

## Claims

1. A metal-organic coordination compound, which is neutrally charged and wherein the coordination compound comprises at least one divalent lanthanide coordinated by a cyclic organic ligand, wherein the cyclic organic ligand contains two monoanionic groups covalently linked with the cyclic organic ligand and being separated from each other by a sequence of at least two intervening atoms.

2. The coordination compound according to claim 1, wherein the divalent lanthanide is Europium or Ytterbium and/or wherein the cyclic organic ligand is bicyclic or polycyclic.

3. The metal organic coordination compound according to one of claims 1 or 2, wherein the coordination compound comprises at least one divalent lanthanide coordinated by a cyclic organic ligand having the formula 1-1 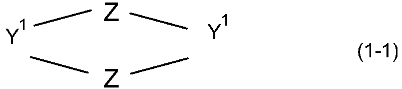
wherein Y¹ for each occurrence is independently a divalent group containing 1 to 30 atoms,
or formula 1-2 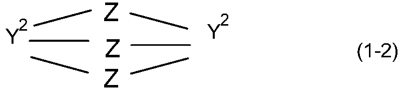
wherein Y² for each occurrence is independently a trivalent group containing 1 to 30 atoms,
Z for each occurrence is independently a divalent organic group, wherein Z has a shortest sequence of at least 3 atoms, preferably 3 to 5 atoms, between the two linkages with Y¹ or Y² in case Y¹ or Y² is a cyclic group connected with each Z via different ring atoms,
wherein Z has a shortest sequence of at least 6 atoms, preferably 6 to 8 atoms, in case Y¹ or Y² is connected with each Z via one atom,
wherein the cyclic organic ligand of formula 1-1 and 1-2 contains two monoanionic groups covalently linked with the cyclic organic ligand and being separated from each other by a sequence of at least two intervening atoms, or wherein the coordination compound comprises at least one divalent lanthanide coordinated by a cyclic organic ligand according to formula 3-1 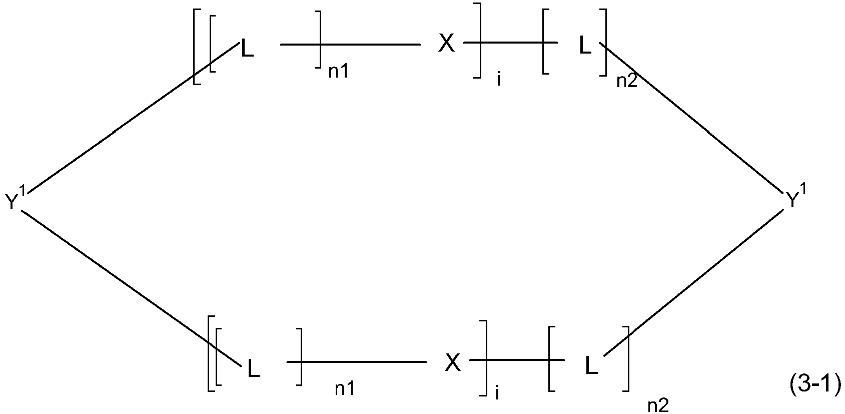
wherein
• Y¹ for each occurrence independently is a divalent organic group, preferably a divalent cyclic group that has two valences at different ring positions, or is 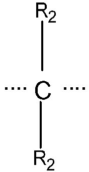
or according to formula 3-2 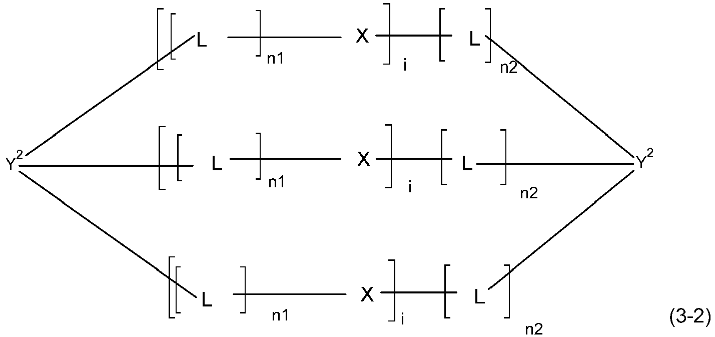
wherein
• Y² for each occurrence independently is a trivalent organic group, preferably C-R₂ or a cyclic group that has three valences at different ring positions, or is B or B-R₂⁻ or N or P,
• X is independently selected for each occurrence from the group of: 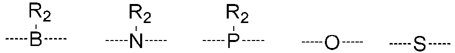
• L for each occurrence independently is a divalent linear, branched or cyclic organic group that can be substituted and that is formed by removing two hydrogen atoms from an organic linear, branched or cyclic molecule that can be substituted, or is a divalent group -CR₁R₁-
• wherein R₁ and R₂ are hydrogen or any covalently bound substituents being identical or different in each occurrence and
• n1, n2, i independently are equal to 1, 2, 3 or 4,
• wherein the cyclic organic ligand of formula 3-1 and R-2 contains two monoanionic groups covalently linked with the cyclic organic ligand and being separated from each other by a sequence of at least two intervening atoms.
wherein in the divalent groups -CR₁R₁- the two R₁ can be covalently linked with each other, thereby forming a cyclic group, and in -CR₂R₂- the two R₂ can be covalently linked to form a cyclic structure,

4. The coordination compound according to claim 3, wherein the compound of formula 3-2 has one or more of the following features:
- Y² is B or B-R₂⁻ or N, wherein preferably R₂ is alkyl, alkoxy, carboxy, aryl, aryloxy or F,
- all three structural elements 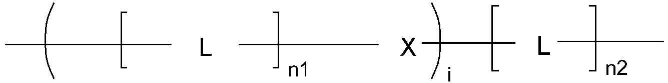 in the compound of formula 3-2 are identical,
- divalent cyclic organic groups L for each occurrence independently are divalent cyclic organic groups that can be substituted and that are formed by removing two hydrogen atoms from neighboring carbon and/or nitrogen atoms in the ring of an organic cyclic molecule that can be substituted, n1 and n2 being 1,
- cyclic organic groups are carbocyclic or heterocyclic groups, the heteroatoms being selected from P, N, Si, O, S
- if in 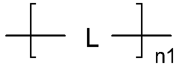 and/or 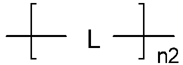 L is -CR¹R¹-, then n1 and/or n2 is 2, 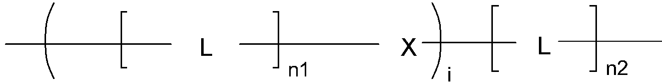 contains two groups X being 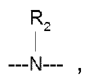 wherein both R₂ together form a group 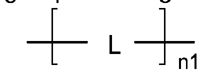 which is identical with group 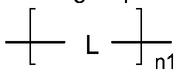 linking the two groups X,
- i is 2.

5. The coordination compound according to any one of claims 3 or 4, the cyclic organic ligand having a structure according to formula 2a, 2c, 2e or 2f: 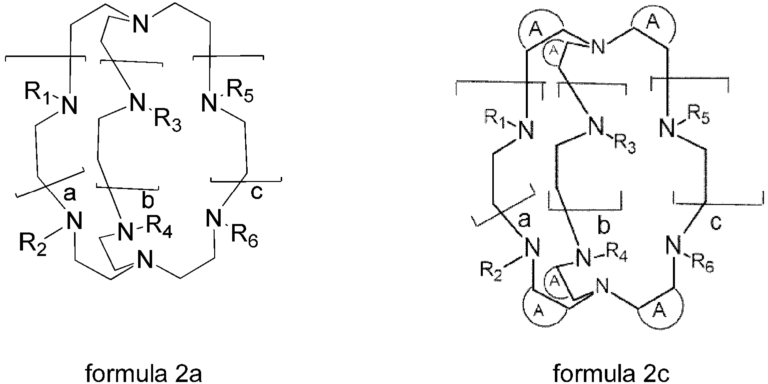 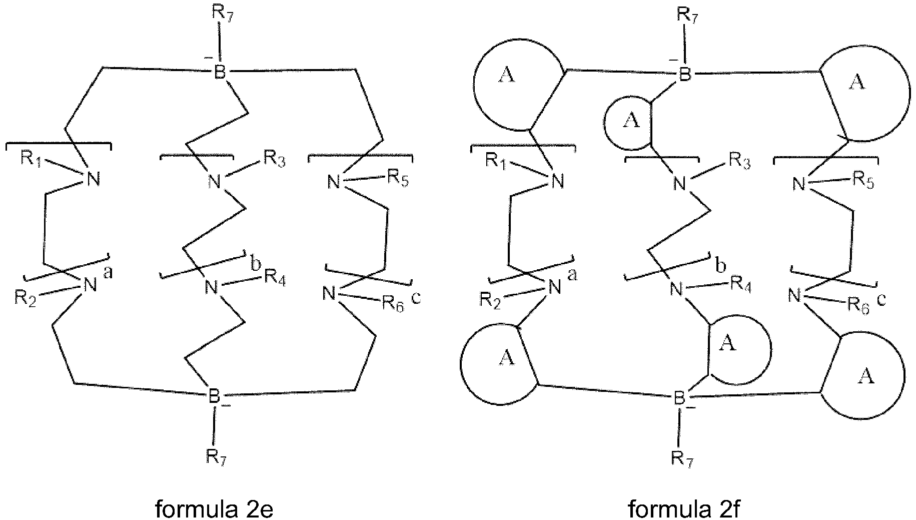 wherein
- R₁, R₂, R₃, R₄, R₅, R₆ in formula 2a and 2c independently in each occurrence represent hydrogen or a substituent, and R₁, R₂, R₃, R₄, R₅, R₆, R₇ in formula 2e, 2f independently in each occurrence represent hydrogen or a substituent,
- **a, b, c** are each independently an integer of 0 or more,
- 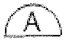 independently in each occurrence represents a divalent cyclic organic group, wherein one or more of the ring-forming carbon atoms can be substituted or can be part of a cyclic group,
wherein the cyclic organic ligand of formula 2a and 2c contains two monoanionic groups covalently linked with the cyclic organic ligand and being separated from each other by a sequence of at least two intervening atoms, wherein preferably the monoanionic group has the anionic charge in a β- or γ-position to the ring carbon or nitrogen atom to which the monoanionic group is covalently bound, or
wherein in formula 2a and 2c,
R₁ or R₂ can be a monoanionic group,
or a ring carbon atom adjacent to N-R₁ or N-R₂ can be substituted by a monoanionic group,
R₃ or R₄ can be a monoanionic group, or a ring carbon atom adjacent to N-R₃ or N-R₄ can be substituted by a monoanionic group, or
R₅ or R₆ can be a monoanionic group, or a ring carbon atom adjacent to N-R₅ or N-R₆ can be substituted by a monoanionic group,
with a total of two monoanionic groups covalently linked with the cyclic organic ligand.

6. The coordination compound according to claim 5, wherein the monoanionic group has the structure
- CH₂-CR'R'-X with X being a monoanionic atom selected from O⁻ and S⁻, or has the structure 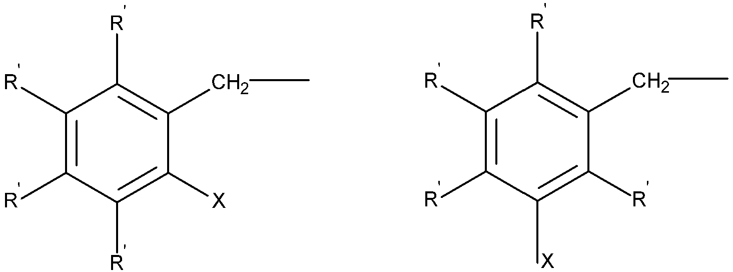
with X being a monoanionic atom selected from O⁻ and S⁻,
with each R' independently being H, substituted or unsubstituted C₁₋₁₂-alkyl, substituted or unsubstituted aryl, halogen, and wherein one R' of the monoanionic group can be NO₂, and wherein two groups R' can be covalently linked to form a cyclic group,
or is selected from the following structures 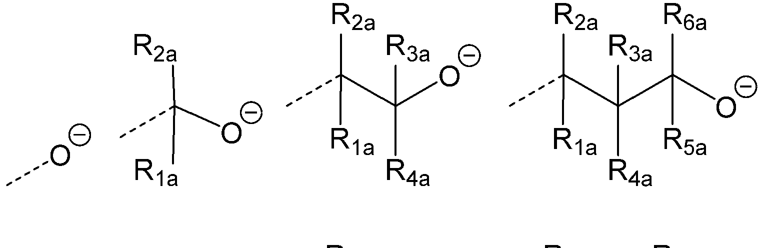 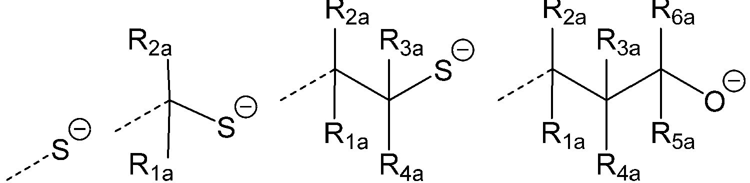 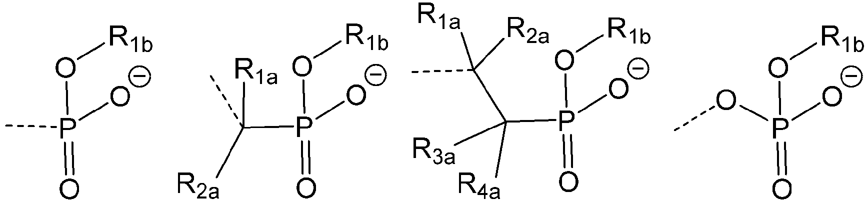 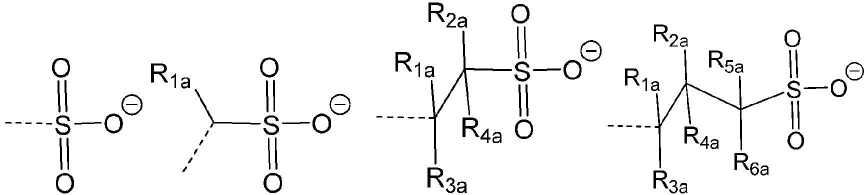 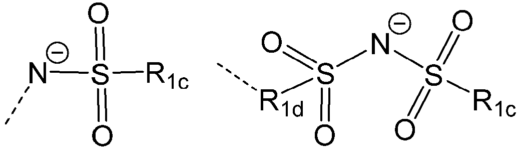 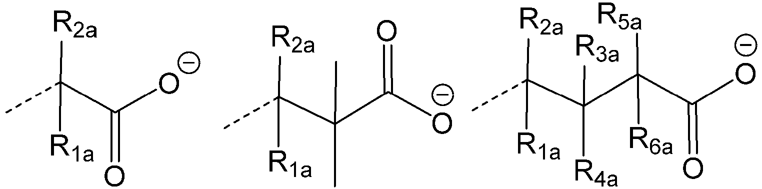 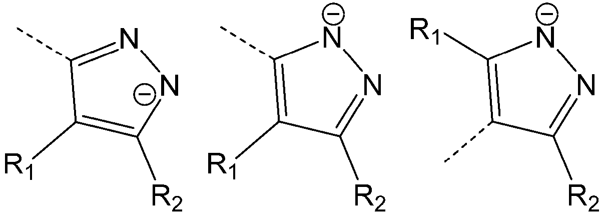 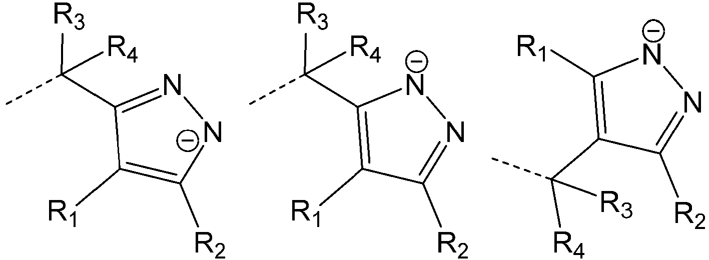 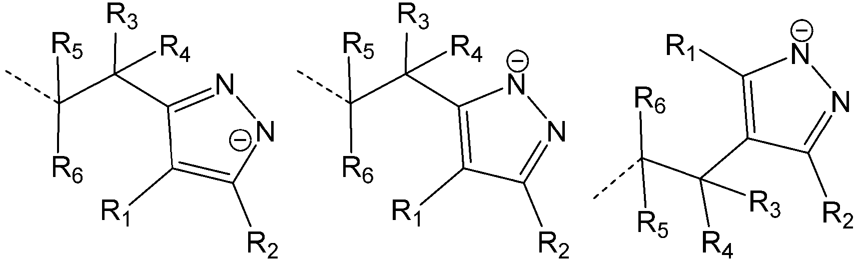 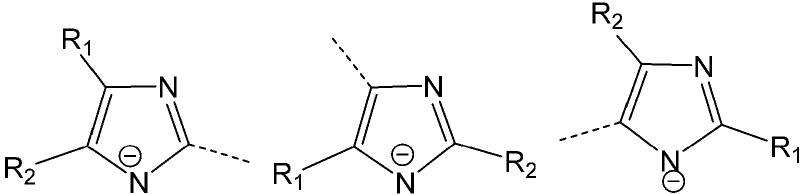 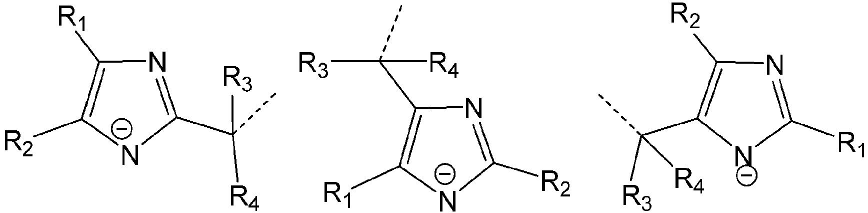 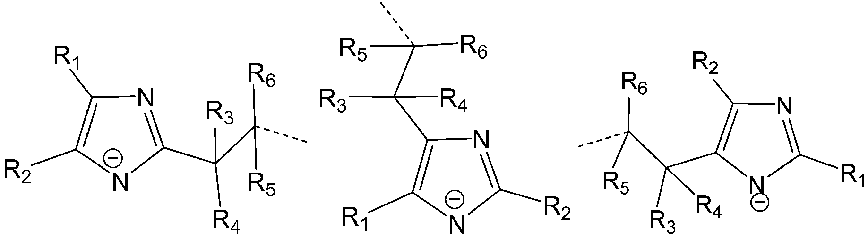 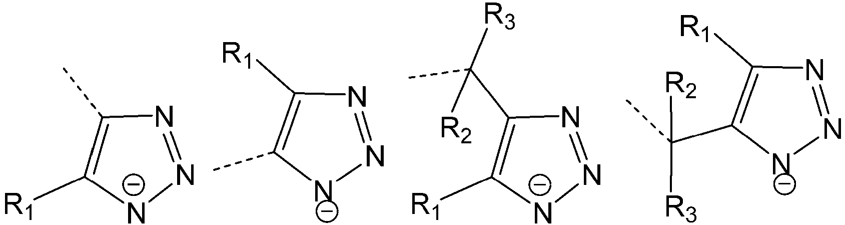 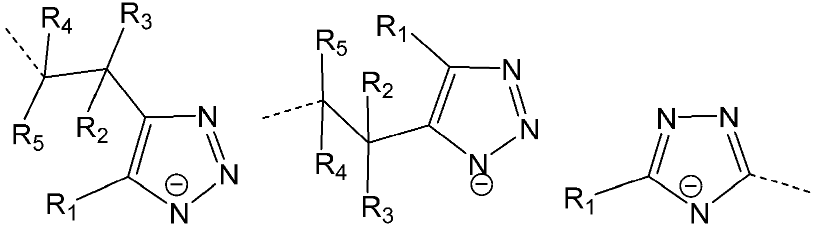 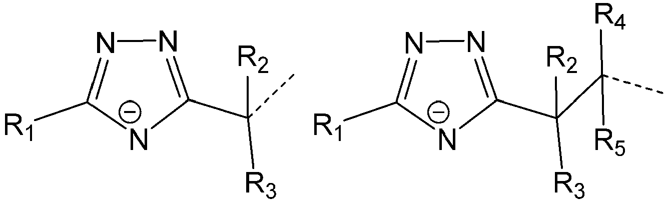 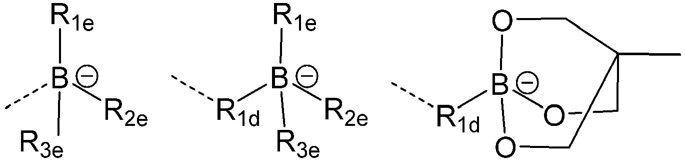 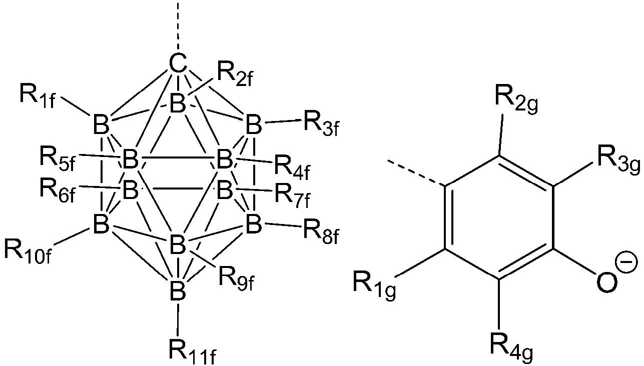 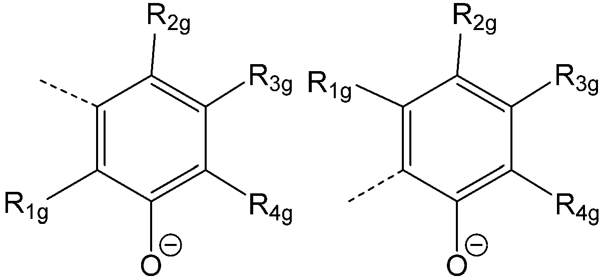 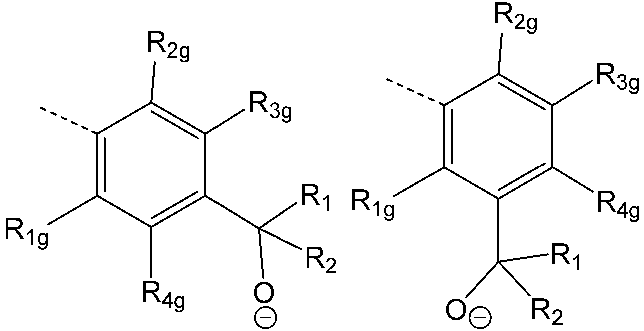 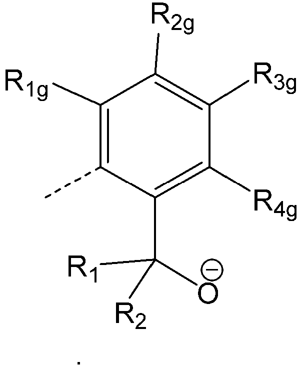
with
R₁ to R₆ independently being H or organyl,
R₁ₐ to R₆ₐ independently being H, CH₃, CF₃, CN, For CₙH₂ₙ₊₁ with n being an integer of 1 to 5,
R_{1b} being CₙH₂ₙ₊₁ with n being an integer of 1 to 5,
R_{1c} being CH₃ or CF₃,
R_{1d} being a divalent organic fragment, preferably selected from alkylene, perfluoroalkylene, which optionally can be substituted,
R₁ₑ to R₃ₑ independently being a 5-membered heteroaryl group with up to 3 heteroatoms, selected from N,S, O, which optionally can be substituted, or a 6-membered aryl or heteroaryl group which can contain up to 4 heteroatoms, selected from N, S, O, preferably from N, and which optionally can be substituted, or a group selected from CF₃, F, H, OMe, OEt R_{1f} to R_{11f} independently being H, Cl, Br, F, CH₃, CF₃ R_{1g} to R_{4g} independently being H, organyl, halogen, preferably F, CF₃,CN, OMe.

7. The coordination compound according to claim 3, wherein each Y¹ is
independently selected from 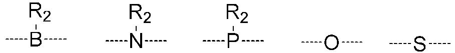 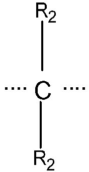
or each Y¹ is independently selected from 3 - to 15-membered, preferably 4-to 12-membered, cyclic organic groups that can contain 1 to 4 hetero atoms selected from N, B, P, O, S and is connected with each Z via a non-neighboring carbon or hetero atom, wherein R₂ is hydrogen or any covalently bound substituent being identical or different in each occurrence, or
wherein each Y² is independently selected from B, B-R₂⁻, N, P, C-R₂, or each Y² is independently selected from 3- to 15-membered, preferably 6- to 12-membered cyclic organic groups that can contain one to four hetero atoms selected from N, B, P, O, S, and is connected with each Z via a non-neighboring carbon or hetero atom,
wherein R₂ is hydrogen or any covalently bound substituent being identical or different in each occurrence.

8. The coordination compound according to one of claims 3 or 7, wherein each Z contains at most one monoanionic group, or wherein one Z contains two monoanionic groups and the others Z, Y¹ and Y² do not contain monoanionic groups.

9. The coordination compound according to one of claims 1 to 8, wherein the cyclic organic ligand contains exactly two monoanionic groups and therefore is twice negatively charged and in combination with the divalent lanthanide forms a complex of neutral charge, wherein preferably the cyclic organic ligand does not contain fluorine atoms and more preferably does not contain halogen atoms.

10. A mixture comprising
a second electrically neutral organic compound, and
the coordination compound according to any one of claims 1 to 9,
wherein the coordination compound is imbedded into the at least one second electrically neutral organic compound,
wherein the second organic compound has a triplet energy higher than 2.5 eV and/or wherein the coordination compound has a higher hole affinity compared to the second organic compound.

11. A compound comprising
the coordination compound according to any one of claims 1 to 9, and
a polymer with a molecular weight Mn above 1000 g/mol, wherein the coordination compound is covalently attached to the polymer backbone.

12. A contrast enhancement medium for magnet resonance tomography (MRT), the contrast enhancement medium comprising the coordination compound according to any one of claims 1 to 9.

13. An organic electronic device, comprising:
a first electrode;
a second electrode; and
an organic layer arranged such that it is electrically interposed between the first and second electrodes, wherein the organic layer comprises the coordination compound according to any one of claims 1 to 9, the mixture of claim 10 or the compound of claim 11,
wherein preferably the organic electronic device is an optoelectronic device, the optoelectronic device being at least one of an organic light emitting diode, an organic photodetector, or a photovoltaic cell.

14. A method of forming an organic device, the method comprising:
forming a layer of the coordination compound according to any one of claims 1 to 9, of the mixture of claim 10 or the compound of claim 11,
wherein the layer is deposited from a gas phase, in particular using an evaporation and/or sublimation process, and/or by a solution-based process.

15. The use of a metal-organic coordination compound according to one of claims 1 to 9 in an organic electronic device, preferably an organic light emitting device.

## Patentansprüche

1. Metallorganische Koordinationsverbindung, die neutral geladen ist und wobei die Koordinationsverbindung mindestens ein zweiwertiges Lanthanid umfasst, das durch einen cyclischen organischen Liganden koordiniert ist, wobei der cyclische organische Ligand zwei monoanionische Gruppen enthält, die kovalent mit dem cyclischen organischen Liganden verknüpft sind und durch eine Abfolge von mindestens zwei dazwischenliegenden Atomen voneinander getrennt sind.

2. Koordinationsverbindung nach Anspruch 1, wobei es sich bei dem zweiwertigen Lanthanid um Europium oder Ytterbium handelt und/oder wobei der cyclische organische Ligand bicyclisch oder polycyclisch ist.

3. Metallorganische Koordinationsverbindung nach Anspruch 1 oder 2, wobei die Koordinationsverbindung mindestens ein zweiwertiges Lanthanid umfasst, das durch einen cyclischen organischen Liganden mit der Formel 1-1 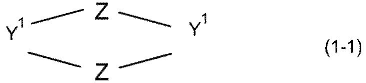
wobei Y¹ für jedes Auftreten unabhängig eine zweiwertige Gruppe mit 1 bis 30 Atomen ist,
oder Formel 1-2 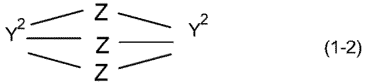
wobei Y² für jedes Auftreten unabhängig eine dreiwertige Gruppe mit 1 bis 30 Atomen ist,
Z für jedes Auftreten unabhängig eine zweiwertige organische Gruppe ist, wobei Z eine kürzeste Abfolge von mindestens 3 Atomen, vorzugsweise 3 bis 5 Atomen, zwischen den beiden Verknüpfungen mit Y¹ bzw. Y² aufweist, wenn Y¹ bzw. Y² eine cyclische Gruppe ist, die über verschiedene Ringatome mit jedem Z verbunden ist,
wobei Z eine kürzeste Abfolge von mindestens 6 Atomen, vorzugsweise 6 bis 8 Atomen, aufweist, wenn Y¹ bzw. Y² über ein Atom mit jedem Z verbunden ist,
wobei der cyclische organische Ligand der Formel 1-1 und 1-2 zwei monoanionische Gruppen enthält, die kovalent mit dem cyclischen organischen Liganden verknüpft sind und durch eine Abfolge von mindestens zwei dazwischenliegenden Atomen voneinander getrennt sind, koordiniert ist, oder
wobei die Koordinationsverbindung mindestens ein zweiwertiges Lanthanid umfasst, das durch einen cyclischen organischen Liganden gemäß Formel 3-1 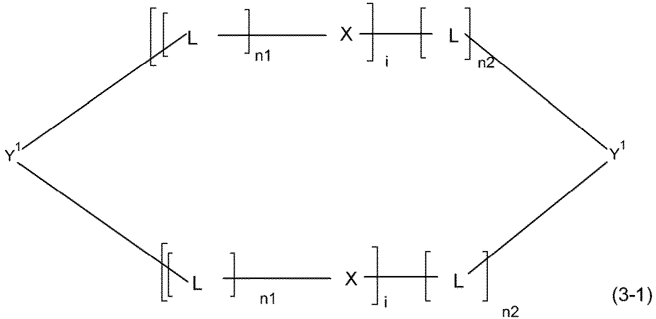
wobei
• Y¹ für jedes Auftreten unabhängig eine zweiwertige organische Gruppe, vorzugsweise eine zweiwertige cyclische Gruppe mit zwei Valenzen an verschiedenen Ringpositionen, ist oder 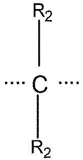 ist,
oder gemäß Formel 3-2 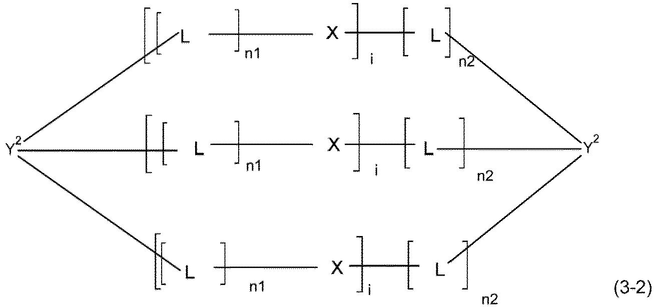
wobei
• Y² für jedes Auftreten unabhängig eine dreiwertige organische Gruppe, vorzugsweise C-R₂ oder eine cyclische Gruppe mit drei Valenzen an verschiedenen Ringpositionen, ist oder B oder B-R₂⁻ oder N oder P ist,
• X unabhängig für jedes Auftreten aus der Gruppe 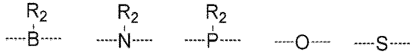 ausgewählt ist,
• L für jedes Auftreten unabhängig eine zweiwertige lineare, verzweigte oder cyclische organische Gruppe, die substituiert sein kann und die durch Entfernung von zwei Wasserstoffatomen von einem organischen linearen, verzweigten oder cyclischen Molekül, das substituiert sein kann, gebildet wird, ist oder eine zweiwertige Gruppe -CR₁R₁- ist,
• wobei R₁ und R₂ Wasserstoff oder beliebige kovalent gebundene Substituenten, die bei jedem Auftreten gleich oder verschieden sind, sind und
• n1, n2 und i unabhängig gleich 1, 2, 3 oder 4 sind,
• wobei der cyclische organische Ligand der Formel 3-1 und R-2 zwei monoanionische Gruppen enthält, die kovalent mit dem cyclischen organischen Liganden verknüpft sind und durch eine Abfolge von mindestens zwei dazwischenliegenden Atomen voneinander getrennt sind, koordiniert ist,
wobei in den zweiwertigen Gruppen -CR₁R₁- die beiden R₁ kovalent miteinander verknüpft sein können und dadurch eine cyclische Gruppe bilden und in -CR₂R₂- die beiden R₂ unter Bildung einer cyclischen Struktur kovalent miteinander verknüpft sein können.

4. Koordinationsverbindung nach Anspruch 3, wobei die Verbindung der Formel 3-2 eines oder mehrere der folgenden Merkmale aufweist:
- Y² ist B oder B-R₂⁻ oder N, wobei vorzugsweise R₂ Alkyl, Alkoxy, Carboxy, Aryl, Aryloxy oder F ist,
- alle drei Strukturelemente 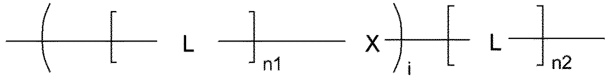 in der Verbindung der Formel 3-2 sind identisch,
- zweiwertige cyclische organische Gruppen L sind für jedes Auftreten unabhängig zweiwertige cyclische organische Gruppen, die substituiert sein können und die durch Entfernung von zwei Wasserstoffatomen von benachbarten Kohlenstoff- und/oder Stickstoffatomen im Ring eines organischen cyclischen Moleküls, das substituiert sein kann, gebildet werden, wobei n1 und n2 1 sind,
- cyclische organische Gruppen sind carbocyclische oder heterocyclische Gruppen, wobei die Heteroatome aus P, N, Si, O, S ausgewählt sind,
- dann, wenn in 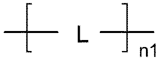 und/oder 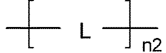 L für -CR₁R₁- steht, ist n1 und/oder n2 2,
- 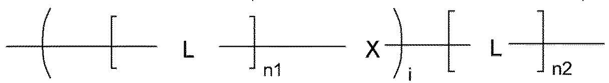 enthält zwei Gruppen X, die für 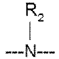 stehen, wobei beide R₂ zusammen eine Gruppe 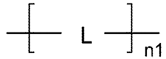 bilden, die mit der Gruppe 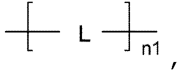 die die beiden Gruppen X verknüpft, identisch ist,
- i ist 2.

5. Koordinationsverbindung nach Anspruch 3 oder 4, wobei der cyclische organische Ligand eine Struktur gemäß Formel 2a, 2c, 2e oder 2f aufweist: 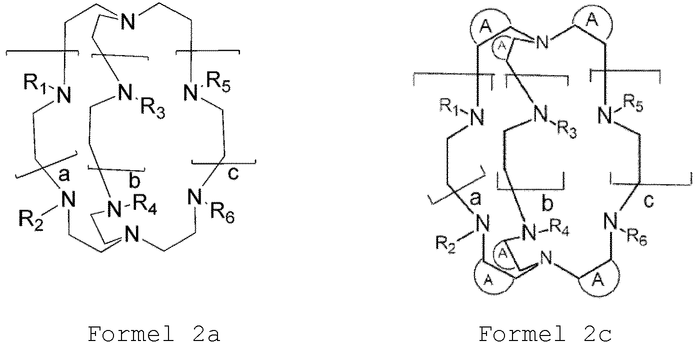 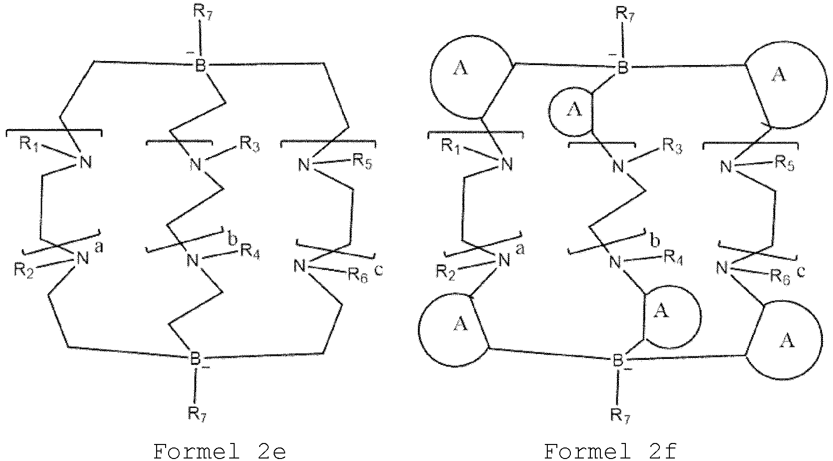 wobei
- R₁, R₂, R₃, R₄, R₅, R₆ in Formel 2a und 2c unabhängig bei jedem Auftreten für Wasserstoff oder einen Substituenten stehen und R₁, R₂, R₃, R₄, R₅, R₆, R₇ in Formel 2e, 2f unabhängig bei jedem Auftreten für Wasserstoff oder einen Substituenten stehen,
- a, b, c jeweils unabhängig für eine ganze Zahl mit einem Wert von 0 oder mehr stehen,
- 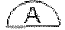 unabhängig bei jedem Auftreten für eine zweiwertige cyclische organische Gruppe steht, wobei eines oder mehrere der ringbildenden Kohlenstoffatomen substituiert sein können oder Teil einer cyclischen Gruppe sein können,
wobei der cyclische organische Ligand der Formel 2a und 2c zwei monoanionische Gruppen enthält, die kovalent mit dem cyclischen organischen Liganden verknüpft sind und durch eine Abfolge von mindestens zwei dazwischenliegenden Atomen voneinander getrennt sind, wobei vorzugsweise die monoanionische Gruppe die anionische Ladung in einer β- oder γ-Position zu dem Ringkohlenstoff- oder - stickstoffatom, an das die monoanionische Gruppe kovalent gebunden ist, aufweist, oder
wobei in Formel 2a und 2c
R₁ oder R₂ eine monoanionische Gruppe sein kann oder ein N-R₁ oder N-R₂ benachbartes Ringkohlenstoffatom durch eine monoanionische Gruppe substituiert sein kann,
R₃ oder R₄ eine monoanionische Gruppe sein kann oder ein N-R₃ oder N-R₄ benachbartes Ringkohlenstoffatom durch eine monoanionische Gruppe substituiert sein kann oder
R₅ oder R₆ eine monoanionische Gruppe sein kann oder ein N-R₅ oder N-R₆ benachbartes Ringkohlenstoffatom durch eine monoanionische Gruppe substituiert sein kann,
wobei insgesamt zwei monoanionische Gruppen kovalent mit dem cyclischen organischen Liganden verknüpft sind.

6. Koordinationsverbindung nach Anspruch 5, wobei die monoanionische Gruppe die Struktur
-CH₂-CR'R'-X aufweist, wobei X ein monoanionisches Atom ist, das aus O⁻ und S⁻ ausgewählt ist,
oder die Struktur 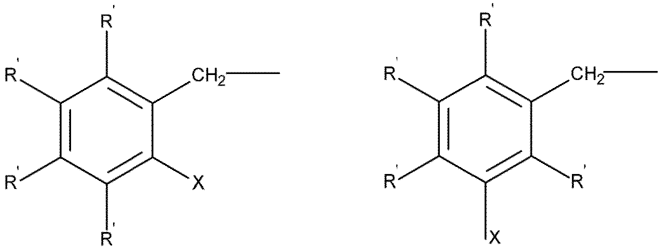
aufweist, wobei X ein monoanionisches Atom ist, das aus O⁻ und S⁻ ausgewählt ist,
wobei R' jeweils unabhängig H, substituiertes oder unsubstituiertes C₁₋₁₂-Alkyl, substituiertes oder unsubstituiertes Aryl, Halogen ist und wobei ein R' der monoanionischen Gruppe NO₂ sein kann und wobei zwei Gruppen R' kovalent zu einer cyclischen Gruppe verknüpft sein können,
oder aus den folgenden Strukturen ausgewählt ist: 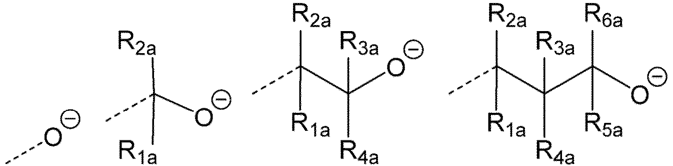 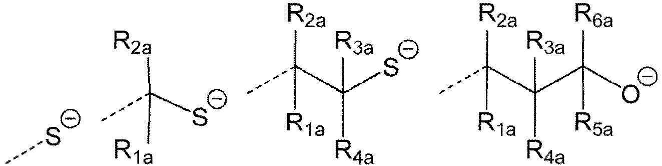 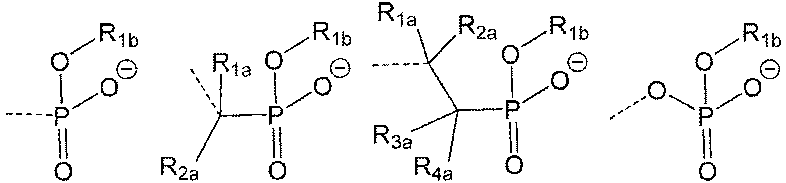 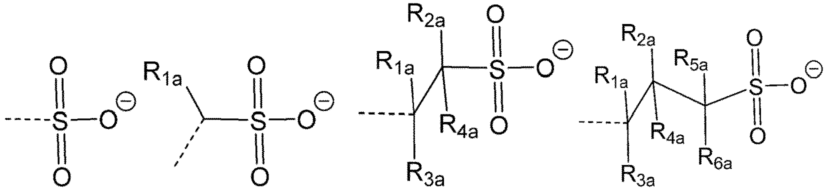 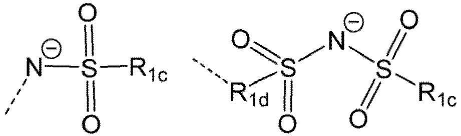 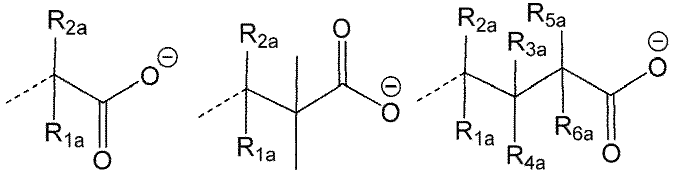 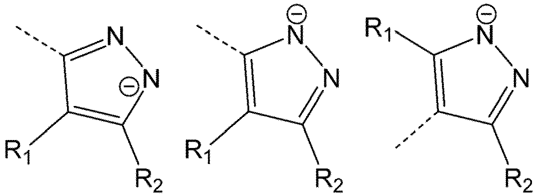 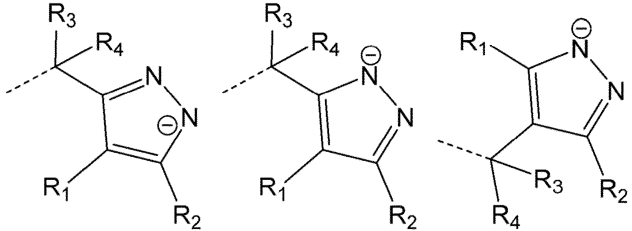 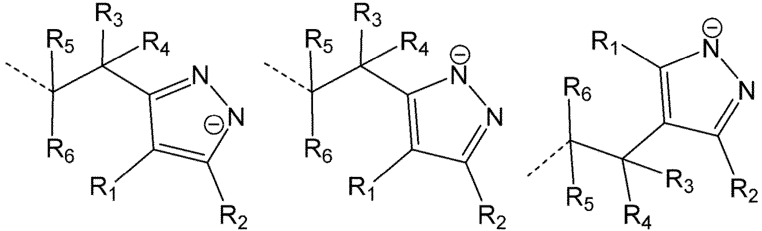 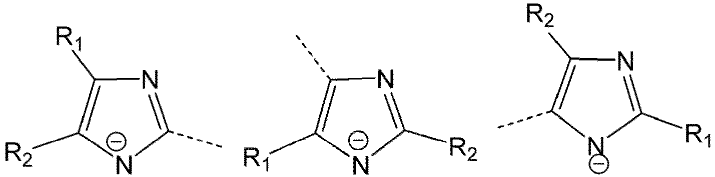 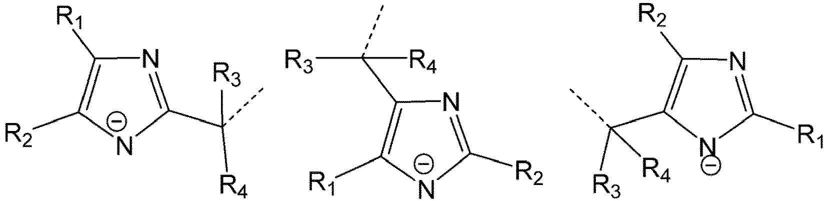 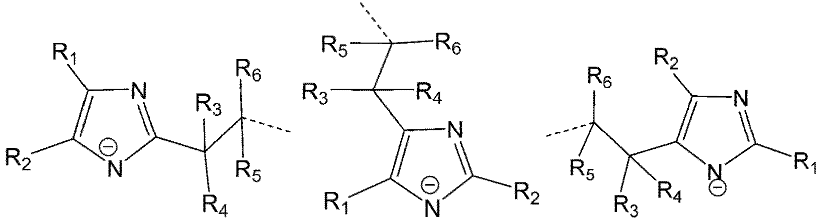 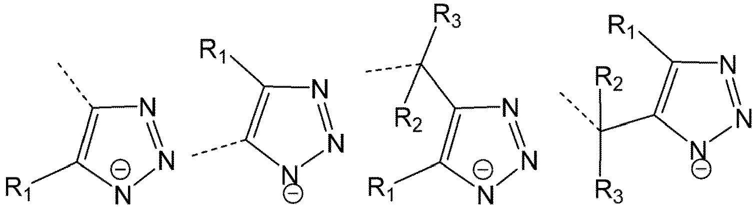 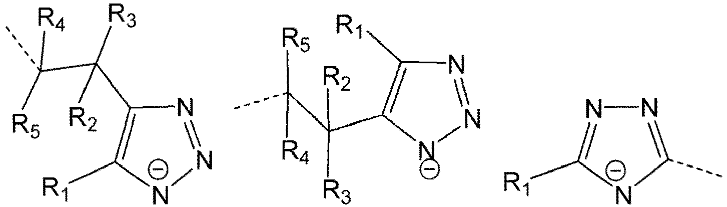 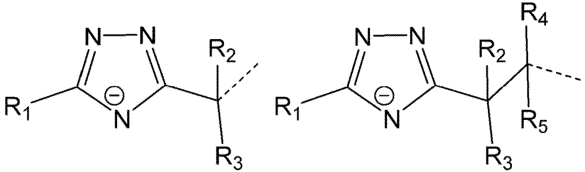 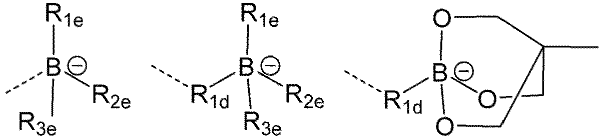 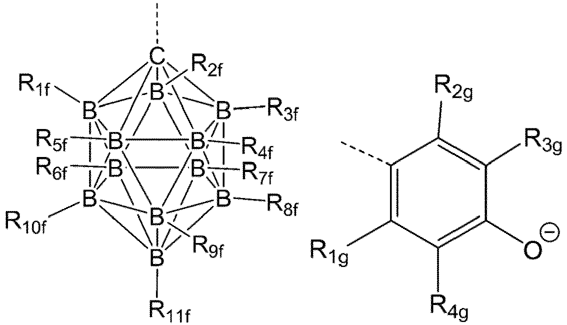 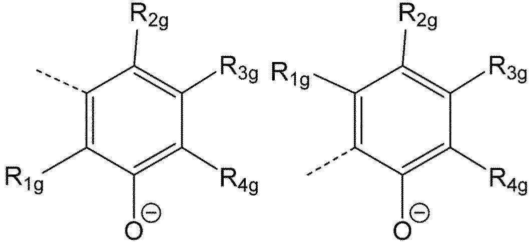 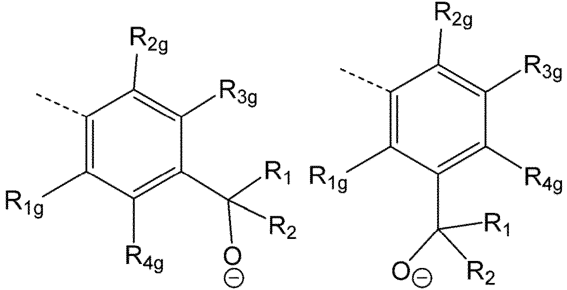 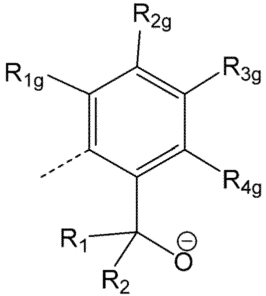
wobei
R₁ bis R₆ unabhängig H oder Organyl sind,
R₁ₐ bis R₆ₐ unabhängig H, CH₃, CF₃, CN, F oder CₙH₂ₙ₊₁ sind, wobei n eine ganze Zahl von 1 bis 5 ist,
R_{1b} CₙH₂ₙ₊₁ ist, wobei n eine ganze Zahl von 1 bis 5 ist,
R_{1c} CH₃ oder CF₃ ist,
R_{1d} ein zweiwertiges organisches Fragment ist, das vorzugsweise aus Alkylen, Perfluoralkylen, das gegebenenfalls substituiert sein kann, ausgewählt ist,
R₁ₑ bis R₃ₑ unabhängig eine 5-gliedrige Heteroarylgruppe mit bis zu 3 aus N, S, O ausgewählten Heteroatomen, die gegebenenfalls substituiert sein kann,
oder eine 6-gliedrige Aryl- oder Heteroarylgruppe, die bis zu 4 aus N, S, O, vorzugsweise aus N, ausgewählte Heteroatome enthalten kann und die gegebenenfalls substituiert sein kann, oder eine aus CF₃, F, H,OMe, OEt ausgewählte Gruppe sind,
R_{1f} bis R_{11f} unabhängig H, Cl, Br, F, CH₃, CF₃ sind, R_{1g} bis R_{4g} unabhängig H, Organyl, Halogen, vorzugsweise F, CF₃, CN, OMe sind.

7. Koordinationsverbindung nach Anspruch 3, wobei Y¹ jeweils unabhängig aus 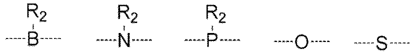 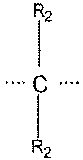 ausgewählt ist
oder Y¹ jeweils unabhängig aus 3- bis 15-gliedrigen, vorzugsweise 4- bis 12-gliedrigen, cyclischen organischen Gruppen, die 1 bis 4 aus N, B, P, O, S ausgewählte Heteroatome enthalten können, ausgewählt ist und über ein nicht benachbartes Kohlenstoff- oder Heteroatom mit jedem Z verbunden ist, wobei R₂ Wasserstoff oder ein beliebiger kovalent gebundener Substituent, der bei jedem Auftreten gleich oder verschieden ist, ist, oder wobei Y² jeweils unabhängig aus B, B-R₂⁻, N, P, C-R₂ ausgewählt ist
oder Y² jeweils unabhängig aus 3- bis 15-gliedrigen, vorzugsweise 6- bis 12-gliedrigen, cyclischen organischen Gruppen, die eins bis vier aus N, B, P, O, S ausgewählte Heteroatome enthalten können, ausgewählt ist und über ein nicht benachbartes Kohlenstoff- oder Heteroatom mit jedem Z verbunden ist,
wobei R₂ Wasserstoff oder ein beliebiger kovalent gebundener Substituent, der bei jedem Auftreten gleich oder verschieden ist, ist.

8. Koordinationsverbindung nach Anspruch 3 oder 7, wobei Z jeweils höchstens eine monoanionische Gruppe enthält oder wobei ein Z zwei monoanionische Gruppen enthält und die anderen Z, Y¹ und Y² keine monoanionischen Gruppen enthalten.

9. Koordinationsverbindung nach einem der Ansprüche 1 bis 8, wobei der cyclische organische Ligand genau zwei monoanionische Gruppen enthält und daher zweifach negativ geladen ist und in Kombination mit dem zweiwertigen Lanthanid einen Komplex mit neutraler Ladung bildet, wobei vorzugsweise der cyclische organische Ligand keine Fluoratome enthält und weiter bevorzugt keine Halogenatome enthält.

10. Mischung, umfassend
eine zweite elektrisch neutrale organische Verbindung und
die Koordinationsverbindung nach einem der Ansprüche 1 bis 9,
wobei die Koordinationsverbindung in die mindestens eine zweite elektrisch neutrale organische Verbindung eingebettet ist,
wobei die zweite organische Verbindung eine Triplettenergie von mehr als 2,5 eV aufweist und/oder wobei die Koordinationsverbindung eine höhere Lochaffinität als die zweite organische Verbindung aufweist.

11. Verbindung, umfassend
die Koordinationsverbindung nach einem der Ansprüche 1 bis 9 und
ein Polymer mit einem Molekulargewicht Mn über 1000 g/mol, wobei die Koordinationsverbindung kovalent an die Polymerhauptkette gebunden ist.

12. Kontrastverstärkungsmedium für die Magnetresonanztomographie (MRT), wobei das Kontrastverstärkungsmedium die Koordinationsverbindung nach einem der Ansprüche 1 bis 9 umfasst.

13. Organische elektronische Vorrichtung, umfassend:
eine erste Elektrode;
eine zweite Elektrode; und
eine organische Schicht, die so angeordnet ist, dass sie sich elektrisch zwischen der ersten Elektrode und der zweiten Elektrode befindet, wobei die organische Schicht die Koordinationsverbindung nach einem der Ansprüche 1 bis 9, die Mischung nach Anspruch 10 oder die Verbindung nach Anspruch 11 umfasst,
wobei vorzugsweise die organische elektronische Vorrichtung eine optoelektronische Vorrichtung ist, wobei es sich bei der optoelektronischen Vorrichtung um eine organische Leuchtdiode, einen organischen Photodetektor und/oder eine Photovoltaikzelle handelt.

14. Verfahren zur Bildung einer organischen Vorrichtung, wobei das Verfahren Folgendes umfasst:
Bilden einer Schicht der Koordinationsverbindung nach einem der Ansprüche 1 bis 9, der Mischung nach Anspruch 10 oder der Verbindung nach Anspruch 11,
wobei die Schicht aus einer Gasphase, insbesondere unter Verwendung eines Verdampfungs- und/oder Sublimationsprozesses, und/oder durch einen lösungsbasierten Prozess abgeschieden wird.

15. Verwendung einer metallorganischen Koordinationsverbindung nach einem der Ansprüche 1 bis 9 in einer organischen elektronischen Vorrichtung, vorzugsweise einer organischen lichtemittierenden Vorrichtung.

## Revendications

1. Composé de coordination métal-organique, qui est chargé de manière neutre et, le composé de coordination comprenant au moins un lanthanide divalent coordiné par un ligand organique cyclique, le ligand organique cyclique contenant deux groupes monoanioniques liés au ligand organique cyclique et étant séparés l'un de l'autre par une séquence d'au moins deux atomes intervenants.

2. Composé de coordination selon la revendication 1, le lanthanide divalent étant l'europium ou l'ytterbium et/ou le ligand organique cyclique étant bicyclique ou polycyclique.

3. Composé de coordination métal-organique selon l'une des revendications 1 ou 2, le composé de coordination comprenant au moins un lanthanide divalent coordiné par un ligand organique cyclique ayant la formule 1-1 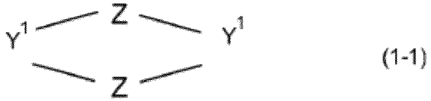
Y¹ pour chaque occurrence étant indépendamment un groupe divalent contenant 1 à 30 atomes,
ou la formule 1-2 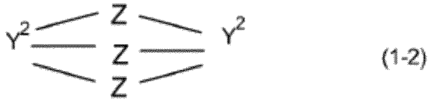
Y² pour chaque occurrence étant indépendamment un groupe trivalent contenant 1 à 30 atomes,
Z pour chaque occurrence étant indépendamment un groupe organique divalent, Z ayant une séquence la plus courte d'au moins 3 atomes, préférablement 3 à 5 atomes, entre les deux liaisons avec Y¹ ou Y² dans le cas où Y¹ ou Y² est un groupe cyclique relié à chaque Z via des atomes de cycle différents,
Z ayant une séquence la plus courte d'au moins 6 atomes, préférablement 6 à 8 atomes, dans le cas où Y¹ ou Y² est relié à chaque Z via un atome,
le ligand organique cyclique de formule 1-1 et 1-2 contenant deux groupes monoanioniques liés de manière covalente au ligand organique cyclique et étant séparés l'un de l'autre par une séquence d'au moins deux atomes intervenants, ou
le composé de coordination comprenant au moins un lanthanide divalent coordiné par un ligand organique cyclique selon la formule 3-1 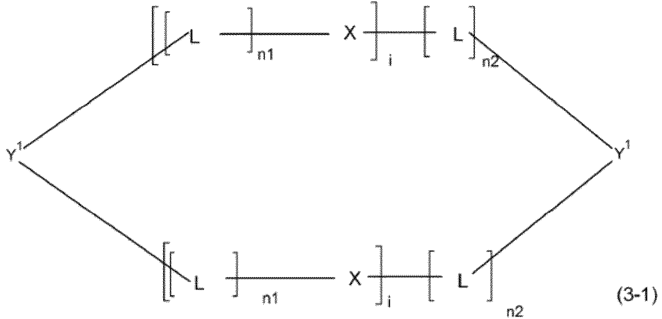
• Y¹ pour chaque occurrence étant indépendamment un groupe organique divalent, préférablement un groupe cyclique divalent qui possède deux valences au niveau de positions de cycle différentes, ou étant 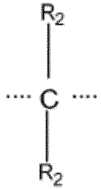 ou selon la formule 3-2 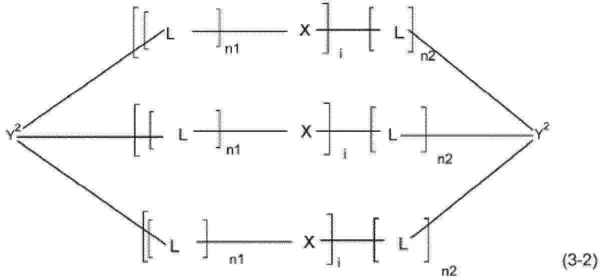
• Y² pour chaque occurrence étant indépendamment un groupe organique trivalent, préférablement C-R₂ ou un groupe cyclique qui possède trois valences au niveau de positions de cycle différentes, ou étant B ou B-R₂⁻ ou N ou P,
• X étant indépendamment choisi pour chaque occurrence dans le groupe de : 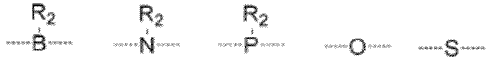
• L pour chaque occurrence indépendamment étant un groupe organique cyclique divalent linéaire, ramifié ou cyclique qui peut être substitué et qui est formé par élimination de deux atomes d'hydrogène d'une molécule organique linéaire, ramifiée ou cyclique qui peut être substituée, ou étant un groupe divalent -CR₁R₁-
• R₁ et R₂ étant hydrogène ou de quelconques substituants liés de manière covalente qui sont identiques ou différents en chaque occurrence et
• n1, n2, i indépendamment étant égaux à 1, 2, 3 ou 4,
• le ligand organique cyclique de formule 3-1 et R-2 contenant deux groupes monoanioniques liés de manière covalente au ligand organique cyclique et étant séparés l'un de l'autre par une séquence d'au moins deux atomes intervenants,
dans les groupes divalents -CR₁R₁-, les deux R₁ pouvant être liés de manière covalente l'un à l'autre, formant ainsi un groupe cyclique, et dans -CR₂R₂-, les deux R₂ pouvant être liés de manière covalente pour former une structure cyclique.

4. Composé de coordination selon la revendication 3, le composé de formule 3-2 ayant l'une ou plusieurs des caractéristiques suivantes :
- Y² étant B ou B-R₂⁻ ou N, préférablement R₂ étant alkyle, alcoxy, carboxy, aryle, aryloxy ou F,
- tous les trois éléments structuraux 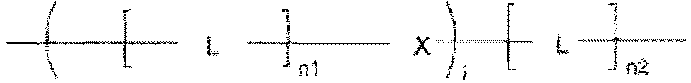 dans le composé de formule 3-2 étant identiques,
- des groupes organiques cycliques divalents L pour chaque occurrence indépendamment étant des groupes organiques cycliques divalents qui peuvent être substitués et qui sont formés par élimination de deux atomes d'hydrogène d'atomes de carbone et/ou d'azote adjacents dans le cycle d'une molécule cyclique organique qui peut être substituée, n1 et n2 étant 1,
- des groupes organiques cycliques étant des groupes carbocycliques ou hétérocycliques, les hétéroatome étant choisis parmi P, N, Si, O, S
- si dans 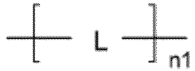 et/ou 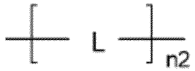 L est -CR¹R¹-, alors n1 et/ou n2 est 2,
- 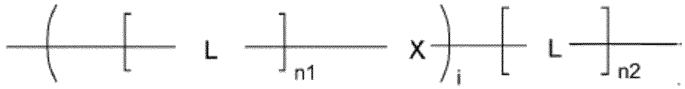 contenant deux groupes X qui sont 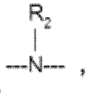 les deux R₂ formant ensemble un groupe 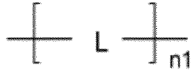 qui est identique au groupe 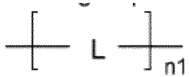 liant les deux groupes X,
- i étant 2.

5. Composé de coordination selon l'une quelconque des revendications 3 ou 4, le ligand organique cyclique ayant une structure selon la formule 2a, 2c, 2e ou 2f : 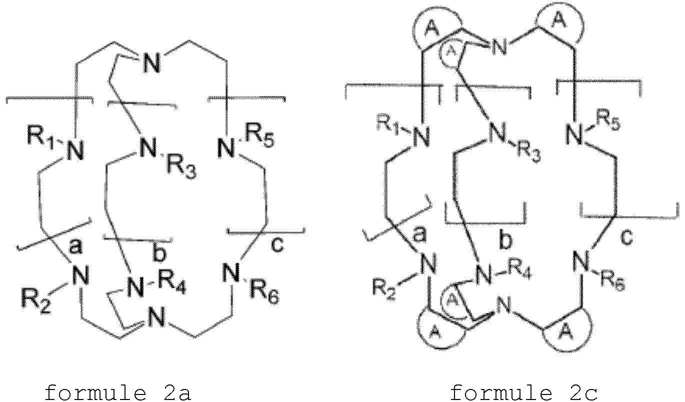 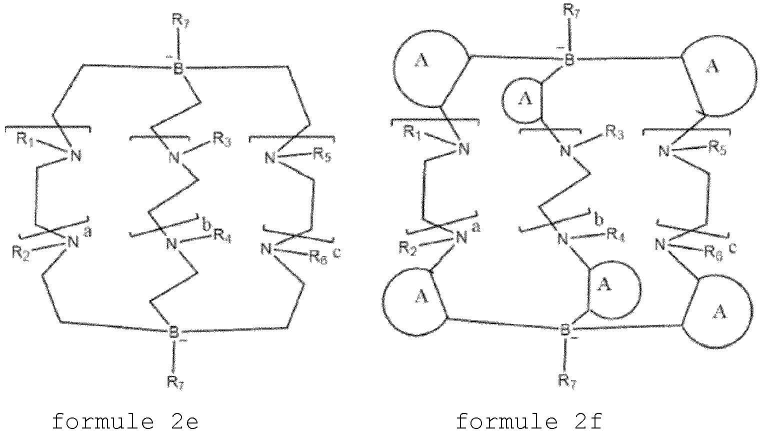
- R₁, R₂, R₃, R₄, R₅, R₆ dans la formule 2a et 2c indépendamment en chaque occurrence représentant hydrogène ou un substituant, et R₁, R₂, R₃, R₄, R₅, R₆, R₇ dans la formule 2e, 2f indépendamment en chaque occurrence représentant hydrogène ou un substituant,
- a, b, c étant chacun indépendamment un entier de 0 ou plus, 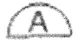
indépendamment en chaque occurrence représentant un groupe organique cyclique divalent, un ou plusieurs des atomes de carbone de formation de cycle pouvant être substitués ou pouvant faire partie d'un groupe cyclique,
ledit ligand organique cyclique de formule 2a et 2c contenant deux groupes monoanioniques liés de manière covalente au ligand organique cyclique et étant séparés l'un de l'autre par une séquence d'au moins deux atomes intervenants, préférablement le groupe monoanionique ayant la charge anionique dans une position β ou γ par rapport à l'atome de carbone ou d'azote de cycle auquel le groupe monoanionique est lié de manière covalente, ou
dans la formule 2a et 2c,
R₁ ou R₂ pouvant être un groupe monoanionique,
ou un atome de carbone de cycle adjacent à N-R₁ ou N-R₂ pouvant être substitué par un groupe monoanionique,
R₃ ou R₄ pouvant être un groupe monoanionique, ou un atome de carbone de cycle adjacent à N-R₃ ou N-R₄ pouvant être substitué par un groupe monoanionique, ou R₅ ou R₆ pouvant être un groupe monoanionique,
ou un atome de carbone de cycle adjacent à N-R₅ ou N-R₆ pouvant être substitué par un groupe monoanionique,
avec un total de deux groupes monoanioniques liés de manière covalente au ligand organique cyclique.

6. Composé de coordination selon la revendication 5, le groupe monoanionique ayant la structure
-CH₂-CR'R'-X, X étant un atome monoanionique choisi parmi O⁻ et S⁻,
ou ayant la structure 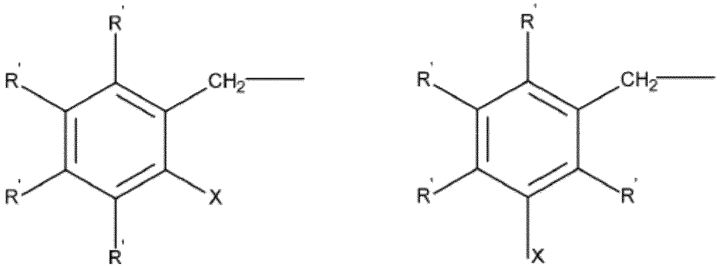
X étant un atome monoanionique choisi parmi O⁻ et S⁻ ,
chaque R' étant indépendamment H, alkyle en C₁₋₁₂ substitué ou non substitué, aryle substitué ou non substitué, halogène, et un R' du groupe monoanionique pouvant être NO₂, et deux groupes R' pouvant être liés de manière covalente pour former un groupe cyclique,
ou étant choisi parmi les structures suivantes 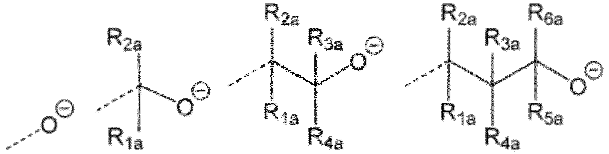 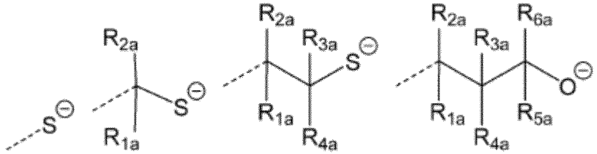 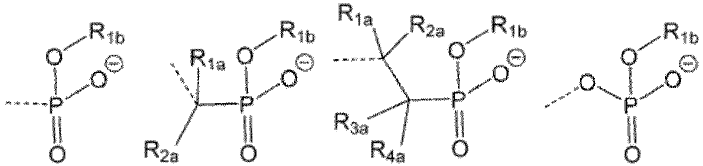 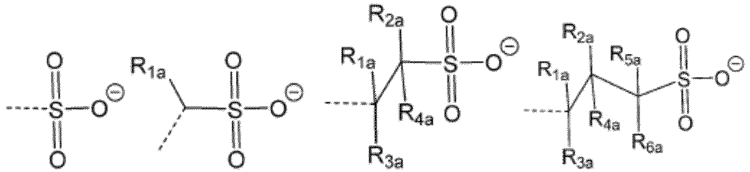 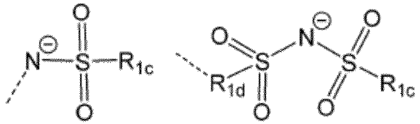 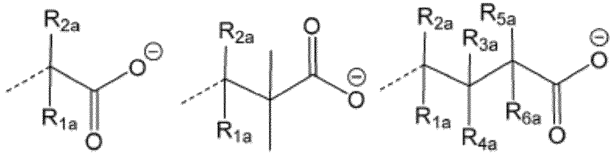 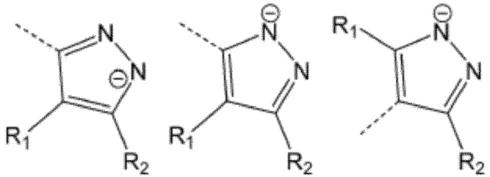 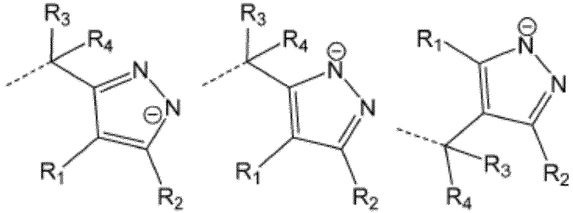 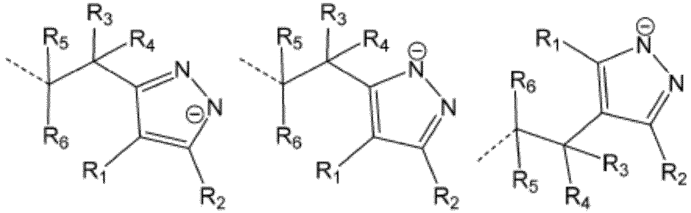 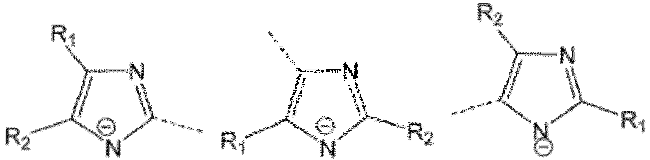 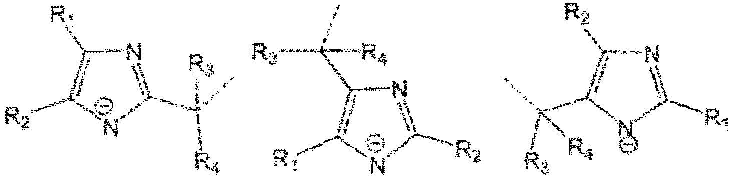 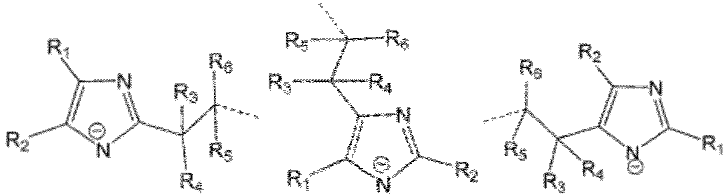 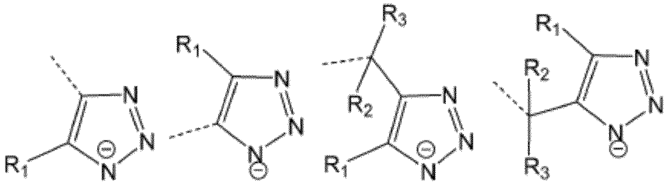 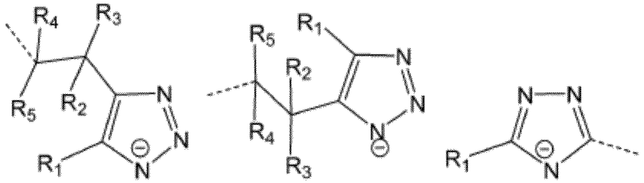 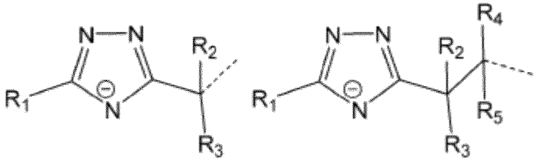 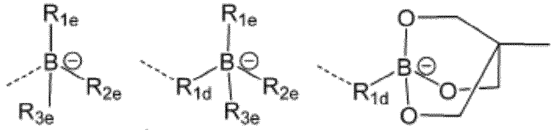 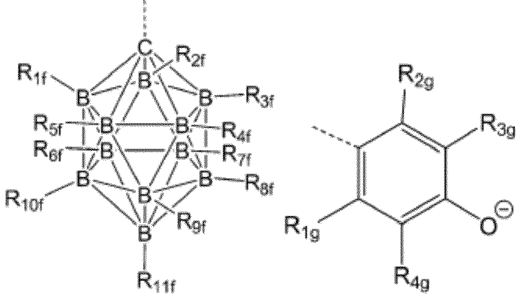 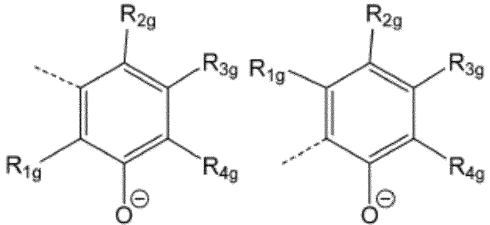 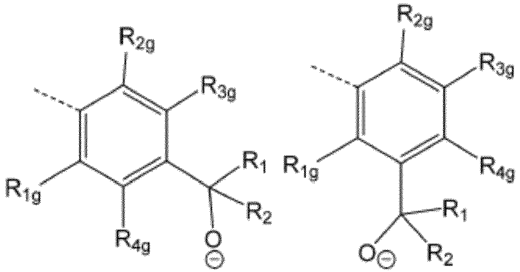 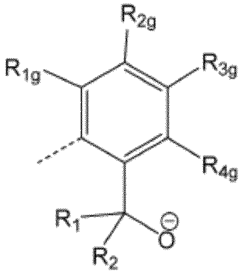
R₁ à R₆ étant indépendamment H ou organyle,
R₁ₐ à R₆ₐ étant indépendamment H, CH₃, CF₃, CN, F ou CₙH₂ₙ₊₁, n étant un entier de 1 à 5,
R_{1b} étant CₙH₂ₙ₊₁, n étant un entier de 1 à 5,
R_{1c} étant CH₃ ou CF₃,
R_{1d} étant un fragment organique divalent, préférablement choisi parmi alkylène, perfluoroalkylène, qui peut éventuellement être substitué,
R₁ₑ à R₃ₑ étant indépendamment un groupe hétéroaryle à 5 chaînons comportant jusqu'à 3 hétéroatomes, choisis parmi N, S, O, qui peut éventuellement être substitué, ou un groupe aryle ou hétéroaryle à 6 chaînons qui peut contenir jusqu'à 4 hétéroatomes, choisis parmi N, S, O, préférablement parmi N, et qui peut éventuellement être substitué, ou un groupe choisi parmi CF₃, F, H, OMe, OEt
R_{1f} à R_{11f} étant indépendamment H, Cl, Br, F, CH₃, CF₃ R_{1g} à R_{4g} étant indépendamment H, organyle, halogène, préférablement F, CF₃, CN, OMe.

7. Composé de coordination selon la revendication 3, chaque Y¹ étant indépendamment choisi parmi 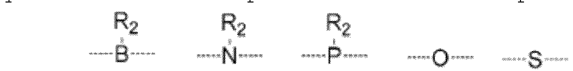 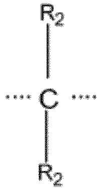
ou chaque Y¹ étant indépendamment choisi parmi des groupes organiques cycliques à 3 à 15 chaînons, préférablement 4 à 12 chaînons qui peuvent contenir 1 à 4 hétéroatomes choisis parmi N, B, P, O, S et étant relié à chaque Z via un carbone ou un hétéroatome non adjacent, R₂ étant hydrogène ou un quelconque substituant lié de manière covalente qui est identique ou différent en chaque occurrence, ou chaque Y² étant indépendamment choisi parmi B, B-R₂⁻, N, P, C-R₂,
ou chaque Y² étant indépendamment choisi parmi des groupes organiques cycliques à 3 à 15 chaînons, préférablement 6 à 12 chaînons qui peuvent contenir un à quatre hétéroatomes choisis parmi N, B, P, O, S et étant relié à chaque Z via un carbone ou un hétéroatome non adjacent,
R₂ étant hydrogène ou un quelconque substituant lié de manière covalente qui est identique ou différent en chaque occurrence.

8. Composé de coordination selon l'une des revendications 3 ou 7, chaque Z contenant au plus un groupe monoanionique, ou un Z contenant deux groupes monoanioniques et les autres Z, Y¹ et Y² ne contenant pas de groupes monoanioniques.

9. Composé de coordination selon l'une des revendications 1 à 8, le ligand organique cyclique contenant exactement deux groupes monoanioniques et par conséquent étant chargé deux fois négativement et en combinaison avec le lanthanide divalent formant un complexe de charge neutre, préférablement le ligand organique cyclique ne contenant pas d'atomes de fluor et plus préférablement ne contenant pas d'atomes d'halogène.

10. Mélange comprenant
un deuxième composé organique électriquement neutre, et
le composé de coordination selon l'une quelconque des revendications 1 à 9,
le composé de coordination étant incorporé dans l'au moins un deuxième composé organique électriquement neutre,
le deuxième composé organique ayant une énergie triplet supérieure à 2,5 eV et/ou le composé de coordination ayant une affinité de trous plus grande que le deuxième composé organique.

11. Composé comprenant
le composé de coordination selon l'une quelconque des revendications 1 à 9, et
un polymère doté d'un poids moléculaire Mn supérieur à 1 000 g/mole, le composé de coordination étant fixé de manière covalente au squelette du polymère.

12. Milieu d'amélioration de contraste pour la tomographie à résonance magnétique (MRT), le milieu d'amélioration de contraste comprenant le composé de coordination selon l'une quelconque des revendications 1 à 9.

13. Dispositif électronique organique, comprenant :
une première électrode ;
une deuxième électrode ; et
une couche organique agencée de sorte qu'elle est interposée électriquement entre la première et la deuxième électrodes, la couche organique comprenant le composé de coordination selon l'une quelconque des revendications 1 à 9, le mélange selon la revendication 10 ou le composé selon la revendication 11,
préférablement le dispositif électronique organique étant un dispositif optoélectronique, le dispositif optoélectronique étant au moins l'un parmi une diode émettrice de lumière organique, un photodétecteur organique ou une cellule photovoltaïque.

14. Procédé de formation d'un dispositif organique, le procédé comprenant :
la formation d'une couche du composé de coordination selon l'une quelconque des revendications 1 à 9, ou du mélange selon la revendication 10 ou du composé selon la revendication 11,
la couche étant déposée à partir d'une phase gazeuse, en particulier en utilisant un processus d'évaporation et/ou de sublimation, et/ou par un processus à base d'une solution.

15. Utilisation d'un composé de coordination métal-organique selon l'une des revendications 1 à 9 dans un dispositif électronique organique, préférablement un dispositif émetteur de lumière organique.
